# EUROPEAN PATENT SPECIFICATION

(11) **EP 4 438 125 B1**
(45) Date of publication and mention of the grant of the patent: **16.09.2026**
(21) Application number: 24190153.7
(22) Date of filing: 18.06.2021
(51) Int. Cl.: A61K 47/54, A61K 47/55, A61P 35/00

(54) **CONJUGATE OF GALNAC AND SAPONIN, THERAPEUTIC COMPOSITION COMPRISING SAID CONJUGATE AND A GALNAC OLIGONUCLEOTIDE CONJUGATE**
KONJUGAT AUS GALNAC UND SAPONIN, THERAPEUTISCHE ZUSAMMENSETZUNG MIT DIESEM KONJUGAT UND EIN GALNAC-OLIGONUKLEOTIDKONJUGAT
CONJUGUÉ DE GALNAC ET DE SAPONINE, COMPOSITION THÉRAPEUTIQUE COMPRENANT LEDIT CONJUGUÉ ET UN CONJUGUÉ D'OLIGONUCLÉOTIDE DE GALNAC

(30) Priority: 24.06.2020 NL 2025899; 10.09.2020 NL 2026442
(43) Date of publication of application: 02.10.2024
(60) Divisional application: 26184555.6 / 4 782 067
(62) Document number(s) of the earlier application(s) in accordance with Art. 76 EPC: 21734241.9 / 4 171 641
(73) Proprietor: Sapreme Technologies B.V., 3584 CM Utrecht (NL)
(72) Inventor: POSTEL, Ruben, 3721 MA Bilthoven (NL); HERMANS, Guy, 3721 MA BILTHOVEN (NL)
(74) Representative: Nederlandsch Octrooibureau

(56) References cited:
- WO-A1-2011/054811
- BACHRAN CHRISTOPHER ET AL: "Saponins in Tumor Therapy", MINI-REVIEWS IN MEDICINAL CHEMISTRY, vol. 8, 1 August 2008 (2008-08-01), pages 575 - 584, XP055775950
- SABINE SEWING ET AL: "GalNAc Conjugation Attenuates the Cytotoxicity of Antisense Oligonucleotide Drugs in Renal Tubular Cells", MOLECULAR THERAPY: NUCLEIC ACIDS., vol. 14, 1 March 2019 (2019-03-01), US, pages 67 - 79, XP055762603, ISSN: 2162-2531, DOI: 10.1016/j.omtn.2018.11.005
- ROGER GILABERT-ORIOL ET AL: "Modified Trastuzumab and Cetuximab Mediate Efficient Toxin Delivery While Retaining Antibody-Dependent Cell-Mediated Cytotoxicity in Target Cells", MOLECULAR PHARMACEUTICS, vol. 10, no. 11, 7 October 2013 (2013-10-07), US, pages 4347 - 4357, XP055677139, ISSN: 1543-8384, DOI: 10.1021/mp400444q
- GILABERT-ORIOL ROGER ET AL: "Dianthin-30 or gelonin versus monomethyl auristatin E, each configured with an anti-calcitonin receptor antibody, are differentially potent in vitro in high-grade glioma cell lines derived from glioblastoma", CANCER IMMUNOLOGY, IMMUNOTHERAPY, SPRINGER, BERLIN/HEIDELBERG, vol. 66, no. 9, 13 May 2017 (2017-05-13), pages 1217 - 1228, XP036309505, ISSN: 0340-7004, [retrieved on 20170513], DOI: 10.1007/S00262-017-2013-Z
- ROGER GILABERT-ORIOL ET AL: "Combinatorial approach to increase efficacy of Cetuximab, Panitumumab and Trastuzumab by dianthin conjugation and co-application of SO1861", BIOCHEMICAL PHARMACOLOGY, vol. 97, no. 3, 1 October 2015 (2015-10-01), US, pages 247 - 255, XP055677222, ISSN: 0006-2952, DOI: 10.1016/j.bcp.2015.07.040
- CHEENU BHARGAVA ET AL: "Targeted dianthin is a powerful toxin to treat pancreatic carcinoma when applied in combination with the glycosylated triterpene SO1861", MOLECULAR ONCOLOGY, vol. 11, no. 11, 15 September 2017 (2017-09-15), pages 1527 - 1543, XP055677214, ISSN: 1574-7891, DOI: 10.1002/1878-0261.12115
- ALEXANDER WENG ET AL: "The toxin component of targeted anti-tumor toxins determines their efficacy increase by saponins", MOLECULAR ONCOLOGY, ELSEVIER, vol. 6, no. 3, 12 January 2012 (2012-01-12), pages 323 - 332, XP028508748, ISSN: 1574-7891, [retrieved on 20120124], DOI: 10.1016/J.MOLONC.2012.01.004
- HENDRIK FUCHS ET AL: "Glycosylated Triterpenoids as Endosomal Escape Enhancers in Targeted Tumor Therapies", BIOMEDICINES, vol. 5, no. 2, 29 March 2017 (2017-03-29), pages 14, XP055429235, DOI: 10.3390/biomedicines5020014

## Description

### TECHNOLOGICAL FIELD

The invention relates to a saponin conjugate comprising a saponin covalently linked to a ligand for ASGPR, the ligand comprising at least one GalNAc. The invention also relates to a pharmaceutical combination comprising a first pharmaceutical composition comprising the saponin conjugate of the invention and a second pharmaceutical composition comprising a second conjugate of an effector molecule and a ligand for ASGPR, or a third conjugate of an effector molecule and a binding molecule for binding to a cell-surface molecule. In addition, the invention relates to a pharmaceutical composition comprising the saponin conjugate of the invention and the second conjugate or the third conjugate. Furthermore, the invention relates to a pharmaceutical combination or pharmaceutical composition of the invention, for use as a medicament. The invention also relates to a pharmaceutical combination or pharmaceutical composition of the invention, for use in the treatment or prophylaxis of a disease or health problem in which an expression product is involved of genes: apoB, TTR, PCSK9, ALAS1, AT3, GO, CC5, X gene of HBV, S gene of HBV, AAT and LDH, and for use in the treatment or prophylaxis of a cancer, an infectious disease, a viral infection, hypercholesterolemia, primary hyperoxaluria, haemophilia A, haemophilia B, AAT related liver disease, acute hepatic porphyria, TTR amyloidosis, complement-mediated disease, hepatitis B infection, or an auto-immune disease. Finally, the invention relates to an *in vitro* or *ex vivo* method for transferring the second conjugate or the third conjugate of the invention from outside a cell to inside said cell.

### BACKGROUND OF THE INVENTION

The ability to inhibit a protein's function, whether in humans or in pathogens, is an integral part of the discovery of new drugs. Conventional small molecule drugs as well as antibodies are strongly limited to a subset of the available targets. Small molecules bind principally to cofactor sites or transmitter sites, effectively sites that are designed to accommodate small molecules. Antibodies can bind a greater variety of proteins but are commonly limited to extracellular targets.

Oligonucleotide therapy is a relatively young and fast-developing field which aims to overcome many of the issues encountered with small-molecule drugs or antibody drugs by directly manipulating the genetic transcription and translation pathways. The potency and versatility of oligonucleotides, in particular the prospect of suppressing genes encoding proteins that are 'undruggable' by classical small molecule drugs, makes them attractive drug candidates. The first oligonucleotide drugs were based on antisense technology, whereby single-stranded nucleic acid molecules would bind with sequence specificity to their complementary mRNA target, thus triggering degradation of the duplex by the RNase H system.

In 1998, Andrew Fire and Craig Mello published a seminal paper identifying double-stranded RNAs (dsRNAs) as the causative agents for post-transcriptional gene silencing (PTGS) in *Caenorhabditis elegans,* a phenomenon they termed RNA interference (RNAi). The discovery of RNAi explained puzzling observations of gene silencing in plants and fungi and kicked off a revolution in biology that eventually showed non-coding RNAs to be central regulators of gene expression in multicellular organisms. Shortly thereafter it was discovered that small dsRNAs (typically 15-30 bp) can catalytically induce RNAi silencing in mammalian cells without eliciting nonspecific interferon responses. Targeting the RNAi pathway through small dsRNAs such as small interfering RNAs (siRNAs) and short hairpin RNA (shRNA) has several theoretical advantages over antisense being notably more efficient (catalytic) and giving longer inhibition of gene expression. This could translate into lower doses and lower cost together with less frequent dosing. Lower exposures could also mean fewer toxicity problems for siRNA.

However, before siRNA reaches its target *in vivo,* it faces a number of significant barriers that block its pathway to the RNA-Induced Silencing Complex (RISC) machinery. Upon entering the bloodstream, siRNA is vulnerable to degradation by endogenous nucleases, and renal excretion due to its small size and highly anionic character. In addition, before reaching its target cell, the siRNA must navigate the tight endothelial junctions of the blood vessels and diffuse through the extracellular matrix. Due to its numerous negative charges, siRNA does not readily bind to or cross the cell membrane, and once inside cells, it must escape from endosomes to interact with its intracellular protein targets.

Hence, the success of oligonucleotide (such as siRNA) therapy strongly depends on an effective delivery of the drug from outside a cell into the cytosol. Consequently, much attention is directed at developing delivery systems which improve oligonucleotide (such as siRNA) delivery. Aside from viral delivery, the main methods employed to enhance siRNA (or other oligonucleotides) delivery to the cell employ liposomes, cell-penetrating peptides (CPPs) and their mimics, or nanoparticles (Gooding, Matt, et al. Chemical biology & drug design 80.6 (2012): 787-809). Safety concerns, such as the possibility of insertion mutagenesis and immunogenesis, are considered to limit the future of viral approaches.

Liposomes are the most commonly used delivery vector for oligonucleotides such as siRNA, where the oligonucleotide is encapsulated within a lipid bilayer. Several problems are encountered with liposomal oligonucleotide delivery, such as oxygen radical-mediated toxicity (typical for of cationic liposomes), cell toxicity, effects on gene regulation and inflammatory responses. Furthermore, *in vivo* delivery using lipids seems to predominantly target the liver and spleen.

Cell-penetrating peptides (CCP), also called protein transduction domains, are short peptides (usually <30 amino-acid residues long) which have the unusual property of being able to cross the cell membrane. Through covalent linking to the oligonucleotide or through the formation of non-covalent complexes with the oligonucleotide, a CPP may enhance cellular uptake of the oligonucleotide. The field of CPP mediated oligonucleotide delivery is extremely complex since it combines the challenges posed by both oligonucleotide technology and peptide technology, two fields which are far from mature, in a single drug. Aside from the oligonucleotide-related issues, typical challenges encountered for CPPs are related to the (lack of) *in vivo* stability of the peptide chain, often requiring the use of non-natural peptide derivatives which may be complex to synthesize and/or exhibit reduced cell-penetrating activity.

Despite the enhanced cellular delivery, overcoming entrapment by endosomes is one of the major challenges to the design of efficient CPPs and other transport systems (Gooding et al).

Nanoparticles and nanocarriers are nanoscale oligonucleotide delivery systems typically comprised of a polymer, biological stabilizers and cell-targeting ligands complexed with the oligonucleotide. An exemplary siRNA nanocarrier system is known under the name siRNA Dynamic Poly-Conjugates. This system is based around an amphipathic polymer linked to polyethylene glycol (PEG) as a biological stabilizer and a hepatocyte-targeting ligand wherein the siRNA is covalently bound to the polymer by a disulfide bond. The targeting moieties and PEG moieties are attached to the polymer via maleamate linkages, forming negatively charged nanoparticles, which do not bind to serum proteins. Following internalization by endocytosis, the maleamate bonds are readily hydrolysed on acidification of the endosome, exposing the cationic amine groups of the polymer and inducing endosomal escape via a proton sponge effect. Nanocarriers are also known in the form of cationic polymers such as polyethyleneimines (PEls) (sometimes combined with cyclodextrins), which can form electrostatic complexes with oligonucleotides such as siRNA, affording protection from degradation and aiding internalisation. However, toxicity at higher concentrations resulting from membrane disruption and apoptosis induction may limit the applications of cationic polymers as therapeutic delivery agent. Aside from complex preparation of the nanoparticle and nanocarriers, their long-term stability is a major barrier to commercially viable use.

Lipoproteins that cause cardiovascular disease, such as LDL, remnant lipoproteins, and Lp(a), can be reduced using gene-silencing therapies by targeting apolipoprotein (ApoB), which protein has an important role in production or removal of these lipoproteins. Antisense oligonucleotide-based approaches and short-interference RNA based approaches have been applied. Clinical studies have assessed the efficacy of such gene-silencing therapies by targeting the apoB gene. Adverse effects of lipid-lowering antisense oligonucleotide and siRNA therapies include injection-site reactions, flu-like symptoms, and low blood platelet counts. Adverse effects are perhaps addressed with newer generations of these drugs that deliver the drugs more efficiently to liver cells using GalNAc conjugation, reducing the levels of the drug in plasma. Despite the improved oligonucleotide (such as siRNA) delivery to the cell which may be achieved by some of these delivery systems, endosomal escape remains a major barrier to the application of oligonucleotide-based therapeutics such as RNAi-based therapeutics. Only a minuscule portion of endocytosed oligonucleotide escapes into the cytoplasmic space where it can exert its intended function, while the vast majority remains trapped in endocytic compartments and is inactive. Recent literature suggests a passive siRNA escape rate of <0.01% (Setten, Ryan L., et al.. Nature Reviews Drug Discovery 18.6 (2019): 421-446). Furthermore, it has been shown that the capacity of cells to functionally internalize oligonucleotides to produce target effect (e.g., knockdown) appears to be independent of the extent to which cells internalize bulk oligonucleotides. These observations have led to the hypothesis of separate 'productive' and 'nonproductive' free-uptake pathways, although the molecular mechanisms distinguishing these pathways remain obscure (Setten, Ryan L., John J. Rossi, and Si-ping Han. Nature Reviews Drug Discovery 18.6 (2019): 421-446).

All in all, there remains a significant need for improved delivery systems of oligonucleotides which result in an increased potency (e.g., target knockdown), less toxicity and/or less off-target effects, regardless of the underlying mechanism (improved endosomal escape, increased 'productive' pathway uptake, or another mechanism). SABINE SEWING ET AL: "GalNAc Conjugation Attenuates the Cytotoxicity of Antisense Oligonucleotide Drugs in Renal Tubular Cells", MOLECULAR THERAPY: NUCLEIC ACIDS., vol. 14, 1 March 2019 (2019-03-01), pages 67-79" also discusses the search for a balance between efficacy and toxicity of oligonucleotides and GalNAc-oligonucleotides
An emerging strategy entails the conjugation of antisense oligonucleotides (ASOs) to receptor ligands in order to increase oligonucleotide potency and distribution to selected tissues. For instance, conjugation of triantennary N-acetyl galactosamine (GalNAc) to oligonucleotide therapeutics yields 10-30-fold increased potency in isolated hepatocytes, as well as in liver *in vivo.* GalNAc is found on damaged glycoproteins that have lost terminal sialic acid residues from their pendant oligosaccharides. The liver functions to clear these proteins from the systemic circulation by expressing trimeric asialoglycoprotein receptors (ASGPRs) at very high levels (order of 10⁵-10⁶ per cell) on the surface of hepatocytes. ASGPRs bind specifically to GalNAc at neutral pH for endocytosis of circulating macromolecules from the blood and release GalNAc at acidic pH (~5-6) for cargo drop- off in the early endosome. Freed ASGPRs are then recycled back to the cell surface for reuse. Approximately one-third of RNAi drugs currently in clinical trials are single- molecule, chemically modified RNAi triggers conjugated to multivalent GalNAc ligands targeting the ASGPRs. The suitable physiology of the liver, the unique properties of ASGPRs, the non- toxic nature of the GalNAc ligand and the simplicity of GalNAc-siRNA conjugates make this an attractive approach for systemic RNAi delivery to hepatocytes.

However, there still exists a need for improved delivery systems which further increase the potency, decrease the toxicity and/or reduce off-target effects of ASGPR targeted oligonucleotide systems (e.g. GalNAc-oligonucleotide conjugates).

### SUMMARY

A first aspect of the invention relates to a saponin conjugate as defined in claim 1, comprising at least one saponin covalently linked to a ligand for asialoglycoprotein receptor (ASGPR), wherein the ligand for ASGPR comprises at least one N-acetylgalactosamine (GalNAc) moiety, preferably three or four GalNAc moieties, more preferably the ligand for ASGPR comprises or consists of (GalNAc)₃Tris, wherein the at least one saponin is selected from monodesmosidic triterpenoid saponins and bidesmosidic triterpenoid saponins. The saponin is a saponin derivative comprising a saccharide chain bound to the aglycone core structure, which is selected from group A:
GlcA-
Gal-(1→2)-[Xyl-(1→3)]-GlcA-,
Glc-(1→2)-[Glc-(1→4)]-GlcA-,
Glc-(1→2)-Ara-(1→3)-[Gal-(1→2)]-GlcA-,
Xyl-(1→2)-Ara-(1→3)-[Gal-(1→2)]-GlcA-,
Rha-(1→2)-Gal-(1→3)-[Glc-(1→2)]-GlcA-,
Ara-(1→4)-Rha-(1→2)-Glc-(1→2)-Rha-(1→2)-GlcA-,
Ara-(1→4)-Fuc-(1→2)-Glc-(1→2)-Rha-(1→2)-GlcA-,
Ara-(1→4)-Rha-(1→2)-Gal-(1→2)-Rha-(1→2)-GlcA-,
Ara-(1→4)-Fuc-(1→2)-Gal-(1→2)-Rha-(1→2)-GlcA-,
Ara-(1→4)-Rha-(1→2)-Glc-(1→2)-Fuc-(1→2)-GlcA-,
Ara-(1→4)-Fuc-(1→2)-Glc-(1→2)-Fuc-(1→2)-GlcA-,
Ara-(1→4)-Rha-(1→2)-Gal-(1→2)-Fuc-(1→2)-GlcA-,
Ara-(1→4)-Fuc-(1→2)-Gal-(1→2)-Fuc-(1→2)-GlcA-,
Xyl-(1→4)-Rha-(1→2)-Glc-(1→2)-Rha-(1→2)-GlcA-,
Xyl-(1→4)-Fuc-(1→2)-Glc-(1→2)-Rha-(1→2)-GlcA-,
Xyl-(1→4)-Rha-(1→2)-Gal-(1→2)-Rha-(1→2)-GlcA-,
Xyl-(1→4)-Fuc-(1→2)-Gal-(1→2)-Rha-(1→2)-GlcA-,
Xyl-(1→4)-Rha-(1→2)-Glc-(1→2)-Fuc-(1→2)-GlcA-,
Xyl-(1→4)-Fuc-(1→2)-Glc-(1→2)-Fuc-(1→2)-GlcA-,
Xyl-(1→4)-Rha-(1→2)-Gal-(1→2)-Fuc-(1→2)-GlcA-,
Xyl-(1→4)-Fuc-(1→2)-Gal-(1→2)-Fuc-(1→2)-GlcA-, and
derivatives thereof, wherein said saccharide chain comprises a carboxyl group which has been derivatised and the carboxyl group which is derivatised is part of a glucuronic acid moiety.

A second aspect of the invention according to claim 15 relates to a pharmaceutical combination comprising:
- a first pharmaceutical composition comprising the saponin conjugate of the invention and optionally comprising a pharmaceutically acceptable excipient and/or a pharmaceutically acceptable diluent; and
- a second pharmaceutical composition comprising a second conjugate of an effector molecule and a ligand for ASGPR, wherein the ligand for ASGPR preferably comprises at least one GalNAc moiety, preferably three or four GalNAc moieties, more preferably the ligand for ASGPR comprises or consists of (GalNAc)₃Tris, or a third conjugate of an effector molecule and a binding molecule comprising a binding site for a cell-surface molecule, and optionally comprising a pharmaceutically acceptable excipient and/or pharmaceutically acceptable diluent.

A third aspect of the invention relates to a pharmaceutical composition comprising:
- the saponin conjugate of the invention;
- a second conjugate of an effector molecule and a ligand for ASGPR wherein the ligand for ASGPR preferably comprises at least one GalNAc moiety, preferably three or four GalNAc moieties, more preferably the ligand for ASGPR comprises or consists of (GalNAc)₃Tris, or a third conjugate of an effector molecule and a binding molecule comprising a binding site for a cell-surface molecule, and optionally comprising a pharmaceutically acceptable excipient and/or pharmaceutically acceptable diluent.

A fourth aspect of the invention relates to a pharmaceutical combination of the invention or pharmaceutical composition of the invention, for use as a medicament.

A fifth aspect of the invention relates to a pharmaceutical combination of the invention or pharmaceutical composition of the invention, for use in the treatment or prophylaxis of a disease or health problem in which an expression product is involved of any one or more of genes: apoB, TTR, PCSK9, ALAS1, AT3, GO, CC5, X gene of HBV, S gene of HBV, AAT and LDH.

A sixth aspect of the invention relates to an *in vitro* or *ex vivo* method for transferring the second conjugate or the third conjugate of the invention from outside a cell to inside said cell, preferably subsequently transferring the effector molecule comprised by the second conjugate or the third conjugate of the invention into the cytosol of said cell, comprising the steps of:
a) providing a cell which expresses ASGPR on its surface, and, when the third conjugate is to be transferred into the cell, which expresses the cell-surface molecule for which the third conjugate comprises a binding molecule for binding to said cell-surface molecule, the cell preferably selected from a liver cell, a virally infected cell and a tumor cell;
b) providing the second conjugate or the third conjugate of any one of the invention for transferring into the cell provided in step a);
c) providing the saponin conjugate of the invention;
d) contacting the cell of step a) *in vitro* or *ex vivo* with the second conjugate or the third conjugate of step b) and the saponin conjugate of step c), therewith effecting the transfer of the second conjugate or the third conjugate from outside the cell into said cell, and preferably therewith subsequently effecting the transfer of the second conjugate or the third conjugate into the cytosol of said cell, or preferably therewith subsequently effecting the transfer of at least the effector molecule comprised by the second conjugate or the third conjugate into the cytosol of said cell.

### DEFINITIONS

The term "GalNAc" has its regular scientific meaning and here refers to N-acetylgalactosamine and to the IUPAC name thereof: 2-(acetylamino)-2-deoxy-D-galactose.

The term "(GalNAc)₃Tris" has its regular scientific meaning in for example the field of siRNA-based therapy, and here refers to a moiety comprising three GalNAc units each separately covalently bound to the hydroxyl groups of tris(hydroxymethyl)aminomethane (Tris) (IUPAC name: 2-amino-2-(hydroxymethyl) propane-1,3-diol), preferably via at least one linker. (GalNAc)₃Tris can exist as a free amine comprising molecule or may be further functionalized via the remaining amine binding site, for example to form the (GalNAc)₃Tris-moiety comprising conjugates described herein.

The term "oligonucleotide" has its regular scientific meaning and here refers to a string of two or more nucleotides, i.e., an oligonucleotides is a short oligomer composed of ribonucleotides or deoxyribonucleotides. Examples are RNA and DNA, and any modified RNA or DNA, such as a string of nucleic acids comprising a nucleotide analogue such as a bridged nucleic acid (BNA), also known as locked nucleic acid (LNA) or a 2'-O,4'-C-aminoethylene or a 2'-O,4'-C-aminomethylene bridged nucleic acid (BNA^{NC}), wherein the nucleotide is a ribonucleotide or a deoxyribonucleotide.

The term "RNAi-mediated gene-targeting" has its regular scientific meaning and here refers to the *in vivo, ex vivo* or *in vitro* approach of influencing functioning of the gene in a cell by transferring into said cell an oligonucleotide, such as a small double-stranded RNA molecule, that targets mRNA involved in transcription of the gene: for example, small double-stranded RNA molecules are capable of efficiently triggering RNAi silencing of specific genes.

The term "bridged nucleic acid", or "BNA" in short, or "locked nucleic acid" or "LNA" in short, has its regular scientific meaning and here refers to a modified RNA nucleotide. A BNA is also referred to as 'constrained RNA molecule' or 'inaccessible RNA molecule'. A BNA monomer can contain a five-membered, six-membered or even a seven-membered bridged structure with a "fixed" C₃'-endo sugar puckering. The bridge is synthetically incorporated at the 2', 4'-position of the ribose to afford a 2', 4'-BNA monomer. A BNA monomer can be incorporated into an oligonucleotide polymeric structure using standard phosphoramidite chemistry known in the art. A BNA is a structurally rigid oligonucleotide with increased binding affinity and stability.

The term antisense oligonucleotide has its regular scientific meaning and may be indicated in short in the description as "AON" or "ASO".

The term "BNA-based antisense oligonucleotide", or in short "BNA-AON", has its regular scientific meaning and here refers to a string of antisense nucleotides wherein at least one of said nucleotides is a BNA.

The term "proteinaceous" has its regular scientific meaning and here refers to a molecule that is protein-like, meaning that the molecule possesses, to some degree, the physicochemical properties characteristic of a protein, is of protein, relating to protein, containing protein, pertaining to protein, consisting of protein, resembling protein, or being a protein. The term "proteinaceous" as used in for example 'proteinaceous molecule' refers to the presence of at least a part of the molecule that resembles or is a protein, wherein 'protein' is to be understood to include a chain of amino-acid residues at least two residues long, thus including a peptide, a polypeptide and a protein and an assembly of proteins or protein domains. In the proteinaceous molecule, the at least two amino-acid residues are for example linked via (an) amide bond(s), such as (a) peptide bond(s). In the proteinaceous molecule, the amino-acid residues are natural amino-acid residues and/or artificial amino-acid residues such as modified natural amino-acid residues. In a preferred embodiment, a proteinaceous molecule is a molecule comprising at least two amino-acid residues, preferably between two and about 2.000 amino-acid residues. In one embodiment, a proteinaceous molecule is a molecule comprising from 2 to 20 (typical for a peptide) amino acids. In one embodiment, a proteinaceous molecule is a molecule comprising from 21 to 1.000 (typical for a polypeptide, a protein, a protein domain, such as an antibody, a Fab, an scFv, a ligand for a receptor such as EGF) amino acids. Preferably, the amino-acid residues are (typically) linked via (a) peptide bond(s). According to the invention, said amino-acid residues are or comprise (modified) (non-)natural amino acid residues.

The term "effector molecule", or "effector moiety" when referring to the effector molecule as part of e.g. a covalent conjugate, has its regular scientific meaning and here refers to a molecule that can selectively bind to for example any one or more of the target molecules: a protein, a peptide, a carbohydrate, a saccharide such as a glycan, a (phospho)lipid, a nucleic acid such as DNA, RNA, an enzyme, and regulates the biological activity of such one or more target molecule(s). The effector molecule is for example a molecule selected from any one or more of a small molecule such as a drug molecule, a toxin such as a protein toxin, an oligonucleotide such as a BNA, a xeno nucleic acid or an siRNA, an enzyme, a peptide, a protein, or any combination thereof. Thus, for example, an effector molecule or an effector moiety is a molecule or moiety selected from any one or more of a small molecule such as a drug molecule, a toxin such as a protein toxin, an oligonucleotide such as a BNA, a xeno nucleic acid or an siRNA, an enzyme, a peptide, a protein, or any combination thereof, that can selectively bind to any one or more of the target molecules: a protein, a peptide, a carbohydrate, a saccharide such as a glycan, a (phospho)lipid, a nucleic acid such as DNA, RNA, an enzyme, and that upon binding to the target molecule regulates the biological activity of such one or more target molecule(s). Typically, an effector molecule can exert a biological effect inside a cell such as a mammalian cell such as a human cell, such as in the cytosol of said cell. Typical effector molecules are thus drug molecules, plasmid DNA, toxins such as toxins comprised by antibody-drug conjugates (ADCs), oligonucleotides such as siRNA, BNA, nucleic acids comprised by an antibody-oligonucleotide conjugate (AOC). For example, an effector molecule is a molecule which can act as a ligand that can increase or decrease (intracellular) enzyme activity, gene expression, or cell signalling.

The term "health problem" has its regular scientific meaning and here refers to any condition of the body of a subject such as a human patient, or of a part, organ, muscle, vein, artery, skin, limb, blood, cell, etc. thereof, that is suboptimal when compared to the condition of that body or part thereof in a healthy subject, therewith hampering the proper functioning and/or well-being of the subject, e.g. impairs normal functioning of a human body.

The term "GalNAc-decorated oligonucleotide drug" has its regular scientific meaning and here refers to an oligonucleotide for interfering in transcription of a gene, wherein one or more GalNAc units are coupled to the oligonucleotide, e.g., via at least one linker.

The term "locked nucleic acid", in short "LNA" has its regular scientific meaning and here refers to an oligonucleotide that contains one or more nucleotide building blocks in which an additional methylene bridge fixes the ribose moiety either in the C3'-endo conformation (beta-D-LNA) or C2'-endo (alpha-L-LNA) conformation, as is known in the art.

The term "bridged nucleic acid NC", in short "BNA^{NC}" has its regular scientific meaning and here refers to an oligonucleotide that contains one or more nucleotide building blocks in which an additional 2'-O,4'-C-aminoethylene bridged nucleic acid is comprised, with different substitutions at the N atom (of which a methyl group is the most commonly used to date), as is known in the art.

The term "chemically modified, metabolically stable siRNA" has its regular scientific meaning and here refers to an siRNA molecule comprising chemical modifications compared to the RNA oligonucleotide consisting of naturally occurring nucleotides, such that the modified siRNA molecule is more resistant towards metabolic degradation, digestion, enzymatic lysis, *etc*., *i.e.*, more stable.

The term "saponin" has its regular scientific meaning and here refers to a group of amphipatic glycosides which comprise one or more hydrophilic glycone moieties combined with a lipophilic aglycon core which is a sapogenin. The saponin may be naturally occurring or synthetic (*i.e.,* non-naturally occurring). The term "saponin" includes naturally occurring saponins, derivatives of naturally occurring saponins as well as saponins synthesized *de novo* through chemical and/or biotechnological synthesis routes.

The term "saponin derivative" (also known as "modified saponin") has its regular scientific meaning and here refers to a compound corresponding to a naturally-occurring saponin which has been derivatised by one or more chemical modifications, such as the oxidation of a functional group, the reduction of a functional group and/or the formation of a covalent bond with another molecule. Preferred modifications include derivatisation of an aldehyde group of the aglycon core; of a carboxyl group of a saccharide chain or of an acetoxy group of a saccharide chain. Typically, the saponin derivative does not have a natural counterpart, i.e. the saponin derivative is not produced naturally by e.g. plants or trees. The term "saponin derivative" includes derivatives obtained by derivatisation of naturally occurring saponins as well as derivatives synthesized *de novo* through chemical and/or biotechnological synthesis routes resulting in a compound corresponding to a naturally occurring saponin which has been derivatised by one or more chemical modifications.

The term "aglycone core structure" has its regular scientific meaning and here refers to the aglycone core of a saponin without the one or two carbohydrate antenna or saccharide chains (glycans) bound thereto. For example, quillaic acid is the aglycone glycoside core structure for SO1861, QS-7, QS-21.

The term "saccharide chain" has its regular scientific meaning and here refers to any of a glycan, a carbohydrate antenna, a single saccharide moiety (monosaccharide) or a chain comprising multiple saccharide moieties (oligosaccharide, polysaccharide). The saccharide chain can consist of only saccharide moieties or may also comprise further moieties such as any one of 4E-Methoxycinnamic acid, 4Z-Methoxycinnamic acid, and 5-O-[5-O-Ara/Api-3,5-dihydroxy-6-methyl-octanoyl]-3,5-dihydroxy-6-methyl-octanoic acid), such as for example present in QS-21.

The term "Api/Xyl-" or "Api- or Xyl-" in the context of the name of a saccharide chain has its regular scientific meaning and here refers to the saccharide chain either comprising an apiose (Api) moiety, or comprising a xylose (Xyl) moiety.

The term "antibody-drug conjugate" or "ADC" has its regular scientific meaning and here refers to any conjugate of an antibody such as an IgG, a Fab, an scFv, an immunoglobulin, an immunoglobulin fragment, one or multiple Vh domains, *etc.,* and any molecule that can exert a therapeutic effect when contacted with cells of a subject such as a human patient, such as an active pharmaceutical ingredient, a toxin, an oligonucleotide, an enzyme, a small molecule drug compound, etc.

The term "antibody-oligonucleotide conjugate" or "AOC" has its regular scientific meaning and here refers to any conjugate of an antibody such as an IgG, a Fab, an scFv, an immunoglobulin, an immunoglobulin fragment, one or multiple Vh domains, etc., with any oligonucleotide molecule that can exert a therapeutic effect when contacted with cells of a subject such as a human patient, such as an oligonucleotide selected from a natural or synthetic string of nucleic acids encompassing DNA, modified DNA, RNA, modified RNA, synthetic nucleic acids, presented as a single-stranded molecule or a double-stranded molecule, such as a BNA, an allele-specific oligonucleotide, a short or small interfering RNA (siRNA; silencing RNA), an anti-sense DNA, anti-sense RNA, etc.

The term "conjugate" has its regular scientific meaning and here refers to at least a first molecule that is covalently bound to at least a second molecule, therewith forming an covalently coupled assembly comprising or consisting of the first molecule and the second molecule. Typical conjugates are tris(GalNAc)₃ - siRNA, an ADC, an AOC, and SO1861-EMCH (EMCH linked to the aldehyde group of the aglycone glycoside core structure of the saponin).

The term "moiety" has its regular scientific meaning and here refers to a molecule that is bound, linked, conjugated to a further molecule, linker, assembly of molecules, *etc.,* and therewith forming part of a larger molecule, conjugate, assembly of molecules. Typically, a moiety is a first molecule that is covalently bound to a second molecule, involving one or more chemical groups initially present on the first and second molecules. For example, saporin is a typical effector molecule. As part of an antibody-drug conjugate, the saporin is a typical effector moiety in the ADC. As part of an antibody-oligonucleotide conjugate, a BNA or an siRNA is a typical effector moiety in the AOC.

The term 'S' as used such as in an antibody-saponin conjugate or construct comprising a linker, represents 'stable linker' which remains intact in the endosome and in the cytosol.

The term 'L' as used such as in an antibody-saponin conjugate or construct comprising a linker, represents 'labile linker' which is cleaved under slightly acid conditions in the endosome.

The term "moiety" has its regular scientific meaning and here refers to a molecule that is bound, linked, conjugated to a further molecule, linker, assembly of molecules, etc., and therewith forming part of a larger molecule, conjugate, assembly of molecules. Typically, a moiety is a first molecule that is covalently bound to a second molecule, involving one or more chemical groups initially present on the first molecule and present on the second molecule. For example, saporin is a typical molecule, and is an example of an effector molecule. As part of an antibody-drug conjugate, the saporin is a typical effector moiety in the ADC. As part of an antibody-oligonucleotide conjugate, a BNA or an siRNA is a typical effector moiety in the AOC.

The term "DAR" normally stands for *Drug Antibody Ratio* and refers to the average *drug* to *antibody ratio* for a given preparation of *antibody drug* conjugate (ADC) and here refers to a ratio of the amount of bound SO1861 moieties, or SPT001, with respect to the conjugate molecule.

The terms first, second, third and the like in the description and in the claims, are used for distinguishing between for example similar elements, compositions, constituents in a composition, or separate method steps, and not necessarily for describing a sequential or chronological order. The terms are interchangeable under appropriate circumstances and the embodiments of the invention can operate in other sequences than described or illustrated herein, unless specified otherwise.

The embodiments of the invention described herein can operate in combination and cooperation, unless specified otherwise.

Furthermore, the various embodiments, although referred to as "preferred" or "e.g." or "for example" or "in particular" and the like are to be construed as exemplary manners in which the invention may be implemented rather than as limiting the scope of the invention.

The term "comprising", used in the claims, should not be interpreted as being restricted to for example the elements or the method steps or the constituents of a compositions listed thereafter; it does not exclude other elements or method steps or constituents in a certain composition. It needs to be interpreted as specifying the presence of the stated features, integers, (method) steps or components as referred to, but does not preclude the presence or addition of one or more other features, integers, steps or components, or groups thereof. Thus, the scope of the expression "a method comprising steps A and B" should not be limited to a method consisting only of steps A and B, rather with respect to the present invention, the only enumerated steps of the method are A and B, and further the claim should be interpreted as including equivalents of those method steps. Thus, the scope of the expression "a composition comprising components A and B" should not be limited to a composition consisting only of components A and B, rather with respect to the present invention, the only enumerated components of the composition are A and B, and further the claim should be interpreted as including equivalents of those components.

In addition, reference to an element or a component by the indefinite article "a" or "an" does not exclude the possibility that more than one of the element or component are present, unless the context clearly requires that there is one and only one of the elements or components. The indefinite article "a" or "an" thus usually means "at least one".

### BRIEF DESCRIPTION OF THE DRAWINGS

**Figure 1****:** synthesis of trivalent-GalNAc.
**Figure 2****:** synthesis of SO1861-DBCO.
**Figure 3****:** synthesis of monovalent-GalNAc-SO1861.
**Figure 4****:** synthesis of trivalent-GalNAc-SO1861.

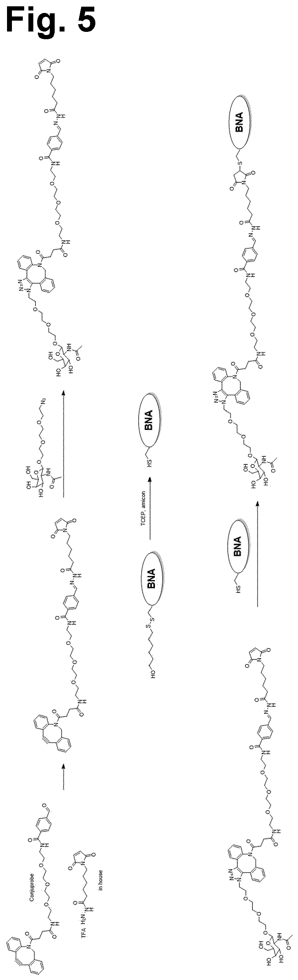

**Figure 5****:** synthesis of monovalent-GalNAc-BNA.
**Figure 6****:** synthesis of trivalent-GaINAc-BNA.
**Figure 7****:** synthesis of trivalent-GaINAc-BNA.
**Figure 8A** **and** **8B****:** synthesis of trivalent GalNAc.
**Figure 9A and 9B****:** General toxicity (MTS) of GalNAc-SO1861 and trivalent-GalNAc-SO1861 on HepG2 (A) and Huh7 (B) cell lines. The legend displayed next to Figure 9B also applies for Figure 9A.
**Figure 10A and 10** **B:** Cell killing assay (MTS) HepG2 (A) and Huh7 (B) cell lines. The legend displayed next to Figure 10B also applies for Figure 10A.
**Figure 11A and 11B****:** Gene expression analysis on HepG2 (A) and Huh7 (B) cell lines.
**Figure 12A and 12B****:** Gene expression analysis on HepG2 (A) and Huh7 (B) cell lines. The legend displayed next to Figure 12B also applies for Figure 12A.
**Figure 13A and 13B****:** cell viability assay on HepG2 (A) and Huh7 (B) cell lines. The legend displayed next to Figure 13B also applies for Figure 13A.
**Figure 14A and 14B****:** Gene expression analysis on HepG2 (A) and Huh7 (B) cell lines. The legend displayed next to Figure 14B also applies for Figure 14A.
**Figure 15A and 15B****:** cell viability assay on HepG2 (A) and Huh7 (B) cell lines. The legend displayed next to Figure 15B also applies for Figure 15A.
**Figure 16A and 16B****:** Gene expression analysis on HepG2 (A) and Huh7 (B) cell lines. The legend displayed next to Figure 16B also applies for Figure 16A.
**Figure 17A and 17B****:** Gene expression analysis on HepG2 (A) and Huh7 (B) cell lines. The legend displayed next to Figure 17B also applies for Figure 17A.
**Figure 18****:** *ApoB* RNA expression analysis in mouse primary hepatocytes.
**Figure 19****:** *ApoB* RNA expression analysis in liver tissue of C57BL/6J mice.
**Figure 20****:** Serum *ApoB* protein analysis in C57BL/6J mice. NB, no analysis values are reported for 1 mg/kg ApoB#02 and 0.01 mg/kg (GalNAc)3-ApoB#03 + 5 mg/kg (GalNAc)3-SO861 groups at 336 hours.
**Figure 21****:** Serum LDL-cholesterol (LDL-C) analysis C57BL/6J mice.
**Figure 22****:** Serum ALT analysis C57BL/6J mice.
**Figure 23****:** Cell viability assay (CTG) on primary human hepatocytes
**Figure 24****:** ApoB RNA expression analysis and cell viability assay on primary human hepatocytes
**Figure 25****:** (GalNAc)3-Ls-SO1861 synthesis, intermediate 22, 23.
**Figure 26:** (GalNAc)3-Ls-SO1861 synthesis, intermediate 24.
**Figure 27****:** (GalNAc)3-Ls-BNA) synthesis, intermediate 25, 26.
**Figure 28****:** (GalNAc)3-Ls-BNA) synthesis.

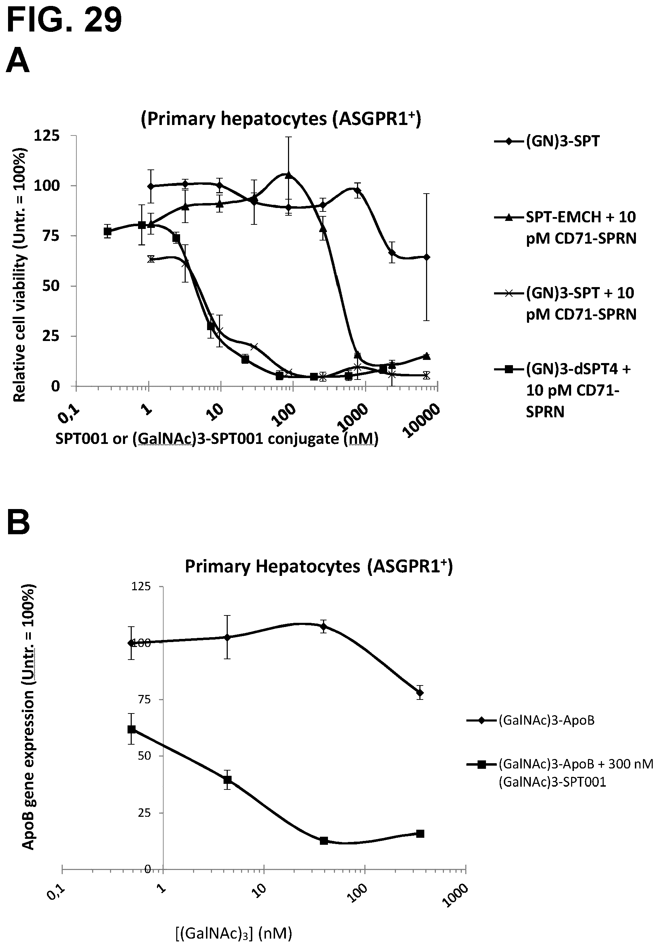

**Figure 29A****:** Relative cell viability of primary human hepatocytes (compared with untreated cells 'Untr', which is set to 100%) upon contacting the cells with a concentration series of trivalent GalNAc conjugated with SO1861 ((GN)₃-SPT), the combination of 10 pM monoclonal antibody anti-CD71 conjugated with saporin (CD71-SPRN) and a concentration series of SO1861 with EMCH bound to the aglycone aldehyde group (SPT-EMCH), the combination of 10 pM CD71-SPRN and a concentration series of (GN)₃-SPT, and the combination of 10 pM CD71-SPRN and a concentration series of (GN)₃ conjugated with a dendron with four SO1861 moieties covalently bound thereto (DAR = 4 for the bound SO1861) ((GN)₃-dSPT4).
**Figure 29B****:** Relative ApoB gene expression of primary human hepatocytes (compared with untreated cells 'Untr', which is set to 100%) upon contacting the cells with a concentration series of trivalent GalNAc conjugated with antisense oligonucleotide for silencing ApoB mRNA and ApoB protein expression ((GalNAc)₃-ApoB), the combination of 300 nM trivalent GalNAc conjugated with SO1861 ((GalNAc)₃-SPT001) and a concentration series of (GalNAc)₃-ApoB. 'Untr' are untreated cells, set at ApoB expression is 100%.
**Figure 30A and 30B****:** Cell viability of primary human hepatocytes (A) and hepatocyte cell line Huh7 cells (B) (both compared with untreated cells 'Untr', which is set to 100%), upon contacting the cells with a concentration series of trivalent GalNAc conjugated with SO1861 (Trivalent GalNAc-SPT001), the combination of 10 pM monoclonal antibody anti-CD71 conjugated with saporin (CD71-saporin) and a concentration series of covalent conjugate Trivalent GaINAc-SPT001.
**Figure 30C****:** Cartoon of covalent Trivalent GalNAc-SPT001 conjugate (DAR = 1 for the SO1861). 'S' is SPT001, SO1861 in the cartoon.
**Figure 30D****:** Cartoon of Anti-CD71 antibody - saporin conjugate (OKT-9), wherein 'T' is the toxin saporin covalently linked to the antibody heavy chain.
**Figure 31A** **and B:** Relative ApoB gene expression of primary human hepatocytes (A) and hepatocyte cell line Huh7 cells (B), upon contacting the cells with a concentration series of trivalent GalNAc conjugated with antisense oligonucleotide for silencing ApoB mRNA and ApoB protein expression (TrivalentGalNAc-*ApoB*BNA), the combination of 300 nM trivalent GalNAc conjugated with SO1861 (TrivalentGaINAc-SPT001) (DAR = 1 with regard to bound SPT001 (SO1861)) and a concentration series TrivalentGaINAc-ApoBBNA.
**Figure 31C****:** Cartoon of covalent TrivalentGalNAc-ApoBBNA conjugate. 'A' is antisense oligonucleotide ApoB (ApoBBNA) in the cartoon.
**Figure 32A** **and B:** Absolute ApoB protein expression by primary human hepatocytes (A) and hepatocyte cell line Huh7 cells (B), under influence of contacting the cells with a concentration series of TrivalentGalNAc-*ApoB*BNA, the combination of 300 nM TrivalentGalNAc-SPT001 and a concentration series of TrivalentGaINAc-ApoBBNA.
**Figure 33A****:** Synthesis of dendron(SPT001)₄-NH₂ (molecule 15 from molecules 13 and 14).
**Figure 33B****:** Synthesis dendron(SPT001)₄-azide (molecule 19 from molecules 15 and 18).
**Figure 33C****:** Synthesis dendron(SPT001)₄-trivalent GalNAc (molecule 24 from molecules 19 and 23).

### DETAILED DESCRIPTION

The present invention will be described with respect to particular embodiments.

A first aspect of the invention relates to a saponin conjugate according to claim 1, which saponin conjugate comprising at least one saponin covalently linked to a ligand for asialoglycoprotein receptor (ASGPR), wherein the ligand for ASGPR comprises at least one N-acetylgalactosamine (GalNAc) moiety, preferably three or four GalNAc moieties, more preferably the ligand for ASGPR comprises or consists of (GalNAc)₃Tris, wherein the at least one saponin is selected from monodesmosidic triterpenoid saponins and bidesmosidic triterpenoid saponins.

Saponins of the triterpene glycoside type have the capacity to stimulate the delivery of effector molecules from outside a target cell to inside said cell, and subsequently into the cytosol of said cell. Saponins facilitate for example receptor mediated uptake of certain effector molecules, such as a protein toxin comprised in an ADC. Endocytosis of the toxin, as part of the ADC, delivers the toxin in the endosome and/or in the lysosome of the target cell. Without wishing to be bound by any theory, subsequent release of the ADC, or at least of the toxin, out of the endosome or lysosome and into the cell cytosol, is stimulated or mediated by the triterpenoid saponin. When a target cell is contacted with the toxin at a toxin dose that is too low for exerting an intracellular effect, co-administration of the toxin, such as part of an ADC, and a free saponin, such that the target cell is contacted with both the ADC and the saponin, can result in efficient toxin mediated killing of the target cell, at the same toxin dose that is ineffective when contacted with the cell in the absence of the saponin. Thus, under influence of the saponin, the therapeutic window of the toxin is improved. Still, the toxin dose required for efficient and sufficient target cell killing, can be accompanied by undesired off-target effects, for example when the tumor cell receptor targeted by the ADC is not a truly tumor-cell specific receptor, though is also expressed to some lower extent at the surface of e.g. healthy cells, elsewhere in a patient's body, or on cells that are present in the same organ where the target cells reside. Furthermore, the required dose of the free saponin, needed to reach a sufficient extent of toxin activation (delivery of the toxin in the target-cell cytosol), may be at a level such that the risk for saponin-mediated side effects are apparent. Since the saponin is administered systemically, and non-targeted when the target (tumor) cell to be treated with the toxin is considered, a relatively high saponin dose is to be administered to the patient in need of the toxin treatment, in order to reach the local saponin dose at the level of the target cells.

The inventors now surprisingly found that the saponin dose required for potentiating the effect of a toxin on tumor cells, can be lowered by a factor of about 100 to 1000, when the saponin is provided with a target cell targeting moiety such as a receptor ligand (e.g. EGF or a cytokine), an antibody, or a binding fragment or domain thereof. For tumor cells, nowadays a series of cell-surface receptors are known for their specific expression on the surface of such aberrant cells. Saponins are successfully coupled by the inventors to antibodies and ligands for binding to tumor-cell specific receptors such as anti-CD71 antibody OKT-9, trastuzumab, cetuximab, etc. Such targeted saponin conjugates indeed are able to stimulate e.g. the cytotoxic effect of a toxin inside a target cell, though at a 100-fold to 1000-fold lower saponin dose compared to the dose required for the same saponin in free form, not provided with a tumor cell targeting binding molecule. The current inventors further invented that target cell specific delivery and subsequent endocytosis of the (targeted) saponin, is also possible for non-aberrant cells or aberrant cells not related to e.g. cancer (tumor cells), auto-immune disease, virally infected cells, etc. That is to say, surprisingly, the inventors found that the ASGPR provides a suitable entrance on cells bearing this receptor, such as liver cells, for entry of saponins provided with a ligand for ASGPR, in particular ASGPR1. Known ligands such as mono-valent GalNAc and tri-valent GalNAc are successfully coupled to one or a multitude (2, 4, 8, etc.) of saponin molecules, providing saponin conjugates comprising the ASGPR ligand and comprising at least one saponin moiety.

Contacting cells that express the ASGPR, such as liver cells, with the saponin conjugates comprising the ligand for the ASGPR, results surprisingly in uptake of the saponin, as evident when such cells are co-targeted with e.g. conjugates comprising a gene-silencing nucleic acid and a binding molecule for a target cell surface molecule, e.g. CD71, ASGPR, resulting in saponin dependent gene silencing in the target cell, or conjugates comprising such a binding molecule for the target cell and a toxin, resulting in saponin dependent cytotoxicity. Providing the saponin with a ligand for ASGPR results in effects in the target cell, exerted by a co-administered effector molecule such as a targeted oligonucleotide or toxin (e.g. anti-CD71 antibody or ASGPR ligand coupled to a nucleic acid or to a toxin). Such effects are apparent to a much lower extent, if at all, when the target cells are contacted with the effector molecule only, in the absence of targeted saponin. Thus, the saponin conjugates of the invention improve the therapeutic efficacy of effector molecules when a target cell comprising ASGPR at its surface is considered, and such target cell is contacted with both the saponin-ASGPR ligand of the invention and the (targeted) effector molecule. Herewith, the therapeutic window of the effector molecule is improved: higher therapeutic effect at the same dose, under influence of co-administration of the saponin conjugate; or similar therapeutic effect at lower dose, under influence of co-administration of the saponin conjugate. The inventors also found that about a 100-fold to 1000-fold lower dose of the saponin conjugate is required to reach a certain level of therapeutic effect in the target ASGPR expressing cell, such as a liver cell, induced by the co-administered (targeted) effector molecule such as an ASO, an siRNA, a BNA, a toxin such as a protein toxin, when compared to the saponin dose required for reaching the similar effect, when the saponin is not provided with a ligand for ASGPR.

Thus, surprisingly, the inventors now provide for improved therapeutic methods using the ASGPR as a target receptor for saponin-mediated delivery of an effector molecule in the cytosol of ASGPR expressing cells, resulting in similar therapeutic effects induced by the effector molecule at lower doses than possible in the absence of the saponin conjugate of the invention. Thus, the present invention provides for improved therapeutic use of the stimulatory effect of saponins, when the delivery of an effector molecule into the cytosol of, e.g., liver cells is considered, by providing the saponin - ASGPR ligand conjugate of the invention. Advantages reached by the inventors are amongst others: lower dose required for the effector molecule by co-administration of the (targeted) effector molecule with saponin, and lower dose required for the saponin by providing the saponin as a covalent conjugate of a ligand for ASGPR, such as tri-valent GalNAc, and one or more saponin moieties. Surprisingly, the ligand for ASGPR can be the same in the saponin conjugate and in the targeted effector molecule conjugate, resulting in efficient effector molecule-mediated effects in the target cell, under stimulatory influence of the saponin. That is to say, the saponin conjugate and the effector molecule conjugate, both comprising covalently linked ligand for ASGPR such as tri-valent GalNAc, can be co-administered to target cells, such as liver cells exposing the ASGPR at their surface, such that the desired therapeutic effect of the effector molecule is efficiently obtained, while both conjugates target the same receptor and use the same route for endocytosis into the cell. This surprising finding is highly beneficial for liver-cell targeting therapies such as therapies involving cytosolic delivery of a nucleic acid (ASO, BNA, siRNA, to list a few), since liver cells do express the ASGPR, which ASGPR is specific for liver cells, whereas further liver cell receptors specific enough for use in liver cell targeting therapy are virtually absent. The current invention thus opens new ways to improve liver-cell directed therapies wherein effector molecules have to be intracellularly delivered and located for their mode of action. Targeted saponin provided as the saponin conjugates of the invention widens the therapeutic window of such effector molecules, and at the same time, by providing the saponin with a liver cell targeting binding molecule, also the therapeutic window of the saponin is improved, according to the invention.

### Saponin conjugate - introduction

The present invention provides a conjugate of a saponin and a ligand for asialoglycoprotein receptor (ASGPR), referred to herein as "saponin conjugate". The ASGPR ligand comprises at least one N-acetylgalactosamine (GalNAc) moiety. In accordance with preferred embodiments of the present invention each GalNAc moiety is bound to the remainder of the ASGPR ligand or - in case the ASGPR ligand consists of a single GalNAc moiety - to the saponin moiety, via a covalent bond to the oxygen on position "1" as indicated in formula (I):

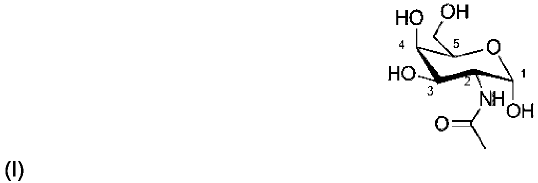

As shown in formula (II)s, the ASGPR ligand consists of a single GalNAc moiety bound to the saponin moiety S, preferably via a saponin moiety linker L_{S}.

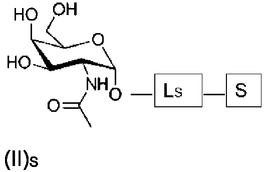

The ASGPR ligand may comprise more than one GalNAc moiety, such as 2, 3, 4, 5 or 6 GalNAc moieties, preferably 3 or 4 GalNAc moieties. In such case, it is preferred that the GalNAc moieties are each separately covalently bound via the oxygen on position "1" to a central bridging moiety B, which effectively forms a bridge between the GalNAc moieties and the saponin moiety, preferably via a saponin moiety linker L_{S}. The GalNAc moieties may be directly bound to the bridging moiety B as shown in formula (III)s: 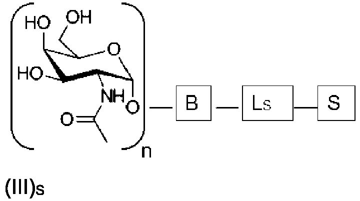 wherein n is an integer larger than or equal to 2, L_{S} is a saponin moiety linker and S is the saponin moiety. More preferably, the GalNAc moieties are bound to the bridging moiety B via GalNAc linkers L_{GAL} as shown in formula (IV)_{S}: 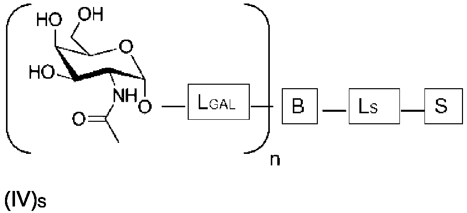 wherein n is an integer larger than or equal to 2, L_{S} is a saponin moiety linker and S is the saponin moiety. While each occurrence of L_{GAL} may be independently chosen, the skilled person will appreciate that the synthesis of the saponin conjugate is simplified in case each occurrence of L_{GAL} represents the same moiety.

### Saponin conjugate - Linker L_{S}

The saponin moiety linker L_{S} represents any chemical moiety suitable for covalently binding a saponin to GalNAc as in formula (II)_{S} or to the bridging moiety B as in formula (III)_{S} and (IV)_{S}. The identity and size of the linker is not particularly limited and covalently binding a saponin to GalNAc as in formula (II)_{S} or to the bridging moiety B as in formula (III)_{S} and (IV)_{S} may be effected by means of a 'regular' chemical functional group (e.g. an ester bond) as well as through "click-chemistry" type linkers which typically have a long chain length, resulting in a linker E_{S} comprising e.g. more than 10 or more than 20 carbon atoms. Suitable linkers Ls and associated coupling reactions for binding molecules to each other are described in the handbook Hermanson, Greg T. Bioconjugate techniques. Academic press, 2013.

As will be understood by the skilled person, the saponin moiety linker L_{S} will typically be the result of a coupling reaction (e.g. "click-chemistry" type) between at least a first precursor L_{S1} covalently bound to GalNAc or the bridging moiety B and a second precursor L_{S2} covalently bound to the saponin moiety. This principle is illustrated in the following reaction scheme for the compound of formula (II)_{S}: 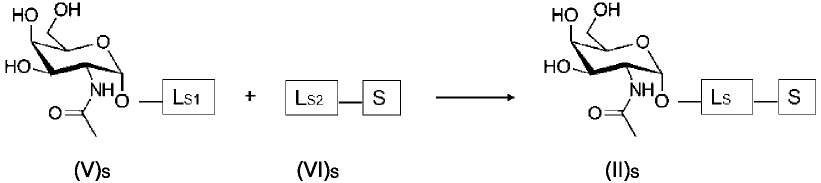 wherein Ls is a saponin moiety linker, L_{S1} is a precursor of the saponin moiety linker Ls which is covalently bound to GalNAc or to the bridging moiety B and L_{S2} is a precursor of the saponin moiety linker L_{S} which is covalently bound to the saponin moiety and S is the saponin moiety.

The corresponding reaction scheme for the compound of formula (III)_{S} is as follows: 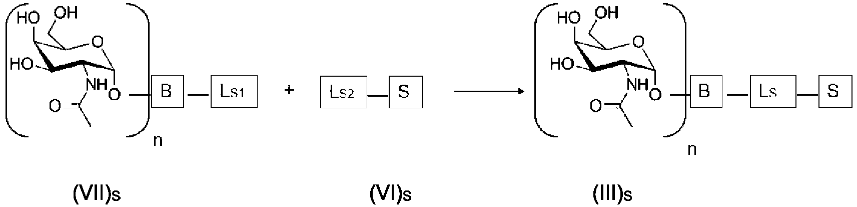 wherein Ls is a saponin moiety linker, L_{S1} is a precursor of the saponin moiety linker Ls which is covalently bound to GalNAc and L_{S2} is a precursor of the saponin moiety linker L_{S} which is covalently bound to the saponin moiety, n is an integer larger than or equal to 2, B is a bridging moiety and S is the saponin moiety.

The corresponding reaction scheme for the compound of formula (IV)s is as follows: 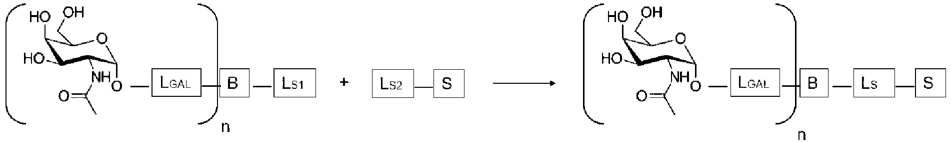 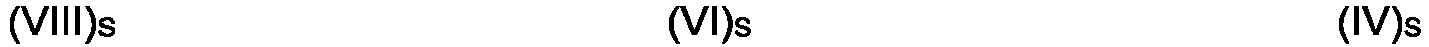 wherein Ls is a saponin moiety linker, L_{S1} is a precursor of the saponin moiety linker Ls which is covalently bound to GalNAc and L_{S2} is a precursor of the saponin moiety linker L_{S} which is covalently bound to the saponin moiety, n is an integer larger than or equal to 2, B is a bridging moiety, S is the saponin moiety and L_{GAL} is a GalNAc linker.

The saponin moiety linker Ls can be the result of a coupling reaction between at least a first precursor L_{S1} covalently bound to GalNAc or the bridging moiety B and a second precursor L_{S2} covalently bound to the saponin moiety, wherein the coupling reaction is for example an azide-alkyne cycloaddition, a thiol maleimide coupling, a Staudinger reaction, a nucleophilic ring-opening of strained heterocyclic electrophiles (such as aziridines, epoxides, cyclic sulfates, aziridinium ions, episulfonium ions), a carbonyl reaction of the non-aldol type (such as urea, thiourea, hydrazon, oxime ether, amide or aromatic heterocycle formation) or an addition to a carbon-carbon double bond (such as epoxidation, aziridination, dihydroxylation, sulfentyl halide addition, nitrosyl halide addition or micheal addition), preferably wherein the coupling reaction is an azide-alkyne cycloaddition, a thiol maleimide coupling, a Staudinger reaction, a nucleophilic ring-opening of strained heterocyclic electrophiles, more preferably wherein the coupling reaction is an azide-alkyne cycloaddition or a thiol maleimide coupling.

In accordance with some embodiments of the invention, the saponin moiety linker L_{S} comprises a hydrazone and/or a 1, 2, 3-triazole, preferably a hydrazone and a 1, 2, 3-triazole. Whenever reference is made to a 1, 2, 3-triazole in the context of the linkers of the present application, this preferably means a 1*H*-1, 2, 3-triazole.

Such a hydrazone is e.g. the result of a coupling between a terminal hydrazide and an aldehyde containing compound (such as an aldehyde containing saponin). This hydrazide/aldehyde coupling is a known 'click'-chemistry tool in the field of bioconjugation and is for example described in Hermanson, Greg T. Bioconjugate techniques. Academic press, 2013.

Such a 1, 2, 3-triazole is e.g. the result of a coupling between an azide and an alkyne containing compound. This azide/alkyne coupling is a commonly known 'click'-chemistry tool in the field of bioconjugation and is for example described in Hermanson, Greg T. Bioconjugate techniques. Academic press, 2013.

Consequently, it is preferred that the saponin moiety linker L_{S} is the result of a coupling reaction between a first precursor L_{S1} covalently bound to GalNAc or the bridging moiety B, the first precursor L_{S1} comprising an azide; and a second precursor L_{S2} covalently bound to the saponin moiety, the second precursor L_{S2} comprising an alkyne and preferably comprising a hydrazon resulting from a hydrazide/aldehyde coupling to an aldehyde of the saponin moiety.

Embodiments of the structure for the precursor L_{S1} present in the compounds of formula (V)_{S} are the following azides: 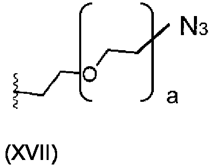 wherein a represents an integer larger than or equal to 0, preferably a represents an integer selected from 1, 2, and 3, more preferably a represents 2.

Embodiments of the structure for the precursor L_{S1} present in the compounds of formula (VII)_{S} or (VIII)_{S} are the following azides: 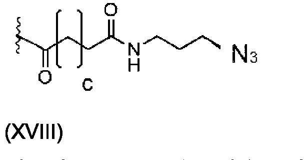 wherein c represents an integer larger than or equal to 0, preferably c represents an integer in the range of 5-15, more preferably c represents 9.

Embodiments of the structure for the precursor L_{S2} present in the compounds of formula (VI)_{S}, are the following hydrazons: 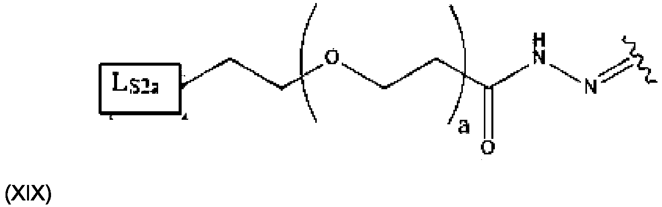 wherein a represents an integer larger than or equal to 0, preferably a represents an integer in the range of 2-6, more preferably a represents 4, and wherein L_{S2a} represents an alkyne-containing moiety. As will be understood by the skilled person in light of the present disclosure, the hydrazon depicted in formula (XIX) results from a reaction of a hydrazide with an aldehyde of the saponin moiety.

L_{S2a} preferably comprises less than 20 carbon atoms, more preferably L_{S2a} represents a moiety according to formula (XX): 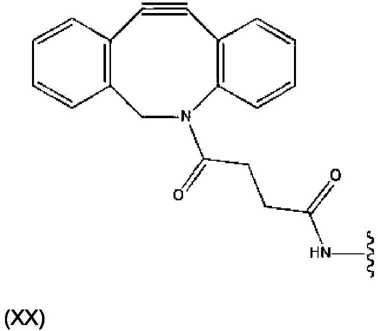 Hence, in some embodiments the saponin conjugate is a compound of formula (II)s, (III)_{S} or (IV)_{S} as described herein, wherein the saponin moiety linker L_{S} is the result of a coupling reaction between a compound of formula (V)_{S}, (VII)_{S} or (VIII)_{S} with a compound of formula (VI)_{S}, wherein the first precursor L_{S1} is an azide, for example an azide of formula (XVII) or formula (XVIII), and wherein the second precursor L_{S2} is a compound of formula (XIX) comprising an alkyn-containing moiety L_{S2a}, for example the alkyn of formula (XX) and a hydrazon resulting from a reaction of a hydrazide with an aldehyde of the saponin moiety.

### Saponin conjugate - saponin

An aspect of the invention relates to a saponin conjugate according to the pending claims comprising at least one saponin covalently linked to a ligand for asialoglycoprotein receptor (ASGPR), wherein the ligand for ASGPR comprises at least one N-acetylgalactosamine (GalNAc) moiety, preferably three or four GalNAc moieties, more preferably the ligand for ASGPR comprises or consists of (GalNAc)₃Tris, wherein the at least one saponin is selected from monodesmosidic triterpenoid saponins and bidesmosidic triterpenoid saponins.

An embodiment is the saponin conjugate of the invention, wherein the saponin comprises an aglycone core structure selected from the group consisting of:
2alpha-hydroxy oleanolic acid;
16alpha-hydroxy oleanolic acid;
hederagenin (23-hydroxy oleanolic acid);
16alpha,23-dihydroxy oleanolic acid;
gypsogenin;
quillaic acid;
protoaescigenin-21(2-methylbut-2-enoate)-22-acetate;
23-oxo-barringtogenol C-21,22-bis(2-methylbut-2-enoate);
23-oxo-barringtogenol C-21(2-methylbut-2-enoate)-16,22-diacetate;
digitogenin;
3,16,28-trihydroxy oleanan-12-en;
gypsogenic acid,
   and
derivatives thereof,
preferably the saponin comprises an aglycone core structure selected from quillaic acid and gypsogenin or derivatives thereof, more preferably the saponin aglycone core structure is quillaic acid or a derivative thereof. Such preferred aglycones comprise an aldehyde group at the C-23 atom or a derivative thereof as described herein elsewhere. Without wishing to be bound by any theory, such an aldehyde group contributes to the endosomal escape enhancing activity of saponins of the triterpene glycoside type, comprising an aglycone selected from Group C, especially quillajic acid and gypsogenin.
An embodiment is the saponin conjugate of the invention, wherein the at least one saponin is a monodesmosidic or bi-desmosidic triterpene saponin belonging to the type of a 12,13-dehydrooleanane with the aldehyde group in position C-23 and optionally comprising a glucuronic acid group in a carbohydrate substituent at the C-3beta-OH group of the saponin, preferably a bi-desmosidic triterpene saponin belonging to the type of a 12,13-dehydrooleanane with the aldehyde group in position C-23 and comprising a glucuronic acid group in a carbohydrate substituent at the C-3beta-OH group of the saponin.

An embodiment is the saponin conjugate of the invention, wherein the at least one saponin comprises a first saccharide chain, which is bound to the C₃ atom or the C₂₈ atom of the aglycone core structure of the at least one saponin, preferably to the C₃ atom, and/or wherein the at least one saponin comprises a second saccharide chain, which is bound to the C₂₈ atom of the aglycone core structure of the at least one saponin, preferably, the saponin comprises the first and second saccharide chain. Thus, when the saponin comprised by the saponin conjugate of the invention bears two glycans (saccharide chains), the first saccharide chain is bound at position C₃ of the aglycone core structure and the second saccharide chain is bound at position C₂₈ of the aglycone core structure.

The saponin conjugate of the invention comprises the saponin, which comprises a saccharide chain bound to the aglycone core structure, which is selected from group A:
GIcA-
Glc-,
Gal-,
Rha-(1→2)-Ara-,
Gal-(1→2)-[Xyl-(1→3)]-GlcA-,
Glc-(1→2)-[Glc-(1→4)]-GlcA-,
Glc-(1→2)-Ara-(1→3)-[Gal-(1→2)]-GlcA-,
Xyl-(1→2)-Ara-(1→3)-[Gal-(1→2)]-GlcA-,
Glc-(1→3)-Gal-(1→2)-[Xyl-(1→3)]-Glc-(1→4)-Gal-,
Rha-(1→2)-Gal-(1→3)-[Glc-(1→2)]-GlcA-,
Ara-(1→4)-Rha-(1→2)-Glc-(1→2)-Rha-(1→2)-GlcA-,
Ara-(1→4)-Fuc-(1→2)-Glc-(1→2)-Rha-(1→2)-GlcA-,
Ara-(1→4)-Rha-(1→2)-Gal-(1→2)-Rha-(1→2)-GlcA-,
Ara-(1→4)-Fuc-(1→2)-Gal-(1→2)-Rha-(1→2)-GlcA-,
Ara-(1→4)-Rha-(1→2)-Glc-(1→2)-Fuc-(1→2)-GlcA-,
Ara-(1→4)-Fuc-(1→2)-Glc-(1→2)-Fuc-(1→2)-GlcA-,
Ara-(1→4)-Rha-(1→2)-Gal-(1→2)-Fuc-(1→2)-GlcA-,
Ara-(1→4)-Fuc-(1→2)-Gal-(1→2)-Fuc-(1→2)-GlcA-,
Xyl-(1 →4)-Rha-(1→2)-Glc-(1→2)-Rha-(1→2)-GlcA-,
Xyl-(1 →4)-Fuc-(1→2)-Glc-(1→2)-Rha-(1→2)-GlcA-,
Xyl-(1→4)-Rha-(1→2)-Gal-(1→2)-Rha-(1→2)-GlcA-,
Xyl-(1→4)-Fuc-(1→2)-Gal-(1→2)-Rha-(1→2)-GlcA-,
Xyl-(1→4)-Rha-(1→2)-Glc-(1→2)-Fuc-(1→2)-GlcA-,
Xyl-(1→4)-Fuc-(1→2)-Glc-(1→2)-Fuc-(1→2)-GlcA-,
Xyl-(1→4)-Rha-(1→2)-Gal-(1→2)-Fuc-(1→2)-GlcA-,
Xyl-(1→4)-Fuc-(1→2)-Gal-(1→2)-Fuc-(1→2)-GlcA-, and
derivatives thereof.
An embodiment is the saponin conjugate of the invention, wherein
- the saponin comprises a saccharide chain bound to the aglycone core structure, which is selected from group B:
   Glc-,
   Gal-,
   Rha-(1→2)-[Xyl-(1→4)]-Rha-,
   Rha-(1→2)-[Ara-(1→3)-Xyl-(1→4)]-Rha-,
   Ara-,
   Xyl-,
   Xyl-(1→4)-Rha-(1→2)-[R1-(→4)]-Fuc- wherein R1 is 4E-Methoxycinnamic acid,
   Xyl-(1→4)-Rha-(1→2)-[R2-(→4)]-Fuc- wherein R2 is 4Z-Methoxycinnamic acid,
   Xyl-(1→4)-[Gal-(1→3)]-Rha-(1→2)-4-OAc-Fuc-,
   Xyl-(1→4)-[Glc-(1→3)]-Rha-(1→2)-3,4-di-OAc-Fuc-,
   Xyl-(1→4)-[Glc-(1→3)]-Rha-(1→2)-[R3-(→4)]-3-OAc-Fuc- wherein R3 is 4E-Methoxycinnamic acid,
   Glc-(1→3)-Xyl-(1→4)-[Glc-(1→3)]-Rha-(1→2)-4-OAc-Fuc-,
   Glc-(1→3)-Xyl-(1→4)-Rha-(1→2)-4-OAc-Fuc-,
   (Ara- or Xyl-)(1→3)-(Ara- or Xyl-)(1→4)-(Rha- or Fuc-)(1→2)-[4-OAc-(Rha- or Fuc-)(1→4)]-(Rha- or Fuc-),
   Xyl-(1→3)-Xyl-(1→4)-Rha-(1→2)-[Qui-(1→4)]-Fuc-,
   Api-(1→3)-Xyl-(1→4)-[Glc-(1→3)]-Rha-(1→2)-Fuc-,
   Xyl-(1→4)-[Gal-(1→3)]-Rha-(1→2)-Fuc-,
   Xyl-(1→4)-[Glc-(1→3)]-Rha-(1→2)-Fuc-,
   Ara/Xyl-(1→4)-Rha/Fuc-(1→4)-[Glc/Gal-(1→2)]-Fuc-,
   Api-(1→3)-Xyl-(1→4)-[Glc-(1→3)]-Rha-(1→2)-[R4-(→4)]-Fuc- wherein R4 is 5-O-[5-O-Ara/Api-3,5-dihydroxy-6-methyl-octanoyl]-3,5-dihydroxy-6-methyl-octanoic acid),
   Api-(1→3)-Xyl-(1→4)-Rha-(1→2)-[R5-(→4)]-Fuc- wherein R5 is 5-O-[5-O-Ara/Api-3,5-dihydroxy-6-methyl-octanoyl]-3,5-dihydroxy-6-methyl-octanoic acid),
   Api-(1→3)-Xyl-(1→4)-Rha-(1→2)-[Rha-(1→3)]-4-OAc-Fuc-,
   Api-(1→3)-Xyl-(1→4)-[Glc-(1→3)]-Rha-(1→2)-[Rha-(1→3)]-4-OAc-Fuc-,
   6-OAc-Glc-(1→3)-Xyl-(1→4)-Rha-(1→2)-[3-OAc-Rha-(1→3)]-Fuc-,
   Glc-(1→3)-Xyl-(1→4)-Rha-(1→2)-[3-OAc--Rha-(1→3)]-Fuc-,
   Xyl-(1→3)-Xyl-(1→4)-Rha-(1→2)-[Qui-(1→4)]-Fuc-,
   Glc-(1→3)-[Xyl-(1→4)]-Rha-(1→2)-[Qui-(1→4)]-Fuc-,
   Glc-(1→3)-Xyl-(1→4)-Rha-(1→2)-[Xyl-(1→3)-4-OAc-Qui-(1→4)]-Fuc-,
   Xyl-(1→3)-Xyl-(1→4)-Rha-(1→2)-[3,4-di-OAc-Qui-(1→4)]-Fuc-,
   Glc-(1→3)-[Xyl-(1→4)]-Rha-(1→2)-Fuc-,
   6-OAc-Glc-(1→3)-[Xyl-(1→4)]-Rha-(1→2)-Fuc-,
   Glc-(1→3)-[Xyl-(1→3)-Xyl-(1→4)]-Rha-(1→2)-Fuc-,
   Xyl-(1→3)-Xyl-(1→4)-Rha-(1→2)-[Xyl-(1→3)-4-OAc-Qui-(1→4)]-Fuc-,
   Api/Xyl-(1→3)-Xyl-(1→4)-[Glc-(1→3)]-Rha-(1→2)-[Rha-(1→3)]-4OAc-Fuc-,
   Api-(1→3)-Xyl-(1→4)-[Glc-(1→3)]-Rha-(1→2)-[Rha-(1→3)]-4OAc-Fuc-,
   Api/Xyl-(1→3)-Xyl-(1→4)-[Glc-(1→3)]-Rha-(1→2)-[R6-(→4)]-Fuc- wherein R6 is 5-O-[5-O-Rha-(1→2)-Ara/Api-3,5-dihydroxy-6-methyl-octanoyl]-3,5-dihydroxy-6-methyl-octanoic acid),
   Api/Xyl-(1→3)-Xyl-(1→4)-[Glc-(1→3)]-Rha-(1→2)-[R7-(→4)]-Fuc- wherein R7 is 5-O-[5-O-Ara/Api-3,5-dihydroxy-6-methyl-octanoyl]-3,5-dihydroxy-6-methyl-octanoic acid),
   Api/Xyl-(1→3)-Xyl-(1→4)-[Glc-(1→3)]-Rha-(1→2)-[R8-(→4)]-Fuc- wherein R8 is 5-O-[5-O-Ara/Api-3,5-dihydroxy-6-methyl-octanoyl]-3,5-dihydroxy-6-methyl-octanoic acid),
   Api-(1→3)-Xyl-(1→4)-Rha-(1→2)-[R9-(→4)]-Fuc- wherein R9 is 5-O-[5-O-Ara/Api-3,5-dihydroxy-6-methyl-octanoyl]-3,5-dihydroxy-6-methyl-octanoic acid),
   Xyl-(1→3)-Xyl-(1→4)-Rha-(1→2)-[R10-(→4)]-Fuc- wherein R10 is 5-O-[5-O-Ara/Api-3,5-dihydroxy-6-methyl-octanoyl]-3,5-dihydroxy-6-methyl-octanoic acid),
   Api-(1→3)-Xyl-(1→4)-Rha-(1→2)-[R11-(→3)]-Fuc- wherein R11 is 5-O-[5-O-Ara/Api-3,5-dihydroxy-6-methyl-octanoyl]-3,5-dihydroxy-6-methyl-octanoic acid),
   Xyl-(1→3)-Xyl-(1→4)-Rha-(1→2)-[R12-(→3)]-Fuc- wherein R12 is 5-O-[5-O-Ara/Api-3,5-dihydroxy-6-methyl-octanoyl]-3,5-dihydroxy-6-methyl-octanoic acid)
   Glc-(1→3)-[Glc-(1→6)]-Gal-, and
   derivatives thereof,
   or
- the saponin is a bidesmosidic triterpene glycoside comprising a first saccharide chain selected from the group A bound to the aglycone core structure and comprising a second saccharide chain selected from the group B bound to the aglycone core structure. Thus, when the saponin comprised by the saponin conjugate of the invention bears two glycans (saccharide chains), the first saccharide chain is bound at position C₃ of the aglycone core structure and the second saccharide chain is bound at position C₂₈ of the aglycone core structure.

Without wishing to be bound by any theory, presence of an aldehyde group or a derivative thereof in the aglycone core structure (here, also referred to as 'aglycone') is beneficial for the capacity of the saponin to stimulate and/or potentiate the endosomal escape of (effector) molecules when such a saponin co-localizes in a cell, in the endosome of said cell, with these (effector) molecules. Therefore, saponin conjugates of the invention comprising saponin which has an aglycone with an aldehyde group is preferred. In quillajic acid and in gypsogenin the aldehyde group is at the C₂₃ atom of the aglycone.

An embodiment is the saponin conjugate of the invention, wherein the saponin is selected from the group consisting of: *Quillaja* bark saponin, dipsacoside B, saikosaponin A, saikosaponin D, macranthoidin A, esculentoside A, phytolaccagenin, aescinate, AS6.2, NP-005236, AMA-1, AMR, alpha-Hederin, NP-012672, NP-017777, NP-017778, NP-017774, NP-018110, NP-017772, NP-018109, NP-017888, NP-017889, NP-018108, SA1641, AE X55, NP-017674, NP-017810, AG1, NP-003881, NP-017676, NP-017677, NP-017706, NP-017705, NP-017773, NP-017775, SA1657, AG2, SO1861, GE1741, SO1542, SO1584, SO1658, SO1674, SO1832, SO1862, SO1904, QS-7, QS1861, QS-7 api, QS1862, QS-17, QS-18, QS-21 A-apio, QS-21 A-xylo, QS-21 B-apio, QS-21 B-xylo, beta-Aescin, Aescin Ia, Teaseed saponin I, Teaseedsaponin J, Assamsaponin F, Digitonin, Primula acid 1 and AS64R, stereoisomers thereof, derivatives thereof, and combinations thereof, preferably the saponin is selected from the group consisting of QS-21, a QS-21 derivative, SO1861, a SO1861 derivative, SA1641, a SA1641 derivative, GE1741, a GE1741 derivative and combinations thereof, more preferably the saponin is selected from the group consisting of a QS-21 derivative, a SO1861 derivative and combinations thereof, most preferably the saponin is a SO1861 derivative.

Such saponins of the triterpene glycoside type are capable of enhancing the endosomal escape of (effector) molecules that are present in the endosome (or lysosome) of a cell, when the saponin co-localizes with such (effector) molecule inside the cell. The inventors established that the endosomal escape enhancing activity of these saponins is about 100 to 1000 times more potent when the saponin is contacted with a cell when the saponin is comprised by the conjugate of the invention. The free saponin is capable of stimulating the delivery of (effector) molecules in the cytosol of cells, when such cells are contacted with the (effector) molecules and the saponin, at 100-1000 times higher saponin concentration, compared to the concentration of the same saponin which is comprised by the saponin conjugate of the invention, required to achieve the same extent of delivery of the (effector) molecule from outside the cell to inside the endsome and finally in the cytosol of said cell. Saponins which display such endosomal escape enhancing activity are listed in Table A1, as well as saponins with high structural similarity with saponins for which the ability to potentiate the cytosolic delivery of (effector) molecules has been established. When the saponin is part of the saponin conjugate of the invention, the targeted delivery of the saponin upon binding of the ligand for ASGPR to the cell-surface binding site on the target cell, on said cell, and after endocytosis, into the endosome of said cell, is thus about 100 to 1000 times more effective compared to contacting the same cell with free, untargeted saponin which is not provided with a ligand for ASGPR for binding to ASGPR of a target cell.

In embodiments the saponin conjugate comprises at least one saponin, wherein the saponin is a derivative wherein
i. the aglycone core structure of the saponin comprises an aldehyde group which has been derivatised;
ii. a saccharide chain of the saponin, preferably the saccharide chain selected from group A comprises a carboxyl group which has been derivatised;
iii. a saccharide chain of the saponin, preferably the saccharide chain selected from group B comprises an acetoxy (Me(CO)O-) group which has been derivatised; or
iv. any combination of derivatisations (i), (ii) and/or (iii) is present.

Exemplary derivatisations (i) for an aldehyde group of the aglycone core structure include reduction to an alcohol, such as a primary alcohol; transformation into a hydrazone; transformation into a hemiacetal; transformation into an acetal; oxidation to a carboxyl; transformation into an imine; transformation into an oxime; transformation into a β-hydroxy ketone; or transformation into an enone, preferably reduction to an alcohol, such as a primary alcohol; or transformation into a hydrazone.

In embodiments the saponin is a derivative wherein the aglycone core structure comprises an aldehyde group which has been derivatised by:
- reduction to an alcohol, such as a primary alcohol; or
- transformation into a hydrazone of formula R^{b}HC=NNHR^{a}; preferably into a hydrazone of formula R^{b}HC=NNH(CO)R^{a}
wherein R^{a} is group comprising less than 20 carbon atoms, preferably less than 10 carbon atoms and R^{b} is the remainder of the saponin. Preferably, R^{a} is an N-alkylated maleimide. In particular embodiments, the aldehyde group has been transformation into a hydrazon bond through reaction with N-ε-maleimidocaproic acid hydrazide (EMCH); N-[ß-maleimidopropionic acid] hydrazide (BMPH); or N-[κ-maleimidoundecanoic acid] hydrazide (KMUH).

Exemplary derivatisations (ii) for a carboxyl group of a saccharide chain include: reduction to an alcohol, such as a primary alcohol; transformation into an amide; transformation into an ester; transformation into an amine, such as a primary or secondary amine; or decarboxylation, preferably reduction to an alcohol, such as a primary alcohol; transformation into an amide; or transformation into an ester; more preferably transformation into an amide.

Preferably, the carboxyl group which is derivatised is part of a glucuronic acid moiety and, preferably, the saccharide chain is selected from group A.

In embodiments the saponin is a derivative wherein a saccharide chain, preferably a saccharide chain selected from group A, comprises a carboxyl group, preferably a carboxyl group of a glucuronic acid moiety, which has been derivatised by:
- reduction to an alcohol, such as a primary alcohol;
- transformation into an amide of formula R^{a}NH(CO)R^{b}; or
- transformation into an ester of formula R^{a}O(CO)R^{b}
wherein R^{a} is group comprising less than 20 carbon atoms, preferably less than 10 carbon atoms and R^{b} is the remainder of the saponin. Preferably, R^{a} is an N-alkylated maleimide or a diol. In particular embodiments, the carboxyl group has been derivatised by transformation into an amide bond through reaction with 2-amino-2-methyl-1,3-propanediol (AMPD) or N-(2-aminoethyl)maleimide (AEM).

Exemplary derivatisations (iii) for an acetoxy group of a saccharide chain include: transformation into an alcohol, such as a secondary alcohol, by deacetylation, optionally followed by transformation of the resulting alcohol into an ether; ester or ketone; preferably transformation into an alcohol, such as a secondary alcohol, by deacetylation.

Preferably, the acetoxy group which is derivatised is part of a saccharide chain selected from group B.

In embodiments the saponin is a derivative wherein a saccharide chain, preferably a saccharide chain selected from group B, comprises an acetoxy group which has been derivatised by:
- transformation into an alcohol, such as a secondary alcohol, by deacetylation; or
- transformation into an alcohol, such as a secondary alcohol, by deacetylation followed by transformation of the resulting alcohol into an ether of formula R^{a}OR^{b}, an ester of formula R^{a}(CO)OR^{b}, or a ketone of formula R^{a}(CO)R^{b}
wherein R^{a} is group comprising less than 20 carbon atoms, preferably less than 10 carbon atoms and R^{b} is the remainder of the saponin. Preferably, R^{a} is an N-alkylated maleimide or a diol.

Hence, in embodiments the saponin is a derivative wherein:
- the aglycone core structure comprises an aldehyde group which has been derivatised by:
   - reduction to an alcohol;
   - transformation into a hydrazone bond through reaction with N-ε-maleimidocaproic acid hydrazide (EMCH);
   - transformation into a hydrazone bond through reaction with N-[ß-maleimidopropionic acid] hydrazide (BMPH); or
   - transformation into a hydrazone bond through reaction with N-[κ-maleimidoundecanoic acid] hydrazide (KMUH);
- the saccharide chain selected from group A comprises a carboxyl group, preferably a carboxyl group of a glucuronic acid moiety, which has been derivatised by transformation into an amide bond through reaction with 2-amino-2-methyl-1,3-propanediol (AMPD) or N-(2-aminoethyl)maleimide (AEM);
- the saccharide chain selected from group B comprises an acetoxy group (Me(CO)O-) which has been derivatised by transformation into a hydroxyl group (HO-); or
- any combination of derivatisations (i), (ii) and/or (iii) is present.

An embodiment is the saponin conjugate of the invention, wherein the saponin is a saponin derivative wherein
i. the saponin derivative comprises an aglycone core structure comprising an aldehyde group which has been derivatised;
ii. the saponin derivative comprises a saccharide chain, preferably a saccharide chain selected from group A, the saccharide chain comprising a carboxyl group which has been derivatised;
iii. the saponin derivative comprises a saccharide chain, preferably a saccharide chain selected from group B, the saccharide chain comprising an acetoxy (Me(CO)O-) group which has been derivatised; or
iv. the saponin derivative comprises any combination of derivatisations i., ii. and iii., preferably any combination of two derivatisations of derivatisations i., ii. and iii.

An embodiment is the saponin conjugate of the invention, wherein the saponin is any one or more of: SO1861, SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, Quillajasaponin, Saponinum album, QS-18, Quil-A, Gyp1, gypsoside A, AG1, AG2, SO1542, SO1584, SO1658, SO1674, SO1832, SO1904, stereoisomers thereof, derivatives thereof and combinations thereof, preferably the saponin is selected from the group consisting of QS-21, a QS-21 derivative, SO1861, a SO1861 derivative, SA1641, a SA1641 derivative, GE1741, a GE1741 derivative and combinations thereof, more preferably the saponin is selected from the group consisting of a QS-21 derivative, a SO1861 derivative and combinations thereof, most preferably the saponin is a SO1861 derivative.

An embodiment is the saponin conjugate of the invention, wherein the saponin is a saponin derivative of the quillaic acid saponin or the gypsogenin saponin according to the invention and is represented by Molecule 1: 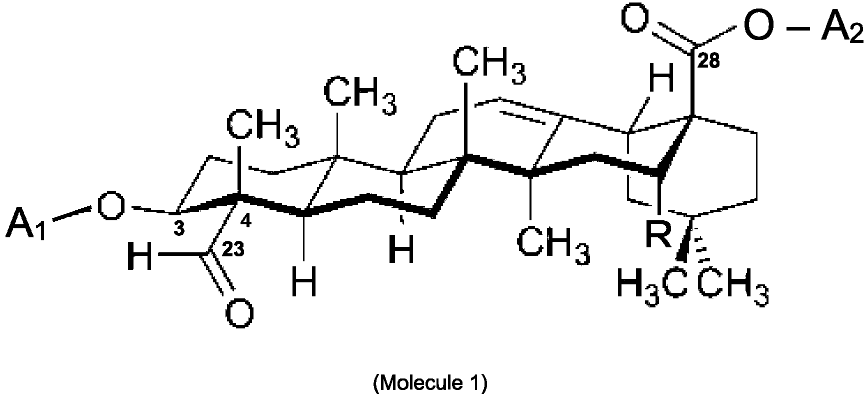 wherein
A₁ represents a saccharide chain selected from group A as defined in claim 1 and A₁ comprises or consists of a glucuronic acid moiety and the carboxyl group of said glucuronic acid moiety of A₁ has been derivatised;
A₂ represents hydrogen, a monosaccharide or a linear or branched oligosaccharide, preferably A₂ represents a saccharide chain selected from group B, more preferably A₂ represents a saccharide chain selected from group B and A₂ comprises at least one acetoxy (Me(CO)O-) group, such as one, two, three or four acetoxy groups,

wherein preferably both A₁ and A₂ are an oligosaccharide chain;
   and R is hydrogen in gypsogenin or hydroxyl in quillaic acid;
wherein the saponin derivative corresponds to the saponin represented by Molecule 1 wherein at least one, preferably one or two, more preferably one, of the following derivatisations is present:
   i. the aldehyde group at position C₂₃ of the quillaic acid or gypsogenin has been derivatised;
   ii. the carboxyl group of a glucuronic acid moiety of A₁ has been derivatised; and
   iii. one or more, preferably all, of acetoxy group(s) of one saccharide moiety or of two or more saccharide moieties of A₂, when A₂ represents a saccharide chain selected from group B and A₂ comprises at least one acetoxy group, has/have been derivatised.

An embodiment is the saponin conjugate of the invention, wherein A₁ represents a saccharide chain selected from group A and comprises or consists of a glucuronic acid moiety and wherein the carboxyl group of a glucuronic acid moiety of A₁ has been derivatised and/or wherein A₂ represents a saccharide chain selected from group B and A₂ comprises at least one acetoxy group and wherein at least one acetoxy group of A₂ has been derivatised.

An embodiment is the saponin conjugate of the invention, wherein the saponin represented by Molecule 1 is a bidesmosidic triterpene saponin.

An embodiment is the saponin conjugate of the invention, wherein the saponin derivative corresponds to the saponin represented by Molecule 1 wherein at least one, preferably one or two, more preferably one, of the following derivatisations is present:
i. the aldehyde group at position C₂₃ of the quillaic acid or gypsogenin has been derivatised by;
   - reduction to an alcohol;
   - transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH, wherein the maleimide group of the EMCH is optionally derivatised by formation of a thioether bond with mercaptoethanol;
   - transformation into a hydrazone bond through reaction with N-[ß-maleimidopropionic acid] hydrazide (BMPH) wherein the maleimide group of the BMPH is optionally derivatised by formation of a thioether bond with mercaptoethanol; or
   - transformation into a hydrazone bond through reaction with N-[κ-maleimidoundecanoic acid] hydrazide (KMUH) wherein the maleimide group of the KMUH is optionally derivatised by formation of a thioether bond with mercaptoethanol;
ii. the carboxyl group of a glucuronic acid moiety of A₁, when A₁ represents a saccharide chain selected from group A and A₁ comprises or consists of a glucuronic acid moiety, has been derivatised by transformation into an amide bond through reaction with 2-amino-2-methyl-1,3-propanediol (AMPD) or N-(2-aminoethyl)maleimide (AEM), therewith providing a saponin-Glu-AMPD such as a QS-21-Glu-AMPD or a SO1861-Glu-AMPD or a saponin-Glu-AEM such as a QS-21-Glu-AEM or a SO1861-Glu-AEM; and
iii. one or more, preferably all, of acetoxy group(s) of one saccharide moiety or of two or more saccharide moieties of A₂, when A₂ represents a saccharide chain selected from group B and A₂ comprises at least one acetoxy group, has/have been derivatised by transformation into a hydroxyl group (HO-) by deacetylation.

An embodiment is the saponin conjugate of the invention, wherein A₁ is Gal-(1→2)-[Xyl-(1→3)]-GlcA and/or A₂ is Glc-(1→3)-Xyl-(1→4)-Rha-(1→2)-[Xyl-(1→3)-4-OAc-Qui-(1→4)]-Fuc, preferably the saponin represented by Molecule 1 is 3-O-beta-D-galactopyranosyl-(1→2)-[beta-D-xylopyranosyl-(1→3)]-beta-D-glucuronopyranosyl quillaic acid 28-O-beta-D-glucopyranosyl-(1→3)-beta-D-xylopyranosyl-(1→4)- alpha-L-rhamnopyranosyl-(1→2)-[beta-D-xylopyranosyl-(1→3)-4OAc-beta-D-quinovopyranosyl-(1→4)]-beta-D-fucopyranoside, more preferably the saponin is any one or more of: SO1861, GE1741, SA1641 and QS-21, or a derivative thereof, most preferably SO1861 or a derivative thereof.

An embodiment is the saponin conjugate of the invention, wherein the saponin is a saponin derivative wherein
i. the saponin derivative comprises an aglycone core structure comprising an aldehyde group which has been derivatised by:
   - reduction to an alcohol;
   - transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH, wherein the maleimide group of the EMCH is optionally derivatised by formation of a thioether bond with mercaptoethanol;
   - transformation into a hydrazon bond through reaction with N-[ß-maleimidopropionic acid] hydrazide (BMPH) wherein the maleimide group of the BMPH is optionally derivatised by formation of a thioether bond with mercaptoethanol; or
   - transformation into a hydrazon bond through reaction with N-[κ-maleimidoundecanoic acid] hydrazide (KMUH) wherein the maleimide group of the KMUH is optionally derivatised by formation of a thioether bond with mercaptoethanol;
ii. the saponin derivative comprises a saccharide chain, preferably a saccharide chain selected from group A, the saccharide chain comprising a carboxyl group, preferably a carboxyl group of a glucuronic acid moiety which has been derivatised by transformation into an amide bond through reaction with 2-amino-2-methyl-1,3-propanediol (AMPD) or N-(2-aminoethyl)maleimide (AEM), therewith providing a saponin-Glu-AMPD such as a QS-21-Glu- AMPD or a SO1861-Glu-AMPD or a saponin-Glu-AEM such as a QS-21-Glu-AEM or a SO1861-Glu-AEM;
iii. the saponin derivative comprises a saccharide chain, preferably a saccharide chain selected from group B, the saccharide chain comprising an acetoxy (Me(CO)O-) group which has been derivatised by transformation into a hydroxyl group (HO-) by deacetylation; or
iv. the saponin derivative comprises any combination of derivatisations i., ii. and iii., preferably any combination of two derivatisations of derivatisations i., ii. and iii.;
preferably, the saponin derivative comprises an aglycone core structure wherein the aglycone core structure comprises an aldehyde group which has been derivatised by transformation into a hydrazone bond through reaction with EMCH wherein the maleimide group of the EMCH is optionally derivatised by formation of a thioether bond with mercaptoethanol.

An embodiment is the saponin conjugate of the invention, wherein the saponin is a saponin derivative wherein
i. the saponin derivative comprises an aglycone core structure comprising an aldehyde group which has been derivatised by transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH;
ii. the saponin derivative comprises a saccharide chain, preferably a saccharide chain selected from group A, the saccharide chain comprising a carboxyl group, preferably a carboxyl group of a glucuronic acid moiety which has been derivatised by transformation into an amide bond through reaction with N-(2-aminoethyl)maleimide (AEM), therewith providing a saponin-Glu-AEM such as a QS-21-Glu-AEM or a SO1861-Glu-AEM; or
iii. the saponin derivative comprises a combination of derivatisations i. and ii.

An embodiment is the saponin conjugate of the invention, wherein the saponin derivative comprises an aglycone core structure wherein the aglycone core structure comprises an aldehyde group and wherein the saponin derivative comprises a saccharide chain, preferably a saccharide chain selected from group A, the saccharide chain comprising a carboxyl group, preferably a carboxyl group of a glucuronic acid moiety, which glucuronic acid moiety has been derivatised by transformation into an amide bond through reaction with N-(2-aminoethyl)maleimide (AEM).

An embodiment is the saponin conjugate of the invention, wherein the saponin derivative is represented by Molecule 2: 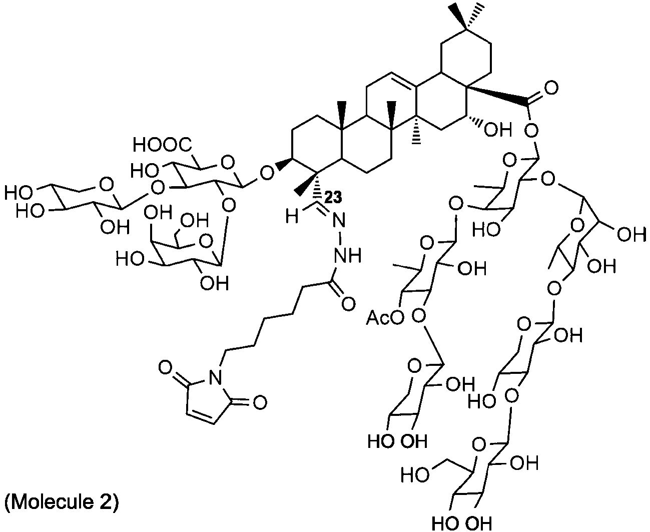 or wherein the saponin derivative is represented by Molecule 3:

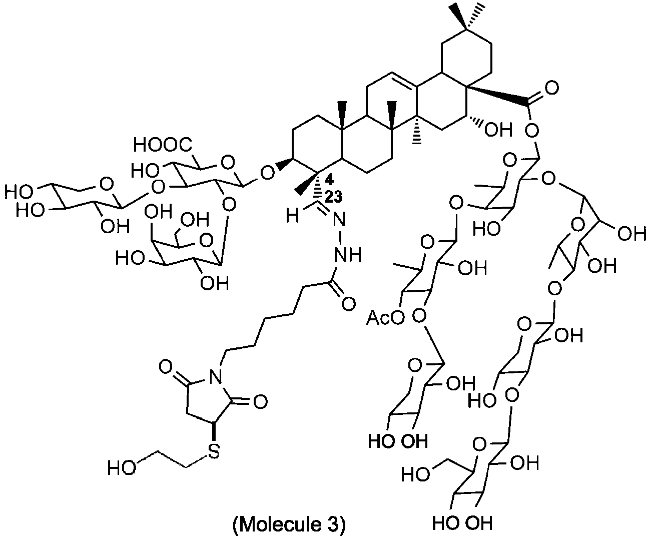

An embodiment is the saponin conjugate of the invention, wherein the at least one saponin and the ligand for ASGPR are covalently linked directly or via at least one linker.

An embodiment is the saponin conjugate of the invention, wherein the GalNAc moiety is bound to the saponin S, preferably via a saponin linker Lₛ, as represented in formula (II)_{S}:

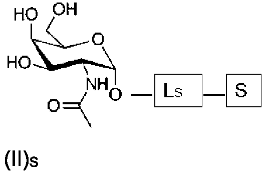

An embodiment is the saponin conjugate of the invention, wherein the GalNAc moieties are each separately covalently bound via the oxygen on position "1" of the GalNAc moiety to a central bridging moiety B, which effectively forms a bridge between the GalNAc moieties and the saponin moiety, preferably via a saponin moiety linker L_{S}, as shown in formula (III)_{S}: 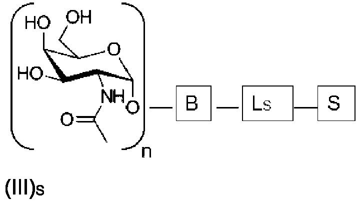 wherein n is an integer larger than or equal to 2, preferably n is 3, L_{S} is a saponin moiety linker and S is the saponin moiety.

An embodiment is the saponin conjugate of the invention, wherein the GalNAc moieties are bound to the bridging moiety B via GalNAc linkers L_{GAL} as shown in formula (IV)_{S}: 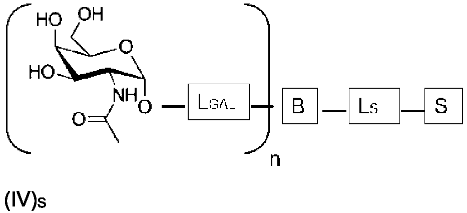 wherein n is an integer larger than or equal to 2, preferably n is 3, L_{S} is a saponin moiety linker and S is the saponin moiety.

An embodiment is the saponin conjugate of the invention, wherein the saponin moiety linker Ls represents any chemical moiety suitable for covalently binding a saponin to GalNAc as in formula (II)_{S} or to the bridging moiety B as in formula (III)_{S} and (IV)_{S}.

An embodiment is the saponin conjugate of the invention, wherein the saponin moiety linker Ls is the result of a coupling reaction between at least a first precursor L_{S1} covalently bound to GalNAc or the bridging moiety B and a second precursor L_{S2} covalently bound to the saponin moiety, wherein L_{S1} is a precursor of the saponin moiety linker Ls which is covalently bound to GalNAc or to the bridging moiety B and L_{S2} is a precursor of the saponin moiety linker Ls which is covalently bound to the saponin moiety.

An embodiment is the saponin conjugate of the invention, wherein the saponin moiety linker Ls is the result of a coupling reaction between at least a first precursor L_{S1} covalently bound to GalNAc or the bridging moiety B and a second precursor L_{S2} covalently bound to the saponin moiety, wherein the coupling reaction is selected from the group consisting of an azide-alkyne cycloaddition, a thiol maleimide coupling, a Staudinger reaction, a nucleophilic ring-opening of strained heterocyclic electrophiles, a carbonyl reaction of the non-aldol type and an addition to a carbon-carbon double bond, preferably wherein the coupling reaction is an azide-alkyne cycloaddition, a thiol maleimide coupling, a Staudinger reaction, a nucleophilic ring-opening of strained heterocyclic electrophiles, more preferably wherein the coupling reaction is an azide-alkyne cycloaddition or a thiol maleimide coupling.

An embodiment is the saponin conjugate of the invention, wherein the saponin moiety linker Ls is the result of a coupling reaction between a first precursor L_{S1} covalently bound to GalNAc or the bridging moiety B, the first precursor L_{S1} comprising an azide; and a second precursor L_{S2} covalently bound to the saponin moiety, the second precursor L_{S2} comprising an alkyne and preferably comprising a hydrazon resulting from a hydrazide/aldehyde coupling to an aldehyde of the saponin moiety.

An embodiment is the saponin conjugate of the invention, wherein the structure for the precursor L_{S1} is the following azide of formula (XVII): 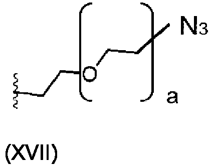
wherein a represents an integer larger than or equal to 0, preferably a represents an integer selected from 1, 2, and 3, more preferably a represents 2, or
wherein the structure for the precursor L_{S1} comprises the following azide of formula (XVIII): 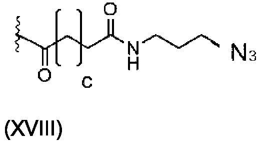
wherein c represents an integer larger than or equal to 0, preferably c represents an integer in the range of 5-15, more preferably c represents 9.

An embodiment is the saponin conjugate of the invention, wherein the structure for the precursor L_{S2} comprises the following hydrazone with formula (XIX): 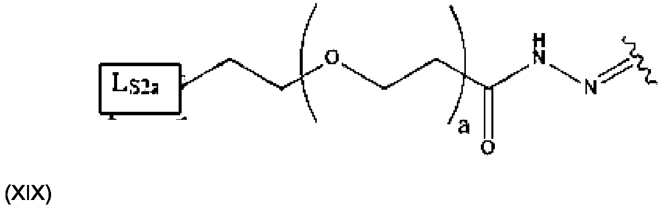 wherein a represents an integer larger than or equal to 0, preferably a represents an integer in the range of 2-6, more preferably a represents 4, and wherein L_{S2a} represents an alkyne-containing moiety.

An embodiment is the saponin conjugate of the invention, wherein L_{S2a} comprises less than 20 carbon atoms, preferably L_{S2a} represents a moiety according to formula (XX):

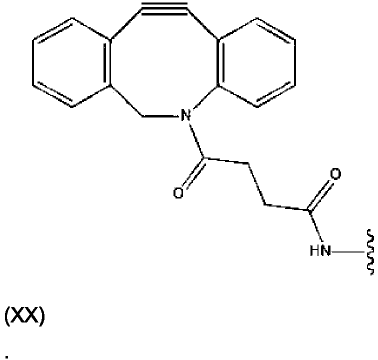

An embodiment is the saponin conjugate of the invention, wherein the bridging moiety is a compound of formula (XV): 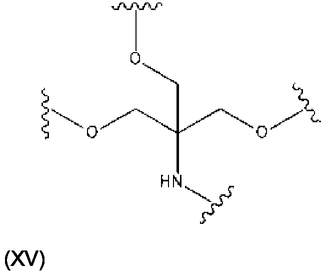 wherein the oxygen atoms of the compound of formula (XV) are bound to GalNAc or to the GalNAc linkers L_{GAL}, and the nitrogen atom of the compound of formula (XV) is bound to the saponin moiety linker L_{S}.

An embodiment is the saponin conjugate of the invention, wherein L_{GAL} represents any chemical moiety suitable for covalently binding GalNAc to the bridging moiety B.

An embodiment is the saponin conjugate of the invention, wherein L_{GAL} comprises 2-25 carbon atoms, preferably 7-15 carbon atoms, more preferably 11 carbon atoms and wherein L_{GAL} comprises at least one, preferably two amide moieties.

An embodiment is the saponin conjugate of the invention, wherein L_{GAL} is a compound according to formula (XVI):

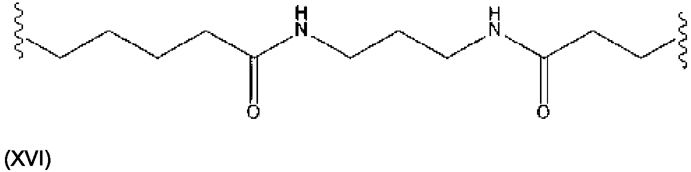

An embodiment is the saponin conjugate of the invention, wherein the ligand for ASGPR is (GalNAc)₃Tris represented by Molecule I': 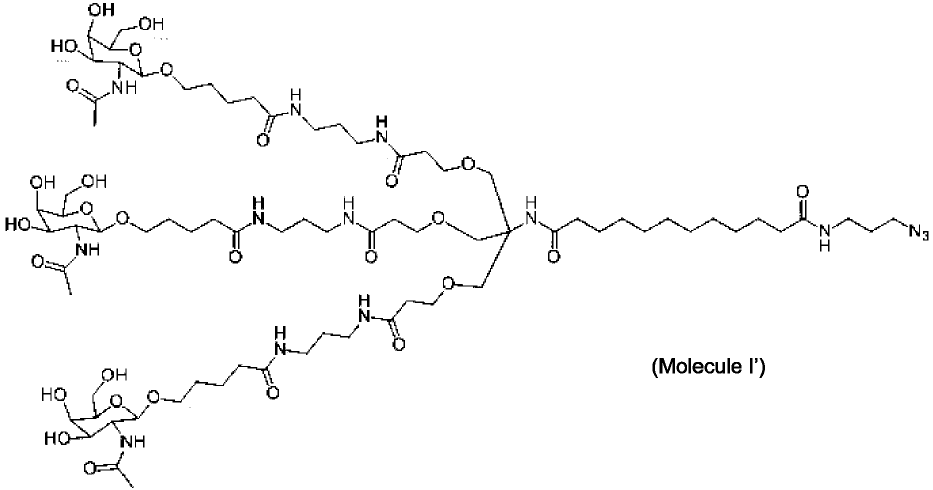 or wherein the ligand for ASGPR is mono-GalNAc represented by Molecule II':

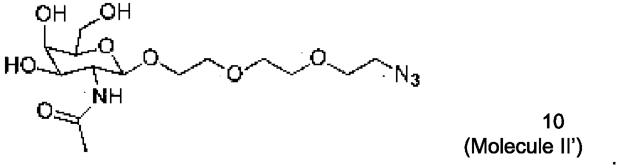

An embodiment is the saponin conjugate of the invention, wherein the at least one saponin is covalently bound to Molecule I' or Molecule II' of the invention via a linker represented by Molecule III': 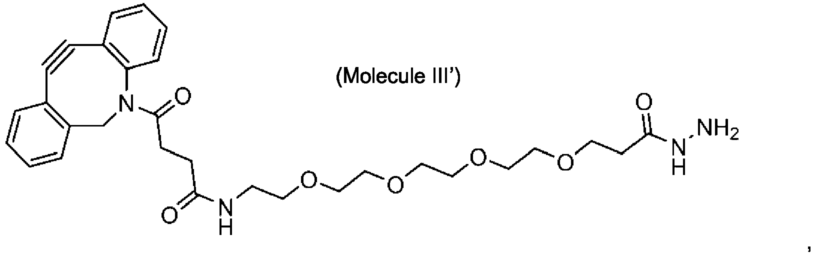 wherein the hydrazide moiety of Molecule III' formed a covalent hydrazone bond with an aldehyde group in the saponin, and wherein the dibenzocyclooctyne group formed a covalent bond with the azide group of Molecule I' or Molecule II'.

An embodiment is the saponin conjugate of the invention, wherein the at least one saponin is covalently bound to the ligand for ASGPR via at least one cleavable linker.

An embodiment is the saponin conjugate of the invention, wherein the cleavable linker is subject to cleavage under acidic conditions, reductive conditions, enzymatic conditions and/or light-induced conditions, and preferably the cleavable linker comprises a cleavable bond selected from a hydrazone bond and a hydrazide bond subject to cleavage under acidic conditions, and/or a bond susceptible to proteolysis, for example proteolysis by Cathepsin B, and/or a bond susceptible for cleavage under reductive conditions such as a disulfide bond.

An embodiment is the saponin conjugate of the invention, wherein the cleavable linker is subject to cleavage *in vivo* under acidic conditions such as for example present in endosomes and/or lysosomes of mammalian cells, preferably human cells, preferably the cleavable linker is subject to cleavage *in vivo* at pH 4.0 - 6.5, and more preferably at pH ≤ 5.5.

An embodiment is the saponin conjugate of the invention, wherein the conjugate comprises 1, 2, 3, 4, 5, 6, 8, 10, 16, 32, 64, 128 or 1-100 saponin moieties, or any number of saponin moieties therein between, such as 7, 9, 12 saponin moieties.

The skilled person will understand that if the saponin comprised in the saponin conjugate of the present invention is a compound of formula (II)s, (III)_{S} or (IV)_{S} as described herein wherein the saponin moiety linker L_{S} is the result of a coupling reaction between a first precursor L_{S1} covalently bound to GalNAc or the bridging moiety and a second precursor L_{S2} covalently bound to the saponin moiety, the second precursor L_{S2} comprising a hydrazone resulting from a hydrazide/aldehyde coupling to an aldehyde of the saponin moiety, as described herein, this means that the aldehyde group is already occupied by the linker, such that suitable saponin derivatives are these wherein saponin is derivatised by (ii) a carboxyl group of a saccharide chain as described herein and/or (iii) an acetoxy group of a saccharide chain as described herein.

### Effector molecule conjugate - introduction

Certain pharmaceutical combinations and certain pharmaceutical compositions of the present invention comprise a second conjugate of an effector molecule and a ligand for asialoglycoprotein receptor (ASGPR), referred to herein as "effector molecule conjugate". When the effector molecule is bound to the remainder of the effector molecule conjugate it is referred to herein as an "effector moiety". The ASGPR ligand comprises at least one N-acetylgalactosamine (GalNAc) moiety. In accordance with preferred embodiments of the present invention each GalNAc moiety is bound to the remainder of the ASGPR ligand or - in case ASGPR ligand consists of a single GalNAc moiety - to the effector moiety, via a covalent bond to the oxygen on position "1" as indicated in formula (I):

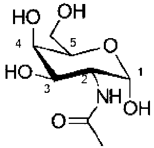

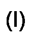

As shown in formula (II)_{E}, the ASGPR ligand consists of a single GalNAc moiety bound to the effector moiety E, preferably via an effector moiety linker L_{E}.

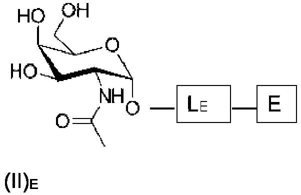

The ASGPR ligand may comprise more than one GalNAc moiety, such as 2, 3, 4, 5 or 6 GalNAc moieties, preferably 3 or 4 GalNAc moieties. In such case, it is preferred that the GalNAc moieties are each separately covalently bound via the oxygen on position "1" to a central bridging moiety B, which effectively forms a bridge between the GalNAc moieties and the effector moiety, preferably via an effector moiety linker L_{E}. The GalNAc moieties may be directly bound to the bridging moiety B as shown in formula (III)_{E}: 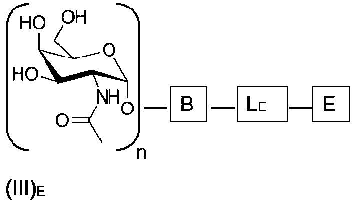 wherein n is an integer larger than or equal to 2, L_{E} is an effector moiety linker and E is the effector moiety. More preferably, the GalNAc moieties are bound to the bridging moiety B via GalNAc linkers L_{GAL} as shown in formula (IV)_{E}: 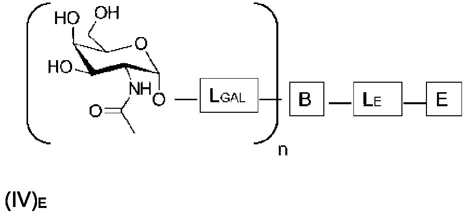 wherein n is an integer larger than or equal to 2, L_{E} is an effector moiety linker and E is the effector moiety. While each occurrence of L_{GAL} may be independently chosen, the skilled person will appreciate that the synthesis of the conjugate is simplified in case each occurrence of L_{GAL} represents the same moiety.

### Effector molecule conjugate - Linker L_{E}

The effector moiety linker L_{E} represents any chemical moiety suitable for covalently binding an effector moiety to GalNAc as in formula (II)_{E} or to the bridging moiety B as in formula (III)_{E} and (IV)_{E}. The identity and size of the linker is not particularly limited and covalently binding an effector molecule to GalNAc as in formula (II)_{E} or to the bridging moiety B as in formula (III)_{E} and (IV)_{E} may be effected by means of a 'regular' chemical functional group (e.g. an ether bond) as well as through "click-chemistry" type linkers which typically have a long chain length, resulting in a linker E_{L} comprising e.g. more than 10 or more than 20 carbon atoms. Suitable effector moiety linkers L_{E} and associated coupling reactions are described in the handbook Hermanson, Greg T. Bioconjugate techniques. Academic press, 2013.

As will be understood by the skilled person, the effector moiety linker L_{E} will typically be the result of a coupling reaction (e.g. "click-chemistry" type) between at least a first precursor L_{E1} covalently bound to GalNAc or the bridging moiety B and a second precursor L_{E2} covalently bound to the effector moiety. This principle is illustrated in the following reaction scheme for the compound of formula (II): 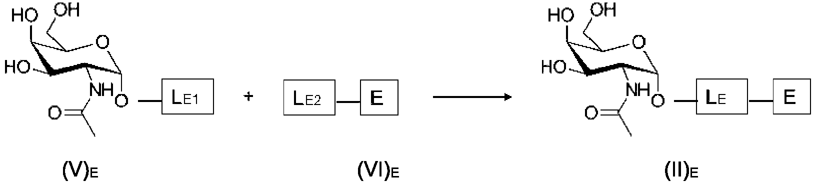 wherein L_{E} is an effector moiety linker, L_{E1} is a precursor of the effector moiety linker L_{E} which is covalently bound to GalNAc and L_{E2} is a precursor of the effector moiety linker L_{E} which is covalently bound to the effector moiety and E is the effector moiety.

The corresponding reaction scheme for the compound of formula (III) is as follows: 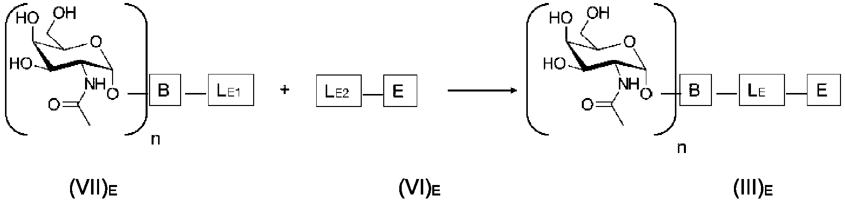 wherein L_{E} is an effector moiety linker, L_{E1} is a precursor of the effector moiety linker L_{E} which is covalently bound to GalNAc and L_{E2} is a precursor of the effector moiety linker L_{E} which is covalently bound to the effector moiety, n is an integer larger than or equal to 2 and E is the effector moiety.

The corresponding reaction scheme for the compound of formula (IV)_{E} is as follows: 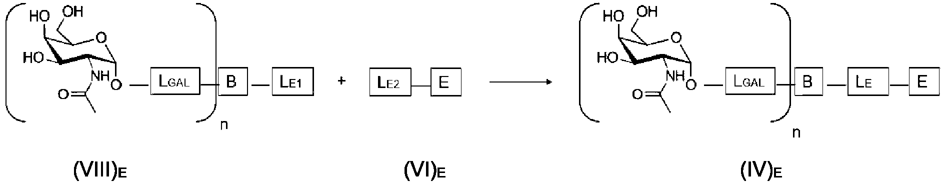 wherein L_{E} is an effector moiety linker, L_{E1} is a precursor of the effector moiety linker L_{E} which is covalently bound to GalNAc and L_{E2} is a precursor of the effector moiety linker L_{E} which is covalently bound to the effector moiety, n is an integer larger than or equal to 2, E is the effector moiety, and L_{GAL} is a GalNAc linker and B is a bridging moiety.

The effector moiety linker L_{E} can be the result of a coupling reaction between at least a first precursor L_{E1} covalently bound to GalNAc or the bridging moiety and a second precursor L_{E2} covalently bound to the effector moiety, wherein the coupling reaction is for example an azide-alkyne cycloaddition, a thiol maleimide coupling, a staudinger reaction, a nucleophilic ring-opening of strained heterocyclic electrophiles (such as aziridines, epoxides, cyclic sulfates, aziridinium ions, episulfonium ions), a carbonyl reaction of the non-aldol type (such as urea, thiourea, hydrazon, oxime ether, amide or aromatic heterocycle formation) or an addition to a carbon-carbon double bond (such as epoxidation, aziridination, dihydroxylation, sulfentyl halide addition, nitrosyl halide addition or micheal addition), preferably wherein the coupling reaction is an azide-alkyne cycloaddition, a thiol maleimide coupling, a staudinger reaction, a nucleophilic ring-opening of strained heterocyclic electrophiles, more preferably wherein the coupling reaction is an azide-alkyne cycloaddition or a thiol maleimide coupling.

In accordance with some embodiments of the invention, the effector moiety linker L_{E} comprises a succinimide thioether moiety. Such a succinimide thioether is e.g. the result of a thiol maleimide coupling between an N-substituted maleimide and a thiol or sulfhydryl containing compound. This thiol maleimide coupling is a commonly known 'click'-chemistry tool in the field of bioconjugation and is for example described in Hermanson, Greg T. Bioconjugate techniques. Academic press, 2013 page 289. Consequently, it is preferred that the effector moiety linker L_{E} is the result of a coupling reaction between a first precursor L_{E1} covalently bound to GalNAc or the bridging moiety, the first precursor L_{E1} comprising an N-substituted maleimide; and a second precursor L_{E2} covalently bound to the effector moiety, the second precursor L_{E2} comprising a thiol or a precursor thereof. A suitable thiol precursor is a disulfide, which may be cleaved (e.g. in-situ) via reduction to the corresponding thiol.

A preferred structure for the precursor L_{E1} present in the compounds of formula (V)_{E}, (VII)_{E} or (VIII)_{E} is the following terminal N-substituted maleimide: 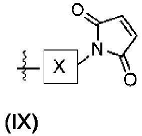 wherein X represents any linker suitable for covalently bonding the terminal N-substituted maleimide to GalNAc or the bridging moiety B. As will be understood by the skilled person, X may be the result of a coupling reaction between a first moiety covalently bound to GalNAc or the bridging moiety and a second moiety covalently bound to the maleimide. X can comprise a hydrazone and/or a 1, 2, 3-triazole. Whenever reference is made to a 1, 2, 3-triazole in the context of the linkers of the present application, this preferably means a 1*H*-1, 2, 3-triazole.

Embodiments of the structure for the precursor L_{E1} present in the compounds of formula (V)_{E}, (VII)_{E} or (VIII)_{E} are the following terminal N-substituted maleimides: 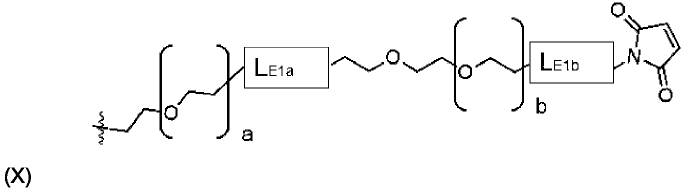
wherein a and b each independently represent an integer larger than or equal to 0, preferably a and b each independently represent an integer selected from 0, 1, 2, and 3, more preferably a and b represent 2, and wherein L_{E1a} represents a hydrazone and/or a 1, 2, 3-triazole, preferably a 1, 2, 3-triazole and wherein L_{E1b} represents a hydrazone and/or a 1, 2, 3-triazole, preferably a hydrazone; 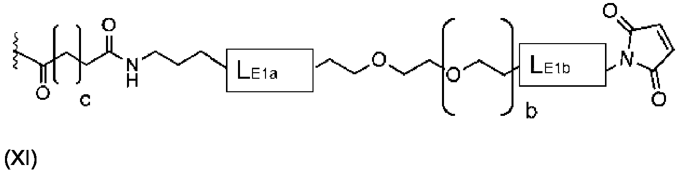
wherein b and c each independently represent an integer larger than or equal to 0, preferably b represents an integer selected from 0, 1, 2, and 3 and c represents an integer in the range of 5-15, more preferably b represents 2, and c represents 9, wherein L_{E1a} represents a hydrazone and/or a 1, 2, 3-triazole, preferably a 1, 2, 3-triazole and wherein L_{E1b} represents a hydrazone and/or a 1, 2, 3-triazole, preferably a hydrazone;
   and 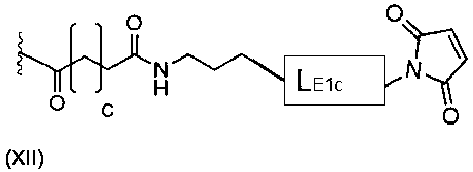
wherein c represents an integer larger than or equal to 0, preferably c represents an integer in the range of 5-15, more preferably c represents 9, wherein L_{E1c} represents a hydrazone and/or a 1, 2, 3-triazole, preferably a 1, 2, 3-triazole.

L_{E1a}, L_{E1b} and L_{E1c} each preferably comprise less than 20 carbon atoms, more preferably L_{E1a}, L_{E1b} and L_{E1c} each independently represent a moiety according to formula (XIII), (XIV) or (XV), preferably L_{E1a} is the 1,2,3-triazole of formula (XIII), L_{E1b} is the hydrazone of formula (XIV) and L_{E1c} is the 1,2,3-triazole of formula (XV):

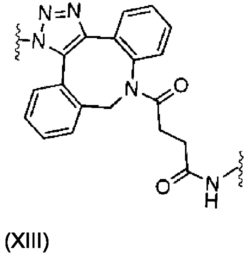

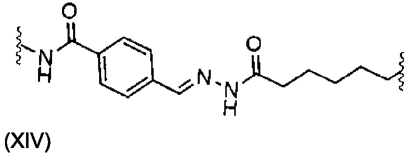

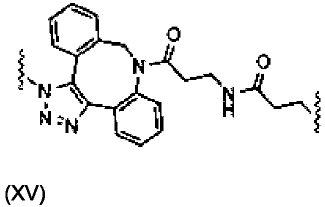

Hence, in some embodiments the effector molecule conjugate is a compound of formula (II)_{E}, (III)_{E} or (IV)_{E} as described herein, wherein the effector moiety linker L_{E} is the result of a coupling reaction between a compound of formula (V)_{E}, (VII)_{E} or (VIII)_{E} with a compound of formula (VI)_{E}, wherein the first precursor L_{E1} is a terminal N-substituted maleimide of formula (X), (XI) or (XII), wherein L_{E1a} is for example the 1,2,3-triazole of formula (XIII), L_{E1b} is for example the hydrazone of formula (XIV) and L_{E1c} is for example the 1,2,3-triazole of formula (XV).

### Effector molecule conjugate - Effector moiety

An aspect of the invention relates to a pharmaceutical combination comprising:
- a first pharmaceutical composition comprising the saponin conjugate of the invention and optionally comprising a pharmaceutically acceptable excipient and/or a pharmaceutically acceptable diluent; and
- a second pharmaceutical composition comprising a second conjugate of an effector molecule and a ligand for ASGPR, wherein the ligand for ASGPR preferably comprises at least one GalNAc moiety, preferably three or four GalNAc moieties, more preferably the ligand for ASGPR comprises or consists of (GalNAc)₃Tris, or a third conjugate of an effector molecule and a binding molecule comprising a binding site for a cell-surface molecule, and optionally comprising a pharmaceutically acceptable excipient and/or pharmaceutically acceptable diluent.

An aspect of the invention relates to a pharmaceutical composition comprising:
- the saponin conjugate of the invention;
- a second conjugate of an effector molecule and a ligand for ASGPR wherein the ligand for ASGPR preferably comprises at least one GalNAc moiety, preferably three or four GalNAc moieties, more preferably the ligand for ASGPR comprises or consists of (GalNAc)₃Tris, or a third conjugate of an effector molecule and a binding molecule comprising a binding site for a cell-surface molecule, and optionally comprising a pharmaceutically acceptable excipient and/or pharmaceutically acceptable diluent.

An embodiment is the pharmaceutical combination of the invention or the pharmaceutical composition of the invention, wherein the effector molecule comprises or consists of at least one of a small molecule such as a drug molecule, a toxin such as a protein toxin, an oligonucleotide such as a BNA, a xeno nucleic acid or an siRNA, an enzyme, a peptide, a protein, or any combination thereof, preferably, the effector molecule is a toxin, an enzyme or an oligonucleotide.

An embodiment is the pharmaceutical combination of the invention or the pharmaceutical composition of the invention, wherein the ligand for ASGPR comprises or is (GalNAc)₃Tris, and/or wherein the ligand for ASGPR and the effector molecule are conjugated via a covalent bond, preferably via at least one linker.

An embodiment is the pharmaceutical combination of the invention or the pharmaceutical composition of the invention, wherein the effector molecule is an oligonucleotide selected from any one or more of a(n): short interfering RNA (siRNA), short hairpin RNA (shRNA), anti-hairpin-shaped microRNA (miRNA), single-stranded RNA, aptamer RNA, double-stranded RNA (dsRNA), anti-microRNA (anti-miRNA, anti-miR), antisense oligonucleotide (ASO), DNA, antisense DNA, locked nucleic acid (LNA), bridged nucleic acid (BNA), 2'-O,4'-aminoethylene bridged nucleic Acid (BNA^{NC}), BNA-based siRNA, and BNA-based antisense oligonucleotide (BNA-AON).

An embodiment is the pharmaceutical combination of the invention or the pharmaceutical composition of the invention, wherein the effector molecule is an oligonucleotide selected from any one or more of a(n): anti-miRNA, a BNA-AON or an siRNA, such as BNA-based siRNA, selected from chemically modified siRNA, metabolically stable siRNA and chemically modified, metabolically stable siRNA.

An embodiment is the pharmaceutical combination of the invention or the pharmaceutical composition of the invention, wherein the effector molecule is an oligonucleotide capable of, for example when present inside a mammalian cell, silencing any one of genes: apolipoprotein B (apoB), HSP27, transthyretin (TTR), proprotein convertase subtilisin/kexin type 9 (PCSK9), delta-aminolevulinate synthase 1 (ALAS1), antithrombin 3 (AT3), glycolate oxidase (GO), complement component C5 (CC5), X gene of hepatitis B virus (HBV), S gene of HBV, alpha-1 antitrypsin (AAT) and lactate dehydrogenase (LDH), and/or is an oligonucleotide capable of, for example when present inside a mammalian cell, targeting an aberrant miRNA.

Despite progress in implementation of lifestyle changes and drug treatment for prevention, cardiovascular disease is still an important cause of total years of life lost [B.G. Nordestgaard et al., Advances in lipid-lowering therapy through gene-silencing technologies, Nature Reviews - Cardiology, V 15, May 2018, pp.261-272]. Substantial residual cardiovascular disease risk remains even after optimal lifestyle intervention, antihypertensive treatment, high-dose statin therapy and after treatment with newer classes of lipid-lowering therapy, such as the cholesterol absorption inhibitor ezetimibe, the PCSK9 inhibitors evolocumab and bococizumab, the lipid-lowering fibrate agent bezafibrate, the cholesteryl ester transfer protein inhibitor anacetrapib, and the anti-inflammatory agent canakinumab. Therapies in development, in particular antisense oligonucleotide inhibition or small interfering RNA (siRNA)-based approaches, employ gene-silencing technologies through mRNA degradation. Whereas small-molecule lipid-lowering agents are administered orally, gene-silencing drugs are usually injected subcutaneously. Gene-silencing technologies inhibit gene expression within the cell nuclei or cytoplasm to reduce protein production, resulting in reduced plasma lipoprotein levels in gram amounts. Antisense oligonucleotides and siRNAs are formulated with the aim of achieving much higher concentrations within liver cells than in plasma. Gene-silencing therapies target proteins both in plasma and within cells.

LDL most likely causes atherosclerosis through intimal deposition of cholesterol. A prerequisite for a myocardial infarction (MI) or an ischaemic stroke event is the development of atherosclerosis within the coronary and other arteries. Atherosclerotic plaque initiation occurs by infiltration of LDL and remnant lipoproteins from plasma into the intima of arteries, facilitated by a blood-pressure gradient. Atherosclerotic plaque progression can occur if LDL and remnant lipoprotein levels remain elevated in plasma over prolonged periods of time. Endothelial dysfunction, which leads to increased flux of these lipoproteins into the intima, can further enhance the progression of atherosclerosis. Plaque rupture leading to MI or ischaemic stroke typically occurs at sites of unstable atherosclerotic plaques with ongoing local inflammation and no fibrous cap. After plaque rupture, activated platelets and fibrin generate a thrombus that can progress further in size if high levels of Lp(a) inhibit fibrinolysis through its homology with plasminogen. Finally, a substantial reduction in LDL and remnant lipoproteins in plasma can stabilize the plaque by reducing plaque size and local inflammation. Gene-silencing technologies take advantage of the central dogma of molecular biology: the transcription of DNA into mRNA and the translation of mRNA into proteins. If the DNA sequence of a gene is known to influence the levels of a lipoprotein fraction causative of cardiovascular disease, it is possible to synthesize a single strand of RNA (antisense oligonucleotide) or a double strand of RNA (siRNA) that will bind to the mRNA product of that gene and inactivate it, effectively turning off - or silencing - that gene. A challenge with the gene-silencing approaches based on antisense oligonucleotides or siRNA is potential off-target or adverse effects, which are minimized by preferentially delivering the medication to liver cells and thereby keeping drug concentrations low in the rest of the body. Currently, the best way of selectively delivering either antisense oligonucleotides or siRNAs to liver cells is to attach these drugs or their delivery systems to N-acetylgalactosamine (GalNAc) moieties, which facilitates selective uptake into liver cells via the asialoglycoprotein receptor. Such gene-silencing drugs with a conjugated GalNAc moiety will substantially increase drug potency, reduce the levels of the drug in plasma, and provide the promise of fewer adverse effects.

Lipid-lowering gene-silencing therapies are assessed in phase II and phase III trials. In addition to silencing the apolipoprotein B gene, also genes ANGPTL3, APOC3, LPA, and PCSK9, which are active in several lipoprotein pathways, are targets for gene-silencing therapies. An example of a lipoprotein gene silencing therapy is Mipomersen, which is an antisense oligonucleotide that binds directly to the mRNA sequence that encodes the apoB protein, which is the predominant protein carried on LDL, remnant lipoproteins, and Lp(a).

Part of the invention is the pharmaceutical combination of the invention or the pharmaceutical composition of the invention, wherein the effector molecule is an oligonucleotide, preferably an siRNA such as a BNA-based siRNA, or an antisense oligonucleotide such as a BNA-based antisense oligonucleotide, for example an AON based on 2'-O,4'-aminoethylene bridged nucleic acid, capable of, preferably when present inside a mammalian cell, silencing gene apolipoprotein B (apoB).

An embodiment is the pharmaceutical combination of the invention or the pharmaceutical composition of the invention, wherein the effector molecule is an oligonucleotide capable of, for example when present inside a mammalian cell, targeting an mRNA involved in expression of any one of proteins: apoB, HSP27, TTR, PCSK9, ALAS1, AT3, GO, CC5, expression product of X gene of HBV, expression product of S gene of HBV, AAT and LDH, or is capable of, for example when present inside a mammalian cell, antagonizing or restoring an miRNA function such as inhibiting an oncogenic miRNA (onco-miR) or suppression of expression of an onco-miR.

Part of the invention is the pharmaceutical combination of the invention or the pharmaceutical composition of the invention, wherein the effector molecule is an oligonucleotide, preferably an siRNA such as a BNA-based siRNA, or an antisense oligonucleotide such as a BNA-based antisense oligonucleotide, for example an AON based on 2'-O,4'-aminoethylene bridged nucleic acid, capable of, preferably when present inside a mammalian cell, targeting an mRNA involved in expression of protein apoB. That is to say, binding of the oligonucleotide comprised by the effector-moiety comprising conjugate to the apoB mRNA results in silencing of the expression of the protein apolipoprotein B.

The inventors now surprisingly established the improved and effective gene silencing of apoB by treatment with a BNA-AON in combination with a conjugate of a ligand for ASGPR, here tri-GalNAc, and a saponin. The saponin, here SO1861 as an example, is known for its activity to facilitate the endosomal escape of molecule entering the endosome, e.g., via receptor-mediated cellular uptake. The gene silencing of apoB resulted in reduced mRNA levels for expressing ApoB protein, lower plasma ApoB level, and resulted in lowered LDL plasma level, after treatment with the therapeutic combination of GalNAc-comprising AON conjugate and GalNAc-comprising saponin conjugate. The gene-silencing effect is apparent at 72 h after a single administration of the combination of said two conjugates, and even after over 10 days, e.g., after 14 days.

An embodiment is the pharmaceutical combination of the invention or the pharmaceutical composition of the invention, wherein the effector molecule is or comprises a toxin which toxin comprises or consists of at least one molecule selected from any one or more of a peptide, a protein, an enzyme such as urease and Cre-recombinase, a proteinaceous toxin, a ribosome-inactivating protein, and/or a bacterial toxin, a plant toxin, more preferably selected from any one or more of a viral toxin such as apoptin; a bacterial toxin such as Shiga toxin, Shiga-like toxin, Pseudomonas aeruginosa exotoxin (PE) or exotoxin A of PE, full-length or truncated diphtheria toxin (DT), cholera toxin; a fungal toxin such as alpha-sarcin; a plant toxin including ribosome-inactivating proteins and the A chain of type 2 ribosome-inactivating proteins such as dianthin e.g. dianthin-30 or dianthin-32, saporin e.g. saporin-S3 or saporin-S6, bouganin or de-immunized derivative debouganin of bouganin, shiga-like toxin A, pokeweed antiviral protein, ricin, ricin A chain, modeccin, modeccin A chain, abrin, abrin A chain, volkensin, volkensin A chain, viscumin, viscumin A chain; or an animal or human toxin such as frog RNase, or granzyme B or angiogenin from humans, or any fragment or derivative thereof; preferably the protein toxin is dianthin and/or saporin, and/or comprises or consists of at least one of a toxin targeting ribosome, a toxin targeting elongation factor, a toxin targeting tubulin, a toxin targeting DNA and a toxin targeting RNA, more preferably any one or more of emtansine, pasudotox, maytansinoid derivative DM1, maytansinoid derivative DM4, monomethyl auristatin E (MMAE, vedotin), monomethyl auristatin F (MMAF, mafodotin), a Calicheamicin, N-Acetyl-γ-calicheamicin, a pyrrolobenzodiazepine (PBD) dimer, a benzodiazepine, a CC-1065 analogue, a duocarmycin, Doxorubicin, paclitaxel, docetaxel, cisplatin, cyclophosphamide, etoposide, docetaxel, 5-fluorouracyl (5-FU), mitoxantrone, a tubulysin, an indolinobenzodiazepine, AZ13599185, a cryptophycin, rhizoxin, methotrexate, an anthracycline, a camptothecin analogue, SN-38, DX-8951f, exatecan mesylate, truncated form of *Pseudomonas aeruginosa* exotoxin (PE38), a Duocarmycin derivative, an amanitin, α-amanitin, a spliceostatin, a thailanstatin, ozogamicin, tesirine, Amberstatin269 and soravtansine, or a derivative thereof.

### Conjugates - Bridging moiety B

The bridging moiety B present in the saponin conjugates of formula (III)_{S} or (IV)_{S} and in the effector molecule conjugates of formula as (III)_{E} or (IV)_{E} described herein represents any moiety suitable for covalently binding 2 or more, preferably 3 or 4, more preferably 3 GalNAc moieties and the saponin moiety linker L_{S} or the effector moiety linker L_{E}.

According to preferred embodiments, the bridging moiety B is a compound of formula (XV): 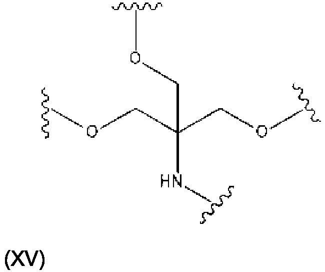 wherein the oxygen atoms of the compound of formula (XV) are bound to GalNAc (corresponding to compounds of formula (III)_{S} or (III)_{E}) or to the GalNAc linkers L_{GAL} (corresponding to compounds of formula (IV)_{S} or (IV)_{E}), and the nitrogen atom of the compound of formula (XV) is bound to the saponin moiety linker L_{S} or the effector moiety linker L_{E}.

The skilled person will understand that these compounds of formula (III)_{S}, (III)_{E}, (IV)_{S} or (IV)_{E} wherein the bridging moiety B is a compound of formula (XV) correspond to effector molecule conjugates wherein the ASGPR ligand consists of (GalNAc)₃Tris.

### Conjugates - Linker L_{GAL}

The GalNAc linkers L_{GAL} represent any chemical moiety suitable for covalently binding GalNAc to the bridging moiety as in formula (IV)_{S} or (IV)_{E}. The identity and size of the linker is not particularly limited.

According to embodiments, the GalNAc linkers L_{GAL} each comprise 2-25 carbon atoms, preferably 7-15 carbon atoms, more preferably 11 carbon atoms. Preferably the GalNAc linkers L_{GAL} comprise at least one, preferably two amide moieties. A particularly preferred GalNAc linker L_{GAL} is a compound according to formula (XVI):

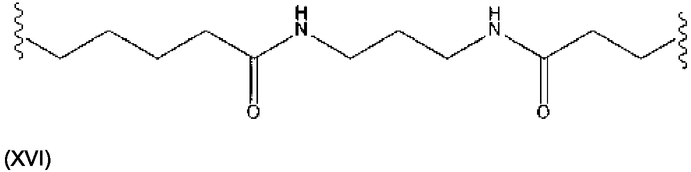

An embodiment is the pharmaceutical combination of the invention or the pharmaceutical composition of the invention, wherein the binding molecule comprising a binding site for a cell-surface molecule, comprised by the third conjugate, is a ligand for a cell-surface molecule or an antibody comprising a binding site for a cell-surface molecule or at least one domain or fragment thereof comprising the binding site.

An embodiment is the pharmaceutical combination of the invention or the pharmaceutical composition of the invention, wherein the third conjugate is any one or more of: an antibody-toxin conjugate, a receptor-ligand - toxin conjugate, an antibody-drug conjugate, a receptor-ligand - drug conjugate, an antibody-nucleic acid conjugate or a receptor-ligand - nucleic acid conjugate.

An embodiment is the pharmaceutical combination of the invention or the pharmaceutical composition of the invention, wherein the binding molecule comprising a binding site for a cell-surface molecule, comprised by the third conjugate, is capable of binding to any one of cell-surface molecules: CD71, CA125, EpCAM(17-1A), CD52, CEA, CD44v6, FAP, EGF-IR, integrin, syndecan-1, vascular integrin alpha-V beta-3, HER2, EGFR, CD20, CD22, Folate receptor 1, CD146, CD56, CD19, CD138, CD27L receptor, PSMA, CanAg, integrin-alphaV, CA6, CD33, mesothelin, Cripto, CD3, CD30, CD239, CD70, CD123, CD352, DLL3, CD25, ephrinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, asialoglycoprotein receptor (ASGPR), preferably any one of: HER2, CD71, ASGPR and EGFR, more preferably CD71.

An embodiment is the pharmaceutical combination of the invention or the pharmaceutical composition of the invention, wherein the binding molecule comprising a binding site for a cell-surface molecule, comprised by the third conjugate, is or comprises any one of: an antibody, preferably a monoclonal antibody such as a human monoclonal antibody, an IgG, a molecule comprising or consisting of a single-domain antibody, at least one V_{HH} domain, preferable a camelid V_{H}, a variable heavy chain new antigen receptor (V_{NAR}) domain, a Fab, an scFv, an Fv, a dAb, an F(ab)2 and a Fcab fragment.

An aspect of the invention relates to the pharmaceutical combination of the invention or to the pharmaceutical composition of the invention, for use as a medicament.

An aspect of the invention relates to the pharmaceutical combination of the invention or to the pharmaceutical composition of the invention, for use in the treatment or prophylaxis of a disease or health problem in which an expression product is involved of any one or more of genes: apoB, TTR, PCSK9, ALAS1, AT3, GO, CC5, X gene of HBV, S gene of HBV, AAT and LDH.

Part of the invention is the pharmaceutical combination of the invention or the pharmaceutical composition of the invention, for use in the treatment or prophylaxis of a disease or health problem in which an expression product is involved of gene apoB. The expression product of the apoB gene is the lipoprotein apolipoprotein B (ApoB), present as part of LDL, remnant lipoproteins, and Lp(a). Typically, the disease is a cardiovascular disease, typically related to build-up and presence of an atherosclerotic plaque involving one or more of ApoB comprising LDL, Lp(a) and remnant lipoproteins. The inventors now surprisingly established the improved and effective gene silencing of apoB by treatment with a BNA-AON in combination with a conjugate of a ligand for ASGPR, here tri-GalNAc, and a saponin. The saponin, here SO1861 as an example, is known for its activity to facilitate the endosomal escape of molecule entering the endosome, e.g. via receptor-mediated cellular uptake. The gene silencing of apoB resulted in reduced mRNA levels for expressing ApoB protein, reduced plasma ApoB protein level, and resulted in lowered LDL plasma level, after treatment with the therapeutic combination of GalNAc-comprising AON conjugate and GalNAc-comprising saponin conjugate. The gene-silencing effect is apparent at 72 h after a single administration of the combination of said two conjugates, and even after over 10 days, e.g. after 14 days.

An embodiment is the pharmaceutical combination of the invention or the pharmaceutical composition of the invention, or the pharmaceutical combination or pharmaceutical composition for use of the invention, for use in the treatment or prophylaxis of a cancer, an infectious disease, a viral infection, hypercholesterolemia, primary hyperoxaluria, haemophilia A, haemophilia B, AAT related liver disease, acute hepatic porphyria, TTR-mediated amyloidosis, hereditary TTR amyloidosis (hATTR), complement-mediated disease, hepatitis B infection, or an auto-immune disease.

An embodiment is the pharmaceutical combination for use of the invention or the pharmaceutical composition for use of the invention, wherein the saponin is a saponin derivative, preferably a QS-21 derivative or an SO1861 derivative according to the invention.

An aspect of the invention relates to an *in vitro* or *ex vivo* method for transferring the second conjugate or the third conjugate of the invention from outside a cell to inside said cell, preferably subsequently transferring the effector molecule comprised by the second conjugate or the third conjugate according to the invention into the cytosol of said cell, comprising the steps of:
a) providing a cell which expresses ASGPR on its surface, and, when the third conjugate is to be transferred into the cell, which expresses the cell-surface molecule for which the third conjugate comprises a binding molecule for binding to said cell-surface molecule, the cell preferably selected from a liver cell, a virally infected cell and a tumor cell;
b) providing the second conjugate or the third conjugate of the invention, for transferring into the cell provided in step a);
c) providing the saponin conjugate of the invention;
d) contacting the cell of step a) *in vitro* or *ex vivo* with the second conjugate or the third conjugate of step b) and the saponin conjugate of step c), therewith effecting the transfer of the second conjugate or the third conjugate from outside the cell into said cell, and preferably therewith subsequently effecting the transfer of the second conjugate or the third conjugate into the cytosol of said cell, or preferably therewith subsequently effecting the transfer of at least the effector molecule comprised by the second conjugate or the third conjugate into the cytosol of said cell.

While the invention has been described in terms of several embodiments, it is contemplated that alternatives, modifications, permutations and equivalents thereof will become apparent to one having ordinary skill in the art upon reading the specification and upon study of the drawings. The invention is not limited in any way to the illustrated embodiments. Changes can be made without departing from the scope which is defined by the appended claims.

The present invention has been described above with reference to a number of exemplary embodiments. The invention is further illustrated by the following examples.

### EXAMPLES AND EXEMPLARY EMBODIMENTS

### Example 1. CD71-saporin + 4000 nM trivalent GaINAc-L-SO1861

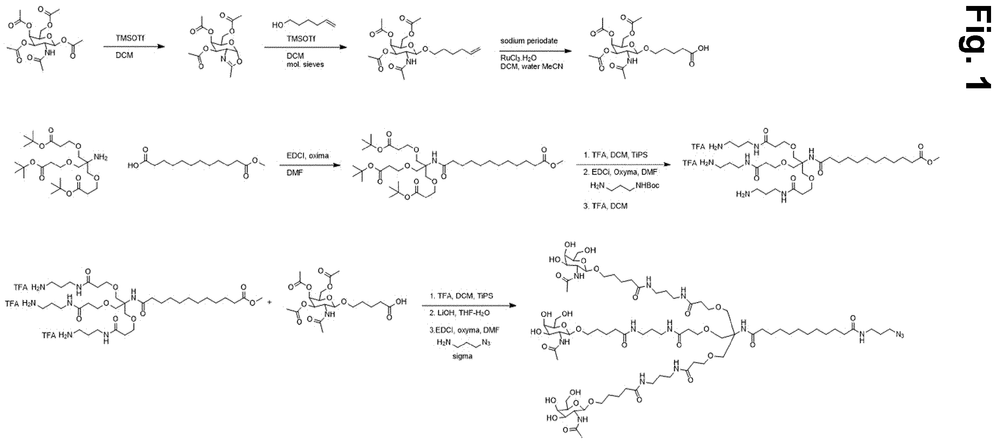

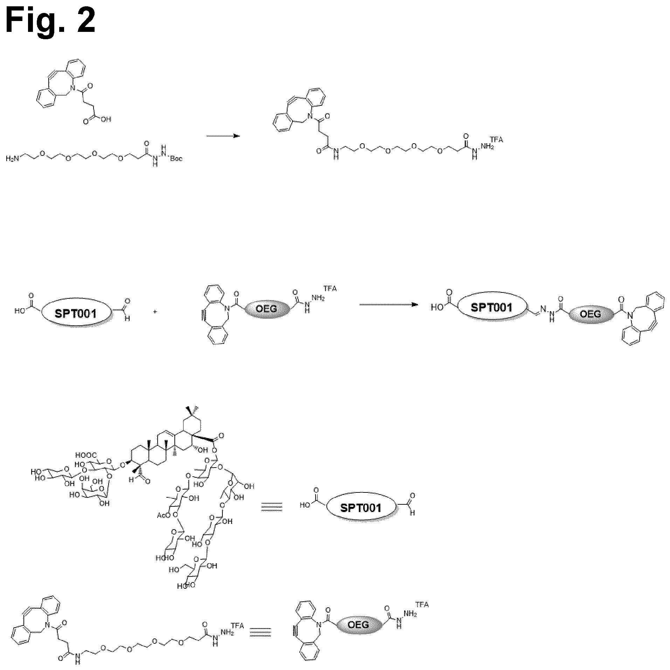

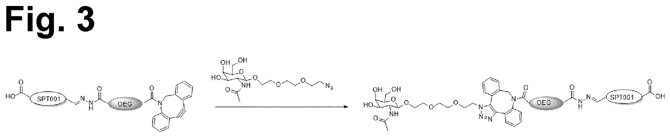

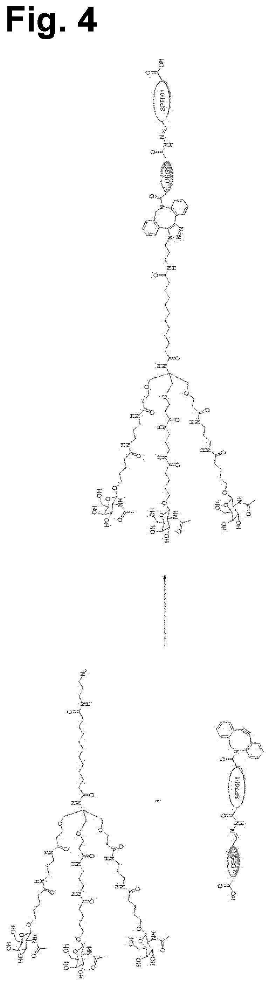

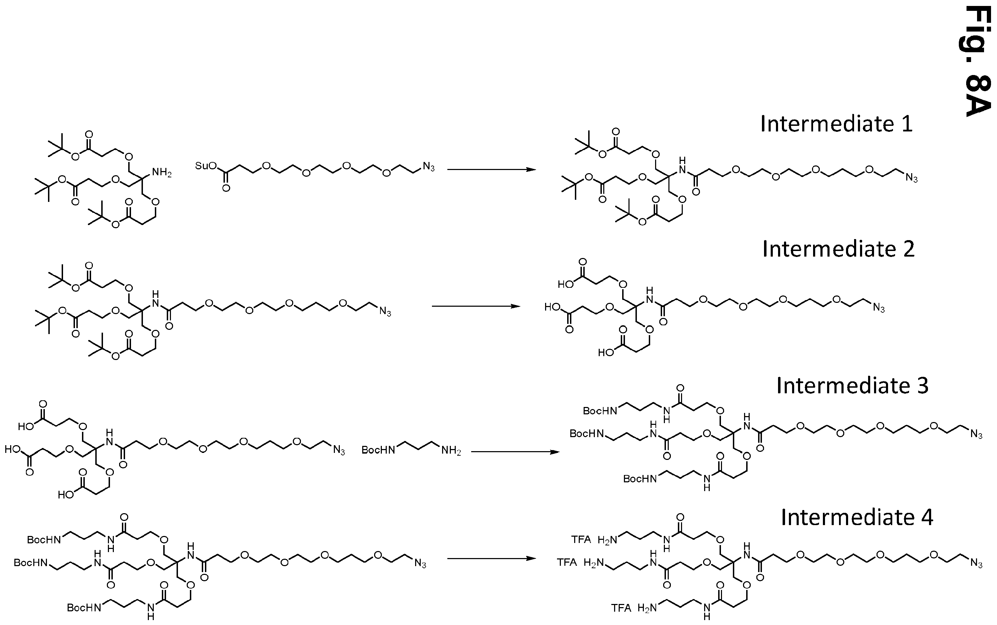

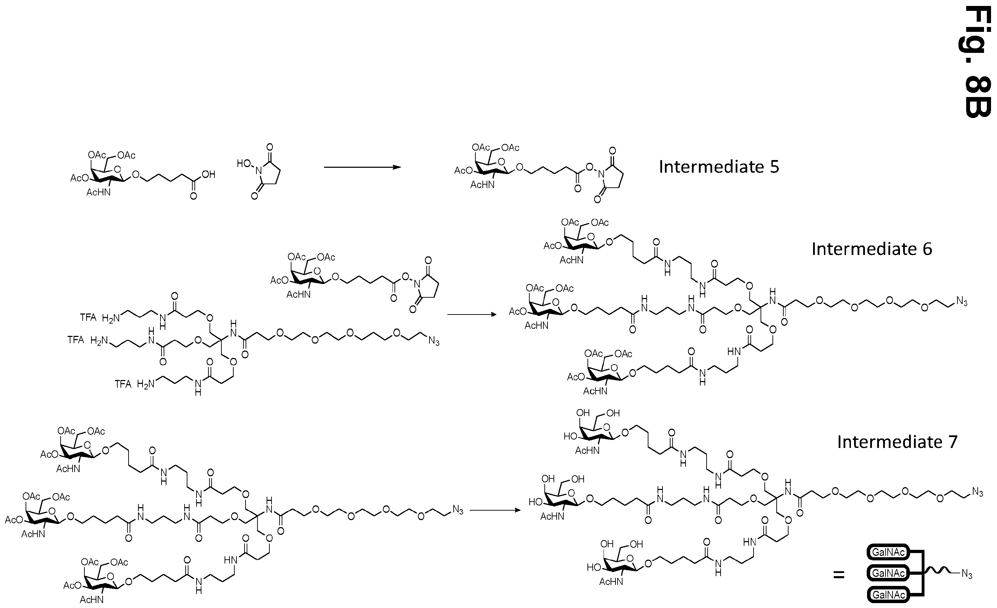

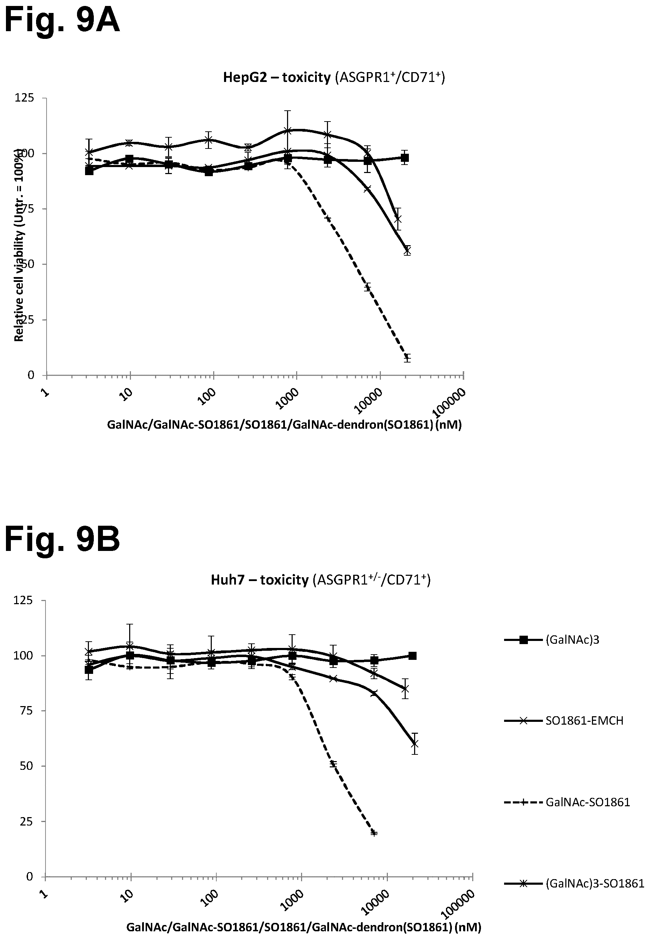

Trivalent-GalNAc is a targeting ligand that recognizes and binds the ASGPR1 receptor on hepatocytes. Trivalent GalNAc was produced (Figure 1 and Figure 8) and SO1861-EMCH was conjugated (labile, in a similar manner as described in Figure 2 and Figure 4 for underivatised SO1861, wherein SPT001 is synonymous to SO1861) to trivalent-GalNAc, with a DAR of 1 with regard to bound SO1861, (trivalent-GalNAc-SO1861). SO1861-EMCH refers to SO1861 wherein the aldehyde group is derivatised by transformation into a hydrazone bond through reaction with N-ε-maleimidocaproic acid hydrazide (EMCH), therewith providing SO1861-Ald-EMCH. The 'trivalent-GalNAc' as depicted in figures 1 and 8 is a typical (GalNAc)₃Tris conjugate suitable for coupling to an effector molecule or to saponin. As a control SO1861-EMCH was also conjugated (labile) to monovalent-GalNAc (Figure 3), with a DAR 1 with regard to bound SO1861, (GalNAc-SO1861). HepG2 (ASGPR1⁺; CD71⁺, Table A3) and Huh7 (ASGPR1⁺¹-; CD71⁺, Table A3) cells were treated with a concentration range of trivalent-GalNAc-L-SO1861 ((GalNAc)3-SO1861) and GalNAc-L-SO1861 (GalNAc-SO1861) in the presence and absence of 10 pM CD71-saporin (monoclonal antibody OKT-9 targeting CD71 conjugated to the protein toxin saporin). Cell treatment in absence of CD71-saporin show that the trivalent-GalNAc-L-SO1861 ((GalNAc)3-SO1861), (or trivalent-GalNAc) as single compound is not toxic up to 15000 nM (Figure 9).

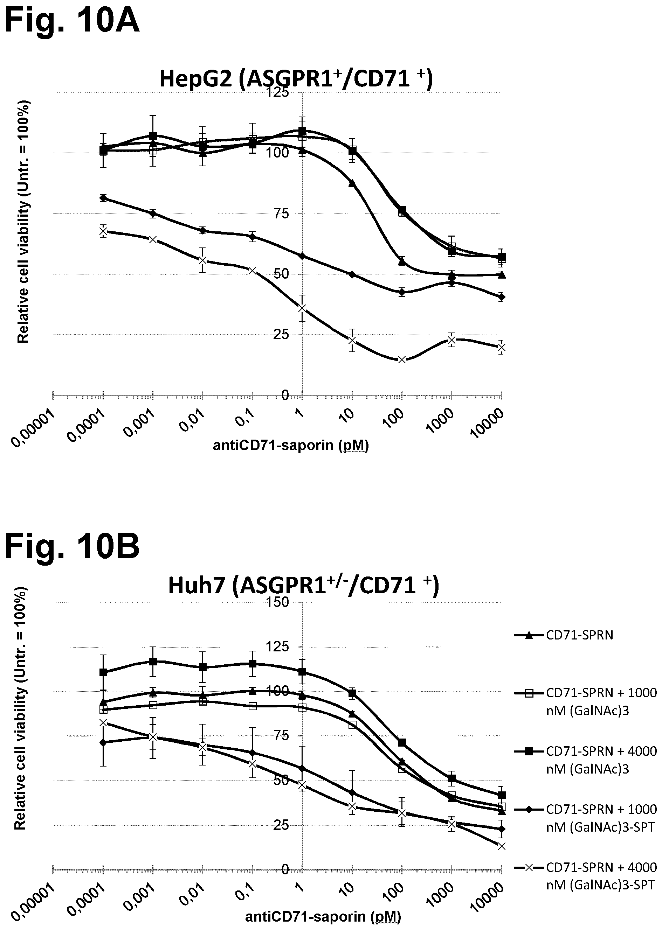

HepG2 and Huh7 cells were also treated with a range of concentrations of CD71-saporin in combination with a fixed concentration of 1000 nM or 4000 nM trivalent-GalNAc-SO1861 (DAR = 1 with regard to bound SO1861). Targeted protein toxin mediated cell killing of ASGPR1/CD71 expressing cells (HepG2 and Huh7) was determined. This revealed strong cell killing at low concentrations of CD71-Saporin (IC50=1 pM) in both cell lines, whereas equivalent concentrations CD71-Saporin (CD71-SPRN), CD71-Saporin + 1000 nM trivalent-GalNAc ((GalNAc)3) or CD71-Saporin + 4000 nM trivalent-GalNAc ((GalNAc)3-SPT) could only induce cell killing at high concentrations CD71-Saporin (IC50=100 pM) in both cell lines (Figure 10).

All this shows that trivalent-GalNAc-SO1861 ((GalNAc)3-SPT) in combination with low concentrations of CD71-Saporin (CD71-SPRN) effectively induce cell killing in ASGPR1/CD71 expressing cells. Thus trivalent-GalNAc-SO1861 effectively induces endosomal escape of a protein toxin in ASGPR1 expressing cells.

### Example 2 trivalent-GalNAc-L/S-BNA + SO1861-EMCH

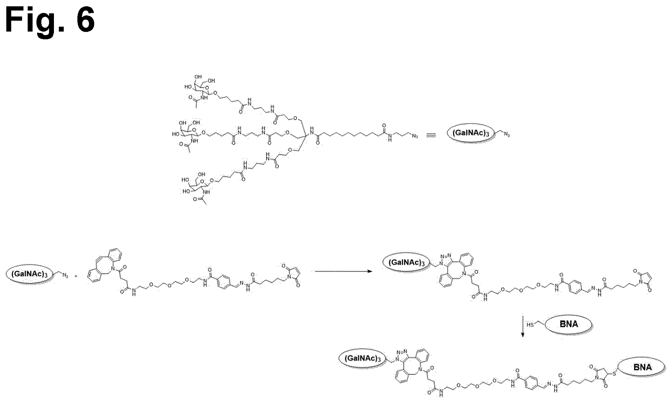

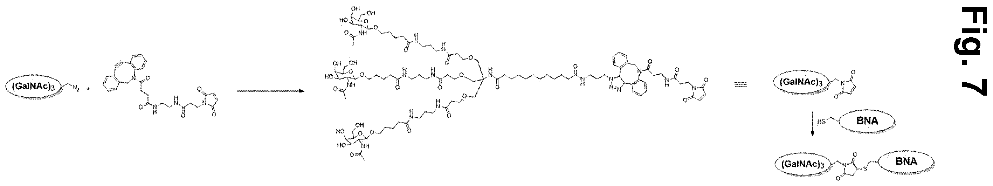

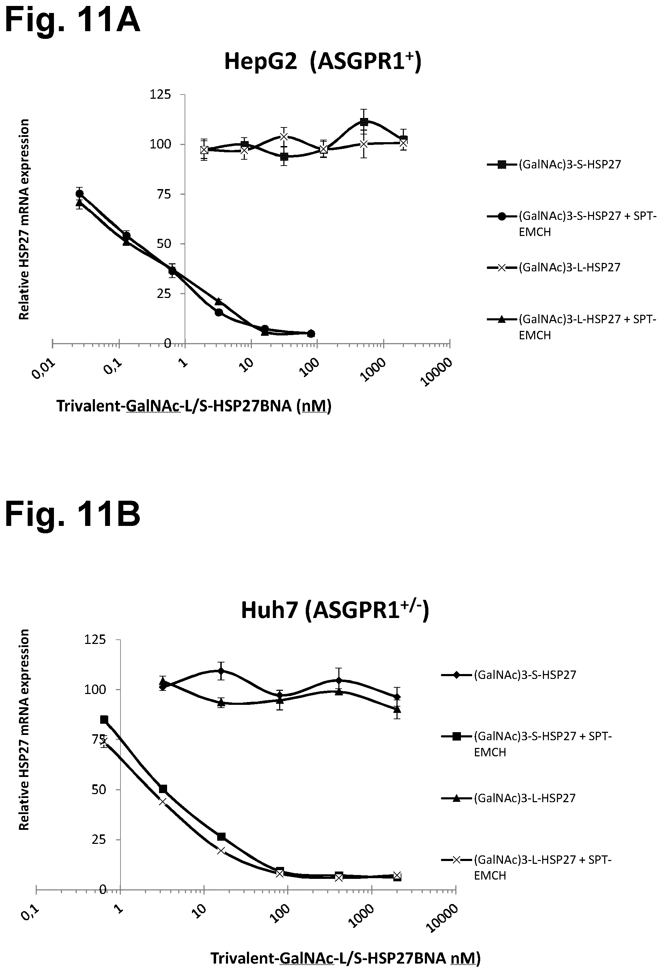

HSP27BNA was conjugated to trivalent-GalNAc (Figure 6-7) and HepG2 (ASGPR1⁺) cells or Huh7 (ASPGR1^{+/-}) were treated with a range of concentrations of trivalent-GalNAc-L-HSP27BNA (labile conjugation, Figure 6) or trivalent-GalNAc-S-HSP27BNA (stable conjugation, Figure 7) in combination with a fixed concentration of 4000 nM SO1861-EMCH. HSP27 gene silencing in HepG2 and Huh7 cells was determined. Only in combination with 4000 nM SO1861-EMCH effective gene silencing was observed at low concentrations of trivalent-GalNAc-L-HSP27BNA (also called (GalNAc)3-L-HSP27) (HepG2: IC50 = 0,1 nM; Huh7: IC50 = 3 nM) or trivalent-GalNAc-S-HSP27BNA (also called (GalNAc)3-S-HSP27) (HepG2: IC50 = 0,1 nM; Huh7: IC50 = 3 nM) in both HepG2 and Huh7 cells, whereas equivalent concentrations trivalent-GalNAc-L-HSP27BNA (HepG2/Huh7: IC50 > 2000 nM) or trivalent-GaINAc-S-HSP27BNA (HepG2/Huh7: IC50 > 2000 nM) did not show gene silencing activity in HepG2 and Huh7 cell lines (Figure 11).

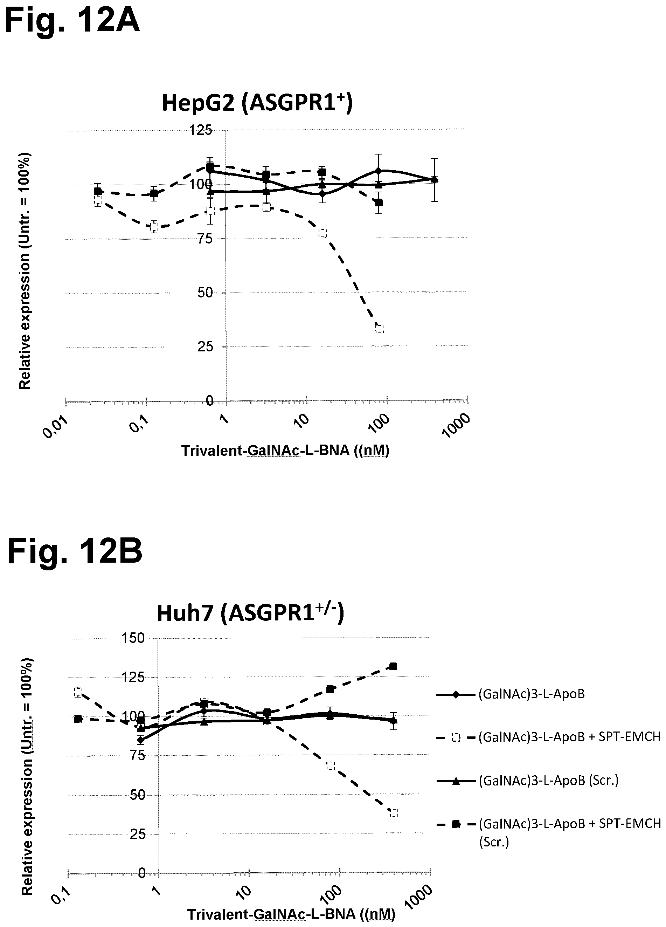

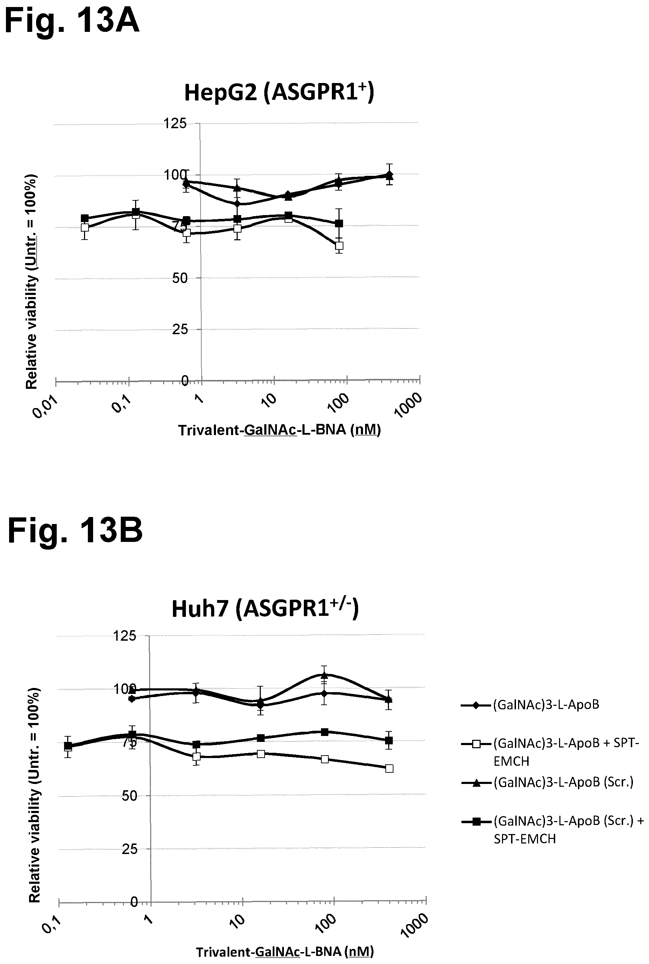

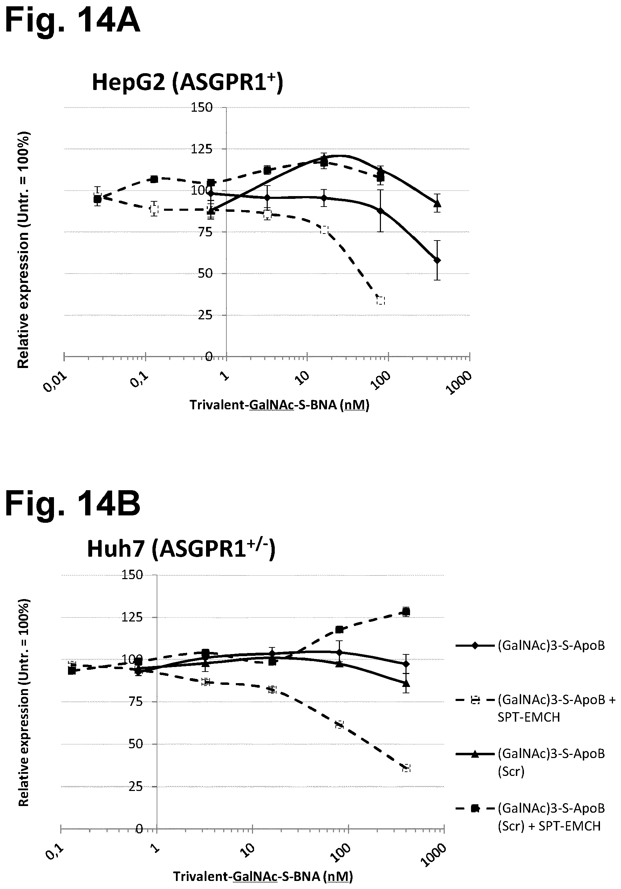

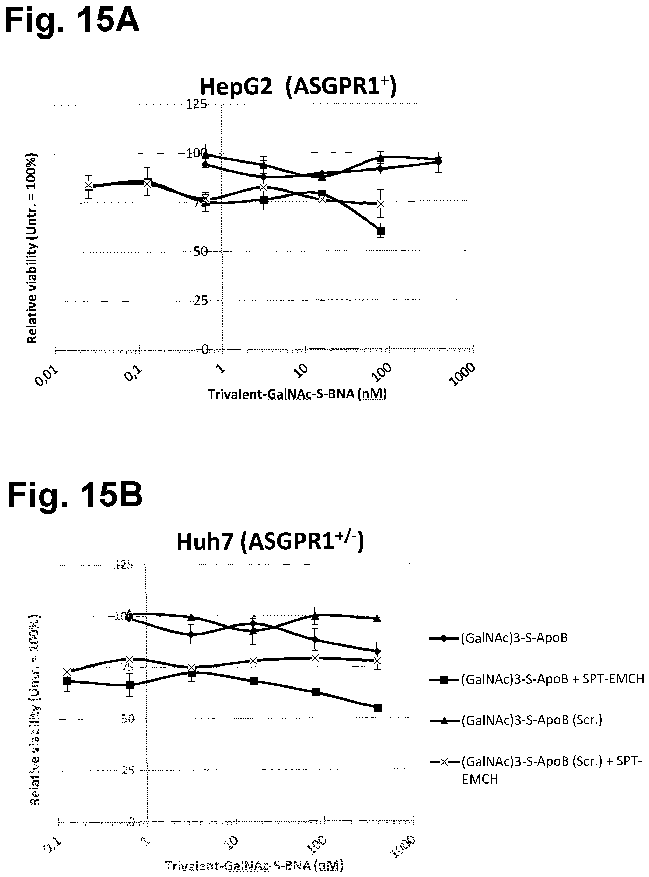

Next, ApoBBNA was conjugated in a similar manner (Figure 6-7) to trivalent-GalNAc and HepG2 (ASGPR1⁺) cells or Huh7 (ASPGR1^{+/-}) were treated with a range of concentrations of trivalent-GalNAc-L-ApoBBNA (also called (GalNAc)3-L-ApoB) (labile conjugation, Figure 6, Figure 12, Figure 13) or trivalent GalNAc-S-ApoBBNA (also called (GalNAc)3-S-ApoB) (stable conjugation, Figure 7, Figure 14, Figure 15) or a trivalent GalNAc-L-ApoB_{scrambled}BNA (also called (GalNAc)3-L-ApoB (Scr.) or trivalent GalNAc-S-ApoB_{scrambled}BNA (also called (GalNAc)3-L-ApoB (Scr.) (where the scrambled ApoBBNA is an antisense scrambled oligo sequence that does not bind the ApoB mRNA; off-target control BNA) in combination with a fixed concentration of 4000 nM SO1861-EMCH (also called SPT-EMCH). ApoB gene silencing in HepG2 (ASGPR1⁺) cells or Huh7 (ASPGR1^{+/-}) was determined. Only in combination with 4000 nM SO1861-EMCH effective gene silencing was observed at low concentrations of trivalent-GalNAc-L-ApoBBNA (IC50 = 10 nM) or trivalent-GalNAc-S-ApoBBNA (IC50 = 10 nM) in HepG2 cells, whereas equivalent concentrations trivalent-GalNAc-L-ApoBBNA (IC50 > 2000 nM) or trivalent-GalNAc-S-ApoBBNA (IC50 > 2000 nM) did not show gene silencing activity in HepG2 cells (Figure 12, Figure 14). The treatments did not affect the viability of both cell lines (Figure 13, Figure 15). The 'L' refers to a 'labile' linker, which indicates that the linker is cleaved in the cell, i.e., at pH as apparent in the endosome and the lysosome. In contrast, the 'S' refers to a 'stable' linker, which indicates that the linker is not cleaved in the cell, i.e., at pH as apparent in the endosome and the lysosome. Thus, a conjugate comprising a labile bond between any two molecules forming the conjugate or comprised by the conjugate, will be cleaved at a position in the labile linker, therewith dissociating the two linked or bound molecules. An example is the cleavage of a hydrazone bond under acidic conditions as apparent in the endosome and lysosome of mammalian cells such as human cells.

All this shows that SO1861-EMCH can enhance endosomal escape and target gene silencing of trivalent-GalNAc-L-BNA or trivalent-GalNAc-S-BNA for two independent gene targets. The gene silencing is more efficient in HepG2 cells compared to Huh7 cells, suggesting that higher levels of ASGPR1 expression at the plasma membrane of the cell facilitates increased uptake of the GalNAc-BNA conjugates.

### Example 3 trivalent-GalNAc-L/S-BNA + trivalent-GalNAc-L-SO1861

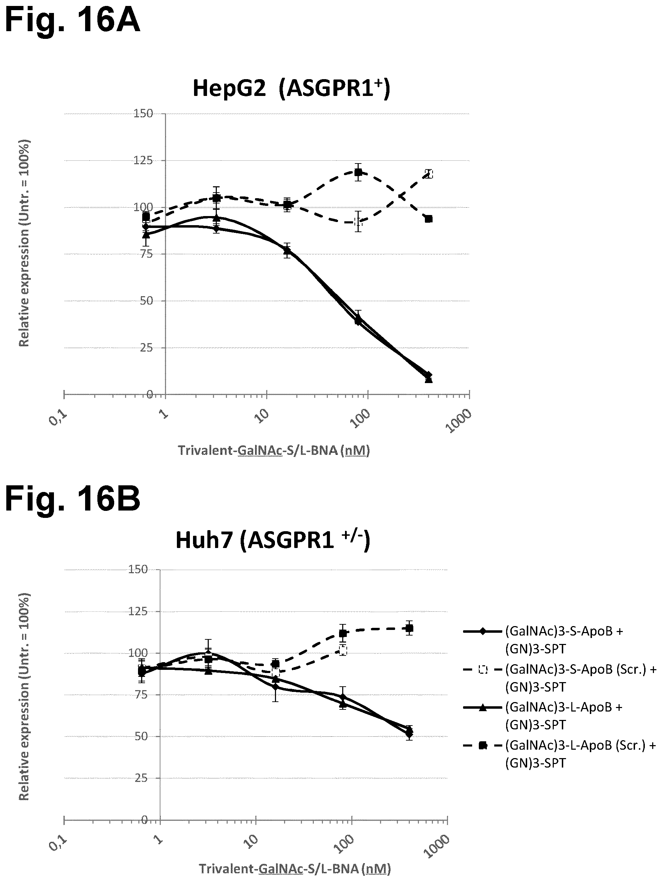

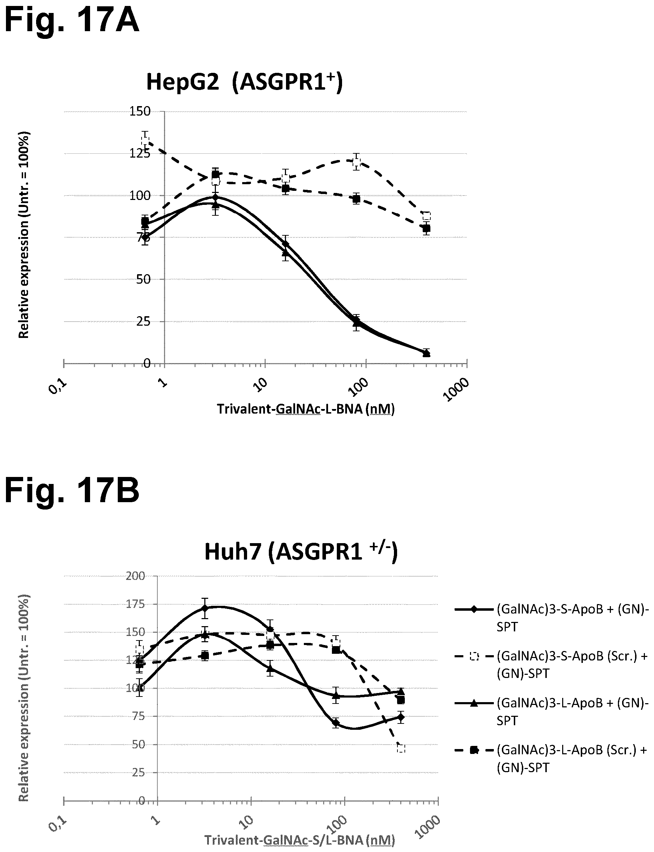

SO1861-EMCH was conjugated (labile, in a similar manner as described in Figure 2 and Figure 4 for underivatised SO1861 (also named SPT001) to trivalent-GalNAc, with a DAR = 1 with regard to bound SO1861, (called trivalent-GalNAc-SO1861, or (GalNAc)3-SO1861, or (GN)3-SPT). HepG2 (ASGPR1⁺) cells or Huh7 (ASPGR1^{+/-}) cells were treated with a range of concentrations of trivalent-GalNAc-L-ApoBBNA (labile conjugation Figure 16), trivalent-GalNAc-S-ApoBBNA (stable conjugation (Figure 17) or the scrambled off-target control conjugates (trivalent-GalNAc-L-ApoB_{scrambled}BNA or trivalent-GalNAc-S-ApoB_{scrambled}BNA) in combination with a fixed concentration of 4000 nM trivalent-GalNAc-L-SO1861 (DAR = 1 with regard to bound SO1861). ApoB gene silencing HepG2 (ASGPR1⁺) cells and Huh7 (ASPGR1^{+/-}) cells was determined. Only in combination with 4000 nM trivalent-GalNAc-L-SO1861 ((GN)3-SPT) effective gene silencing was observed at low concentrations of trivalent-GalNAc-L-ApoBBNA (IC50 = 50 nM) or trivalent-GalNAc-S-ApoBBNA (IC50= 50 nM) in HepG2 cells (ASGPR1⁺). Both combination treatments in Huh7 cells (ASGPR1^{+/-}) showed less effective gene silencing (trivalent-GalNAc-L-ApoBBNA: IC50 = 700 nM; trivalent-GalNAc-S-ApoBBNA: IC50= 700 nM). Equivalent concentrations of trivalent-GalNAc-L-ApoBBNA (HepG2/Huh7: IC50> 1000nM) or trivalent-GalNAc-S-ApoBBNA (HepG2/Huh7: IC50 > 1000 nM) did not show gene silencing activity in HepG2 and Huh7 cell lines, nor did 4000 nM trivalent-GalNAc-L-SO1861 induce/enhance gene silencing of scrambled trivalent-GalNAc-L-ApoB_{scrambled}BNA + (HepG2/Huh7: IC50 > 100 0nM) or scrambled trivalent-GalNAc-S-ApoB_{scrambled}BNA (HepG2/Huh7: IC50 > 1000 nM) (Figure 16). T

All this shows that in combination with trivalent-GalNAc-L-SO1861 ((GN)3-SPT), very low concentrations of trivalent-GalNAc-L-HSP27BNA or trivalent-GalNAc-S-HSP27BNA effectively induce gene silencing in ASGPR1 expressing cells, and again stronger effects are seen in cells with a higher ASGPR1 expression.

### Example 4 Potency of (GalNAc)3-BNA (GalNAc)3-SO1861 in primary mouse hepatocytes

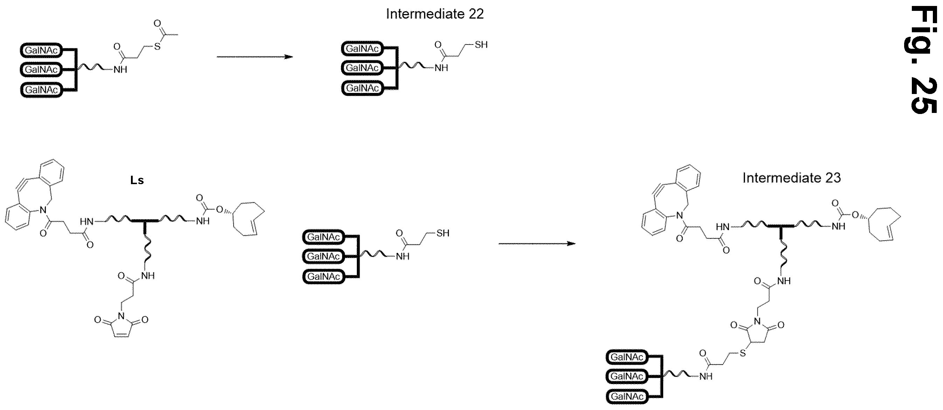

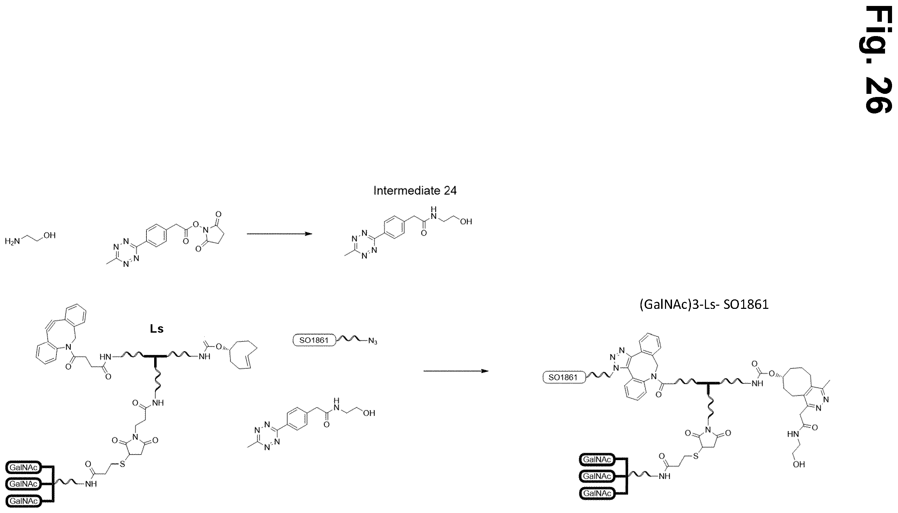

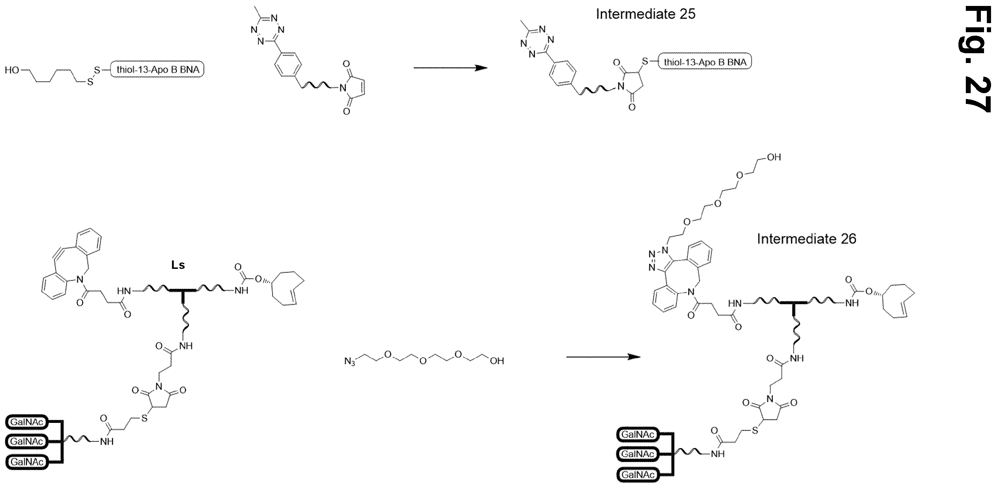

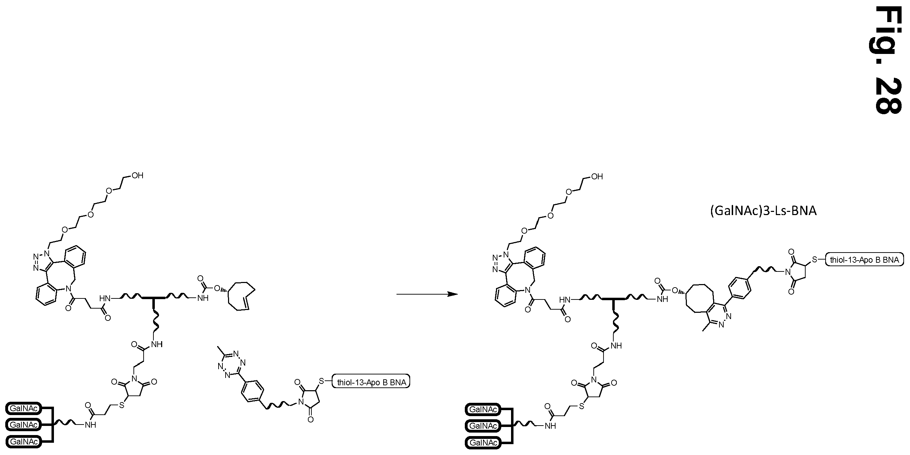

(GalNAc)3 was conjugated to a linker ('Ls') with SO1861-EMCH revealing (GalNAc)3-SO1861, with a DAR = 1 for the bound SO1861 (FIG 25, 26). Next, a murine ApoB-sequence targeting BNA, more specifically ApoB#02 BNA^{NC}, or ApoB#02 or thiol13-ApoB BNA, was conjugated to a linker ('Ls') revealing (GaINAc)3-ApoB#02 with a DAR = 1 for the bound SO1861 (FIG 27, 28).

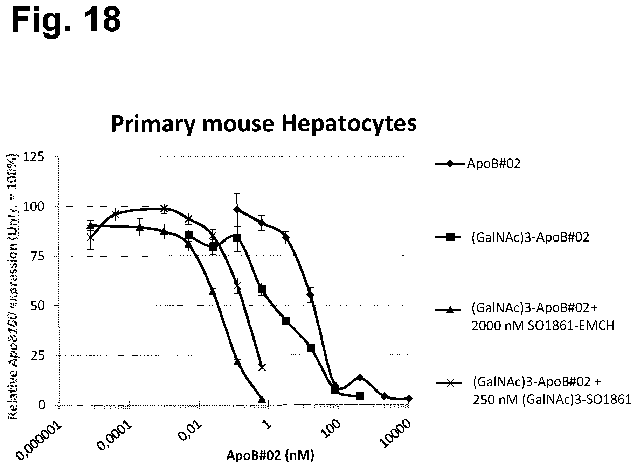

Primary mouse hepatocytes were treated with a range of concentrations of either ApoB#02 BNA alone, or (GaINAc)3-ApoB#02, or (GalNAc)3-ApoB#02 + 2000 nM SO1861-EMCH, or (GalNAc)3-ApoB#02 + 250 nM (GalNAc)3-SO1861 and *ApoB* RNA expression was determined by qPCR after 72 hrs incubation (Figure 18). Only in combination with 2000 nM SO1861-EMCH or in combination with 250 nM (GalNAc)3-SO1861, effective down-modulation of *ApoB* mRNA expression, i.e., gene expression inhibition, was observed at low concentrations of (GaINAc)3-ApoB#02 BNA; IC50 ca 0.03 nM in combination with 2000 nM SO1861-EMCH, and IC50 ca 0.2 nM in combination with 250 nM (GalNAc)3-SO1861. Treatment with ApoBBNA#02 alone was approximately 100 - 667-fold less potent in *ApoB* RNA down-modulation (ApoB#02: IC50 = 20 nM), while (GalNAc)3-ApoB#02 with an IC50 of ca. 2 nM was about 10 - 67-fold less potent.

This shows that only in combination with (GalNAc)3-SO1861 or SO1861-EMCH, very low concentrations of (GaINAc)3-ApoB#02 BNA are effectively inducing *ApoB* RNA down-modulation, i.e., gene silencing in murine primary hepatocytes.

### Example 5 in vivo efficay of (GalNAc)3-BNA + (GalNAc)3-SO1861 in C57BL/6J mice

C57BL/6J mice were treated as described and dosed according to Table A5. The conjugates used in this study, (GalNAc)3-ApoB#02 and (GalNAc)3-SO1861 were produced as described in Figure 25-28. Clinical observations were recorded and different biomarkers of efficacy and tolerability were analysed.

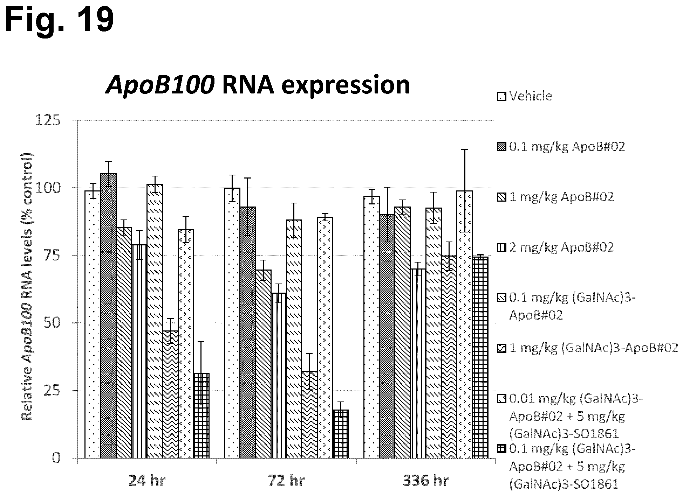

As Figure 19 shows, after 24 hrs, the *ApoB* RNA levels, i.e. gene expression, was not noticeably reduced in animals treated with 0.1 mg/kg ApoB#02 BNA (ApoB#02), and ca 15% and 21% reduced in groups dosed with 1 mg/kg or 2 mg/kg ApoB#02 BNA, respectively (all n = 3 animals per group), compared to the vehicle-dosed group (n = 5 animals). Likewise, (GalNAc)3-ApoB#02, at a dose of 1 mg/kg (corresponding to 0.58 mg/kg ApoB#02 BNA) reduced *ApoB* expression by ca 53% (n = 3 animals) compared to the vehicle control after 24 hrs, while 0.1 mg/kg (GaINAc)3-ApoB#02 alone did not show a noticeable reduction (n = 3) compared to vehicle. In contrast, when 0.1 mg/kg (GalNAc)3-ApoB#02 was co-administered with 5 mg/kg (GalNAc)3-SO1861, *ApoB* expression was readily reduced by ca 69% (n = 2 animals) already after 24 hrs; while 0.01 mg/kg (GalNAc)3-ApoB#02, when co-administered with 5 mg/kg (GalNAc)3-SO1861 still show a minor reduction of 16% (n = 2 animals), compared to vehicle. After 72 hrs, the highest dose of ApoB#02 BNA administered (2 mg/kg) showed a maximal reduction of 39% (n = 3 animals) compared to vehicle (n = 5 animals), while 1 mg/kg (GalNAc)3-ApoB#02 resulted in a 68% reduction of *ApoB* expression (n = 3 animals). Again, in contrast, the 10-fold lower dose of 0.1 mg/kg (GalNAc)3-ApoB#02, when co-administered with 5 mg/kg (GalNAc)3-SO1861, resulted in even 82% reduction of *ApoB* expression levels (n = 3 animals), while 0.1 mg/kg ((GalNAc)3-ApoB#02 alone only show 12% reduction (n = 3 animals). The effect was durable, and after 336 hrs, *ApoB* expression levels were reduced by 30% for 2 mg/kg ApoB#2 BNA (n = 2 animals) and 25% for 1 mg/kg (GalNAc)3-ApoB#02. The co-administration group of 10-fold less payload (ie, 0.1 mg/kg (GalNAc)3-ApoB#02 + 5 mg/kg (GalNAc)3-SO1861 still showed 25% decrease and hence, constitute a 10-fold improved ApoB expression reduction reduction based in administered payload.

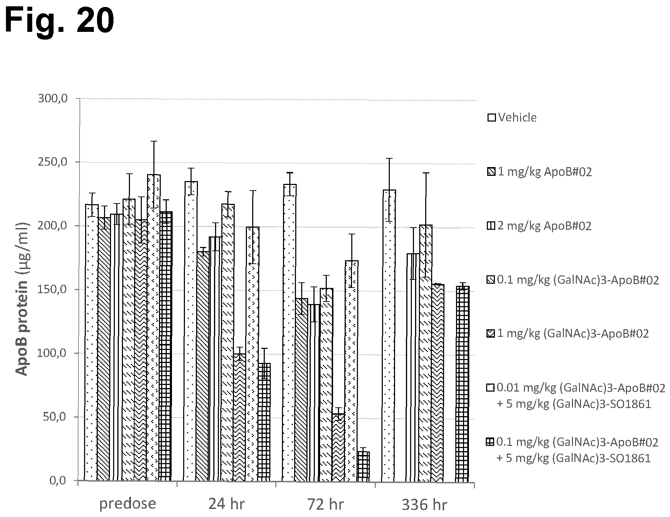

As Figure 20 shows, the *ApoB* RNA down-modulation (shown in Figure 19) translated well to ApoB protein level. After 24 hrs, the ApoB protein levels were most noticeably reduced by ca 50% for both, the 1 mg/kg (GaINAc)3-ApoB#02 (n = 8 animals) and equally so for the co-administration group with 10-fold less (GalNAc)3-ApoB#02 + 5 mg/kg (GalNAc)3-SO1861, ie, 0.1 mg/kg (GaINAc)3-ApoB#02 + 5 mg/kg (GalNAc)3-SO1861 (n = 8 animals), compared to controls (n = 14 animals). After 72 hours, while all treatment groups showed reduction in ApoB protein, compared to vehicle controls, the most pronounced effect was observed only when co-administering 0.1 mg/kg (GaINAc)3-ApoB#02 with 5 mg/kg (GalNAc)3-SO1861, resulting in an ApoB reduction of ca 90% (n = 5 animals). Even 0.01 mg/kg (GaINAc)3-ApoB#02 reduced ApoB protein by ca 25% when co-administering it with 5 mg/kg (GalNAc)3-SO1861 (n = 4 animals), while the 10-fold higher dose of 0.1 mg/kg (GalNAc)3-ApoB#02 alone reduced ApoB protein by 35% (n = 5 animals) after 72 hrs post-dose. Again, the effect was durable and ApoB protein reduction was still observable at 336 hrs for all treatment groups analyzed (all n = 2 animals per group; no samples were analysed from 1 mg/kg ApoB#02 and 0.01 mg/kg (GalNAc)3-ApoB#02 + 5 mg/kg (GalNAc)3-SO861 groups at 336 hours). Also here, 1 mg/kg (GalNAc)3-ApoB#02 and the co-administration group of the 10-fold lower dose of 0.1 mg/kg (GaINAc)3-ApoB#02 + 5 mg/kg (GalNAc)3-SO1861 showed similar durability (23% reduction in ApoB protein after 336 hrs).

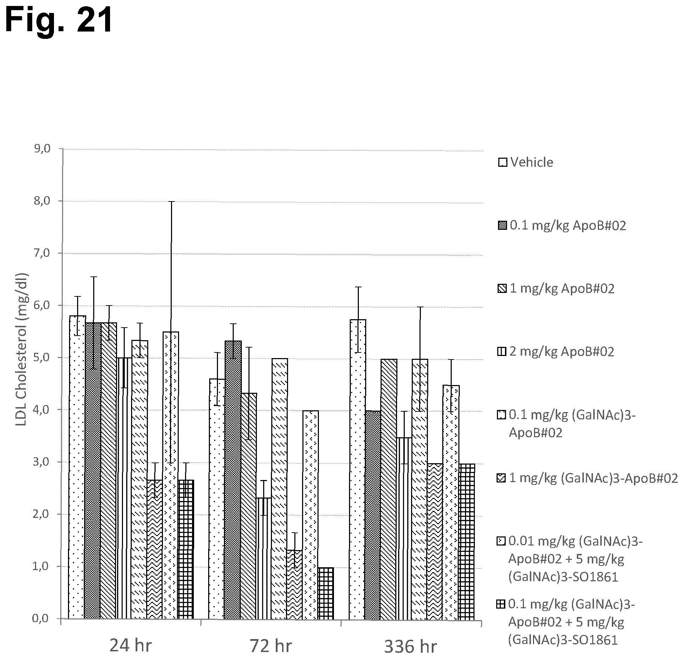

As Figure 21 shows, the observed ApoB protein down-modulation (shown in Figure 20) translated well to LDL-cholesterol (LDL-C) levels. After 24 hrs, LDL-C levels were not noticeably reduced in animals treated with either 0.1, 1, or 2 mg/kg ApoB#02 BNA (n = 3 animals per group) compared to vehicle (n = 6 animals). Targeted (GalNAc)3-ApoB#02 at a dose of 1 mg/kg reduced LDL-C to an average of 2.7 mg/dl within 24 hrs already (n = 3 animals), i.e., a 53% reduction compared to vehicle and ApoB#02 BNA groups. The same reduction of LDL-C to an average of 2.7 mg/dl within 24 hrs (n = 3 animals), *i.e.* a 53% reduction, was already observed with a 10-fold lower dose of 0.1 mg/kg (GalNAc)3-ApoB#02 when co-administering it with 5 mg/kg (GalNAc)3-SO1861, while the group of 0.01 mg/kg (GalNAc)3-ApoB#02 + 5 mg/kg (GalNAc)3-SO1861 had a too large variation for conclusion (n = 2 animals). After 72 hrs, the highest dose of ApoB#02 BNA (2 mg/kg) showed a 50% reduction (n = 3 animals) in LDL-C compared to vehicle (n = 6 animals), while 1 mg/kg (GaINAc)3-ApoB#02 was 72% reduced (n = 3 animals), and the 10-fold lower dose of 0.1 mg/kg (GaINAc)3-ApoB#02 + 5 mg/kg (GalNAc)3-SO1861 was even 78% reduced (n = 3 animals); while the 0.01 mg/kg (GalNAc)3-ApoB#02 + 5 mg/kg (GalNAc)3-SO1861 was 13% reduced (n = 2 animals). The effect was durable, and after 336 hrs, LDL-C levels were reduced by 40% for 2 mg/kg ApoB#02 BNA, and 48% for 1 mg/kg (GalNAc)3-ApoB#02 and for the co-administration group of 0.1 mg/kg (GaINAc)3-ApoB#02 + 5 mg/kg (GalNAc)3-SO1861, showing a 10-fold improved LDL-C reduction based in administered payload.

In conclusion, this shows that only in combination with 5 mg/kg (GalNAc)3-SO1861, very low concentrations of trivalent-GalNAc-ApoB#02 BNA ((GalNAc)3-ApoB#02 BNA) are effectively inducing *ApoB* RNA down-modulation, i.e., gene silencing in the livers of C57BL/6J mice, and all 'downstream' biomarkers of efficacy, such as ApoB protein and LDL-cholesterol.

### Example 5 in vivo tolerability of (GalNAc)3-BNA + (GalNAc)3-SO1861 in C57BL/6J mice

The conjugates used in this study, (GalNAc)3-ApoB#02 and (GalNAc)3-SO1861, were produced as described in Figure 25-28.

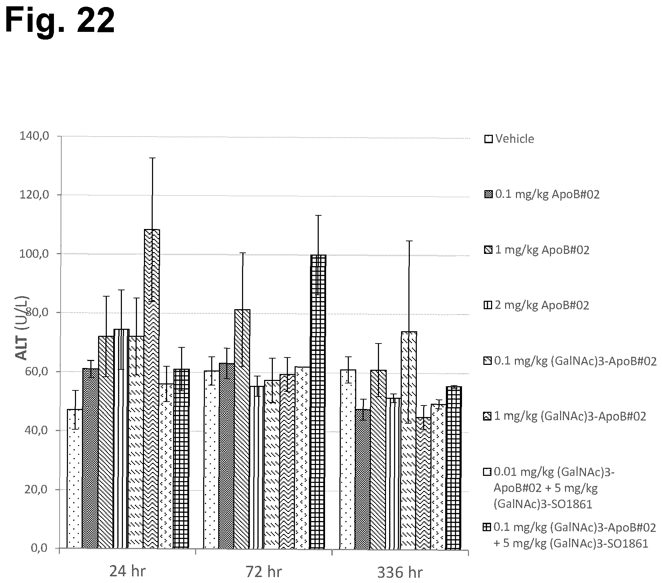

During the course of the study, all treatments, doses, and dose regimens as described in Table A5 were well tolerated and showed no treatment-specific adverse findings or any specific clinical observations indicative of the conjugates not being tolerated. The mice in treatment groups gained weight comparable to the mice receiving vehicle. Serum ALT levels were determined and results are shown in Figure 22. During the study period, ALT levels for vehicle mice ranged from an average 47 to 61 U/L per group (n = 6 animals for vehicle). Levels of ALT transiently increased in the different dose groups at different time points, with sometimes large per-animal variation per group, without showing specific compound or dose relationship (n = 3 for all groups at 24 hrs and 72 hrs, respectively, except for 0.01 mg/kg (GalNAc)3-ApoB#02 + 5 mg/kg (GalNAc)3-SO1861, where n = 2 for both time points). Notably, after 336 hrs, ALT levels in all dosing groups were back to baseline and comparable to vehicle controls (n = 2 for all groups).

In conclusion, this shows that ApoB#02, (GalNAc)3-ApoB#02, and (GalNAc)3-SO1861 (alone or in combination with (GalNAc)3-ApoB#02) are well tolerated in the doses and dose regimen applied, and particularly, that co-administration of (GalNAc)3-SO1861 with (GalNAc)3-ApoB#02 has no immediate tolerability issues, while showing high potency in the C57BL/6J mouse model.

### Example 6. (GalNAc)3-SO1861 + 10 pM CD71-saporin and (GalNAc)3-BNA + 250 nM (GalNAc)3-SO1861

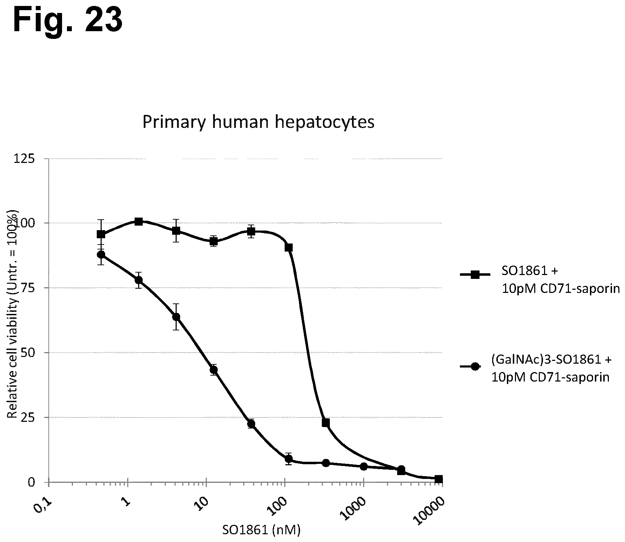

SO1861-EMCH was conjugated (labile, in a similar manner as described in Figure 2 and Figure 4 for underivatised SO1861 to trivalent-GalNAc ((GalNAc)3), with a DAR = 1 for the bound SO1861, called (GalNAc)3-SO1861. SO1861-EMCH refers to SO1861 wherein the aldehyde group is derivatised by transformation into a hydrazone bond through reaction with N-ε-maleimidocaproic acid hydrazide (EMCH), therewith providing SO1861-Ald-EMCH. The 'trivalent-GalNAc' as depicted in figures 1 and 8 is a typical (GalNAc)₃Tris conjugate suitable for coupling to an effector molecule or to saponin. Primary human hepatocytes were treated with a concentration range of SO1861 (underivatized) or (GalNAc)3-SO1861 in the presence and absence of 10 pM (non-effective concentration) CD71-saporin (monoclonal antibody clone OKT-9 targeting CD71 conjugated to the protein toxin saporin) (Figure 23). This revealed that efficient CD71-saporin mediated cell killing of primary human hepatocytes was effective at SO1861 IC50 = 200 nM, whereas targeted (GalNAc)3-SO1861 was already effective at much lower concentrations (SO1861 IC50 = 10 nM) (Figure 23). All this revealed that ASGPR1 receptor-mediated delivery of conjugated, acid-sensitive cleavable (EMCH) SO1861 requires reduced amounts of SO1861 to reveal enhanced potency.

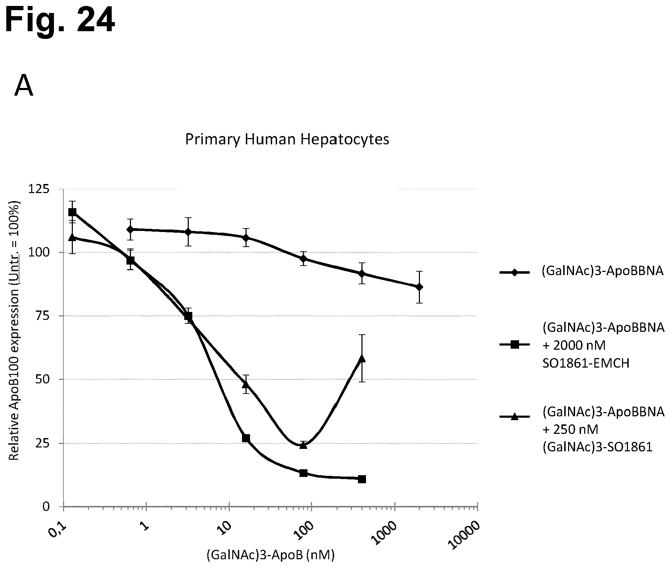

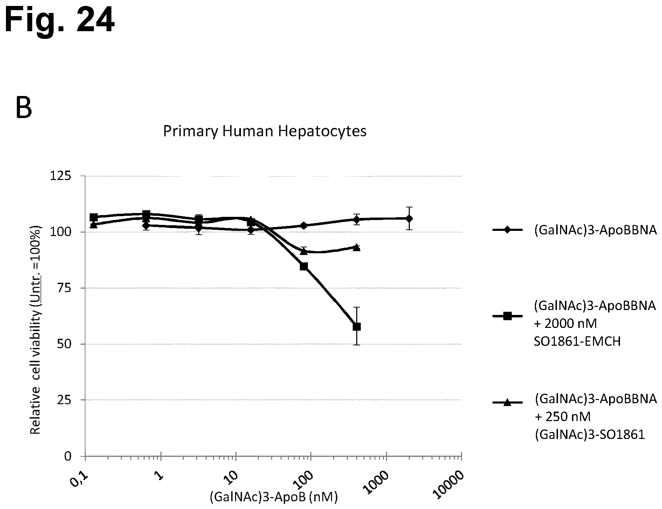

Next, ApoB BNA (SEQ ID NO: 2) was conjugated to trivalent-GalNAc ((GaINAc)3) to produce (GalNAc)3-ApoBBNA (Figure 6) and tested in combination with 2000 nM SO1861-EMCH or 250 nM (GalNAc)3-SO1861 on primary human hepatocytes for modulation of the ApoB RNA expression (Figure 24A) as well as the cell viability (Figure 24B). This revealed that (GalNAc)3-ApoBBNA alone was not able to efficiently reduce the ApoB RNA expression (IC50> 1000nM) (Figure 24 A), and no effects on cell viability were observed (Figure 24 B). However, (GalNAc)3-ApoBBNA + 2000nM SO1861-EMCH were highly effective and showed strong downregulation of *ApoB* RNA expression at low concentrations of (GalNAc)3-ApoBBNA (IC50 = 8 nM; Figure 24 A) and decreased cell viability at IC50 = 600 nM was observed (Figure 24 B). (GalNAc)3-ApoBBNA + 250 nM (GalNAc)3-SO1861 showed also strong downregulation of *ApoB* RNA at low concentrations of (GalNAc)3-ApoBBNA (IC50=15nM; Figure 24 A) without affecting cell viability significantly (Figure 24 B). Interestingly, in the (GalNAc)3-ApoBBNA + 250 nM (GalNAc)3-SO1861 combination, above 100 nM (GalNAc)3-ApoBBNA, competition of the (GalNAc)3 conjugates for the binding to the ASGPR1 receptor was observed, thereby limiting the optimal concentrations of both conjugates to access the cell, thereby reverting the ApoB RNA downregulation.

### Example 7. CD71-saporin + concentration series of trivalent GalNAc-L-SO1861, trivalent GalNAc-(L-SO1861)4, SO1861-EMCH

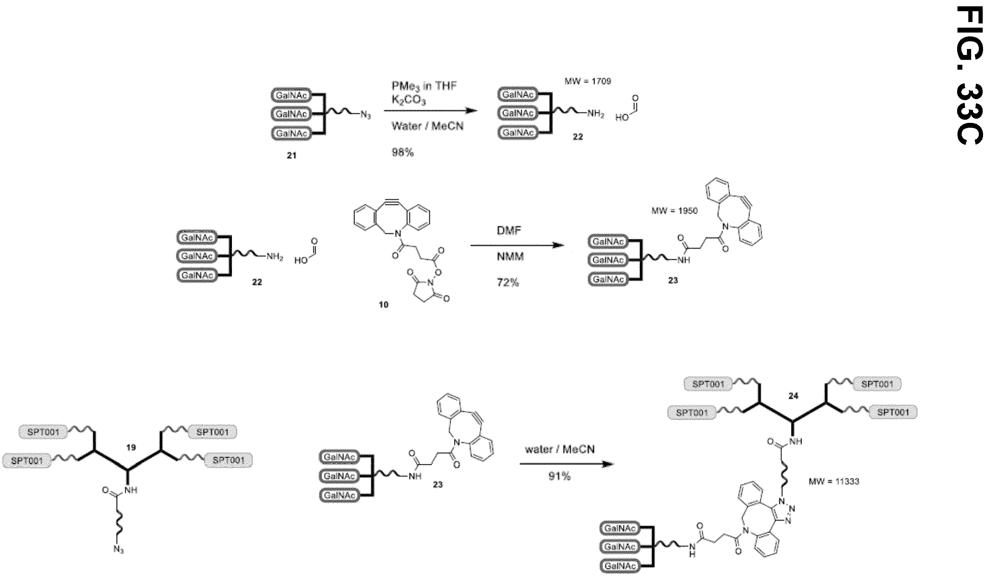

Trivalent GalNAc was produced (Figure 1 and Figure 8) and SO1861-EMCH was conjugated (labile, in a similar manner as described in Figure 2 and Figure 4 for underivatised SO1861, wherein SPT and SPT001 are synonymous to SO1861) to trivalent-GalNAc, with a DAR = 1 with regard to bound SO1861, (trivalent-GalNAc-SO1861; (GN)3-SPT)) or with a DAR 4 by first coupling the SO1861-EMCH to a dendron (trivalent-GalNAc-dendron(SO1861)4; (GN)3-dSPT4; Figure 33C). Again, SO1861-EMCH refers to SO1861 wherein the aldehyde group is derivatised by transformation into a hydrazone bond through reaction with N-ε-maleimidocaproic acid hydrazide (EMCH), therewith providing SO1861-Ald-EMCH. The 'trivalent-GalNAc' as depicted in figures 1 and 8 is a typical (GalNAc)₃Tris conjugate suitable for coupling to an effector molecule or to saponin. As a control SO1861-EMCH was also conjugated (labile) to monovalent-GalNAc (Figure 3), with a DAR = 1 with regard to bound SO1861or with a DAR = 4 with regard to bound SO1861 (using a dendron), (GalNAc-SO1861 and GalNAc-(SO1861)4). Primary hepatocytes express receptor at high density - allow for targeting. Primary hepatocytes which highly express ASGPR1 (ASGPR1⁺) were treated with a concentration range of trivalent-GalNAc-L-SO1861, SO1861-EMCH, trivalent GalNAc-(SO1861)4 in the presence of 10 pM CD71-saporin (monoclonal antibody OKT-9 targeting CD71 conjugated to the protein toxin saporin). Cell treatment in absence of CD71-saporin show that the trivalent-GalNAc-L-SO1861 as single compound is not toxic up to at least 1.000 nM (Figure 29A; relative cell viability). Cell viability of cells treated with the combination of anti CD71 MoAb-saporin ('MoAb' = monoclonal antibody) and GalNAc conjugated with SO1861 (either DAR = 1 with regard to bound SO1861, or DAR = 4 with regard to bound SO1861) is about a factor 100 lower compared to cell viability of cells treated with anti CD71 MoAb-saporin (CD71-SPRN) combined with SO1861-EMCH (SPT-EMCH) (Figure 29A).

All this shows that trivalent-GalNAc-SO1861 and trivalent-GalNAc-(SO1861)₄ in combination with low concentrations of CD71-Saporin effectively induce cell killing in ASGPR1/CD71 expressing cells. Thus, trivalent-GalNAc-SO1861 effectively induces endosomal escape of a protein toxin in ASGPR1 expressing cells. Thus, SPT001 and GalNAc targeted SPT001 (DAR = 1 with regard to bound SO1861) both increase potency of industry standard GalNAc targeted ASO; SO1861-EMCH with about a factor 10, and both (GN)3-SPT and (GN)3-dSPT4 about 100x, when cell viability of liver cells is considered.

### Example 8. Trivalent GalNAc conjugated with ApoB antisense oligonucleotide + trivalent GalNAc-L-SO1861

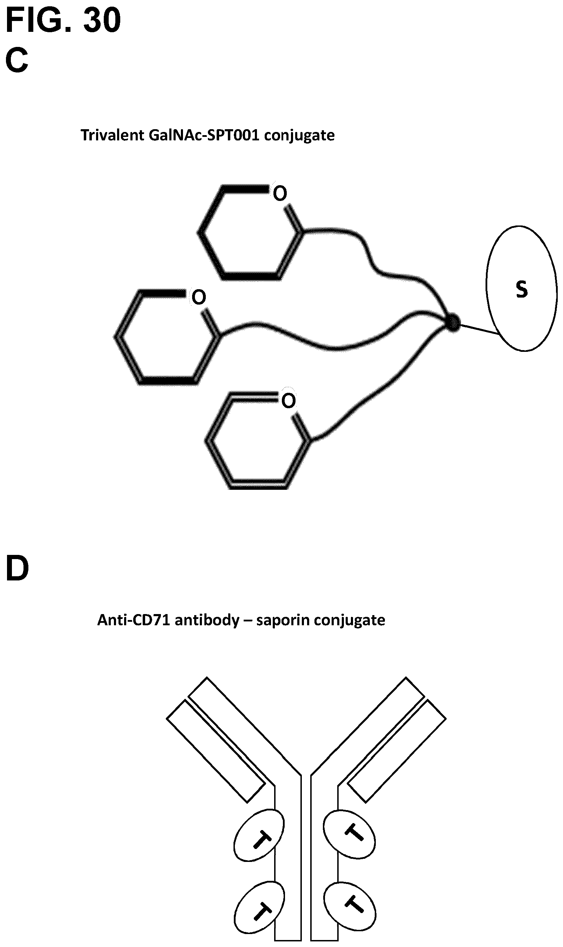

Trivalent GalNAc was produced (Figure 1 and Figure 8) and SO1861-EMCH was conjugated (labile, in a similar manner as described in Figure 2 and Figure 4 for underivatised SO1861, wherein SPT and SPT001 are synonymous to SO1861) to trivalent-GalNAc, with a DAR 1, (trivalent-GalNAc-SO1861; (GalNAc)3-SPT001); Figure 30C). Again, SO1861-EMCH refers to SO1861 wherein the aldehyde group is derivatised by transformation into a hydrazone bond through reaction with N-ε-maleimidocaproic acid hydrazide (EMCH), therewith providing SO1861-Ald-EMCH. Also, the covalent conjugate of trivalent GalNAc coupled to both the ApoB antisense BNA oligonucleotide ApoBBNA (SEQ ID NO: 2) and a dendron comprising four covalently coupled SO1861-EMCH, was prepared. See for the outlines for preparing the conjugates also the section **'Trivalent-GalNAc-SO1861 and GalNAc-SO1861 synthesis (****Figure 2****, 4)'** and the section **'Trivalent GaINAc-BNA oligo synthesis (****Figure 6****, 7)'** and the sections **'Trivalent GalNAc-S-ApoB (or HSP27) BNA oligo synthesis'** and **'Trivalent GalNAc-L-ApoB (or HSP27) BNA oligo synthesis'** and the section **'Dendron(-L-SO1861)₄-trivalent GalNAc synthesis',** here below.

Primary hepatocytes which highly express ASGPR1 (ASGPR1⁺) were treated with a concentration range of trivalent-GalNAc-*Apo*BBNA ((GaINAc)3-ApoB), either or not in the presence of 300 nM trivalent-GalNAc-SO1861 ((GalNAc)3-SPT001) (Figure 29B). Cell treatment in absence of (GalNAc)3-SPT001 show that the trivalent-GalNAc-L-SO1861 is highly effective in reducing the ApoB gene expression in the primary hepatocytes compared to ApoB gene expression in cells treated with (GalNAc)3-ApoB only (Figure 29B).

All this shows that trivalent-GalNAc-SO1861 in combination with (GalNAc)₃-ApoB effectively induces silencing of ApoB gene expression in ASGPR1 expressing cells. Thus trivalent-GalNAc-SO1861 effectively induces endosomal escape of a BNA in ASGPR1 expressing cells.

### Example 9. Trivalent GalNAc conjugated with SO1861, or a concentration series of conjugate trivalent GalNAc-(L-SO1861) in the presence of a fixed dose of OKT-9 anti-CD71 monoclonal antibody

Trivalent GalNAc was produced (Figure 1 and Figure 8) and SO1861-EMCH was conjugated (labile, in a similar manner as described in Figure 2 and Figure 4 for underivatised SO1861, wherein SPT and SPT001 are synonymous to SO1861) to trivalent-GalNAc, with a DAR = 1 with regard to bound SO1861, (trivalent-GalNAc-SO1861; (GaINAc)3-SPT001); Figure 30C). Again, SO1861-EMCH refers to SO1861 wherein the aldehyde group is derivatised by transformation into a hydrazone bond through reaction with N-ε-maleimidocaproic acid hydrazide (EMCH), therewith providing SO1861-Ald-EMCH. See for the outline for preparing the conjugate also the section **'Trivalent-GalNAc-SO1861** and **GalNAc-SO1861 synthesis (****Figure 2****, 4)'** here below.

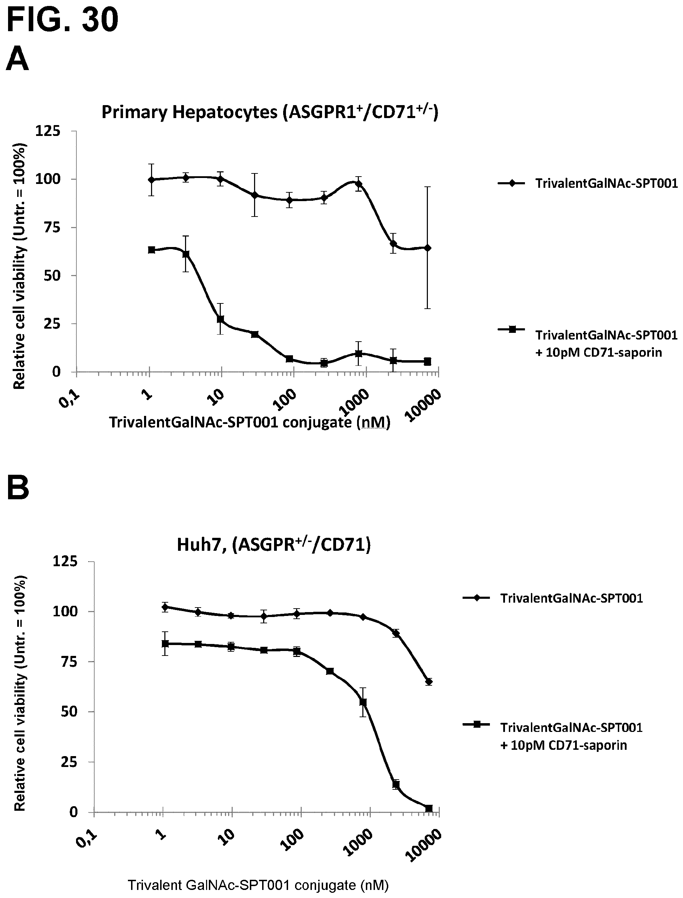

Primary hepatocytes which highly express ASGPR1 (ASGPR1⁺) and hepatocyte cells of cell-line Huh7 (ASGPR1^{+/-}) were treated with a concentration range of trivalent-GalNAc-SO1861 (TrivalentGaINAc-SPT001), either or not in the presence of 10 pM CD71-saporin (monoclonal antibody OKT-9 targeting CD71 conjugated to the protein toxin saporin; Figure 30D). Cell treatment in absence of CD71-saporin show that the trivalent-GalNAc-L-SO1861 as single compound is not toxic up to at least 1.000 nM (Figure 30A, 30B; relative cell viability). Cell viability of cells treated with the combination of anti CD71 MoAb-saporin and a low nM dose of GalNAc conjugated with SO1861 (DAR = 1 with regard to bound SO1861; TrivalentGaINAc-SPT001) is highly effective in killing the primary hepatocytes compared to cell viability of Huh7 cells treated with the same dose of anti CD71 MoAb-saporin (CD71-saporin) combined with the same dose of TrivalentGalNAc-SPT001 (Figure 30A and 30B).

All this shows that low dose of trivalent-GalNAc-SO1861 in combination with a low concentration of CD71-Saporin effectively induce cell killing in ASGPR1 expressing cells. Thus trivalent-GalNAc-SO1861 effectively induces endosomal escape of a protein toxin in ASGPR1 expressing cells. Thus, low nM TrivalentGalNAc-SPT001 enhances cytoplasmic delivery of low pM CD71MoAb-saporin in primary hepatocytes; and Hepatocytes that lack ASGPR1 expression are not responsive at low nM concentrations of TrivalentGaINAc-SPT001.

### Example 10. Trivalent GalNAc conjugated with ApoB antisense oligonucleotide + trivalent GaINAc-L-SO1861

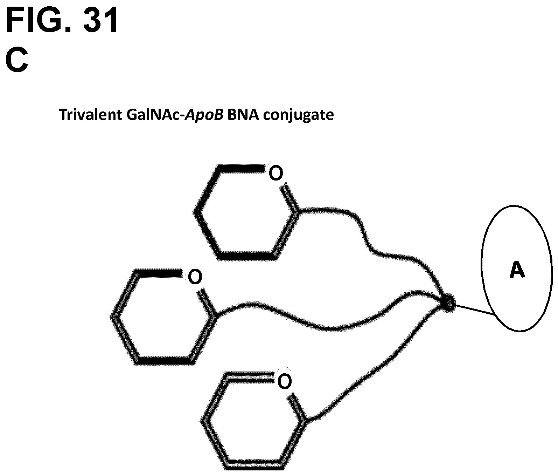

Trivalent GalNAc was produced and SO1861-EMCH was conjugated to trivalent-GalNAc, with a DAR = 1 with regard to bound SO1861, as described in Example 8 (trivalent-GalNAc-SO1861; (GalNAc)3-SPT001); Figure 30C). In addition, the conjugate of trivalent GalNAc and the ApoB antisense BNA oligonucleotide ApoBBNA (SEQ ID NO: 2) was prepared (Figure 31C). See for the outlines for preparing the conjugates also the section **'Trivalent-GalNAc-SO1861 and GalNAc-SO1861 synthesis (****Figure 2****, 4)'** and the section **'Trivalent GalNAc-BNA oligo synthesis (****Figure 6****, 7)'** and the sections **'Trivalent GalNAc-S-ApoB (or HSP27) BNA oligo synthesis'** and **'Trivalent GalNAc-L-ApoB (or HSP27) BNA oligo synthesis'** and the section **'Dendron(-L-SO1861)₄-trivalent GalNAc** and **dendron(-L-SO1861)₈-trivalent GalNAc synthesis'** and **'Dendron(-L-SO1861)₄-trivalent GalNAc synthesis',** here below.

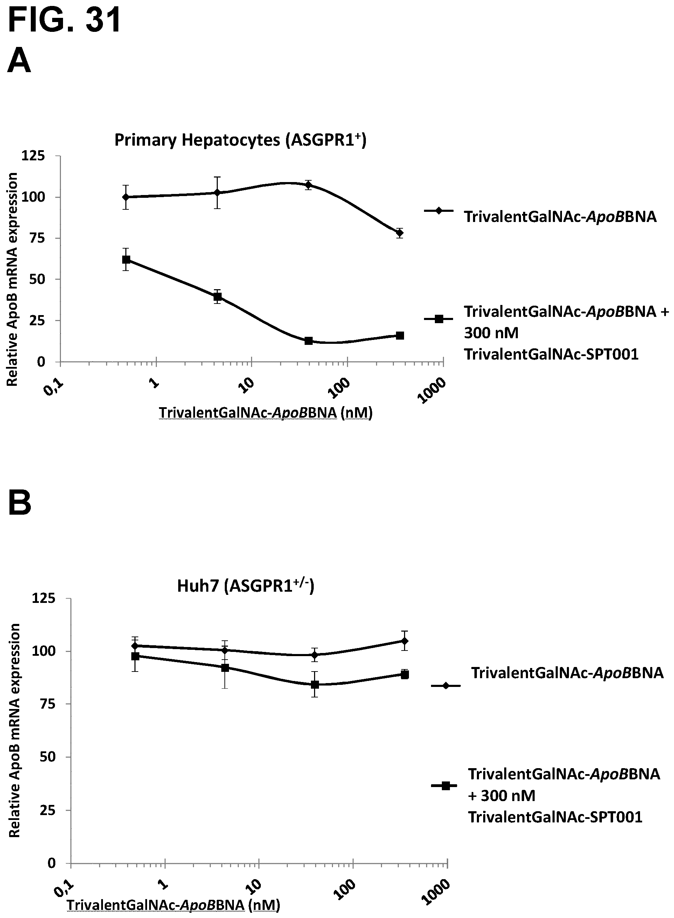

Primary hepatocytes which highly express ASGPR1 (ASGPR1⁺) and hepatocyte cells of cell-line Huh7 (ASGPR1^{+/-}) were treated with a concentration range of trivalent-GalNAc-ApoBBNA (TrivalentGaINAc-ApoBBNA), either or not in the presence of 300 nM trivalent-GalNAc-SO1861 (TrivalentGaINAc-SPT001) (Figure 31A and B). Cell treatment in absence of TrivalentGalNAc-SPT001 show that the TrivalentGalNAc-SPT001 is highly effective (~factor 100 more potent) in reducing the ApoB mRNA expression in the primary hepatocytes compared to ApoB mRNA expression in the primary liver cells treated with TrivalentGalNAc-*Apo*BBNA only (Figure 31A). Neither TrivalentGalNAc-ApoBBNA, nor the combination of TrivalentGalNAc-*Apo*BBNA and TrivalentGalNAc-SPT001 induce lowering of the ApoB mRNA expression in these liver cells.

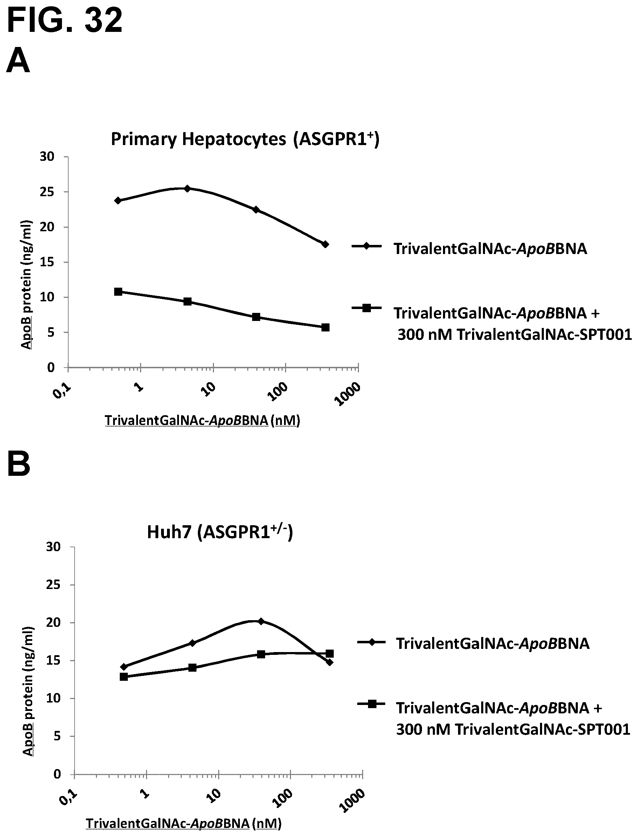

All this shows that TrivalentGalNAc-SPT001 in combination with TrivalentGalNAc-*Apo*BBNA effectively induces silencing of ApoB mRNA expression in ASGPR1 expressing cells. Thus TrivalentGalNAc-SPT001 effectively induces endosomal escape of a BNA in ASGPR1 expressing cells. Primary hepatocytes which highly express ASGPR1 (ASGPR1⁺) and hepatocyte cells of cell-line Huh7 (ASGPR1^{+/-}) were treated with a concentration range of trivalent-GalNAc-ApoBBNA (TrivalentGalNAc-ApoBBNA), either or not in the presence of 300 nM trivalent-GalNAc-SO1861 (TrivalentGaINAc-SPT001) (Figure 32A and B) and ApoB protein expression was assessed and compared. Cell treatment in absence of TrivalentGalNAc-SPT001 show that the TrivalentGalNAc-SPT001 is highly effective (with a factor of over 1000 more potency) in reducing the ApoB protein expression in the primary hepatocytes compared to ApoB protein expression in the primary liver cells treated with TrivalentGalNAc-*Apo*BBNA only (Figure 32A). Neither TrivalentGaINAc-ApoBBNA, nor the combination of TrivalentGalNAc-ApoBBNA and TrivalentGalNAc-SPT001 induce lowering of the ApoB protein expression in these liver cells (Figure 32B).

All this shows that TrivalentGalNAc-SPT001 in combination with TrivalentGalNAc-*Apo*BBNA effectively induces silencing of ApoB protein expression in ASGPR1 expressing cells. Thus, TrivalentGalNAc-SPT001 effectively induces endosomal escape of a BNA in ASGPR1 expressing cells. Thus, low nM trivalentGalNAc-*Apo*BBNA + trivalentGalNAc-SPT001 can enhance endosomal escape and cytoplasmic delivery of ApoBBNA resulting in ApoB mRNA silencing in primary hepatocytes; Low nM trivalentGalNAc-ApoBBNA + trivalentGalNAc-SPT001 can enhance endosomal escape and cytoplasmic delivery of ApoBBNA resulting in ApoB protein decay in primary hepatocytes; Cells with lowASGPR1 expression are not responsive for the therapy when ApoB expression is considered.

### Materials and methods

### Abbreviations

- AEM: N-(2-Aminoethyl)maleimide trifluoroacetate salt
- AMPD: 2-Amino-2-methyl-1,3-propanediol
- BOP: (Benzotriazol-1-yloxy)tris(dimethylamino)phosphonium hexafluorophosphate
- DIPEA: N,N-diisopropylethylamine
- DMF: N,N-dimethylformamide
- EDCI.HCl: 3-((Ethylimino)methyleneamino)-N,N-dimethylpropan-1-aminium chloride
- EMCH.TFA: N-(ε-maleimidocaproic acid) hydrazide, trifluoroacetic acid salt
- HATU: 1-[Bis(dimethylamino)methylene]-1H-1,2,3-triazolo[4,5-b]pyridinium 3-oxid hexafluorophosphate
- min: minutes
- NMM: 4-Methylmorpholine
- r.t.: retention time
- TCEP: tris(2-carboxyethyl)phosphine hydrochloride
- Temp: temperature
- TFA: trifluoroacetic acid

### Analytical methods

### LC-MS method 1

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product: neg or neg/pos within in a range of 1500-2400 or 2000-3000; ELSD: gaspressure 40 psi, drift tube temp: 50°C; column: Acquity C18, 50×2.1 mm, 1.7 µm Temp: 60°C, Flow: 0.6 mL/min, lin. Gradient depending on the polarity of the product:
^{A}t₀ = 2% A, t_{5.0min} = 50% A, t_{6.0min} = 98% A
^{B}t₀ = 2% A, t_{5.0min} = 98% A, t_{6.0min} = 98% A
Posttime: 1.0 min, Eluent A: acetonitrile, Eluent B: 10 mM ammonium bicarbonate in water (pH=9.5).

### LC-MS method 2

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product: pos/neg 100-800 or neg 2000-3000; ELSD: gaspressure 40 psi, drift tube temp: 50 °C; column: Waters XSelect^{™} CSH C18, 50×2.1 mm, 2.5 µm, Temp: 25°C, Flow: 0.5 mL/min, Gradient: t₀ₘᵢₙ = 5% A, t_{2.0min} = 98% A, t_{2.7min} = 98% A, Posttime: 0.3 min, Eluent A: acetonitrile, Eluent B: 10 mM ammonium bicarbonate in water (pH = 9.5).

### LC-MS method 3

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product pos/neg 105-800, 500-1200 or 1500-2500; ELSD: gaspressure 40 psi, drift tube temp: 50 °C; column: Waters XSelect^{™} CSH C18, 50×2.1 mm, 2.5µm, Temp: 40 °C, Flow: 0.5 mL/min, Gradient: t₀ₘᵢₙ = 5% A, t_{2.0min} = 98% A, t_{2.7min}= 98% A, Posttime: 0.3 min, Eluent A: 0.1% formic acid in acetonitrile, Eluent B: 0.1% formic acid in water.

### LC-MS method 4

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product: pos/neg 100-800 or neg 2000-3000; ELSD: gaspressure 40 psi, drift tube temp: 50 °C column: Waters Acquity Shield RP18, 50×2.1 mm, 1.7µm, Temp: 25°C, Flow: 0.5 mL/min, Gradient: t₀ₘᵢₙ = 5% A, t_{2.0min} = 98% A, t_{2.7min}= 98% A, Posttime: 0.3 min, Eluent A: acetonitrile, Eluent B: 10 mM ammonium bicarbonate in water (pH=9.5).

### LC-MS method 5

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges: pos/neg 1500-2500; ELSD: gaspressure 40 psi, drift tube temp: 50 °C; column: Waters XSelect^{™} CSH C18, 50×2.1mm, 2.5µm, Temp: 25 °C, Flow: 0.6 mL/min, Gradient: t₀ₘᵢₙ = 5% A, t_{1.3mln} = 98% A, t_{1.7min} = 98% A, Posttime: 0.3 min, Eluent A: acetonitrile, Eluent B: 10 mM ammonium bicarbonate in water (pH=9.5).

### Preparative methods

### Preparative MP-LC method 1

Instrument type: Reveleris^{™} prep MPLC; column: Waters XSelect^{™} CSH C18 (145×25 mm, 10µm); Flow: 40 mL/min; Column temp: room temperature; Eluent A: 10 mM ammoniumbicarbonate in water pH = 9.0); Eluent B: 99% acetonitrile + 1% 10 mM ammoniumbicarbonate in water; Gradient:
^{A}t₀ₘᵢₙ = 5% B, t₁ₘᵢₙ = 5% B, t₂ₘᵢₙ = 10% B, t₁₇ₘᵢₙ = 50% B, t₁₈ₘᵢₙ = 100% B, t₂₃ₘᵢₙ = 100% B
^{A}t₀ₘᵢₙ = 5% B, t₁ₘᵢₙ = 5% B, t₂ₘᵢₙ = 20% B, t₁₇ₘᵢₙ = 60% B, t₁₈ₘᵢₙ = 100% B, t₂₃ₘᵢₙ = 100% B
; Detection UV: 210, 235, 254 nm and ELSD.

### Preparative MP-LC method 2

Instrument type: Reveleris^{™} prep MPLC; Column: Phenomenex LUNA C18(3) (150×25 mm, 10µm); Flow: 40 mL/min; Column temp: room temperature; Eluent A: 0.1% (v/v) Formic acid in water, Eluent B: 0.1% (v/v) Formic acid in acetonitrile; Gradient:
^{A}t₀ₘᵢₙ = 5% B, t₁ₘᵢₙ = 5% B, t₂ₘᵢₙ = 20% B, t₁₇ₘᵢₙ = 60% B, t₁₈ₘᵢₙ = 100% B, t₂₃ₘᵢₙ = 100% B
^{B}t₀ₘᵢₙ = 2% B, t₁ₘᵢₙ = 2% B, t₂ₘᵢₙ = 2% B, t₁₇ₘᵢₙ = 30% B, t₁₈ₘᵢₙ = 100% B, t₂₃ₘᵢₙ = 100% B
^{C}t₀ₘᵢₙ = 5% B, t₁ₘᵢₙ = 5% B, t₂ₘᵢₙ = 10% B, t₁₇ₘᵢₙ = 50% B, t₁₈ₘᵢₙ = 100% B, t₂₃ₘᵢₙ = 100% B
^{B}t₀ₘᵢₙ = 5% B, t₁ₘᵢₙ = 5% B, t₂ₘᵢₙ = 5% B, t₁₇ₘᵢₙ = 40% B, t₁₈ₘᵢₙ = 100% B, t₂₃ₘᵢₙ = 100% B
; Detection UV: 210, 235, 254 nm and ELSD.

### Preparative LC-MS method 3

MS instrument type: Agilent Technologies G6130B Quadrupole; HPLC instrument type: Agilent Technologies 1290 preparative LC; Column: Waters XSelect^{™} CSH (C18, 150×19mm, 10µm); Flow: 25 ml/min; Column temp: room temperature; Eluent A: 100% acetonitrile; Eluent B: 10 mM ammonium bicarbonate in water pH=9.0; Gradient:
^{A}t₀ = 20% A, t_{2.5min} = 20% A, t₁₁ₘᵢₙ = 60% A, t₁₃ₘᵢₙ = 100% A, t₁₇ₘᵢₙ = 100% A
^{B}t₀ = 5% A, t_{2.5min} = 5% A, t₁₁ₘᵢₙ= 40% A, t₁₃ₘᵢₙ = 100% A, t₁₇ₘᵢₙ= 100% A
; Detection: DAD (210 nm); Detection: MSD (ESI pos/neg) mass range: 100 - 800; Fraction collection based on DAD.

### Preparative LC-MS method 4

MS instrument type: Agilent Technologies G6130B Quadrupole; HPLC instrument type: Agilent Technologies 1290 preparative LC; Column: Waters XBridge Protein (C4, 150×19mm, 10µm); Flow: 25 ml/min; Column temp: room temperature; Eluent A: 100% acetonitrile; Eluent B: 10 mM ammonium bicarbonate in water pH=9.0; Gradient:
^{A}t₀ = 2% A, t_{2.5min} = 2% A, t₁₁ₘᵢₙ= 30% A, t₁₃ₘᵢₙ = 100% A, t₁₇ₘᵢₙ = 100% A
^{B}t₀ = 10% A, t_{2.5min} = 10% A, t₁₁ₘᵢₙ = 50% A, t₁₃ₘᵢₙ = 100% A, t₁₇ₘᵢₙ = 100% A
^{C}t₀ = 5% A, t_{2.5min} = 5% A, t₁₁ₘᵢₙ = 40% A, t₁₃ₘᵢₙ = 100% A, t₁₇ₘᵢₙ = 100% A
; Detection: DAD (210 nm); Detection: MSD (ESI pos/neg) mass range: 100 - 800; Fraction collection based on DAD

### Preparative LC-MS method 1,

MS instrument type: Agilent Technologies G6130B Quadrupole; HPLC instrument type: Agilent Technologies 1290 preparative LC; Column: Waters XBridge Protein (C4, 150×19mm, 10µm); Flow: 25 ml/min; Column temp: room temperature; Eluent A: 100% acetonitrile; Eluent B: 10 mM ammonium bicarbonate in water pH=9.0; Gradient:
^{A}t₀ = 2% A, t_{2.5min} = 2% A, t₁₁ₘᵢₙ**=** 30% A, t₁₃ₘᵢₙ = 100% A, t₁₇ₘᵢₙ = 100% A
^{B}t₀ = 10% A, t_{2.5min} = 10% A, t₁₁ₘᵢₙ = 50% A, t₁₃ₘᵢₙ = 100% A, t₁₇ₘᵢₙ = 100% A
; Detection: DAD (210 nm); Detection: MSD (ESI pos/neg) mass range: 100 - 800; Fraction collection based on DAD

### Preparative MP-LC method 1,

Instrument type: Reveleris^{™} prep MPLC; Column: Phenomenex LUNA C18(3) (150×25 mm, 10µm); Flow: 40 mL/min; Column temp: room temperature; Eluent A: 0.1% (v/v) Formic acid in water, Eluent B: 0.1% (v/v) Formic acid in acetonitrile; Gradient:
^{A}t₀ₘᵢₙ = 2% B, t₁ₘᵢₙ = 2% B, t₂ₘᵢₙ = 2% B, t₁₇ₘᵢₙ = 30% B, t₁₈ₘᵢₙ = 100% B, t₂₃ₘᵢₙ = 100% B
^{B}t₀ₘᵢₙ = 5% B, t₁ₘᵢₙ = 5% B, t₂ₘᵢₙ = 5% B, t₁₇ₘᵢₙ = 40% B, t₁₈ₘᵢₙ = 100% B, t₂₃ₘᵢₙ = 100% B
^{C}t₀ₘᵢₙ = 5% B, t₁ₘᵢₙ = 5% B, t₂ₘᵢₙ = 10% B, t₁₇ₘᵢₙ = 50% B, t₁₈ₘᵢₙ = 100% B, t₂₃ₘᵢₙ = 100% B
; Detection UV: 210, 235, 254 nm and ELSD.

### Flash chromatography

Grace Reveleris X2^{®} C-815 Flash; Solvent delivery system: 3-piston pump with auto-priming, 4 independent channels with up to 4 solvents in a single run, auto-switches lines when solvent depletes; maximum pump flow rate 250 mL/min; maximum pressure 50bar (725psi); Detection: UV 200-400nm, combination of up to 4 UV signals and scan of entire UV range, ELSD; Column sizes: 4-330g on instrument, luer type, 750g up to 3000g with optional holder.

### SO1861-EMCH synthesis (SO1861-EMCH is also referred to as SO1861-Ald-EMCH)

To SO1861 (121 mg, 0.065 mmol) and EMCH.TFA (110 mg, 0.325 mmol) was added methanol (extra dry, 3.00 mL) and TFA (0.020 mL, 0.260 mmol). The reaction mixture stirred at room temperature. After 1.5 hours the reaction mixture was subjected to preparative MP-LC. Fractions corresponding to the product were immediately pooled together, frozen and lyophilized overnight to give the title compound (120 mg, 90%) as a white fluffy solid. Purity based on LC-MS 96%.
LRMS (m/z): 2069 [M-1]¹⁻
LC-MS r.t. (min): 1.08⁴

### SO1861-EMCH-mercaptoethanol (SO1861-EMCH (blocked))

To SO1861-EMCH (0.1 mg, 48 nmol) 200 µL mercaptoethanol (18 mg, 230 µmol) was added and the solution was shaken for 1 h at 800 rpm and room temperature on a ThermoMixer C (Eppendorf). After shaking for 1 h, the solution was diluted with methanol and dialyzed extensively for **4** h against methanol using regenerated cellulose membrane tubes (Spectra/Por 7) with a MWCO of 1 kDa. After dialysis, an aliquot was taken out and analyzed via MALDI-TOF-MS.

MALDI-TOF-MS (RP mode): *m*/*z* 2193 Da ([M+K]⁺, SO1861-EMCH-mercaptoethanol), *m*/*z* 2185 Da ([M+K]⁺, SO1861-EMCH-mercaptoethanol), *m*/*z* 2170 Da ([M+Na]⁺, SO1861-EMCH-mercaptoethanol).

### Trivalent GalNAc-azide synthesis

### Intermediate 1:

### tert-butyl 1-azido-17,17-bis((3-(tert-butoxy)-3-oxopropoxy)methyl)-15-oxo-3,6,9,12,19-pentaoxa-16-azadocosan-22-oate

To di-tert-butyl 3,3'-((2-amino-2-((3-(tert-butoxy)-3-oxopropoxy)methyl)propane-1,3-diyl)bis(oxy))dipropionate (1.27 g, 2.51 mmol) was added a solution of 3-Azido(peg4)propionic acid N-hydroxysuccinimide ester (977 mg, 2.51 mmol) in DMF (10 mL). Next, DIPEA (657 µL, 3.77 mmol) was added and the reaction mixture was stirred overnight at room temperature. The reaction mixture was evaporated *in vacuo* and the residue was dissolved in ethyl acetate (100 mL). The resulting solution was washed with 0.5 N potassium bisulphate solution (2 × 100 mL) and brine (100 mL), dried over Na₂SO₄, filtered and evaporated in vacuo. The residue was purified by flash chromatography (DCM - 10% methanol in DCM (v/v) gradient 100:0 rising to 0:100) to give the title compound (1.27 g, 65%) as a colorless oil. Purity based on LC-MS 100% (ELSD).
LRMS (m/z): 780 [M+1]¹⁺
LC-MS r.t. (min): 2.10²

### Intermediate 2:

### 1-azido-17,17-bis((2-carboxyethoxy)methyl)-15-oxo-3,6,9,12,19-pentaoxa-16-azadocosan-22-oic acid

To a solution of tert-butyl 1-azido-17,17-bis((3-(tert-butoxy)-3-oxopropoxy)methyl)-15-oxo-3,6,9,12,19-pentaoxa-16-azadocosan-22-oate (1.27 g, 1.63 mmol) in DCM (5.0 mL) was added TFA (5.0 mL, 65 mmol). The reaction mixture was stirred at room temperature. After 1.5 hours the reaction mixture was evaporated *in vacuo,* co-evaporated with toluene (3 × 10 mL) and DCM (3 × 10 mL) to give the crude title product as a colorless oil.
LRMS (m/z): 611 [M+1]¹⁺

### Intermediate 3:

### di-tert-butyl (10-(1-azido-3,6,9,12-tetraoxapentadecan-15-amido)-10-(13,13-dimethyl-5,11-dioxo-2,12-dioxa-6,10-diazatetradecyl)-5,15-dioxo-8,12-dioxa-4,16-diazanonadecane-1,19-diyl)dicarbamate

1-azido-17,17-bis((2-carboxyethoxy)methyl)-15-oxo-3,6,9,12,19-pentaoxa-16-azadocosan-22-oic acid (997 mg, 1.63 mmol), Oxyma Pure (1.04 g, 7.35 mmol) and EDCI.HCl (1.17 g, 6.12 mmol) were dissolved in DMF (10.0 mL). Next, DIPEA (1.99 mL, 11.4 mmol) was added, followed directly by the addition of a solution of N-BOC-1,3-propanediamine (1.07 g, 6.12 mmol) in DMF (10.0 mL). The reaction mixture was stirred overnight at room temperature. The reaction mixture was evaporated *in vacuo* and the residue was dissolved in ethyl acetate (100 mL). The resulting solution was washed with 0.5 **N** potassium bisulphate solution (100 mL), saturated sodium bicarbonate solution (2 × 100 mL) and brine (100 mL), dried over Na₂SO₄, filtered and evaporated *in vacuo.* The residue was purified by flash chromatography (DCM - 10% methanol in DCM (v/v) gradient 0:100 rising to 100:0, staying at 100:0 until the product eluted) to give the title compound (1.16 g, 66%) as a yellowish viscous oil. LC-MS 99% (ELSD).
LRMS (m/z): 1080 [M+1]¹⁺
LC-MS r.t. (min): 1.51³

### Intermediate 4:

### 3,3'-((2-((3-((3-aminopropyl)amino)-3-oxopropoxy)methyl)-2-(1-azido-3,6,9,12-tetraoxapentadecan-15-amido)propane-1,3-diyl)bis(oxy))bis(N-(3-aminopropyl)propanamide) tris(2,2,2-trifluoroacetate) synthesis

To a solution of di-tert-butyl (10-(1-azido-3,6,9,12-tetraoxapentadecan-15-amido)-10-(13,13-dimethyl-5,11-dioxo-2,12-dioxa-6,10-diazatetradecyl)-5,15-dioxo-8,12-dioxa-4,16-diazanonadecane-1,19-diyl)dicarbamate (1.16 g, 1.08 mmol) in DCM (10 mL) was added TFA (10 mL, 131 mmol). The reaction mixture was stirred at room temperature. After 2 hours the reaction mixture was evaporated *in vacuo,* co-evaporated with toluene (3 × 10 mL) and DCM (3 × 10 mL) to give the crude title product as a yellowish viscous oil.
LRMS (m/z): 260 [M+3]³⁺, 390 [M+2]²⁺, 780 [M+1]¹⁺,

### Intermediate 5:

### (2R,3R,4R,5R,6R)-5-acetamido-2-(acetoxymethyl)-6-((5-((2,5-dioxopyrrolidin-1-yl)oxy)-5-oxopentyl)oxy)tetrahydro-2H-pyran-3,4-diyl diacetate

5-(((2R,3R,4R,5R,6R)-3-acetamido-4,5-diacetoxy-6-(acetoxymethyl)tetrahydro-2H-pyran-2-yl)oxy)pentanoic acid (obtain according J. Am. Chem Soc., 2014, 136, 16958-16961, 3.00 g, 6.70 mmol) and N-Hydroxysuccinimide (926 mg, 8.05 mmol) were dissolved in DCM (50 mL). Next, EDCI.HCl (1.54 g, 8.05 mmol) and 4-(Dimethylamino)pyridine (82 mg, 0.67 mmol) were added and the reaction mixture was stirred overnight at room temperature. The reaction mixture was diluted with DCM and the resulting solution was washed with 0.5 N potassium bisulphate solution (150 mL), saturated sodium bicarbonate solution (150 mL) and brine (150 mL), dried over Na₂SO₄, filtered and evaporated *in vacuo* to give the title compound (3.60 g, 99%) as a white foam. Purity based on LC-MS 99% (ELSD).
LRMS (m/z): 545 [M+1]¹⁺
LC-MS r.t. (min): 1.07³

### Intermediate 6:

### [(3R,6R)-3,4-bis(acetyloxy)-6-{4-[(3-{3-[2-(1-azido-3,6,9,12-tetraoxapentadecan-15-amido)-3-(2-{[3-(5-{[(2R,5R)-4,5-bis(acetyloxy)-6-[(acetyloxy)methyl]-3-acetamidooxan-2-yl]oxy}pentanamido)propyl]carbamoyl}ethoxy)-2-[(2-{[3-(5-{[(2R,5R)-4,5-bis(acetyloxy)-6-[(acetyloxy)methyl]-3-acetamidooxan-2-yl]oxy}pentanamido)propyl]carbamoyl}ethoxy)methyl]propoxy]propanamido}propyl)carbamoyl ]butoxy}-5-acetamidooxan-2-yl]methyl acetate

3,3'-((2-((3-((3-aminopropyl)amino)-3-oxopropoxy)methyl)-2-(1-azido-3,6,9,12-tetraoxapentadecan-15-amido)propane-1,3-diyl)bis(oxy))bis(N-(3-aminopropyl)propanamide) tris(2,2,2-trifluoroacetate) (1.21 g, 1.08 mmol) was dissolved in a mixture of DMF (10 mL) and DIPEA (1.69 mL, 9.70 mmol). Next, (2R,3R,4R,5R,6R)-5-acetamido-2-(acetoxymethyl)-6-((5-((2,5-dioxopyrrolidin-1-yl)oxy)-5-oxopentyl)oxy)tetrahydro-2H-pyran-3,4-diyl diacetate (2.20 g, 4.04 mmol) was added and the reaction mixture was stirred over the weekend at room temperature. Next, the reaction mixture was evaporated *in vacuo* and the residue was purified by flash chromatography (DCM - 30% methanol in DCM (v/v) gradient 0:100 rising to 100:0) to give the title compound (1.84 g, 83%) as a yellowish foam. LC-MS 95% (ELSD).
LRMS (m/z): 2068 [M+1]¹⁺
LC-MS r.t. (min): 1.18³

### Intermediate 7:

### Trivalent GalNAc-azlde

[(3R,6R)-3,4-bis(acetyloxy)-6-{4-[(3-{3-[2-(1-azido-3,6,9,12-tetraoxapentadecan-15-amido)-3-(2-{[3-(5-{[(2R,5R)-4,5-bis(acetyloxy)-6-[(acetyloxy)methyl]-3-acetamidooxan-2-yl]oxy)pentanamido)propyl]carbamoyl)ethoxy)-2-[(2-f[3-(5-{[(2R,5R)-4,5-bis(acetyloxy)-6-[(acetyloxy)methyl]-3-acetamidooxan-2-yl]oxy}pentanamido)propyl]carbamoyl}ethoxy)methyl]propoxy]propanamido}propyl)carbamoyl]butoxy}-5-acetamidooxan-2-yl]methyl acetate (300 mg, 0.145 mmol) was dissolved in a mixture of triethylamine (2.00 mL, 14.4 mmol), methanol (2.00 mL) and water (2.00 mL) and the reaction mixture was stirred at room temperature. After 2 hours the reaction mixture was evaporated *in vacuo.* The residue was purified by preparative MP-LC.^{2B} Fractions corresponding to the product were immediately pooled together, frozen and lyophilized overnight to give the title compound (164 mg, 67%) as a white solid. Purity based on LC-MS 97%.
LRMS (m/z): 1688 [M-1]¹⁻
LC-MS r.t. (min): 1.99^{1A}

### Trivalent-GalNAc-SO1861 and GalNAc-SO1861 synthesis (Figure 2, 4)

### Intermediate 8:

### SO1861-NH₂

SO1861-azide (6.89 mg, 3.20 µmol) was dissolved in mixture of 32 mM potassium carbonate (150 µL, 4.80 µmol) and acetonitrile (150 µL). Directly afterwards, a 1.0 M trimethylphosphine solution in THF (32 µL, 32 µmol) was added and the resulting mixture was shaken for 1 min and left standing at room temperature. After 30 min. the reaction mixture was subjected to preparative MP-LC. Fractions corresponding to the product were immediately pooled together, frozen and lyophilized overnight to give the title compound (5.30 mg, 78%) as a white fluffy solid. Purity based on LC-MS 94%.
LRMS (m/z): 1062 [M-2]²⁻
LC-MS r.t. (min): 2.51^{1B}

### Intermediate 9:

### SO1861-DBCO

To SO1861-amine (48.6 mg, 22.9 µmol) and DBCO-NHS (13.0 mg, 32.4 µmol) was added to a solution of DIPEA (5.98 µL, 34.3 µmol) and DMF (2.0 mL). The reaction mixture was shaken for 1 min and left standing at room temperature. After 4 hours the reaction mixture was diluted with a solution of 50 mM sodium bicarbonate (1.00 mL, 50 µmol). The resulting mixture was shaken for 1 min and left standing at room temperature. After 30 min the reaction mixture was subjected to preparative MP-LC.^{1B} Fractions corresponding to the product were immediately pooled together, frozen and lyophilized overnight to give the title compound (16.9 mg, 31%) as a white fluffy solid. Purity based on LC-MS 94%.
LRMS (m/z): 2412 [M-1]¹⁻
LC-MS r.t. (min): 2.45^{1B}

### SO1861-L-trivalent GalNAc synthesis

SO1861-DBCO (7.50 mg, 3.11 µmol) and trivalent GalNAc-azide (5.31 mg, 3.14 µmol) were dissolved in a mixture of water/acetonitrile (2:1, v/v, 0.90 mL). The reaction mixture was shaken for 1 min and left standing at room temperature. After 2 hours the reaction mixture was subjected to preparative LC-MS.^{3B} Fractions corresponding to the product were immediately pooled together, frozen and lyophilized overnight to give the title compound (9.60 mg, 75%) as a white fluffy solid. Purity based on LC-MS 99%.
LRMS (m/z): 2050 [M-2]²⁻
LC-MS r.t. (min): 2.04^{1B}

### SO1861-L-GalNAc synthesis

SO1861-DBCO (1.75 mg, 0.73 µmol) and GalNAc-PEG3-azide (0.82 mg, 2.18 µmol) were dissolved in a mixture of water/acetonitrile (1:1, v/v, 1.00 mL). The reaction mixture was shaken for 1 min and left standing at room temperature. After 2 hours the reaction mixture was subjected to preparative LC-MS.^{3B} Fractions corresponding to the product were immediately pooled together, frozen and lyophilized overnight to give the title compound (1.63 mg, 81%) as a white fluffy solid. Purity based on LC-MS 100%.
LRMS (m/z): 2790 [M-1]¹⁻
LC-MS r.t. (min): 2.15^{1B}

### Trivalent GalNAc-BNA oligo synthesis (Figure 6, 7)

### Intermediate 10:

### 4-{2-azatricyclo[10.4.0.0^{4,9}]hexadeca-1(12),4(9),5,7,13,15-hexaen-10-yn-2-yl}-N-[2-(2-{2-[2-({4-[(E)-{[6-(2,5-dioxo-2,5-dihydro-1H-pyrrol-1-yl)hexanamido]imino}methyl]phenyl}formamido)ethoxy]ethoxy}ethoxy)ethyl]-4-oxobutanamide

4-(2-azatricyclo[10.4.0.0^{4,9}]hexadeca-1(12),4(9),5,7,13,15-hexaen-10-yn-2-yl)-N-{2-[2-(2-{2-[(4-formylphenyl)formamido]ethoxy}ethoxy)ethoxy]ethyl}-4-oxobutanamide (25.0 mg, 40.9 µmol) and EMCH.TFA (20.8 mg, 61.3 µmol) were dissolved in methanol (extra dry, 2.00 mL). Next, TFA (9.39 µL, 123 µmol) was added. The reaction mixture was shaken for 1 min and left standing overnight at room. The reaction mixture was subjected to preparative MP-LC.^{1B} Fractions corresponding to the product were immediately pooled together, frozen and lyophilized overnight to give the title compound (16.2 mg, 48%) as a white solid. Purity based on LC-MS 91%.
LRMS (m/z): 410.2 [M+2]²⁺
LC-MS r.t. (min): 1.41²

### Intermediate 11:

### Trivalent GalNAc-L-maleimide

Trivalent GalNAc-azide (20.3 mg, 12.0 µmol) and 4-{2-azatricyclo[10.4.0.0^{4,9}]hexadeca-1(12),4(9),5,7,13,15-hexaen-10-yn-2-yl}-N-[2-(2-{2-[2-({4-[(E)-{[6-(2,5-dioxo-2,5-dihydro-1H-pyrrol-1-yl)hexanamido]imino}methyl]phenyl}formamido)ethoxy]ethoxy}ethoxy)ethyl]-4-oxobutanamide (11.8 mg, 14.4 µmol) were dissolved in a mixture of water/acetonitrile (2:1, v/v, 0.90 mL). The reaction mixture was shaken for 1 min and left standing at room temperature. After 2 hours the reaction mixture was subjected to preparative MP-LC.^{2C} Fractions corresponding to the product were immediately pooled together, frozen and lyophilized overnight to give the title compound (25.6 mg, 85%) as a white solid. Purity based on LC-MS 88%.
LRMS (m/z): 2101 [M-405]¹⁻, 2304 [M-202]¹⁻, 2507 [M-1]¹⁻
LC-MS r.t. (min): 1.90^{1B} (double peaks due to isomers)

### Intermediate 12:

### Trivalent GalNAc-S-maleimide

Trivalent GalNAc-azide (20.3 mg, 12.0 µmol) and DBCO-maleimide (10.3 mg, 24.0 µmol) were dissolved in a mixture of water/acetonitrile (2:1, v/v, 0.90 mL). The reaction mixture was shaken for 1 min and left standing at room temperature. After 2 hours the reaction mixture was subjected to preparative MP-LC.^{2D} Fractions corresponding to the product were immediately pooled together, frozen and lyophilized overnight to give the title compound (22.2 mg, 87%) as a white solid. Purity based on LC-MS 85%. Contains 10% of the hydrolysed maleimide.
LRMS (m/z): 2115 [M-1]¹⁻
LC-MS r.t. (min): 1.60^{1B} (double peaks due to isomers)

### Trivalent GalNAc-S-ApoB (or HSP27) BNA oligo synthesis

To ApoB BNA oligo disulfide (5.00 mg, 0.686 µmol) was added a solution of 20 mM ammonium bicarbonate with 2.5 mM TCEP (1.00 mL, 2.5 µmol). The reaction mixture was shaken for 1 min and left standing at room temperature. After 1 hour the reaction mixture was filtered by using a centrifugal filter with a molecular weight cut-off of 3000 Da (5000 × g for 30 min, 2 × 0.50 mL ). Next, the residue solution was washed twice with a solution of 20 mM ammonium bicarbonate with 2.5 mM TCEP (0.50 mL), each time filtered under the same conditions described above. As next, the residue solution was diluted with 20 mM ammonium bicarbonate/acetonitrile (3:1, v/v, 1.00 mL) and the resulting solution was directly added to trivalent GalNAc-S-maleimide (3.02 mg, 1.43 µmol). The reaction mixture was shaken for 1 min and left standing at room temperature. After 1.5 hours the reaction mixture was subjected to preparative LC-MS.^{4A} Fractions corresponding to the product were immediately pooled together, frozen and lyophilized overnight to give the title compound (6.75 mg, quant.) as a white fluffy solid. Purity based on LC-MS 94% (very broad peak).
LRMS (m/z): 2318 [M-4]⁴⁻
LC-MS r.t. (min): 1.65^{1A}

### Trivalent GalNAc-S-ApoB (or HSP27) scrambled BNA oligo synthesis

To ApoB scrambled BNA oligo disulfide (5.00 mg, 0.688 µmol) was added a solution of 20 mM ammonium bicarbonate with 2.5 mM TCEP (1.00 mL, 2.5 µmol). The reaction mixture was shaken for 1 min and left standing at room temperature. After 1 hour the reaction mixture was filtered by using a centrifugal filter with a molecular weight cut-off of 3000 Da (5000 × g for 30 min, 2 × 0.50 mL ). Next, the residue solution was washed twice with a solution of 20 mM ammonium bicarbonate with 2.5 mM TCEP (0.50 mL), each time filtered under the same conditions described above. As next, the residue solution was diluted with 20 mM ammonium bicarbonate/acetonitrile (3:1, v/v, 1.00 mL) and the resulting solution was directly added to trivalent GaINAc-S-maleimide (3.09 mg, 1.46 µmol). The reaction mixture was shaken for 1 min and left standing at room temperature. After 1.5 hours the reaction mixture was subjected to preparative LC-MS.^{4A} Fractions corresponding to the product were immediately pooled together, frozen and lyophilized overnight to give the title compound (5.91 mg, 93%) as a white fluffy solid. Purity based on LC-MS 88% (very broad peak).
LRMS (m/z): 2311 [M-4]⁴⁻
LC-MS r.t. (min): 1.60^{1A}

### Trivalent GalNAc-L-ApoB (or HSP27) BNA oligo synthesis

To ApoB BNA oligo disulfide (5.00 mg, 0.686 µmol) was added a solution of 20 mM ammonium bicarbonate with 2.5 mM TCEP (1.00 mL, 2.5 µmol). The reaction mixture was shaken for 1 min and left standing at room temperature. After 1 hour the reaction mixture was filtered by using a centrifugal filter with a molecular weight cut-off of 3000 Da (5000 × g for 30 min, 2 × 0.50 mL ). Next, the residue solution was washed twice with a solution of 20 mM ammonium bicarbonate with 2.5 mM TCEP (0.50 mL), each time filtered under the same conditions described above. As next, the residue solution was diluted with 20 mM ammonium bicarbonate/acetonitrile (3:1, v/v, 1.00 mL) and the resulting solution was directly added to trivalent GalNAc-L-maleimide (3.63 mg, 1.45 µmol). The reaction mixture was shaken for 1 min and left standing at room temperature. After 1.5 hours the reaction mixture was subjected to preparative LC-MS.^{4A} Fractions corresponding to the product were immediately pooled together, frozen and lyophilized overnight to give the title compound (6.68 mg, quant.) as a white fluffy solid. Purity based on LC-MS 99% (very broad peak).
LRMS (m/z): 2416 [M-4]⁴⁻
LC-MS r.t. (min): 1.97^{1A}

### Trivalent GalNAc-L-ApoB (or HSP27) scrambled BNA oligo synthesis

To ApoB scrambled BNA oligo disulfide (5.00 mg, 0.688 µmol) was added a solution of 20 mM ammonium bicarbonate with 2.5 mM TCEP (1.00 mL, 2.5 µmol). The reaction mixture was shaken for 1 min and left standing at room temperature. After 1 hour the reaction mixture was filtered by using a centrifugal filter with a molecular weight cut-off of 3000 Da (5000 × g for 30 min, 2 × 0.50 mL ). Next, the residue solution was washed twice with a solution of 20 mM ammonium bicarbonate with 2.5 mM TCEP (0.50 mL), each time filtered under the same conditions described above. As next, the residue solution was diluted with 20 mM ammonium bicarbonate/acetonitrile (3:1, v/v, 1.00 mL) and the resulting solution was directly added to trivalent GalNAc-L-maleimide (3.68 mg, 1.47 µmol). The reaction mixture was shaken for 1 min and left standing at room temperature. After 1.5 hours the reaction mixture was subjected to preparative LC-MS.^{4A} Fractions corresponding to the product were immediately pooled together, frozen and lyophilized overnight to give the title compound (4.71 mg, 71%) as a white fluffy solid. Purity based on LC-MS 96% (very broad peak).
LRMS (m/z): 2409 [M-4]⁴⁻
LC-MS r.t. (min): 1.93^{1A}

### Dendron(-L-SO1861)₄-trivalent GalNAc and dendron(-L-SO1861)₈-trivalent GalNAc synthesis

### Intermediate 13:

### Trivalent GalNAc-amine formate

Trivalent GalNAc-azide (36.5 mg, 21.6 µmol) was dissolved in a solution of potassium carbonate (5.97 mg, 43.2 µmol) in water (1.00 mL) and acetonitrile (1.00 mL). Next, a 1.0 M trimethylphosphine solution in THF (216 µL, 216 µmol) was added and the resulting mixture was shaken for 1 min and left standing at room temperature. After 45 min the reaction mixture was evaporated *in vacuo* and the residue was dissolved in water/acetonitrile (9:1, v/v, 1 mL). The resulting solution was directly subjected to preparative MP-LC.^{2B} Fractions corresponding to the product were immediately pooled together, frozen and lyophilized overnight to give the title compound (36.1 mg, 98%) as a white solid. Purity based on LC-MS 100%.
LRMS (m/z): 1662 [M-1]¹⁻
LC-MS r.t. (min): 1.62^{1A}

### Intermediate 14:

### Trivalent GalNAc-DBCO

Trivalent GalNAc-amine formate (17.4 mg, 10.2 µmol) and DBCO-NHS (6.14 mg, 15.3 µmol) were dissolved in a solution of NMM (2.24 µL, 20.3 µmol) in DMF (0.50 mL). The reaction mixture was shaken for 1 min and left standing at room temperature. After 2 hours the reaction mixture was evaporated *in vacuo* and the residue was dissolved in water/acetonitrile (8:2, v/v, 1 mL). The resulting solution was directly subjected to preparative MP-LC.^{2C} Fractions corresponding to the product were immediately pooled together, frozen and lyophilized overnight to give the title compound (14.2 mg, 72%) as a white solid. Purity based on LC-MS 96%.
LRMS (m/z): 1950 [M-1]¹
LC-MS r.t. (min): 1.86^{1B}

### Intermediate 15:

### dendron(-L-SO1861)₈-azide

Dendron(-L-SO1861)₈-amine (19.6 mg, 1.08 µmol) and 2,5-dioxopyrrolidin-1-yl 1-azido-3,6,9,12-tetraoxapentadecan-15-oate (4.17 mg, 10.8 µmol) were dissolved in DMF (1.50 mL). Next, DIPEA (1.87 µL, 10.8 µmol) was added and the mixture was shaken for 1 min and left standing overnight at room temperature. The reaction mixture was subjected to preparative LC-MS.^{4B} Fractions corresponding to the product were immediately pooled together, frozen and lyophilized overnight to give the title compound (11.7 mg, 59%) as a white fluffy solid. Purity based on LC-MS 92% (very broad peak).
LRMS (m/z): 2316 [M-8]⁸⁻, ): 2647 [M-7]⁷⁻
LC-MS r.t. (min): 4.29^{1A}

### Dendron(-L-SO1861)₄-trivalent GalNAc synthesis

Dendron(-L-SO1861)₄-azide (2.50 mg, 0.266 µmol) and trivalent GalNAc-DBCO (1.56 mg, 0.799 µmol) were dissolved in a mixture of water/acetonitrile (3:1, v/v, 1.00 mL). The reaction mixture was shaken for 1 min and left standing at room temperature. After 2 hours the reaction mixture was subjected to preparative LC-MS.^{4B} Fractions corresponding to the product were immediately pooled together, frozen and lyophilized overnight to give the title compound (2.74 mg, 91%) as a white fluffy solid. Purity based on LC-MS 86% (very broad peak).
LRMS (m/z): 2832 [M-4]⁴⁻
LC-MS r.t. (min): 4.07^{1A}

### Dendron(-L-SO1861)₈-trivalent GalNAc synthesis

Dendron(-L-SO1861)₈-azide (2.50 mg, 0.135 µmol) and trivalent GalNAc-DBCO (0.79 mg, 0.405 µmol) were dissolved in a mixture of water/acetonitrile (3:1, v/v, 1.00 mL). The reaction mixture was shaken for 1 min and left standing at room temperature. After 2 hours the reaction mixture was subjected to preparative LC-MS.^{4B} Fractions corresponding to the product were immediately pooled together, frozen and lyophilized overnight to give the title compound (2.03 mg, 74%) as a white fluffy solid. Purity based on LC-MS 100% (very broad peak).
LRMS (m/z): 2559 [M-8]⁸⁻, 2925 [M-7]⁷⁻
LC-MS r.t. (min): 4.18^{1A}

### Dendron(-L-SO1861)₄-L-BNA oligo-trivalent GalNAc and Dendron(-L-SO1861)₈-L-BNA oligo-trivalent GalNAc synthesis

### Intermediate 16:

### Trivalent GalNAc-thloacetate

Trivalent GalNAc-amine formate (18.7 mg, 11.0 µmol) and 4-nitrophenyl 3-(acetylthio)propanoate (5.90 mg, 21.9 µmol) were dissolved in a solution of NMM (2.41 µL, 21.9 µmol) in DMF (0.50 mL). The reaction mixture was shaken for 1 min and left standing at room temperature. After 2 hours the reaction mixture was evaporated *in vacuo* and the residue was dissolved in a mixture of 0.1% formic acid in water and 0.1% formic acid in acetonitrile (9:1, v/v, 1 mL). The resulting solution was directly subjected to preparative MP-LC.^{2B} Fractions corresponding to the product were immediately pooled together, frozen and lyophilized overnight to give the title compound (13.0 mg, 66%) as a white solid. Purity based on LC-MS 100%.
LRMS (m/z): 1749 [M-43]¹⁻, 1792 [M-1]¹
LC-MS r.t. (min): 1.24^{1B}

### Intermediate 17:

### DBCO-TCO-trivalent GalNAc

Trivalent GalNAc-thioacetate (13.0 mg, 7.25 µmol) was dissolved in methanol (0.50 mL). Next, a 1 M solution of sodium hydroxide (7.98 µL, 7.98 µmol) was added. The reaction mixture was shaken for 1 min and left standing at room temperature. After 30 min the reaction mixture was added to a freshly prepared solution of trifunctional linker (Ls-t) (i.e., is an example of a saponin moiety linker Ls) (7.39 mg, 6.13 µmol) in 20 mM ammonium bicarbonate/acetonitrile (3:1, v/v, 2.00 mL).

The trifunctional linker has the IUPAC name: 5,8,11,18,21,24,27-Heptaoxa-2,14,30-triazatritriacontanoic acid, 14-[16-(11,12-didehydrodibenz[*b,f*]azocin-5(6*H*)-yl)-13,16-dioxo-3,6,9-trioxa-12-azahexadec-1-yl]-33-(2,5-dihydro-2,5-dioxo-1*H*-pyrrol-1-yl)-15,31-dioxo-, (1R,4E)-4-cycloocten-1-yl ester. The trifunctional linker has the following formula (XXI):

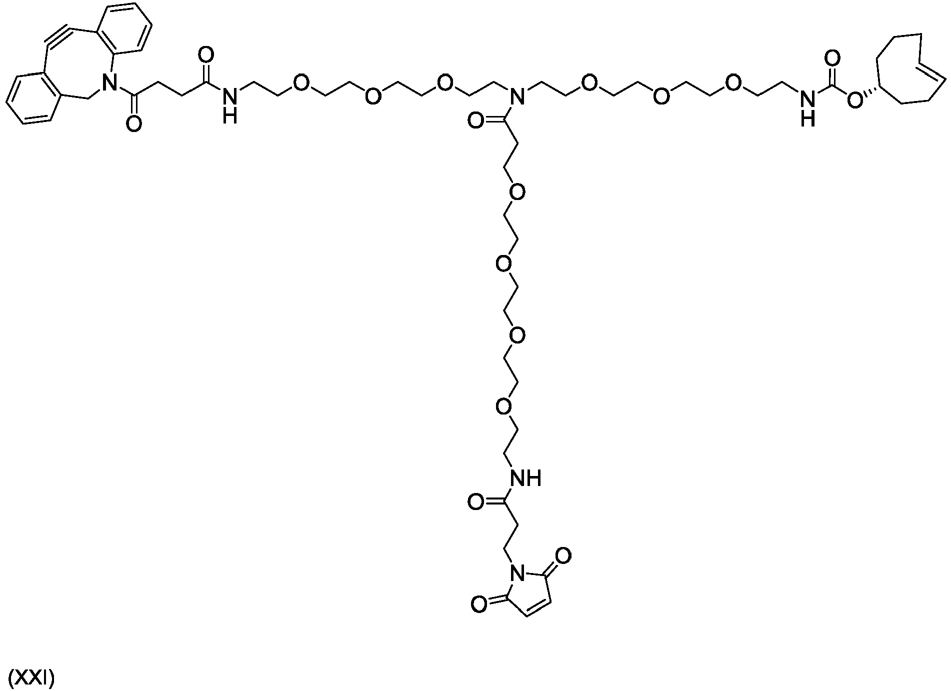

The resulting mixture was shaken for 1 min and left standing at room temperature. After 2 hours the reaction mixture was subjected to preparative MP-LC.^{2A} Fractions corresponding to the product were immediately pooled together, frozen and lyophilized overnight to give the title compound (3.83 mg, 21%) as a white solid. Purity based on LC-MS 89%.
LRMS (m/z): 2409 [M-546]¹⁻, 2955 [M-1]¹⁻
LC-MS r.t. (min): 2.66^{1B}

### Intermediate 18:

**Methyltetrazine-L-ApoB BNA oligo**

To ApoB BNA oligo disulfide (5.00 mg, 0.686 µmol) was added a solution of 20 mM ammonium bicarbonate with 2.5 mM TCEP (1.00 mL, 2.5 µmol). The reaction mixture was shaken for 1 min and left standing at room temperature. After 1 hour the reaction mixture was filtered by using a centrifugal filter with a molecular weight cut-off of 3000 Da (5000 × g for 30 min, 2 × 0.50 mL ). Next, the residue solution was washed twice with a solution of 20 mM ammonium bicarbonate with 2.5 mM TCEP (0.50 mL), each time filtered under the same conditions described above. As next, the residue solution was diluted with 20 mM ammonium bicarbonate (1.50 mL) and the resulting mixture was directly added to a solution of (E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide

(1.36 mg, 1.73 µmol) in acetonitrile (0.5 mL). The reaction mixture was shaken for 1 min and left standing at room temperature. After 30 min the reaction mixture was frozen and lyophilized overnight to yield the crude title product as a pink fluffy solid. To the crude product was added a solution of 20 mM ammonium bicarbonate (1.50 mL) and the resulting suspension was filtered over a 0.45 µm syringe filter. The filtrate was lyophilized overnight to yield the title product (5.44 mg, quant) as a pink fluffy solid. Purity based on LC-MS 90% (very broad peak).
LRMS (m/z): 2648 [M-3]³⁻
LC-MS r.t. (min): 0.62⁴

### Intermediate 19:

### Methyltetrazine-L-ApoB scrambled BNA oligo

To ApoB scrambled BNA oligo disulfide (5.00 mg, 0.688 µmol) was added a solution of 20 mM ammonium bicarbonate with 2.5 mM TCEP (1.00 mL, 2.5 µmol). The reaction mixture was shaken for 1 min and left standing at room temperature. After 1 hour the reaction mixture was filtered by using a centrifugal filter with a molecular weight cut-off of 3000 Da (5000 × g for 30 min, 2 × 0.50 mL ). Next, the residue solution was washed twice with a solution of 20 mM ammonium bicarbonate with 2.5 mM TCEP (0.50 mL), each time filtered under the same conditions described above. As next, the residue solution was diluted with 20 mM ammonium bicarbonate (1.50 mL) and the resulting mixture was directly added to a solution of (E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide (1.34 mg, 1.70 µmol) in acetonitrile (0.5 mL). The reaction mixture was shaken for 1 min and left standing at room temperature. After 30 min the reaction mixture was frozen and lyophilized overnight to yield the crude title product as a pink fluffy solid. To the crude product was added a solution of 20 mM ammonium bicarbonate (1.50 mL) and the resulting suspension was filtered over a 0.45 µm syringe filter. The filtrate was lyophilized overnight to yield the title product (5.48 mg, quant) as a pink fluffy solid. Purity based on LC-MS 84% (very broad peak).
LRMS (m/z): 2639 [M-3]³⁻
LC-MS r.t. (min): 0.58⁴

### Intermediate 20:

### DBCO-L-ApoB BNA oligo-trivalent GalNAc

To methyltetrazine-L-ApoB BNA oligo (5.44 mg, 0.684 µmol) and DBCO-TCO-trivalent GalNAc (2.03 mg, 0.687 µmol) was added 20 mM ammonium bicarbonate/acetonitrile (3:1, v/v, 1.00 mL). The reaction mixture was shaken for 1 min and left standing at room temperature. After 2 hours the reaction mixture was subjected to preparative LC-MS.^{4C} Fractions corresponding to the product were immediately pooled together, frozen and lyophilized overnight to give the title compound (2.87 mg, 39%) as a white fluffy solid. LC-MS shows multiple broad peaks with the correct m/z values.
LRMS (m/z): 2175 [M-5]⁵⁻, 2718 [M-6]⁴⁻
LC-MS r.t. (min): 2.60-3.00^{1A}

### Intermediate 21:

### DBCO-L-ApoB scrambled BNA oligo-trivalent GalNAc

To methyltetrazine-L-ApoB scrambled BNA oligo (5.48 mg, 0.692 µmol) and DBCO-TCO-trivalent GalNAc (1.80 mg, 0.609 µmol) was added 20 mM ammonium bicarbonate/acetonitrile (3:1, v/v, 1.00 mL). The reaction mixture was shaken for 1 min and left standing at room temperature. After 2 hours the reaction mixture was subjected to preparative LC-MS.^{4C} Fractions corresponding to the product were immediately pooled together, frozen and lyophilized overnight to give the title compound (2.15 mg, 33%) as a white fluffy solid. LC-MS shows multiple broad peaks with the correct m/z values.
LRMS (m/z): 2169 [M-5]⁵⁻, 2711 [M-6]⁴⁻
LC-MS r.t. (min): 2.58-3.20^{1A}

### Trivalent linker-L-SPT001-(blocked TCO)-trivalent GalNAc synthesis

### Intermediate 22 (Figure 25)

### Trivalent GalNAc-thiol

Trivalent GalNAc-thioacetate (16.2 mg, 9.02 µmol) was dissolved in methanol (500 µL). Next, a 1.00 M solution of sodium hydroxide (11.0 µL, 11.0 µmol) was added. The reaction mixture was shaken for 1 min and left standing at room temperature. After 30 min the reaction mixture was submitted to preparative MP-LC.^{1A} Fractions corresponding to the product were immediately pooled together, frozen and lyophilized overnight to give the title compound (13.1 mg, 83%) as a white solid. Purity based on LC-MS 98%.
LRMS (m/z): 1748 [M-H]¹⁻
LC-MS r.t. (min): 1.78^{1A}

### Intermediate 23: (Figure 25)

### DBCO-TCO-trivalent GalNAc

Trifunctional linker (15.0 mg, 12.4 µmol) was dissolved in acetonitrile (0.50 mL). Next, a solution of 20 mM ammonium bicarbonate (1.50 mL) was added and the resulting solution was directly transferred to trivalent GalNAc-thiol (22.7 mg, 13.0 µmol). The reaction mixture was shaken for 1 min and left standing at room temperature. After 1 hour the reaction mixture was frozen and lyophilized overnight to yield the crude title product as a white solid. Purity based on LC-MS 84%.
LRMS (m/z): 2409 [M-546]¹⁻, 2955 [M-1]¹⁻
LC-MS r.t. (min): 2.63^{1B}

### Intermediate 24 (Figure 26):

### N-(2-hydroxyethyl)-2-(4-(6-methyl-1,2,4,5-tetrazin-3-yl)phenyl)acetamide

To a solution of methyltetrazine-NHS ester (30.0 mg, 91.7 µmol) in DMF (1.00 mL) was added ethanolamine (11.1 µL, 0.184 mmol) and triethylamine (25.5 µL, 0.183 mmol). The reaction mixture was shaken for 1 min and left standing at room temperature. After 1 hour the reaction mixture was submitted to preparative MP-LC.^{1B} Fractions corresponding to the product were immediately pooled together, frozen and lyophilized overnight to give the title compound (19.2 mg, 77%) as a purple solid. Purity based on LC-MS 89%.
LRMS (m/z): 274 [M+1]¹⁺
LC-MS r.t. (min): 0.85²

### Trivalent linker-L-SPT001-(blocked TCO)-trivalent GalNAc

A solution of DBCO-TCO-trivalent GalNAc (36.8 mg, 12.5 µmol) in DMF (2.0 mL) was added to SO1861-L-azide (26.8 mg, 12.5 µmol). The reaction mixture was shaken for 1 min and left standing at room temperature. After 30 min N-(2-hydroxyethyl)-2-(4-(6-methyl-1,2,4,5-tetrazin-3-yl)phenyl)acetamide (4.08 mg, 14.9 µmol) was added. The reaction mixture was shaken for 1 min and left standing at room temperature. After 30 min the reaction mixture was submitted to preparative MP-LC.^{1C} Fractions corresponding to the product were immediately pooled together, frozen and lyophilized overnight to give the title compound (37.3 mg, 56%) as a white fluffy solid. Purity based on LC-MS 96% (double peaks due to regioisomers).
LRMS (m/z): 2676 [M-2]²⁻
LC-MS r.t. (min): 2.23^{1B}

### Trivalent linker-(blocked DBCO)-BNA oligo-trivalent GalNAc synthesis

### Intermediate 25 (Figure 27):

### methyltetrazine-BNA oligo

To Thiol-13-ApoB BNA oligo disulfide (20 mg, 4.17 µmol) was added a solution of 20 mM ammonium bicarbonate with 5.0 mM TCEP (2.00 mL, 10.0 µmol). The reaction mixture was shaken for 1 min and left standing at room temperature. After 1 hour the reaction mixture was filtered by using a centrifugal filter with a molecular weight cut-off of 3000 Da (5000 × g for 30 min, 4x 0.50 mL). Next, the residue solution was diluted with 20 mM ammonium bicarbonate (3.00 mL) and the resulting mixture was directly added to a solution of (E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-,6,9,12-tetraoxapentadecan-15-amide ( 7.22mg, 9.16 µmol) in acetonitrile (1.0 mL). The reaction mixture was shaken for 1 min and left standing at room temperature. After 1 hour the reaction mixture was frozen and lyophilized overnight to yield the crude title product as a pink fluffy solid. To the crude product was added a 20 mM ammonium bicarbonate/acetonitrile (2:1, v/v, 2.00 mL) and the resulting solution was subjected to preparative LC-MS.^{1A} Fractions corresponding to the product were immediately pooled together, frozen and lyophilized overnight to give the title compound (20.3 mg, 89%) as a pink fluffy solid. Purity based on LC-MS 93% (broad peak).
LRMS (m/z): 1817 [M-3]³⁻
LC-MS r.t. (min): 0.59³

### Intermediate 26 (Figure 27):

### (blocked DBCO)-TCO-trivalent GalNAc

A solution of 1-azido-3,6,9-trioxaundecane-11-ol (2.17 mg, 9.88 µmol) in DMF (0.50 mL) was added to DBCO-TCO-trivalent GalNAc (14.6 mg, 4.94 µmol. The reaction mixture was shaken for 1 min and left standing at room temperature. After 2 hours the reaction mixture was submitted to preparative MP-LC.^{1C} Fractions corresponding to the product were immediately pooled together, frozen and lyophilized overnight to give the title compound (10.00 mg, 64%) as a white solid. Purity based on LC-MS 98%.
LRMS (m/z): 1750 [fragment]
LC-MS r.t. (min): 2.33^{1B}

### Trivalent linker-(blocked DBCO)-BNA oligo-trivalent GalNAc

(blocked DBCO)-TCO-trivalent GalNAc (10.0 mg, 3.15 µmol) and Methyltetrazine-BNA oligo (10.0 mg, 1.83 µmol) were dissolved in a solution of 20 mM ammonium bicarbonate/acetonitrile (3:1, v/v, 1.00 mL). The reaction mixture was shaken for 1 min and left standing at room temperature. After 3 hours the reaction mixture was frozen and lyophilized overnight. The crude product was dissolved in 20 mM ammonium bicarbonate (1 mL) and the resulting solution was directly subjected to preparative LC-MS.^{1B} Fractions corresponding to the product were immediately pooled together, frozen and lyophilized overnight to give the title compound (11.3 mg, 72%) as a white fluffy solid. Purity based on LC-MS 95% (multiple (broad) peaks due to regioisomers).
LRMS (m/z): 2149 [M-4]⁴⁻, 2866 [M-3]³⁻
LC-MS r.t. (min): 2.92^{1A}

### dendron(-L-SO1861)₄-amine (molecule 15)

N,N'-((9S,19S)-14-(6-aminohexanoyl)-1-mercapto-9-(3-mercaptopropanamido)-3,10,18-trioxo-4,11,14,17-tetraazatricosane-19,23-diyl)bis(3-mercaptopropanamide) formate (2.73 mg, 3.13 µmol) was dissolved in a mixture of 20 mM NH₄HCO₃ with 0.5 mM TCEP/acetonitrile (3:1, v/v, 3.00 mL). Next, SO1861-EMCH (29.2 mg, 0.014 mmol) was added and the reaction mixture was stirred at room temperature. After 1.5 hours the reaction mixture was subjected to preparative LC-MS.^{3B} Fractions corresponding to the product were immediately pooled together, frozen and lyophilized overnight to give the title compound (12.3 mg, 43%) as a white fluffy solid. Purity based on LC-MS 97%.
LRMS (m/z): 1517 [M-6]⁶⁻, 1821 [M-5]⁵⁻, 2276 [M-4]⁴⁻
LC-MS r.t. (min): 4.39^{5A}

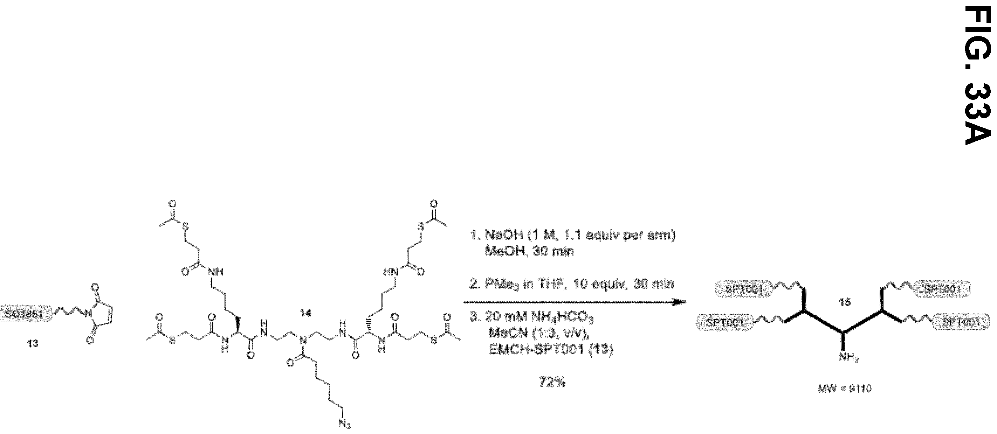

### Dendron(-L-SO1861)₄-trivalent GalNAc (Figure 33A-C) and dendron(-L-SO1861)₈-trivalent GalNAc synthesis

### Dendron(-L-SO1861)₄-azide (molecule 19)

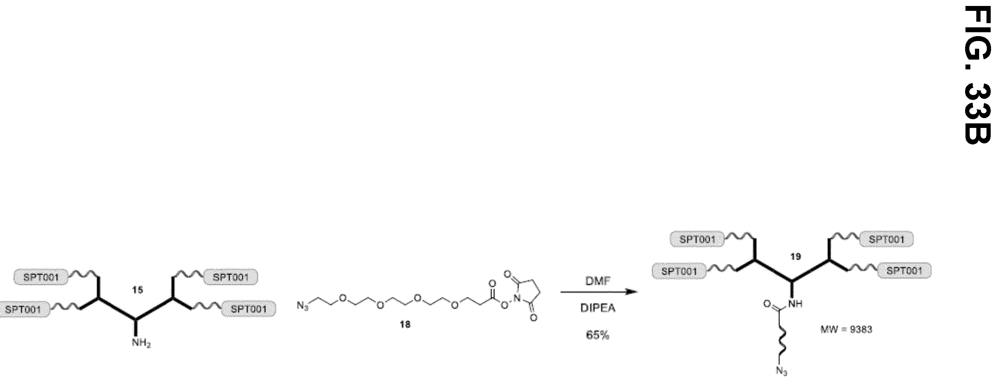

The dendron(-L-SO1861)₄-azide is synthesized based on dendron(SPT001)₄-NH₂ (molecule 15, Figure 33B) wherein this amine dendron was treated with azido-PEG₄-NHS ester (molecule 18) in DMF with DIPEA as a base.

### Trivalent GalNAc-amine formate (molecule 22 in Figure 33C)

Trivalent GalNAc-azide (36.5 mg, 21.6 µmol; molecule 21 in Figure 33C) was dissolved in a solution of potassium carbonate (5.97 mg, 43.2 µmol) in water (1.00 mL) and acetonitrile (1.00 mL). Next, a 1.0 M trimethylphosphine solution in THF (216 µL, 216 µmol) was added and the resulting mixture was shaken for 1 min and left standing at room temperature. After 45 min the reaction mixture was evaporated *in vacuo* and the residue was dissolved in water/acetonitrile (9:1, v/v, 1 mL). The resulting solution was directly subjected to preparative MP-LC.^{2B} Fractions corresponding to the product were immediately pooled together, frozen and lyophilized overnight to give the title compound (36.1 mg, 98%) as a white solid. Purity based on LC-MS 100%.
LRMS (m/z): 1662 [M-1]¹⁻
LC-MS r.t. (min): 1.62^{1A}

### Trivalent GalNAc-DBCO (molecule 23 in Figure 33C)

Trivalent GalNAc-amine formate (17.4 mg, 10.2 µmol; molecule 22) and DBCO-NHS (6.14 mg, 15.3 µmol; molecule 10 in Figure 33C) were dissolved in a solution of NMM (2.24 µL, 20.3 µmol) in DMF (0.50 mL). The reaction mixture was shaken for 1 min and left standing at room temperature. After 2 hours the reaction mixture was evaporated *in vacuo* and the residue was dissolved in water/acetonitrile (8:2, v/v, 1 mL). The resulting solution was directly subjected to preparative MP-LC.^{2C} Fractions corresponding to the product were immediately pooled together, frozen and lyophilized overnight to give the title compound (14.2 mg, 72%) as a white solid. Purity based on LC-MS 96%.
LRMS (m/z): 1950 [M-1]¹
LC-MS r.t. (min): 1.86^{1B}

### dendron(-L-SO1861)₈-azide

Dendron(-L-SO1861)₈-amine (19.6 mg, 1.08 µmol) and 2,5-dioxopyrrolidin-1-yl 1-azido-3,6,9,12-tetraoxapentadecan-15-oate (4.17 mg, 10.8 µmol) were dissolved in DMF (1.50 mL). Next, DIPEA (1.87 µL, 10.8 µmol) was added and the mixture was shaken for 1 min and left standing overnight at room temperature. The reaction mixture was subjected to preparative LC-MS.^{4B} Fractions corresponding to the product were immediately pooled together, frozen and lyophilized overnight to give the title compound (11.7 mg, 59%) as a white fluffy solid. Purity based on LC-MS 92% (very broad peak).
LRMS (m/z): 2316 [M-8]⁸⁻, ): 2647 [M-7]⁷⁻
LC-MS r.t. (min): 4.29^{1A}

### Dendron(-L-SO1861)₄-trivalent GalNAc (molecule 24 in Figure 33C) synthesis

Dendron(-L-SO1861)₄-azide (2.50 mg, 0.266 µmol; synthesis depicted in Figure 33A-C, molecule 19 in Figure 33C) and trivalent GalNAc-DBCO (1.56 mg, 0.799 µmol; molecule 23 in Figure 33C)) were dissolved in a mixture of water/acetonitrile (3:1, v/v, 1.00 mL). The reaction mixture was shaken for 1 min and left standing at room temperature. After 2 hours the reaction mixture was subjected to preparative LC-MS.^{4B} Fractions corresponding to the product were immediately pooled together, frozen and lyophilized overnight to give the title compound (2.74 mg, 91%) as a white fluffy solid. Purity based on LC-MS 86% (very broad peak).
LRMS (m/z): 2832 [M-4]⁴⁻
LC-MS r.t. (min): 4.07^{1A}

### Dendron(-L-SO1861)₈-trivalent GalNAc synthesis

Dendron(-L-SO1861)₈-azide (2.50 mg, 0.135 µmol) and trivalent GalNAc-DBCO (0.79 mg, 0.405 µmol; molecule 23 in Figure 33C) were dissolved in a mixture of water/acetonitrile (3:1, v/v, 1.00 mL). The reaction mixture was shaken for 1 min and left standing at room temperature. After 2 hours the reaction mixture was subjected to preparative LC-MS.^{4B} Fractions corresponding to the product were immediately pooled together, frozen and lyophilized overnight to give the title compound (2.03 mg, 74%) as a white fluffy solid. Purity based on LC-MS 100% (very broad peak).
LRMS (m/z): 2559 [M-8]⁸⁻, 2925 [M-7]⁷⁻
LC-MS r.t. (min): 4.18^{1A}

### AntiCD71-saporin synthesis

Custom CD71mab-saporin conjugate was produced and purchased from Advanced Targeting Systems (San Diego, CA). CD71 monoclonal antibody was purchased from InVivoMab, anti-human CD71 (OKT-9), BioXCell.

### HSP27BNA, ApoB and ApoB_{scrambled}, and ApoB#02 oligo sequences

HSP27 (5'-GGCacagccagtgGCG-3') [SEQ ID NO: 1] (The antisense BNA (HSP27) was BNA, more specifically BNA^{NC}, with oligo nucleic acid sequence 5'-GGCacagccagtgGCG-3' according to Zhang et al. (2011) [Y Zhang, Z Qu, S Kim, V Shi, B Liao1, P Kraft, R Bandaru, Y Wu, LM Greenberger and ID Horak, Down-modulation of cancer targets using locked nucleic acid (LNA)-based antisense oligonucleotides without transfection, Gene Therapy (2011) 18, 326-333]), ApoB (5'-GCCTCagtctgcttcGCACC-3') [SEQ ID NO: 2] and ApoB_{scrambled} (5'-GGCCTctctacaccgCTCGT-3') [SEQ ID NO: 3], and murine and human sequence-compatible ApoB#02 (5'-GCattggtatTCA-3') [SEQ ID NO: 12] BNA^{NC} oligos were ordered with 5'-Thiol C6 linker at Bio-Synthesis Inc, with BNA bases in capitals, and fully phosphorothioate backbones. (Lewisville, Texas).

### RNA isolation and gene expression analysis from human cell culture

RNA from cells was isolated and analysed according to standard protocols (Biorad). qPCR primers that were used are indicated in Table A2.

**Table A2. Primers used in qPCR are shown below:**

| Gene | Primer | Sequence (5'-3') |
|---|---|---|
| HSP27 | Forward | GCAGTCCAACGAGATCACCA [SEQ ID NO: 4] |
| | Reverse | TAAGGCTTTACTTGGCGGCA [SEQ ID NO: 5] |
| ApoB | Forward | AGGGTCCGGGAATCTGATGA [SEQ ID NO: 6] |
| | Reverse | TGGGCACGTTGTCTTTCAGAG [SEQ ID NO: 7] |
| HBMS | Forward | CACCCACACACAGCCTACTT [SEQ ID NO: 8] |
| | Reverse | GTACCCACGCGAATCACTCT [SEQ ID NO: 9] |
| GUSB | Forward | GAAAATACGTGGTTGGAGAGCT [SEQ ID NO: 10] |
| | Reverse | CCGAGTGAAGATCCCCTTTTTA [SEQ ID NO: 11] |

### Cell viability assay (MTS)

After treatment the cells were incubated for 72 hr at 37°C before the cell viability was determined by an MTS-assay, performed according to the manufacturer's instruction (CellTiter 96^{®} AQueous One Solution Cell Proliferation Assay, Promega). Briefly, the MTS solution was diluted 20× in DMEM without phenol red (PAN-Biotech GmbH) supplemented with 10% FBS. The cells were washed once with 200 µL/PBS well, after which 100 µL diluted MTS solution was added/well. The plate was incubated for approximately 20-30 minutes at 37°C. Subsequently, the OD at 492 nm was measured on a Thermo Scientific Multiskan FC plate reader (Thermo Scientific). For quantification the background signal of 'medium only' wells was subtracted from all other wells, before the cell viability percentage of treated/untreated cells was calculated, by dividing the background corrected signal of treated wells over the background corrected signal of the untreated wells (x 100).

### Cell viability assay (CTG)

After treatment the cells were incubated for 72 hr at 37°C before the cell viability was determined by a CTG-assay, performed according to the manufacturer's instruction (CellTiter-Glo^{®} 2.0 Cell Viability Assay, Promega). Briefly, the cell plate was first equilibarated to RT for 30 minutes. Next, to each well containing 120 µL treatment media 120 µL CTG solution was added. The plate briefly mixed (10 sec, 600 rpm) and incubated for approximately 10 minutes in the dark at RT. Subsequently, the luminescence signal was measured on a Spectramax ID5 plate reader (Molecular Devices). For quantification, the background signal of 'medium only' wells was subtracted from all other wells before the cell viability percentage of treated/untreated cells was calculated by dividing the background corrected signal of treated wells over the background corrected signal of the untreated wells (x 100).

### FACS analysis

Cells were seeded in DMEM (PAN-Biotech GmbH) supplemented with 10% fetal bovine serum (PAN-Biotech GmbH) and 1% penicillin/streptomycin (PAN-Biotech GmbH), in T75 flasks at appropriate density for each cell-line and incubated for 72-96 hrs (5% CO₂, 37°C), until a confluency of 90% was reached. Next, the cells were trypsinized (TrypIE Express, Gibco Thermo Scientific) to single cells, transferred to a 15 mL falcon tube, and centrifuged (1,400 rpm, 3 min). The supernatant was discarded while leaving the cell pellet submerged. 500.000 Cells were transferred to round bottom FACS tubes and the washed with 3 mL cold DPBS (Mg²⁺ and Ca²⁺ free, 2% FBS). The cells were centrifuged at 1800 rpm, 3 min 4°C and resuspended in 200 µL cold DPBS (Mg²⁺ and Ca²⁺ free, 2% FBS) or 200 µL antibody solution; containing 5 µL antibody in 195 µL cold DPBS (Mg²⁺ and Ca²⁺ free, 2% FBS). PE anti-human CD71 (#334106, Biolegend) was used to stain the transferrin receptor, PE Mouse IgG2a, κ Isotype Ctrl FC (#400212, Biolegend) was used as its matched isotype control. PE anti-human ASGPR1 (#130-122-963, Miltenyi) was used to stain the ASGPR1 receptor and PE Mouse IgG1, Isotype Ctrl (#130-113-762, Miltenyi) was used as its matched isotype control. Samples were incubated for 30 min. at 4°C. Afterwards, the cells were washed 2x with cold DPBS (Mg²⁺ and Ca²⁺ free, 2% FBS) and fixated for 20 min at room temperature using a 2% PFA solution in DPBS (Mg²⁺ and Ca²⁺ free, 2% FBS). Cells were washed 1x with cold DPBS and resuspended in 1000 µL cold DPBS for FACS analysis. Samples were analyzed with a Sysmex Cube 8 flow cytometry system (Sysmex) and FCS Express 7 Research edition software. Results of the FACS analyses are summarized in Table A3.

**Table A3. Cell membrane receptor expression levels of ASPGR1 and CD71 of HepG2 and Huh7 cells**

| **Cell line** | **ASPGR1 expression level (MFI)** | **CD71 expression level (MFI)** |
|---|---|---|
| **HepG2** | 8.8 | 74.7 |
| **Huh7** | 1.6 | 109 |

### Mouse primary hepatocyte treatment

Cryopreserved primary mouse hepatocytes (PRIMACYT Cell Culture Technology GmbH, Germany) were thawed in hepatocyte thawing media (HTM, PRIMACYT Cell Culture Technology GmbH, Germany) and washed 1x with hepatocyte wash media (HWM, PRIMACYT Cell Culture Technology GmbH, Germany). Cells were re-suspended in hepatocyte plating media (HPM Cryo, PRIMACYT Cell Culture Technology GmbH, Germany) at a density of approx. 0.275 x 10⁶ cells/ml. Cells were seeded onto collagen-I coated plates at a density of 88.000 cells/well or 26.400 cells/well for the 48 or 96-well plates (Greiner BioOne). Cells were pre-cultured for 4-6 hrs at 37°C allowing for cell attachment to cell culture plates before the start of treatment. Plating medium was replaced by 315 µl or 108 µl assay media (MHM, PRIMACYT Cell Culture Technology GmbH, Germany), after which conjugates were added from a 10x concentrated stock solution in PBS. Plates were incubated for 72 hr at 37°C being harvested for gene expression and cell viability analysis.

### Human primary hepatocyte treatment

Cryopreserved primary human hepatocytes (Cytes Biotechnologies S.L., Spain) were thawed in hepatocyte thawing media (Cytes Biotechnologies S.L., Spain). Cells were re-suspended in hepatocyte plating media (Cytes Biotechnologies S.L., Spain). Cells were seeded onto collagen-I coated plates at a density of 215.600 cells/well or 66.600 cells/well for the 48 or 96-well plates (Greiner BioOne). Cells were pre-cultured for 4-6 hrs at 37°C allowing for cell attachment to cell culture plates before the start of treatment. Plating medium was replaced by 315 µl or 108 µl maintenance media (Cytes Biotechnologies S.L., Spain), after which conjugates were added from a 10x concentrated stock solution in PBS. Plates were incubated for 72 hr at 37°C being harvested for gene expression and cell viability analysis.

### RNA isolation and gene expression analysis from primary mouse and human hepatocytes

RNA from cells was isolated using TRI Reagent^{®} Solution (Thermo Scientific) according to the manufacturer's instruction. Conversion into cDNA was performed using iScript^{™} cDNA Synthesis Kit (BioRad) using standard protocols. *ApoB* expression levels and levels of specific hepatocyte housekeeping genes were determined using quantitative real-time PCR assays (qRT-PCR) using iTaq^{™} Universal SYBR^{®} Green Supermix (BioRad) and the Light Cycler 480 (Roche Diagnostics, Rotkreuz, Switzerland) with specific DNA primers, listed in Table A4. Analysis was done by the ΔCt method to determine ApoB expression relative to 2 hepatocyte-specific housekeeping control mRNAs. Each analysis reaction was performed in triplicate.

**Table A4. Primers used in qPCR analysis of mouse primary hepatocytes**

| | | |
|---|---|---|
| ApoB#02 | Forward | GCTAACACTAAGAACCAGAAGATC [SEQ ID NO: 13] |
| | Reverse | TGTCCGTCTAAGGATCCTGC [SEQ ID NO: 14] |
| Mm PPIA | Forward | GCGGCAGGTCCATCTACG [SEQ ID NO: 15] |
| | Reverse | GCCATCCAGCCATTCAGTC [SEQ ID NO: 16] |
| Mm SDHA | Forward | GAGGAAGCACACCCTCTCAT [SEQ ID NO: 17] |
| | Reverse | GGAGCGGATAGCAGGAGGTA [SEQ ID NO: 18] |

### In vivo tolerability and efficacy of trivalent GalNAc-conjugates in a C57BL/6J model

- Dose administration and in-life phase. Per group, 8 C57BL/6J male mice for treatment groups and 14 in vehicle control group, approximately 6 - 7 weeks at arrival and 8 - 9 weeks at dosing, were dosed. The compounds, formulated in PBS or PBS alone (vehicle) according to Table A5, were dosed at 5 ml/kg in the tail vain (intravenously, iv), with dose volumes corresponding to the individual bodyweights. Where applicable, trivalent GalNAc-SO1861-EMCH was co-administered at 5 ml/kg in the tail vain (iv) right after the trivalent GalNAc-ApoB conjugate. Animals were weighed weekly and clinical observations were performed 2 - 3 times daily within the first 48 hrs, and at least once daily thereafter until study end.
- Sampling of serum, liver, and kidney tissue. Blood was sampled before dosing (predose) and at 24 hrs, 72 hrs, 196 hrs, and at 336 hrs post dose, and serum was generated from the collected blood for biomarker analysis, using standard procedures. At the post-dose timepoints of 24 hrs and 72 hrs, 3 animals per timepoint per treatment group and 6 animals in the vehicle control group were sacrificed to collect terminal bleeds and liver, kidney and mesenteric lymph node tissues for further analyses. at end of study at 336 hrs, the remaining 2 animals (4 in the control group) per group were sacrificed. One half of the liver and one kidney was frozen and used for tissue RNA analysis.

**Table A5. Experimental design in vivo tolerability and efficacy of trivalent GalNAc-conjugates**

| Group | Conjugate | Dose [mg/kg] | Relative BNA dose administered [mg/kg] | Co-administered trivalent GalNAc-SO1861-EMCH dose [mg/kg] |
|---|---|---|---|---|
| 1 | PBS (vehicle) | n/a | 0 | |
| 2 | ApoBBNA#02 | 0,1 | 0,1 | |
| 3 | ApoBBNA#02 | 1 | 1 | |
| 4 | ApoBBNA#02 | 2 | 2 | |
| 5 | (GalNAc)3-ApoBBNA#02 | 0.1 | 0.058 | |
| 6 | (GalNAc)3-ApoBBNA#02 | 1 | 0.58 | |
| 7 | (GalNAc)3-ApoBBNA#02 | 0.01 | 0.0058 | 5 |
| 8 | (GalNAc)3-ApoBBNA#02 | 0.1 | 0.058 | 5 |

### Biomarker RNA analysis in liver tissue

The mRNA was isolated from frozen liver tissue using TRIzol^{®} Reagent (Thermo Scientific) according to the manufacturer's instruction. Conversion into cDNA was performed using iScript^{™} cDNA Synthesis Kit (BioRad). *ApoB* expression levels and levels of specific liver housekeeping genes were determined using quantitative real-time PCR assays (qRT-PCR) using iTaq^{™} Universal SYBR^{®} Green Supermix (BioRad) and the Light Cycler 480 (Roche Diagnostics, Rotkreuz, Switzerland) with specific DNA primers, listed in Table A5. Analysis was done by the ΔCt method to determine *ApoB* expression relative to 2 liver-specific housekeeping control mRNAs. Each analysis reaction was performed in triplicate.

**Table A6. Primers used in qPCR analysis of liver tissue**

| | | |
|---|---|---|
| ApoB#02 | Forward | GCTAACACTAAGAACCAGAAGATC [SEQ ID NO: 13] |
| | Reverse | TGTCCGTCTAAGGATCCTGC [SEQ ID NO: 14] |
| Mm RSP17 | Forward | GTGCGAGGAGATCGCCATTA [SEQ ID NO: 19] |
| | Reverse | ATCCGCTTCATCAGATGCGT [SEQ ID NO: 20] |
| Mm SDHA | Forward | GAGGAAGCACACCCTCTCAT [SEQ ID NO: 17] |
| | Reverse | GGAGCGGATAGCAGGAGGTA [SEQ ID NO: 18] |

### Biomarker analysis ApoB protein

ApoB protein levels in mouse serum was determined using an ApoB ELISA (ab230932, Abcam, Cambridge, UK), according to the manufacturer's instruction. Note that no samples were analysed from 1 mg/kg ApoB#02 and 0.01 mg/kg (GalNAc)3-ApoB#03 + 5 mg/kg (GalNAc)3-SO861 groups at 336 hours.

### Biomarker analysis cholesterol

LDL-cholesterol was measured in mouse serum, using an LDL-cholesterol specific two-step AU480 assay kit, respectively, from Beckman Coulter, with a photometric measurement according to the manufacturer's instructions.

### Biomarker analysis cholesterol

Serum alanine transaminase (ALT) levels was measured using an AU480 assay kit from Beckman Coulter (photometric measurements), according to the manufacturer's instructions.

## Claims

1. Saponin conjugate comprising at least one saponin covalently linked to a ligand for asialoglycoprotein receptor (ASGPR), wherein the ligand for ASGPR comprises at least one N-acetylgalactosamine (GalNAc) moiety, preferably three or four GalNAc moieties, more preferably the ligand for ASGPR comprises or consists of (GalNAc)₃Tris, wherein the at least one saponin is selected from monodesmosidic triterpenoid saponins and bidesmosidic triterpenoid saponins, wherein the saponin is a saponin derivative comprising a saccharide chain bound to the aglycone core structure, which is selected from group A:
GlcA-
Gal-(1→2)-[Xyl-(1→3)]-GlcA-,
Glc-(1→2)-[Glc-(1→4)]-GlcA-,
Glc-(1→2)-Ara-(1→3)-[Gal-(1→2)]-GlcA-,
Xyl-(1→2)-Ara-(1→3)-[Gal-(1→2)]-GlcA-,
Rha-(1→2)-Gal-(1→3)-[Glc-(1→2)]-GlcA-,
Ara-(1→4)-Rha-(1→2)-Glc-(1→2)-Rha-(1→2)-GlcA-,
Ara-(1→4)-Fuc-(1→2)-Glc-(1→2)-Rha-(1→2)-GlcA-,
Ara-(1→4)-Rha-(1→2)-Gal-(1→2)-Rha-(1→2)-GlcA-,
Ara-(1→4)-Fuc-(1→2)-Gal-(1→2)-Rha-(1→2)-GlcA-,
Ara-(1→4)-Rha-(1→2)-Glc-(1→2)-Fuc-(1→2)-GlcA-,
Ara-(1→4)-Fuc-(1→2)-Glc-(1→2)-Fuc-(1→2)-GlcA-,
Ara-(1→4)-Rha-(1→2)-Gal-(1→2)-Fuc-(1→2)-GlcA-,
Ara-(1→4)-Fuc-(1→2)-Gal-(1→2)-Fuc-(1→2)-GlcA-,
Xyl-(1→4)-Rha-(1→2)-Glc-(1→2)-Rha-(1→2)-GlcA-,
Xyl-(1→4)-Fuc-(1→2)-Glc-(1→2)-Rha-(1→2)-GlcA-,
Xyl-(1→4)-Rha-(1→2)-Gal-(1→2)-Rha-(1→2)-GlcA-,
Xyl-(1→4)-Fuc-(1→2)-Gal-(1→2)-Rha-(1→2)-GlcA-,
Xyl-(1→4)-Rha-(1→2)-Glc-(1→2)-Fuc-(1→2)-GlcA-,
Xyl-(1→4)-Fuc-(1→2)-Glc-(1→2)-Fuc-(1→2)-GlcA-,
Xyl-(1→4)-Rha-(1→2)-Gal-(1→2)-Fuc-(1→2)-GlcA-,
Xyl-(1→4)-Fuc-(1→2)-Gal-(1→2)-Fuc-(1→2)-GlcA-, and
derivatives thereof,
wherein said saccharide chain comprises a carboxyl group which has been derivatised and the carboxyl group which is derivatised is part of a glucuronic acid moiety.

2. Saponin conjugate of claim 1, wherein the saponin comprises an aglycone core structure selected from the group consisting of:
2alpha-hydroxy oleanolic acid;
16alpha-hydroxy oleanolic acid;
hederagenin (23-hydroxy oleanolic acid);
16alpha,23-dihydroxy oleanolic acid;
gypsogenin;
quillaic acid;
protoaescigenin-21(2-methylbut-2-enoate)-22-acetate;
23-oxo-barringtogenol C-21,22-bis(2-methylbut-2-enoate);
23-oxo-barringtogenol C-21(2-methylbut-2-enoate)-16,22-diacetate;
digitogenin;
3,16,28-trihydroxy oleanan-12-en;
gypsogenic acid,
and
derivatives thereof,
preferably the saponin comprises an aglycone core structure selected from quillaic acid and gypsogenin or derivatives thereof, more preferably the saponin aglycone core structure is quillaic acid or a derivative thereof.

3. Saponin conjugate of claim 1 or 2, wherein
• the saponin comprises a saccharide chain bound to the aglycone core structure, which is selected from group B:
Glc-,
Gal-,
Rha-(1→2)-[Xyl-(1→4)]-Rha-,
Rha-(1->2)-[Ara-(1->3)-Xyl-(1->4)]-Rha-,
Ara-,
Xyl-,
Xyl-(1→4)-Rha-(1→2)-[R1-(→4)]-Fuc- wherein **R1** is 4E-Methoxycinnamic acid,
Xyl-(1→4)-Rha-(1→2)-[R2-(→4)]-Fuc- wherein R2 is 4Z-Methoxycinnamic acid,
Xyl-(1→4)-[Gal-(1→3)]-Rha-(1→2)-4-OAc-Fuc-,
Xyl-(1→4)-[Glc-(1→3)]-Rha-(1→2)-3,4-di-OAc-Fuc-,
Xyl-(1->4)-[Glc-(1->3)]-Rha-(1->2)-[R3-(->4)]-3-OAc-Fuc- wherein R3 is 4E-Methoxycinnamic acid,
Glc-(1→3)-Xyl-(1→4)-[Glc-(1→3)]-Rha-(1→2)-4-OAc-Fuc-,
Glc-(1→3)-Xyl-(1→4)-Rha-(1→2)-4-OAc-Fuc-,
(Ara- or Xyl-)(1→3)-(Ara- or Xyl-)(1→4)-(Rha- or Fuc-)(1→2)-[4-OAc-(Rha- or Fuc-)(1→4)]-(Rha- or Fuc-),
Xyl-(1→3)-Xyl-(1→4)-Rha-(1→2)-[Qui-(1→4)]-Fuc-,
Api-(1→3)-Xyl-(1→4)-[Glc-(1→3)]-Rha-(1→2)-Fuc-,
Xyl-(1->4)-[Gal-(1->3)]-Rha-(1->2)-Fuc-,
Xyl-(1→4)-[Glc-(1→3)]-Rha-(1→2)-Fuc-,
Ara/Xyl-(1→4)-Rha/Fuc-(1→4)-[Glc/Gal-(1→2)]-Fuc-,
Api-(1->3)-Xyl-(1->4)-[Glc-(1->3)]-Rha-(1->2)-[R4-(->4)]-Fuc- wherein R4 is 5-O-[5-O-Ara/Api-3,5-dihydroxy-6-methyl-octanoyl]-3,5-dihydroxy-6-methyl-octanoic acid),
Api-(1->3)-Xyl-(1->4)-Rha-(1->2)-[R5-(->4)]-Fuc- wherein R5 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[Rha-(1→3)]-4-OAc-Fuc-,
Api-(1→3)-Xyl-(1→4)-[Glc-(1→3)]-Rha-(1→2)-[Rha-(1→3)]-4-OAc-Fuc-,
6-OAc-Glc-(1→3)-Xyl-(1→4)-Rha-(1→2)-[3-OAc-Rha-(1→3)]-Fuc-,
Glc-(1→3)-Xyl-(1→4)-Rha-(1→2)-[3-OAc--Rha-(1→3)]-Fuc-,
Xyl-(1→3)-Xyl-(1→4)-Rha-(1→2)-[Qui-(1→4)]-Fuc-,
Glc-(1→3)-[Xyl-(1→4)]-Rha-(1→2)-[Qui-(1→4)]-Fuc-,
Glc-(1→3)-Xyl-(1→4)-Rha-(1→2)-[Xyl-(1→3)-4-OAc-Qui-(1→4)]-Fuc-,
Xyl-(1→3)-Xyl-(1→4)-Rha-(1→2)-[3,4-di-OAc-Qui-(1→4)]-Fuc-,
Glc-(1->3)-[Xyl-(1->4)]-Rha-(1->2)-Fuc-,
6-OAc-Glc-(1→3)-[Xyl-(1→4)]-Rha-(1→2)-Fuc-,
Glc-(1->3)-[Xyl-(1->3)-Xyl-(1->4)]-Rha-(1->2)-Fuc-,
Xyl-(1→3)-Xyl-(1→4)-Rha-(1→2)-[Xyl-(1→3)-4-OAc-Qui-(1→4)]-Fuc-,
Api/Xyl-(1→3)-Xyl-(1→4)-[Glc-(1→3)]-Rha-(1→2)-[Rha-(1→3)]-4OAc-Fuc-,
Api-(1→3)-Xyl-(1→4)-[Glc-(1→3)]-Rha-(1→2)-[Rha-(1→3)]-4OAc-Fuc-,
Api/Xyl-(1→3)-Xyl-(1→4)-[Glc-(1→3)]-Rha-(1→2)-[R6-(→4)]-Fuc- wherein R6 is 5-O-[5-O-Rha-(1->2)-Ara/Api-3,5-dihydroxy-6-methyl-octanoyl]-3,5-dihydroxy-6-methyl-octanoic acid),
Api/Xyl-(1→3)-Xyl-(1→4)-[Glc-(1→3)]-Rha-(1→2)-[R7-(→4)]-Fuc- wherein R7 is 5-O-[5-O-Ara/Api-3,5-dihydroxy-6-methyl-octanoyl]-3,5-dihydroxy-6-methyl-octanoic acid),
Api/Xyl-(1→3)-Xyl-(1→4)-[Glc-(1→3)]-Rha-(1→2)-[R8-(→4)]-Fuc- wherein R8 is 5-O-[5-O-Ara/Api-3,5-dihydroxy-6-methyl-octanoyl]-3,5-dihydroxy-6-methyl-octanoic acid),
Api-(1->3)-Xyl-(1->4)-Rha-(1->2)-[R9-(->4)]-Fuc- wherein R9 is 5-O-[5-O-Ara/Api-3,5-dihydroxy-6-methyl-octanoyl]-3,5-dihydroxy-6-methyl-octanoic acid),
Xyl-(1→3)-Xyl-(1→4)-Rha-(1→2)-[R10-(→4)]-Fuc- wherein R10 is 5-O-[5-O-Ara/Api-3,5-dihydroxy-6-methyl-octanoyl]-3,5-dihydroxy-6-methyl-octanoic acid),
Api-(1->3)-Xyl-(1->4)-Rha-(1->2)-[R11-(->3)]-Fuc- wherein R11 is 5-O-[5-O-Ara/Api-3,5-dihydroxy-6-methyl-octanoyl]-3,5-dihydroxy-6-methyl-octanoic acid),
Xyl-(1->3)-Xyl-(1->4)-Rha-(1->2)-[R12-(->3)]-Fuc- wherein R12 is 5-O-[5-O-Ara/Api-3,5-dihydroxy-6-methyl-octanoyl]-3,5-dihydroxy-6-methyl-octanoic acid)
Glc-(1->3)-[Glc-(1->6)]-Gal-, and
derivatives thereof,
or
• the saponin is a bidesmosidic triterpene glycoside comprising a first saccharide chain selected from the group A bound to the aglycone core structure and comprising a second saccharide chain selected from the group B bound to the aglycone core structure.

4. Saponin conjugate of any one of the claims 1-3, wherein the saponin is selected from the group consisting of: *Quillaja* bark saponin, NP-005236, NP-012672, NP-017777, NP-017778, NP-017774, NP-018110, NP-017772, NP-018109, NP-017888, NP-017889, NP-018108, SA1641, AE X55, NP-017674, NP-017810, AG1, NP-003881, NP-017676, NP-017677, NP-017706, NP-017705, NP-017773, NP-017775, SA1657, AG2, SO1861, GE1741, SO1542, SO1584, SO1658, SO1674, SO1832, SO1862, SO1904, QS-7, QS1861, QS-7 api, QS1862, QS-17, QS-18, QS-21 A-apio, QS-21 A-xylo, QS-21 B-apio, QS-21 B-xylo, beta-Aescin, Aescin Ia, Teaseed saponin I, Teaseedsaponin J, Assamsaponin F, Primula acid 1 and stereoisomers thereof, derivatives thereof, and combinations thereof, preferably the saponin is selected from the group consisting of QS-21, a QS-21 derivative, SO1861, a SO1861 derivative, SA1641, a SA1641 derivative, GE1741, a GE1741 derivative and combinations thereof, more preferably the saponin is selected from the group consisting of a QS-21 derivative, a SO1861 derivative and combinations thereof, most preferably the saponin is a SO1861 derivative.

5. Saponin conjugate of any one of the claims 1 - 4, wherein the saponin is a saponin derivative wherein
i. the saponin derivative comprises an aglycone core structure comprising an aldehyde group which has been derivatised;
ii. the saponin derivative comprises a saccharide chain, preferably a saccharide chain selected from group B as defined in claim 3, the saccharide chain comprising an acetoxy (Me(CO)O-) group which has been derivatised; or
iii. the saponin derivative comprises both derivatisations i. and ii..

6. Saponin conjugate of any one of the claims 1-5, wherein the saponin is any one or more of: SO1861, SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, Quillajasaponin, Saponinum album, QS-18, Quil-A, Gyp1, gypsoside A, AG1, AG2, SO1542, SO1584, SO1658, SO1674, SO1832, SO1904, stereoisomers thereof, derivatives thereof and combinations thereof, preferably the saponin is selected from the group consisting of QS-21, a QS-21 derivative, SO1861, a SO1861 derivative, SA1641, a SA1641 derivative, GE1741, a GE1741 derivative and combinations thereof, more preferably the saponin is selected from the group consisting of a QS-21 derivative, a SO1861 derivative and combinations thereof, most preferably the saponin is a SO1861 derivative.

7. Saponin conjugate of any one of the claims 1-6, wherein the saponin is a saponin derivative of the quillaic acid saponin or the gypsogenin saponin of claim 2 and is represented by Molecule 1: 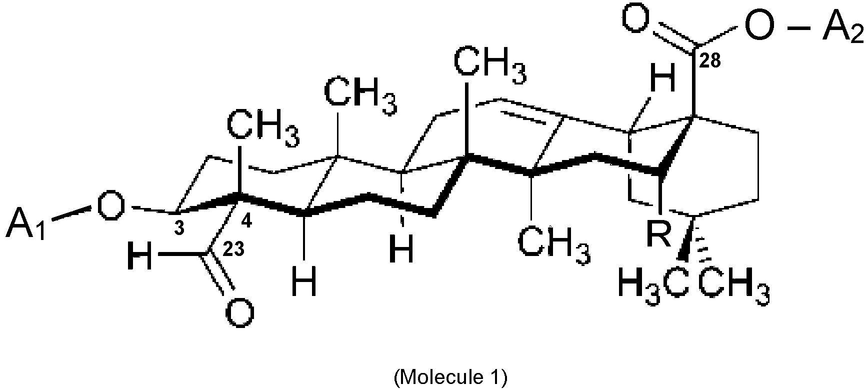 wherein
A₁ represents a saccharide chain selected from group A as defined in claim 1 and A₁ comprises or consists of a glucuronic acid moiety and the carboxyl group of said glucuronic acid moiety of A₁ has been derivatised;
A₂ represents hydrogen, a monosaccharide or a linear or branched oligosaccharide, preferably A₂ represents a saccharide chain selected from group B as defined in claim 3, more preferably A₂ represents a saccharide chain selected from group B as defined in claim 3 and A₂ comprises at least one acetoxy (Me(CO)O-) group, such as one, two, three or four acetoxy groups,
wherein preferably both A₁ and A₂ are an oligosaccharide chain;
and R is hydrogen in gypsogenin or hydroxyl in quillaic acid;
wherein optionally the saponin derivative corresponds to the saponin represented by Molecule 1 wherein the following derivatisation is present:
- the carboxyl group of a glucuronic acid moiety of A₁ has been derivatised by transformation into an amide bond through reaction with 2-amino-2-methyl-1,3-propanediol (AMPD) or N-(2-aminoethyl)maleimide (AEM), therewith providing a saponin-Glu-AMPD such as a QS-21-Glu-AMPD or a SO1861-Glu-AMPD or a saponin-Glu-AEM such as a QS-21-Glu-AEM or a SO1861-Glu-AEM,
wherein preferably A₁ is Gal-(1→2)-[Xyl-(1→3)]-GlcA-.

8. Saponin conjugate of any one of the claims 1-7, wherein the at least one saponin and the ligand for ASGPR are covalently linked directly or via at least one linker, and optionally
wherein the GalNAc moiety is bound to the saponin S, preferably via a saponin linker Lₛ, as shown in formula (II)s: 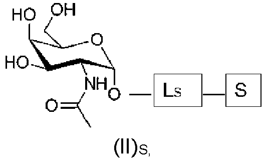 or
wherein the GalNAc moieties are each separately covalently bound via the oxygen on position "1" of the GalNAc moiety to a central bridging moiety B, which effectively forms a bridge between the GalNAc moieties and the saponin moiety, preferably via a saponin moiety linker L_{S}, as shown in formula (III)_{S}: 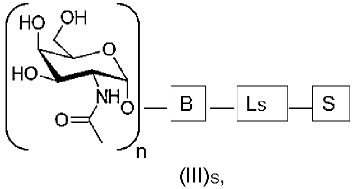
wherein n is an integer larger than or equal to 2, preferably n is 3, L_{S} is a saponin moiety linker and S is the saponin moiety, and optionally
wherein the GalNAc moieties are bound to the bridging moiety B via GalNAc linkers L_{GAL} as shown in formula (IV)s: 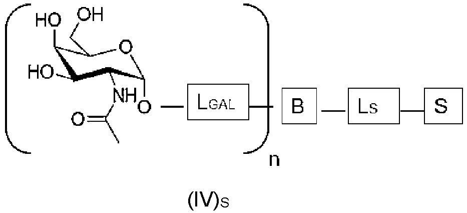
wherein n is an integer larger than or equal to 2, preferably n is 3, L_{S} is a saponin moiety linker and S is the saponin moiety.

9. Saponin conjugate of claim 8, wherein the saponin moiety linker Ls represents any chemical moiety suitable for covalently binding a saponin to GalNAc as in formula (II)s or to the bridging moiety B as in formula (III)s and (IV)s, and optionally
wherein the saponin moiety linker Ls is the result of a coupling reaction between at least a first precursor L_{S1} covalently bound to GalNAc or the bridging moiety B and a second precursor L_{S2} covalently bound to the saponin moiety, wherein L_{S1} is a precursor of the saponin moiety linker L_{S} which is covalently bound to GalNAc or to the bridging moiety B and L_{S2} is a precursor of the saponin moiety linker Ls which is covalently bound to the saponin moiety, and optionally
wherein the saponin moiety linker Ls is the result of a coupling reaction between at least a first precursor L_{S1} covalently bound to GalNAc or the bridging moiety B and a second precursor L_{S2} covalently bound to the saponin moiety, wherein the coupling reaction is selected from the group consisting of an azide-alkyne cycloaddition, a thiol maleimide coupling, a Staudinger reaction, a nucleophilic ring-opening of strained heterocyclic electrophiles, a carbonyl reaction of the non-aldol type and an addition to a carbon-carbon double bond, preferably wherein the coupling reaction is an azide-alkyne cycloaddition, a thiol maleimide coupling, a Staudinger reaction, a nucleophilic ring-opening of strained heterocyclic electrophiles, more preferably wherein the coupling reaction is an azide-alkyne cycloaddition or a thiol maleimide coupling, and optionally,
wherein the saponin moiety linker Ls is the result of a coupling reaction between a first precursor L_{S1} covalently bound to GalNAc or the bridging moiety B, the first precursor L_{S1} comprising an azide; and a second precursor L_{S2} covalently bound to the saponin moiety, the second precursor L_{S2} comprising an alkyne and preferably comprising a hydrazon resulting from a hydrazide/aldehyde coupling to an aldehyde of the saponin moiety, and optionally
wherein the structure for the precursor L_{S1} is the following azide of formula (XVII): 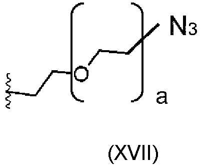
wherein a represents an integer larger than or equal to 0, preferably a represents an integer selected from 1, 2, and 3, more preferably a represents 2, or
wherein the structure for the precursor L_{S1} comprises the following azide of formula (XVIII): 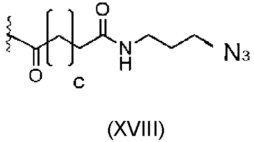
wherein c represents an integer larger than or equal to 0, preferably c represents an integer in the range of 5-15, more preferably c represents 9, and optionally wherein the structure for the precursor L_{S2} comprises the following hydrazone with formula (XIX): 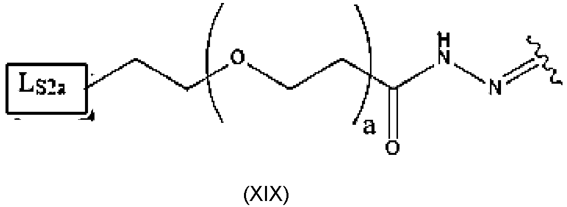
wherein a represents an integer larger than or equal to 0, preferably a represents an integer in the range of 2-6, more preferably a represents 4, and wherein L_{S2a} represents an alkyne-containing moiety.

10. Saponin conjugate of claim 9, wherein L_{S2a} comprises less than 20 carbon atoms, preferably L_{S2a} represents a moiety according to formula (XX):

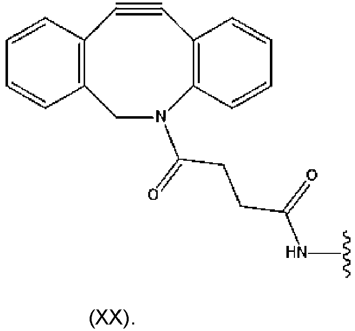

11. Saponin conjugate of any one of claims 8-10, wherein the bridging moiety is a compound of formula (XV): 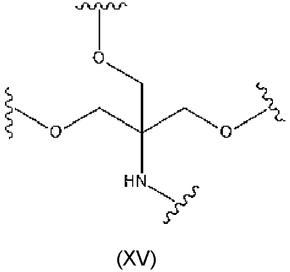
wherein the oxygen atoms of the compound of formula (XV) are bound to GalNAc or to the GalNAc linkers L_{GAL}, and the nitrogen atom of the compound of formula (XV) is bound to the saponin moiety linker Ls, and optionally
wherein L_{GAL} represents any chemical moiety suitable for covalently binding GalNAc to the bridging moiety B, and optionally
wherein L_{GAL} comprises 2-25 carbon atoms, preferably 7-15 carbon atoms, more preferably 11 carbon atoms and wherein L_{GAL} comprises at least one, preferably two amide moieties, and optionally
wherein L_{GAL} is a compound according to formula (XVI):

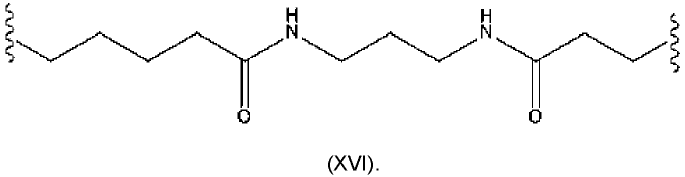

12. Saponin conjugate of any one of claims 8 - 11, wherein the ligand for ASGPR is the (GalNAc)₃Tris represented by Molecule I': 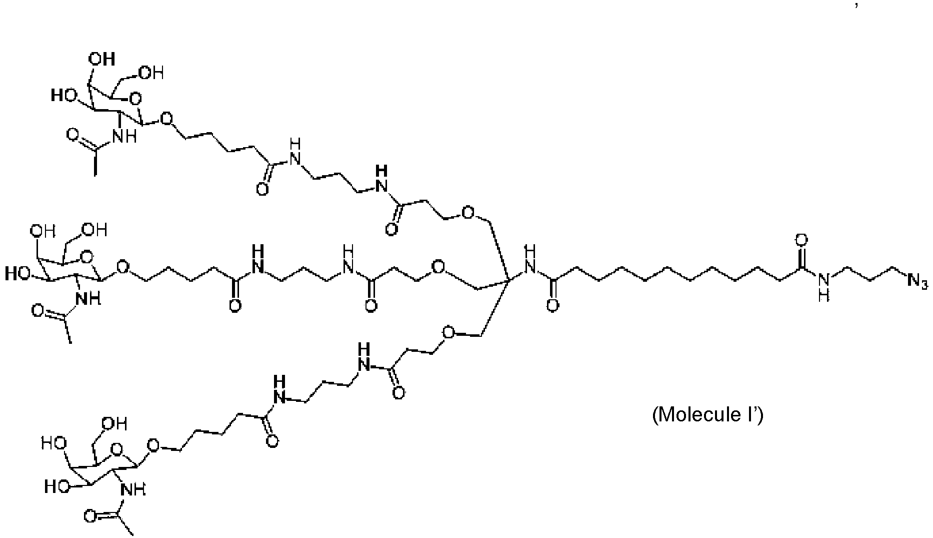 or wherein the ligand for ASGPR is mono-GalNAc represented by Molecule II':

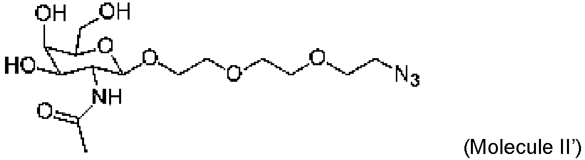

13. Saponin conjugate of any one of the claims 1-12, wherein the at least one saponin is covalently bound to Molecule I' or Molecule II' of claim 12 via a linker represented by Molecule III': 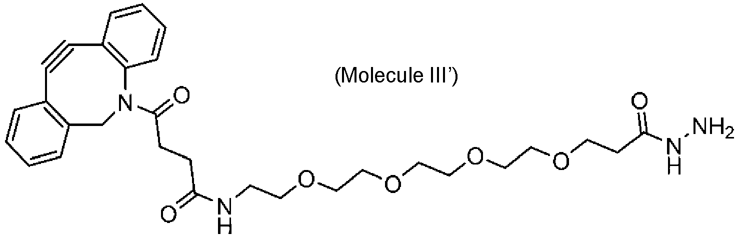 wherein the hydrazide moiety of Molecule III' formed a covalent hydrazone bond with an aldehyde group in the saponin, and wherein the dibenzocyclooctyne group formed a covalent bond with the azide group of Molecule I' or Molecule II'.

14. Saponin conjugate of any one of the claims 1-13, wherein the at least one saponin is covalently bound to the ligand for ASGPR via at least one cleavable linker, and optionally
wherein the cleavable linker is subject to cleavage under acidic conditions, reductive conditions, enzymatic conditions and/or light-induced conditions, and preferably the cleavable linker comprises a cleavable bond selected from a hydrazone bond and a hydrazide bond subject to cleavage under acidic conditions, and/or a bond susceptible to proteolysis, for example proteolysis by Cathepsin B, and/or a bond susceptible for cleavage under reductive conditions such as a disulfide bond, or the cleavable linker is subject to cleavage *in vivo* under acidic conditions such as for example present in endosomes and/or lysosomes of mammalian cells, preferably human cells, preferably the cleavable linker is subject to cleavage *in vivo* at pH 4.0 - 6.5, and more preferably at pH ≤ 5.5.

15. Pharmaceutical combination comprising:
- a first pharmaceutical composition comprising the saponin conjugate of any one of the claims 1-14 and optionally comprising a pharmaceutically acceptable excipient and/or a pharmaceutically acceptable diluent; and
- a second pharmaceutical composition comprising a second conjugate of an effector molecule and a ligand for ASGPR, wherein the ligand for ASGPR preferably comprises at least one GalNAc moiety, preferably three or four GalNAc moieties, more preferably the ligand for ASGPR comprises or consists of (GalNAc)₃Tris, or a third conjugate of an effector molecule and a binding molecule comprising a binding site for a cell-surface molecule, and optionally comprising a pharmaceutically acceptable excipient and/or pharmaceutically acceptable diluent, or
pharmaceutical composition comprising:
- the saponin conjugate of any one of the claims 1-14;
- a second conjugate of an effector molecule and a ligand for ASGPR wherein the ligand for ASGPR preferably comprises at least one GalNAc moiety, preferably three or four GalNAc moieties, more preferably the ligand for ASGPR comprises or consists of (GalNAc)₃Tris, or a third conjugate of an effector molecule and a binding molecule comprising a binding site for a cell-surface molecule, and optionally comprising a pharmaceutically acceptable excipient and/or pharmaceutically acceptable diluent, and optionally
wherein the effector molecule comprises or consists of at least one of a small molecule such as a drug molecule, a toxin such as a protein toxin, an oligonucleotide such as a BNA, a xeno nucleic acid or an siRNA, an enzyme, a peptide, a protein, or any combination thereof, preferably, the effector molecule is a toxin, an enzyme or an oligonucleotide, and optionally
wherein the ligand for ASGPR comprises or is (GalNAc)₃Tris, and/or wherein the ligand for ASGPR and the effector molecule are conjugated via a covalent bond, preferably via at least one linker, and optionally
wherein the effector molecule is an oligonucleotide selected from any one or more of a(n): short interfering RNA (siRNA), short hairpin RNA (shRNA), anti-hairpin-shaped microRNA (miRNA), single-stranded RNA, aptamer RNA, double-stranded RNA (dsRNA), anti-microRNA (anti-miRNA, anti-miR), antisense oligonucleotide (ASO), DNA, antisense DNA, locked nucleic acid (LNA), bridged nucleic acid (BNA), 2'-O,4'-aminoethylene bridged nucleic Acid (BNA^{NC}), BNA-based siRNA, and BNA-based antisense oligonucleotide (BNA-AON), and optionally
wherein the effector molecule is an oligonucleotide selected from any one or more of a(n): anti-miRNA, a BNA-AON or an siRNA, such as BNA-based siRNA, selected from chemically modified siRNA, metabolically stable siRNA and chemically modified, metabolically stable siRNA, and optionally
wherein the effector molecule is an oligonucleotide capable of, for example when present inside a mammalian cell, silencing any one of genes: apolipoprotein B (apoB), HSP27, transthyretin (TTR), proprotein convertase subtilisin/kexin type 9 (PCSK9), delta-aminolevulinate synthase 1 (ALAS1), antithrombin 3 (AT3), glycolate oxidase (GO), complement component C5 (CC5), X gene of hepatitis B virus (HBV), S gene of HBV, alpha-1 antitrypsin (AAT) and lactate dehydrogenase (LDH), and/or is an oligonucleotide capable of, for example when present inside a mammalian cell, targeting an aberrant miRNA, and optionally
wherein the effector molecule is an oligonucleotide, preferably an siRNA such as a BNA-based siRNA, or an antisense oligonucleotide such as a BNA-based antisense oligonucleotide, for example an AON based on 2'-O,4'-aminoethylene bridged nucleic acid, capable of, preferably when present inside a mammalian cell, silencing gene apolipoprotein B (apoB), and optionally
wherein the effector molecule is an oligonucleotide capable of, for example when present inside a mammalian cell, targeting an mRNA involved in expression of any one of proteins: apoB, HSP27, TTR, PCSK9, ALAS1, AT3, GO, CC5, expression product of X gene of HBV, expression product of S gene of HBV, AAT and LDH, or is capable of, for example when present inside a mammalian cell, antagonizing or restoring an miRNA function such as inhibiting an oncogenic miRNA (onco-miR) or suppression of expression of an onco-miR, and optionally,
wherein the effector molecule is an oligonucleotide, preferably an siRNA such as a BNA-based siRNA, or an antisense oligonucleotide such as a BNA-based antisense oligonucleotide, for example an AON based on 2'-O,4'-aminoethylene bridged nucleic acid, capable of, preferably when present inside a mammalian cell, targeting an mRNA involved in expression of protein apoB.

16. Pharmaceutical combination or pharmaceutical composition of claim 15, wherein the binding molecule comprising a binding site for a cell-surface molecule, comprised by the third conjugate, is a ligand for a cell-surface molecule or an antibody comprising a binding site for a cell-surface molecule or at least one domain or fragment thereof comprising the binding site, and optionally wherein the third conjugate is any one or more of: an antibody-toxin conjugate, a receptor-ligand - toxin conjugate, an antibody-drug conjugate, a receptor-ligand - drug conjugate, an antibody-nucleic acid conjugate or a receptor-ligand - nucleic acid conjugate, and optionally
wherein the binding molecule comprising a binding site for a cell-surface molecule, comprised by the third conjugate, is capable of binding to any one of cell-surface molecules: CD71, CA125, EpCAM(17-1A), CD52, CEA, CD44v6, FAP, EGF-IR, integrin, syndecan-1, vascular integrin alphaV beta-3, HER2, EGFR, CD20, CD22, Folate receptor 1, CD146, CD56, CD19, CD138, CD27L receptor, PSMA, CanAg, integrin-alphaV, CA6, CD33, mesothelin, Cripto, CD3, CD30, CD239, CD70, CD123, CD352, DLL3, CD25, ephrinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, asialoglycoprotein receptor (ASGPR), preferably any one of: HER2, CD71, ASGPR and EGFR, more preferably CD71, and optionally
wherein the binding molecule comprising a binding site for a cell-surface molecule, comprised by the third conjugate, is or comprises any one of: an antibody, preferably a monoclonal antibody such as a human monoclonal antibody, an IgG, a molecule comprising or consisting of a single-domain antibody, at least one V_{HH} domain, preferable a camelid V_{H}, a variable heavy chain new antigen receptor (V_{NAR}) domain, a Fab, an scFv, an Fv, a dAb, an F(ab)2 and a Fcab fragment.

17. Pharmaceutical combination or pharmaceutical composition of claims 15 or 16, for use as a medicament, and optionally
for use in the treatment or prophylaxis of a disease or health problem in which an expression product is involved of any one or more of genes: apoB, TTR, PCSK9, ALAS1, AT3, GO, CC5, X gene of HBV, S gene of HBV, AAT and LDH, and optionally for use in the treatment or prophylaxis of a disease or health problem in which an expression product is involved of gene apoB, and optionally for use in the treatment or prophylaxis of a cancer, an infectious disease, a viral infection, hypercholesterolemia, primary hyperoxaluria, haemophilia A, haemophilia B, AAT related liver disease, acute hepatic porphyria, TTR-mediated amyloidosis, hereditary TTR amyloidosis (hATTR), complement-mediated disease, hepatitis B infection, or an auto-immune disease.

18. *In vitro* or ex *vivo* method for transferring the second conjugate or the third conjugate of any one of the claims 14-15 from outside a cell to inside said cell, preferably subsequently transferring the effector molecule comprised by the second conjugate or the third conjugate of any one of the claims 14-15 into the cytosol of said cell, comprising the steps of:
providing a cell which expresses ASGPR on its surface, and, when the third conjugate is to be transferred into the cell, which expresses the cell-surface molecule for which the third conjugate comprises a binding molecule for binding to said cell-surface molecule, the cell preferably selected from a liver cell, a virally infected cell and a tumor cell;
providing the second conjugate or the third conjugate of any one of the claims 14-15 for transferring into the cell provided in step a);
providing the saponin conjugate of any one of the claims 1-14;
contacting the cell of step a) *in vitro* or *ex vivo* with the second conjugate or the third conjugate of step b) and the saponin conjugate of step c), therewith effecting the transfer of the second conjugate or the third conjugate from outside the cell into said cell, and preferably therewith subsequently effecting the transfer of the second conjugate or the third conjugate into the cytosol of said cell, or preferably therewith subsequently effecting the transfer of at least the effector molecule comprised by the second conjugate or the third conjugate into the cytosol of said cell.

## Patentansprüche

1. Saponinkonjugat, das mindestens ein Saponin umfasst, das kovalent an einen Liganden für Asialoglykoproteinrezeptor (ASGPR) gebunden ist, wobei der Ligand für ASGPR mindestens eine N-Acetylgalactosamin (GalNAc)-Gruppe umfasst, bevorzugt drei oder vier GalNAc-Gruppen, mehr bevorzugt umfasst oder besteht der Ligand für ASGPR aus (GalNAc)₃Tris, wobei das mindestens eine Saponin aus monodesmosidischen Triterpenoidsaponinen und bidesmosidischen Triterpenoidsaponinen ausgewählt ist, wobei das Saponin ein Saponinderivat ist, das eine an die Aglykon-Kernstruktur gebundene Saccharidkette umfasst, die aus Gruppe A ausgewählt ist:
GlcA-
Gal-(1→2)-[Xyl-(1→3)]-GlcA-,
Glc-(1→2)-[Glc-(1→4)]-GlcA-,
Glc-(1→2)-Ara-(1→3)-[Gal-(1→2)]-GlcA-,
Xyl-(1→2)-Ara-(1→3)-[Gal-(1→2)]-GlcA-,
Rha-(1→2)-Gal-(1→3)-[Glc-(1→2)]-GlcA-,
Ara-(1→4)-Rha-(1→2)-Glc-(1→2)-Rha-(1→2)-GlcA-,
Ara-(1→4)-Fuc-(1→2)-Glc-(1→2)-Rha-(1→2)-GlcA-,
Ara-(1→4)-Rha-(1→2)-Gal-(1→2)-Rha-(1→2)-GlcA-,
Ara-(1→4)-Fuc-(1→2)-Gal-(1→2)-Rha-(1→2)-GlcA-,
Ara-(1→4)-Rha-(1→2)-Glc-(1→2)-Fuc-(1→2)-GlcA-,
Ara-(1→4)-Fuc-(1→2)-Glc-(1→2)-Fuc-(1→2)-GlcA-,
Ara-(1→4)-Rha-(1→2)-Gal-(1→2)-Fuc-(1→2)-GlcA-,
Ara-(1→4)-Fuc-(1→2)-Gal-(1→2)-Fuc-(1→2)-GlcA-,
Xyl-(1→4)-Rha-(1→2)-Glc-(1→2)-Rha-(1→2)-GlcA-,
Xyl-(1→4)-Fuc-(1→2)-Glc-(1→2)-Rha-(1→2)-GlcA-,
Xyl-(1→4)-Rha-(1→2)-Gal-(1→2)-Rha-(1→2)-GlcA-,
Xyl-(1→4)-Fuc-(1→2)-Gal-(1→2)-Rha-(1→2)-GlcA-,
Xyl-(1→4)-Rha-(1→2)-Glc-(1→2)-Fuc-(1→2)-GlcA-,
Xyl-(1→4)-Fuc-(1→2)-Glc-(1→2)-Fuc-(1→2)-GlcA-,
Xyl-(1→4)-Rha-(1→2)-Gal-(1→2)-Fuc-(1→2)-GlcA-,
Xyl-(1→4)-Fuc-(1→2)-Gal-(1→2)-Fuc-(1→2)-GlcA-, und
Derivaten davon,
wobei besagte Saccharidkette eine Carboxygruppe umfasst, die derivatisiert wurde und die Carboxygruppe, die derivatisiert wurde, ist Teil einer Glucuronsäuregruppe.

2. Saponinkonjugat nach Anspruch 1, wobei das Saponin eine Aglykon-Kernstruktur umfasst, ausgewählt aus der Gruppe bestehend aus:
2alpha-Hydroxyoleanolsäure;
16alpha-Hydroxyoleanolsäure;
Hederagenin (23-Hydroxyoleanolsäure);
16alpha,23-Dihydroxyoleanolsäure;
Gypsogenin;
Quillainsäure;
Protoaescigenin-21(2-methylbut-2-enoat)-22-acetat;
23-Oxo-Barringtogenol C-21,22-bis(2-methylbut-2-enoat);
23-Oxo-Barringtogenol C-21 (2-Methylbut-2-enoat)-16,22-diacetat;
Digitogenin;
3,16,28-Trihydroxyoleanan-12-en;
Gypsogensäure,
und
Derivaten davon,
bevorzugt umfasst das Saponin eine Aglykon-Kernstruktur, ausgewählt aus Quillainsäure und Gypsogenin oder Derivaten davon, mehr bevorzugt ist die Saponin-Aglykon-Kernstruktur Quillainsäure oder ein Derivat davon.

3. Saponinkonjugat nach Anspruch 1 oder 2, wobei
• das Saponin eine an die Aglykon-Kernstruktur gebundene Saccharidkette umfasst, die aus Gruppe B ausgewählt ist:
Glc-,
Gal-,
Rha-(1→2)-[Xyl-(1→4)]-Rha-,
Rha-(1→2)-[Ara-(1→3)-Xyl-(1→4)]-Rha-,
Ara-,
Xyl-,
Xyl-(1→4)-Rha-(1→2)-[R1-(→4)]-Fuc-, wobei R1 4E-Methoxyzimtsäure ist,
Xyl-(1→4)-Rha-(1→2)-[R2-(→4)]-Fuc-, wobei R2 4Z-Methoxyzimtsäure ist,
Xyl-(1→4)-[Gal-(1→3)]-Rha-(1→2)-4-OAc-Fuc-,
Xyl-(1→4)-[Glc-(1→3)]-Rha-(1→2)-3,4-di-OAc-Fuc-,
Xyl-(1→4)-[Glc-(1→3)]-Rha-(1→2)-[R3-(→4)]-3-OAc-Fuc- wobei R3 4E-Methoxyzimtsäure ist,
Glc-(1→3)-Xyl-(1→4)-[Glc-(1→3)]-Rha-(1→2)-4-OAc-Fuc-,
Glc-(1→3) -Xyl-(1→4)-Rha-(1→2)-4-OAc-Fuc-,
(Ara- oder Xyl-)(1→3)-(Ara- oder Xyl-)(1→4)-(Rha- oder Fuc-)(1→2)-[4-OAc-(Rha- oder Fuc-)(1→4)]-(Rha- oder Fuc-),
Xyl-(1→3)-Xyl-(1→4)-Rha-(1→2)-[Qui-(1→4)]-Fuc-,
Api-(1→3)-Xyl-(1→4)-[Glc-(1→3)]-Rha-(1→2)-Fuc-,
Xyl-(1→4)-[Gal-(1→3)]-Rha-(1→2)-Fuc-,
Xyl-(1→4)-[Glc-(1→3)]-Rha-(1→2)-Fuc-,
Ara/Xyl-(1→4)-Rha/Fuc-(1→4)-[Glc/Gal-(1→2)]-Fuc-,
Api-(1→3)-Xyl-(1→4)-[Glc-(1→3)]-Rha-(1→2)-[R4-(→4)]-Fuc- wobei R4 5-O-[5-O-Ara/Api-3,5-Dihydroxy-6-Methyl-Octanoyl]-3,5-Dihydroxy-6-Methyl-Octansäure) ist,
Api-(1→3)-Xyl-(1→4)-Rha-(1→2)-[R5-(→4)]-Fuc- wobei R5 5-O-[5-O-Ara/Api-3,5-dihydroxy-6-methyl-octanoyl]-3,5-dihydroxy-6-methyl-octansäure) ist,
Api-(1→3)-Xyl-(1→4)-Rha-(1→2)-[Rha-(1→3)]-4-OAc-Fuc-,
Api-(1→3)-Xyl-(1→4)-[Glc-(1→3)]-Rha-(1→2)-[Rha-(1→3)]-4-OAc-Fuc-,
6-OAc-Glc-(1→3)-Xyl-(1→4) -Rha-(1→2)-[3-OAc-Rha-(1→3)]-Fuc-,
Glc-(1→3)-Xyl-(1→4)-Rha-(1→2)-[3-OAc-Rha-(1→3)]-Fuc-,
Xyl-(1→3)-Xyl-(1→4)-Rha-(1→2)-[Qui-(1→4)]-Fuc-,
Glc-(1→3)-[Xyl-(1→4)]-Rha-(1→2)-[Qui-(1→4)]-Fuc-,
Glc-(1→3)-Xyl-(1→4)-Rha-(1→2)-[Xyl-(1→3)-4-OAc-Qui-(1→4)]-Fuc-,
Xyl-(1→3)-Xyl-(1→4)-Rha-(1→2)-[3,4-di-OAc-Qui-(1 →4)]-Fuc-,
Glc-(1→3)-[Xyl-(1→4)]-Rha-(1→2)-Fuc-,
6-OAc-Glc-(1→3)-[Xyl-(1→4)]-Rha-(1→2)-Fuc-,
Glc-(1→3)-[Xyl-(1→3)-Xyl-(1→4)]-Rha-(1→2)-Fuc-,
Xyl-(1→3)-Xyl-(1→4)-Rha-(1→2)-[Xyl-(1→3)-4-OAc-Qui-(1→4)]-Fuc-,
Api/Xyl-(1→3)-Xyl-(1→4)-[Glc-(1→3)]-Rha-(1→2)-[Rha-(1→3)]-4OAc-Fuc-,
Api-(1→3)-Xyl-(1→4)-[Glc-(1→3)]-Rha-(1→2)-[Rha-(1→3)]-4OAc-Fuc-,
Api/Xyl-(1→3)-Xyl-(1→4)-[Glc-(1→3)]-Rha-(1→2)-[R6-(→4)]-Fuc-wobei R6 5-O-[5-O-Rha-(1→2)-Ara/Api-3,5-dihydroxy-6-methyl-octanoyl]-3,5-dihydroxy-6-methyl-octansäure) ist,
Api/Xyl-(1→3)-Xyl-(1→4)-[Glc-(1→3)]-Rha-(1→2)-[R7-(→4)]-Fuc- wobei R7 5-O-[5-O-Ara/Api-3,5-dihydroxy-6-methyl-octanoyl]-3,5-dihydroxy-6-methyl-octansäure),
Api/Xyl-(1→3)-Xyl-(1→4)-[Glc-(1→3)]-Rha-(1→2)-[R8-(→4)]-Fuc- wobei R8 5-O-[5-O-Ara/Api-3,5-dihydroxy-6-methyl-octanoyl]-3,5-dihydroxy-6-methyl-octansäure) ist,
Api-(1→3)-Xyl-(1→4)-Rha-(1→2)-[R9-(→4)]-Fuc- wobei R9 5-O-[5-O-Ara/Api-3,5-dihydroxy-6-methyl-octanoyl]-3,5-dihydroxy-6-methyl-octansäure) ist,
Xyl-(1→3)-Xyl-(1→4)-Rha-(1→2)-[R10-(→4)]-Fuc- wobei R10 5-O-[5-O-Ara/Api-3,5-dihydroxy-6-methyl-octanoyl]-3,5-dihydroxy-6-methyl-octansäure ist),
Api-(1→3)-Xyl-(1→4)-Rha-(1→2)-[R11-(→3)]-Fuc- wobei R11 5-O-[5-O-Ara/Api-3,5-dihydroxy-6-methyl-octanoyl]-3,5-dihydroxy-6-methyl-octansäure ist),
Xyl-(1→3)-Xyl-(1→4)-Rha-(1→2)-[R12-(→3)]-Fuc- wobei R12 5-O-[5-O-Ara/Api-3,5-dihydroxy-6-methyl-octanoyl]-3,5-dihydroxy-6-methyl-octansäure) ist,
Glc-(1→3)-[Glc-(1→6)]-Gal- und
Derivaten davon,
oder
• das Saponin ein bidesmosidisches Triterpenglykosid ist, das eine erste Saccharidkette umfasst, die aus der Gruppe A ausgewählt ist, die an die Aglykon-Kernstruktur gebunden ist, und eine zweite Saccharidkette umfasst, die aus der Gruppe B ausgewählt ist, die an die Aglykon-Kernstruktur gebunden ist.

4. Saponinkonjugat nach einem der Ansprüche 1-3, wobei das Saponin ausgewählt ist aus der Gruppe bestehend aus: Quillaja-Rindensaponin, NP-005236, NP-012672, NP-017777, NP-017778, NP-017774, NP-018110, NP-017772, NP-018109, NP-017888, NP-017889, NP-018108, SA1641, AE X55, NP-017674, NP-017810, AG1, NP-003881, NP-017676, NP-017677, NP-017706, NP-017705, NP-017773, NP-017775, SA1657, AG2, SO1861, GE1741, SO1542, SO1584, SO1658, SO1674, SO1832, SO1862, SO1904, QS-7, QS1861, QS-7 api, QS1862, QS-17, QS-18, QS-21 A-apio, QS-21 A-xylo, QS-21 B-apio, QS-21 B-xylo, beta-Aescin, Aescin la, Teesaat-Saponin l, Teesaat-Saponin J, Assam-Saponin F, Primula-Säure 1 und Steroisomere davon, Derivaten davon und Kombinationen davon, wobei das Saponin bevorzugt aus der Gruppe ausgewählt ist, die aus QS-21, einem QS-21-Derivat, SO1861, einem SO1861-Derivat, SA1641, einem SA1641-Derivat, GE1741, einem GE1741-Derivat und Kombinationen davon, mehr bevorzugt ist das Saponin ausgewählt aus der Gruppe bestehend aus einem QS-21-Derivat, einem SO1861-Derivat und Kombinationen davon, am meisten bevorzugt ist das Saponin ein SO1861-Derivat.

5. Saponinkonjugat nach einem der Ansprüche 1-4, wobei das Saponin ein Saponinderivat ist, wobei
i. das Saponinderivat eine Aglykon-Kernstruktur umfasst, die eine Aldehydgruppe umfasst, die derivatisiert wurde;
ii. das Saponinderivat eine Saccharidkette umfasst, bevorzugt eine Saccharidkette, ausgewählt aus Gruppe B, wie in Anspruch 3 definiert, wobei die Saccharidkette eine Acetoxygruppe (Me(CO)O-) umfasst, die derivatisiert wurde; oder
iii. das Saponinderivat beide Derivatisierungen i. und ii. umfasst.

6. Saponinkonjugat nach einem der Ansprüche 1-5, wobei das Saponin eines oder mehrere ist von: SO1861, SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, Quillaja-Saponin, Saponinum album, QS-18, Quil-A, Gyp1, Gypsosid A, AG1, AG2, SO1542, SO1584, SO1658, SO1674, SO1832, SO1904, deren Stereoisomere, Derivate und Kombinationen davon, bevorzugt ist das Saponin ausgewählt aus der Gruppe bestehend aus QS-21, einem QS-21-Derivat, SO1861, einem SO1861-Derivat, SA1641, einem SA1641-Derivat, GE1741, einem GE1741-Derivat und Kombinationen davon, mehr bevorzugt wird das Saponin aus der Gruppe bestehend aus einem QS-21-Derivat, einem SO1861-Derivat und Kombinationen davon ausgewählt, am meisten bevorzugt ist das Saponin ein SO1861-Derivat.

7. Saponinkonjugat nach einem der Ansprüche 1-6, wobei das Saponin ein Saponinderivat des Quillainsäure-Saponins oder des Gypsogenin-Saponins nach Anspruch 2 ist und durch Molekül 1 dargestellt wird: 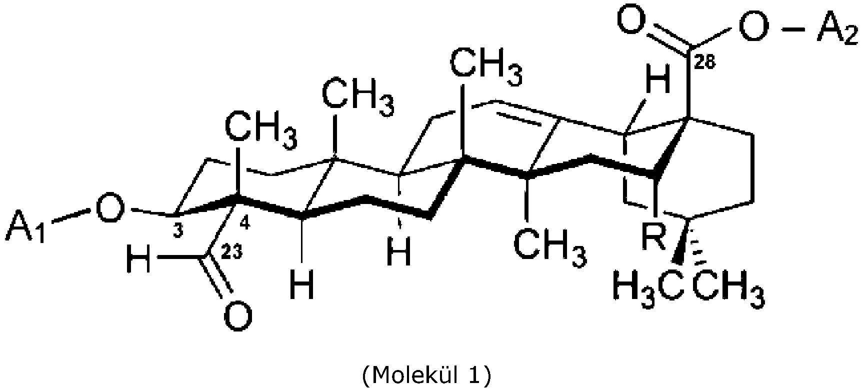 wobei
A₁ eine Saccharidkette darstellt, die aus der in Anspruch 1 definierten Gruppe A ausgewählt ist, und A₁ eine Glucuronsäureeinheit umfasst oder aus dieser besteht und die Carboxylgruppe besagter Glucuronsäureeinheit von A₁ derivatisiert wurde;
A₂ Wasserstoff, ein Monosaccharid oder ein lineares oder verzweigtes Oligosaccharid darstellt, bevorzugt A₂ eine Saccharidkette darstellt, die aus Gruppe B wie in Anspruch 3 definiert ausgewählt ist, mehr bevorzugt A₂ eine Saccharidkette darstellt, die aus Gruppe B, wie in Anspruch 3 definiert, ausgewählt ist, und A₂ mindestens eine Acetoxygruppe (Me(CO)O-) umfasst, wie etwa eine, zwei, drei oder vier Acetoxygruppen,
bevorzugt wobei sowohl A₁ als auch A₂ eine Oligosaccharidkette sind;
und R ist Wasserstoff in Gypsogenin oder Hydroxyl in Quillainsäure ist;
wobei das Saponinderivat optional dem durch Molekül 1 dargestellten Saponin entspricht, wobei die folgende Derivatisierung vorliegt:
- die Carboxylgruppe einer Glucuronsäureeinheit von A₁ durch Umwandlung in eine Amidbindung derivatisiert wurde indem sie mit 2-Amino-2-methyl-1,3-propandiol (AMPD) oder N-(2-Aminoethyl)maleimid (AEM) reagiert wurde, wodurch ein Saponin-Glu-AMPD wie etwa ein QS-21-Glu- AMPD oder ein SO1861-Glu-AMPD oder ein Saponin-Glu-AEM wie etwa ein QS-21-Glu-AEM oder ein SO1861- Glu-AEM bereitgestellt wird,
bevorzugt wobei A₁ Gal-(1→2)-[Xyl-(1→3)]-GlcA- ist.

8. Saponinkonjugat nach einem der Ansprüche 1-7, wobei das mindestens eine Saponin und der Ligand für ASGPR direkt oder über mindestens einen Linker kovalent gebunden sind und wobei optional die GalNAc-Gruppe an das Saponin S gebunden ist, bevorzugt über einen Saponin-Linker L_{S}, wie in Formel (ll)s dargestellt: 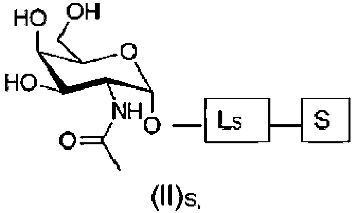
wobei die GalNAc-Gruppen jeweils separat kovalent über den Sauerstoff an Position "1" der GalNAc-Gruppe an eine zentrale Brückengruppe B gebunden sind, die effektiv eine Brücke zwischen den GalNAc-Gruppen und der Saponin-Gruppe bildet, bevorzugt über einen Saponin-Gruppen-Linker L_{S}, wie in Formel (III)s gezeigt: 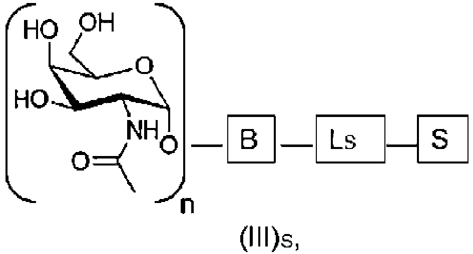
wobei n eine ganze Zahl größer oder gleich 2 ist, bevorzugt n 3 ist, L_{S} ein Saponin-Gruppen-Linker ist und S die Saponin-Gruppe ist, und optional
wobei die GalNAc-Gruppen über GalNAc-Linker L_{GAL} an die Brückengruppe B gebunden sind, wie in Formel (IV)s gezeigt: 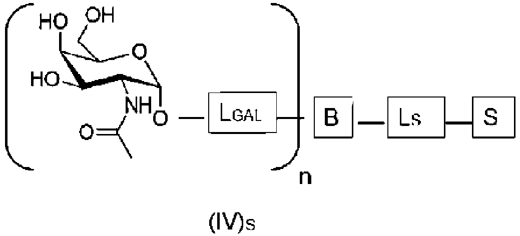
wobei n eine ganze Zahl größer oder gleich 2 ist, bevorzugt n gleich 3 ist, L_{S} ein Saponin-Gruppen-Linker ist und S die Saponin-Gruppe ist.

9. Saponinkonjugat nach Anspruch 8, wobei der Saponin-Gruppen-Linker L_{S} beliebige chemische Gruppen darstellt, die zur kovalenten Bindung eines Saponins an GalNAc wie in Formel (ll)s oder an die Brückengruppe B wie in Formel (lll)s und (lV)s geeignet sind, und optional
wobei der Saponin-Gruppen-Linker L_{S} das Ergebnis einer Kupplungsreaktion zwischen mindestens einem ersten Vorgänger L_{S1}, der kovalent an GalNAc oder die Brückengruppe B gebunden ist, und einem zweiten Vorgänger L_{S2}, der kovalent an die Saponin-Gruppe gebunden ist, wobei L_{S1} ein Vorgänger des Saponin-Gruppen-Linkers L_{S} ist, der kovalent an GalNAc oder die Brückengruppe B gebunden ist, und L_{S2} ein Vorgänger des Saponin-Gruppen-Linkers L_{S} ist , der kovalent an die Saponin-Gruppe gebunden ist, und optional,
wobei der Saponin-Gruppen-Linker L_{S} das Ergebnis einer Kupplungsreaktion zwischen mindestens einem ersten Vorgänger L_{S1}, der kovalent an GalNAc oder die Brückengruppe B gebunden ist, und einem zweiten Vorgänger L_{S2}, der kovalent an die Saponin-Gruppe gebunden ist, wobei die Kupplungsreaktion ausgewählt ist aus der Gruppe bestehend aus einer Azid-Alkin-Cycloaddition, einer Thiol-Maleimid-Kupplung, einer Staudinger-Reaktion, einer nukleophilen Ringöffnung von gespannten heterocyclischen Elektrophilen, einer Carbonylreaktion des Nicht-Aldoltyps und eine Addition an eine Kohlenstoff-Kohlenstoff-Doppelbindung, bevorzugt, wobei die Kupplungsreaktion eine Azid-Alkin-Cycloaddition, eine Thiol-Maleimid-Kupplung, eine Staudinger-Reaktion, eine nukleophile Ringöffnung von gespannten heterocyclischen Elektrophilen, mehr bevorzugt, wobei die Kupplungsreaktion eine Azid-Alkin-Cycloaddition oder eine Thiol-Maleimid-Kupplung ist, und optional,
wobei der Saponin-Linker L_{S} das Ergebnis einer Kupplungsreaktion zwischen einem ersten Vorgänger L_{S1}, der kovalent an GalNAc oder die Brückengruppe B gebunden ist, wobei der erste Vorgänger L_{S1} ein Azid umfasst; und einem zweiten Vorgänger L_{S2}, der kovalent an die Saponin-Gruppe gebunden ist, wobei der zweite Vorgänger L_{S2} ein Alkin und bevorzugt ein Hydrazon umfasst, das aus einer Hydrazid/Aldehyd-Kupplung an einen Aldehyd der Saponin-Gruppe resultiert, und optional
wobei die Struktur für den Vorgänger L_{S1} das folgende Azid der Formel (XVII) ist: 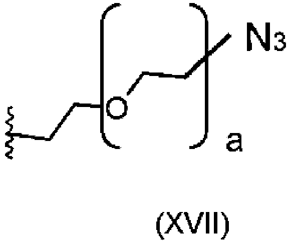
wobei a eine ganze Zahl größer oder gleich 0 darstellt, bevorzugt a eine ganze Zahl darstellt, ausgewählt aus 1, 2 und 3, noch bevorzugter a 2 darstellt, oder
wobei die Struktur für den Vorgänger L_{S1} das folgende Azid der Formel (XVIII) umfasst: 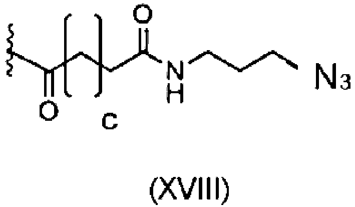
wobei c eine ganze Zahl größer oder gleich 0 darstellt, bevorzugt c eine ganze Zahl im Bereich von 5-15 darstellt, mehr bevorzugt c 9 darstellt, und optional, wobei die Struktur für den Vorgänger L_{S2} das folgende Hydrazon mit Formel (XIX) umfasst: 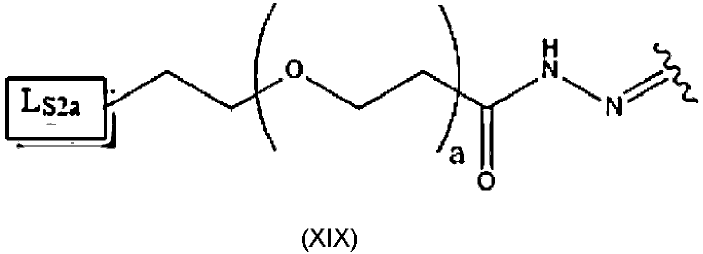
wobei a eine ganze Zahl größer oder gleich 0 darstellt, bevorzugt a eine ganze Zahl im Bereich von 2-6 darstellt, mehr bevorzugt a 4 darstellt, und wobei L_{S2a} eine Alkin-enthaltende Gruppe darstellt.

10. Saponinkonjugat nach Anspruch 9, wobei L_{S2a} weniger als 20 Kohlenstoffatome umfasst, bevorzugt L_{S2a} eine Gruppe nach Formel (XX) darstellt:

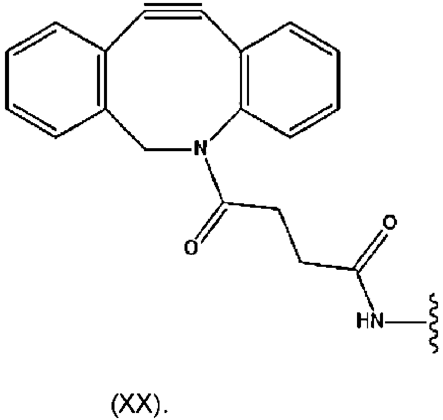

11. Saponinkonjugat nach einem der Ansprüche 8-10, wobei die Brückengruppe eine Verbindung der Formel (XV) ist: 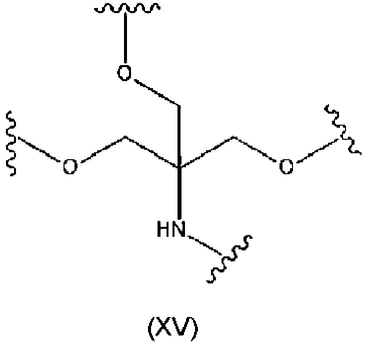
wobei die Sauerstoffatome der Verbindung der Formel (XV) an GalNAc oder an die GalNAc-Linker L_{GAL} gebunden sind und das Stickstoffatom der Verbindung der Formel (XV) an den Saponin-Gruppen-Linker L_{S} gebunden ist, und optional
wobei L_{GAL} einen chemischen Rest darstellt, der zur kovalenten Bindung von GalNAc an die Brückengruppe B geeignet ist, und optional
wobei L_{GAL} 2-25 Kohlenstoffatome, bevorzugt 7-15 Kohlenstoffatome, mehr bevorzugt 11 Kohlenstoffatome umfasst und wobei L_{GAL} mindestens eine, bevorzugt zwei Amidgruppen umfasst, und optional
wobei L_{GAL} eine Verbindung nach Formel (XVI) ist:

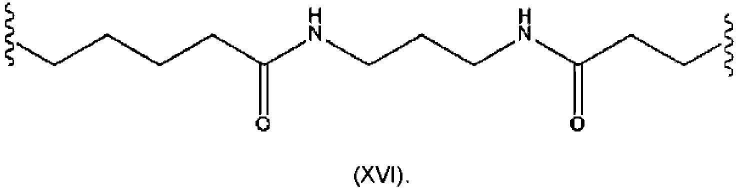

12. Saponinkonjugat nach einem der Ansprüche 8-11, wobei der Ligand für ASGPR das (GalNAc)₃Tris, dargestellt durch Molekül I', ist: 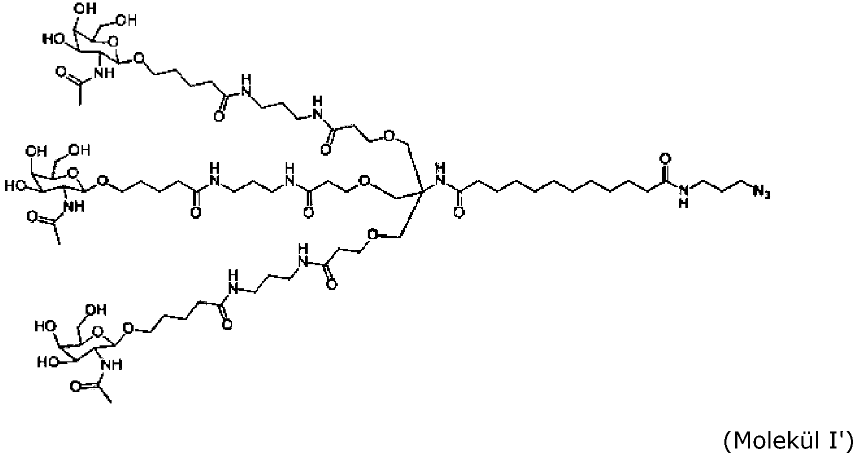 oder wobei der Ligand für ASGPR mono-GalNAc, dargestellt durch Molekül II', ist:

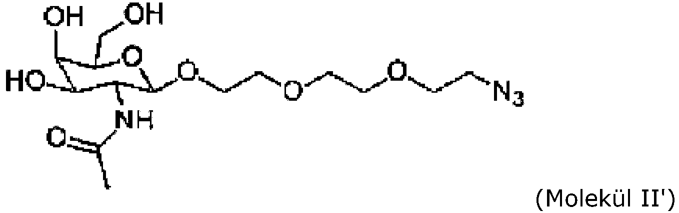

13. Saponinkonjugat nach einem der Ansprüche 1-12, wobei das mindestens eine Saponin kovalent an Molekül I' oder Molekül II' nach Anspruch 12 über einen durch Molekül III' dargestellten Linker gebunden ist: 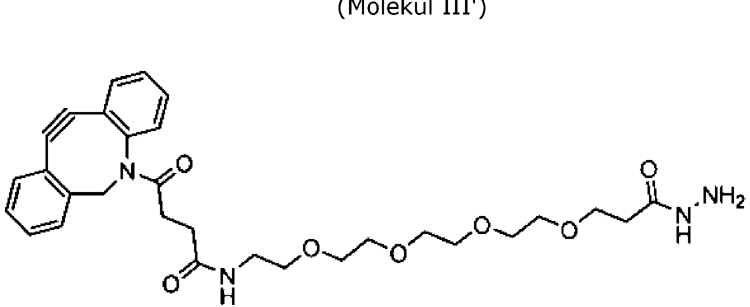 wobei die Hydrazidgruppe von Molekül III' eine kovalente Hydrazonbindung mit einer Aldehydgruppe im Saponin bildete und wobei die Dibenzocyclooctingruppe eine kovalente Bindung mit der Azidgruppe von Molekül I' oder Molekül II' bildete.

14. Saponinkonjugat nach einem der Ansprüche 1-13, wobei das mindestens eine Saponin über mindestens einen spaltbaren Linker kovalent an den Liganden für ASGPR gebunden ist, und optional,
wobei der spaltbare Linker unter sauren Bedingungen, reduktiven Bedingungen, enzymatischen Bedingungen und/oder lichtinduzierten Bedingungen Spaltung unterliegt und der spaltbare Linker bevorzugt eine spaltbare Bindung umfasst, ausgewählt aus einer Hydrazonbindung und einer Hydrazidbindung, die unter sauren Bedingungen gespalten wird, und/oder eine Bindung, die für Proteolyse empfänglich ist, zum Beispiel Proteolyse durch Cathepsin B, und/oder eine Bindung, die für Spaltung unter reduzierenden Bedingungen empfänglich ist, wie eine Disulfidbindung, oder der spaltbare Linker unterliegt einer Spaltung in vivo unter sauren Bedingungen, wie etwa, zum Beispiel gegenwärtig in Endosomen und/oder Lysosomen von Säugetierzellen, bevorzugt menschlichen Zellen, bevorzugt, wobei der spaltbare Linker einer Spaltung in vivo bei einem pH von 4,0-6,5 und mehr bevorzugt bei einem pH ≤ 5 unterliegt.

15. Pharmazeutische Kombination, umfassend:
- eine erste pharmazeutische Zusammensetzung, die das Saponinkonjugat aus einem der Ansprüche 1-14 und optional einen pharmazeutisch akzeptablen Hilfsstoff und/oder ein pharmazeutisch akzeptables Verdünnungsmittel umfasst; und
- eine zweite pharmazeutische Zusammensetzung, die ein zweites Konjugat eines Effektormoleküls und eines Liganden für ASGPR umfasst, wobei der Ligand für ASGPR bevorzugt mindestens eine GalNAc-Gruppe, bevorzugt drei oder vier GalNAc-Gruppen umfasst, mehr bevorzugt umfasst oder besteht der Ligand für ASGPR aus (GalNAc)₃Tris, oder ein drittes Konjugat aus einem Effektormolekül und einem Bindungsmolekül, das eine Bindungsstelle für ein Zelloberflächenmolekül umfasst, und gegebenenfalls einen pharmazeutisch akzeptablen Hilfsstoff und/oder ein pharmazeutisch akzeptables Verdünnungsmittel umfasst, oder
pharmazeutische Zusammensetzung, umfassend:
- das Saponinkonjugat nach einem der Ansprüche 1-14;
- ein zweites Konjugat aus einem Effektormolekül und einem Liganden für ASGPR, wobei der Ligand für ASGPR bevorzugt mindestens eine GalNAc-Gruppe, bevorzugt drei oder vier GalNAc-Gruppen, umfasst, mehr bevorzugt umfasst oder besteht der Ligand für ASGPR aus (GalNAc)₃Tris, oder ein drittes Konjugat aus einem Effektormolekül und einem Bindungsmolekül, das eine Bindungsstelle für ein Zelloberflächenmolekül umfasst, und optional einen pharmazeutisch akzeptablen Hilfsstoff und/oder ein pharmazeutisch akzeptables Verdünnungsmittel umfasst, und optional
wobei das Effektormolekül mindestens eines umfasst oder daraus besteht von einem kleinen Molekül wie etwa einem Arzneimittelmolekül, einem Toxin wie etwa einem Protein-Toxin, einem Oligonukleotid wie etwa einem BNA, einer Xenonukleinsäure oder einer siRNA, einem Enzym, einem Peptid, einem Protein oder einer beliebigen Kombination davon, bevorzugt ist das Effektormolekül ein Toxin, ein Enzym oder ein Oligonukleotid, und optional wobei der Ligand für ASGPR (GalNAc)₃Tris umfasst oder ist und/oder wobei der Ligand für ASGPR und das Effektormolekül über eine kovalente Bindung, bevorzugt über mindestens einen Linker, konjugiert sind, und optional
wobei das Effektormolekül ein Oligonukleotid ist, das aus einem oder mehreren ausgewählt ist von: kurze interferierende RNA (siRNA), kurze Haarnadel-RNA (shRNA), anti-Haarnadel-förmige microRNA (miRNA), einsträngige RNA, Aptamer-RNA, doppelsträngige RNA (dsRNA), AntimicroRNA (Anti-miRNA, Anti-miR), Antisense-Oligonukleotid (ASO), DNA, Antisense-DNA, Locked Nucleic Acid (LNA), Bridged Nucleic Acid (BNA), 2'-O,4'-Aminoethylen-verbrückte Nukleinsäure (BNA^{NC}), BNA-basierte siRNA und BNA-basiertes Antisense-Oligonukleotid (BNA-AON), und optional
wobei das Effektormolekül ein Oligonukleotid ist, das aus einem oder mehreren ausgewählt ist von Anti-miRNA, ein BNA-AON oder eine siRNA, wie zum Beispiel eine BNA-basierte siRNA, die aus chemisch modifizierten siRNA, metabolisch stabiler siRNA und chemisch modifizierter, metabolisch stabiler siRNA, ausgewählt ist, und optional
wobei das Effektormolekül ein Oligonukleotid ist, das zum Beispiel, wenn es in einer Säugetierzelle vorhanden ist, eines der folgenden Gene stilllegen kann: Apolipoprotein B (apoB), HSP27, Transthyretin (TTR), Proproteinkonvertase Subtilisin/Kexin Typ 9 (PCSK9), Delta-Aminolävulinat-Synthase 1 (ALAS1), Antithrombin 3 (AT3), Glykolatoxidase (GO), Komplementkomponente C5 (CC5), X-Gen des Hepatitis- B-Virus (HBV), S-Gen des HBV, Alpha-1-Antitrypsin (AAT) und Laktatdehydrogenase (LDH) und/oder ein Oligonukleotid ist, das in der Lage ist, zum Beispiel, wenn es in einer Säugetierzelle vorhanden ist, ein aberrantes miRNA als Ziel hat, und optional
wobei das Effektormolekül ein Oligonukleotid ist, bevorzugt eine siRNA wie etwa eine BNA-basierte siRNA, oder ein Antisense-Oligonukleotid wie etwa ein BNA-basiertes Antisense-Oligonukleotid, zum Beispiel ein AON-basiertes 2'-O,4'-Aminoethylen-verbrücktes Nukleinsäuremolekül, das in der Lage ist, bevorzugt wenn es in einer Säugetierzelle vorhanden ist, das Gen Apolipoprotein B (apoB) zu silenzieren, und optional
wobei das Effektormolekül ein Oligonukleotid ist, das in der Lage ist, zum Beispiel wenn es in einer
Säugetierzelle vorhanden ist, eine mRNA als Ziel hat, die an der Expression eines der Proteine beteiligt ist von: ApoB, HSP27, TTR, PCSK9, ALAS1, AT3, GO, CC5, Expressionsprodukt des X-Gens von HBV, Expressionsprodukt des S-Gens von HBV, AAT und LDH, oder in der Lage ist, zum Beispiel wenn es in einer Säugetierzelle vorhanden ist, eine miRNA-Funktion zu antagonisieren oder wiederherzustellen, wie etwa Hemmung einer onkogenen miRNA (Onco-miR) oder Unterdrückung der Expression einer Onco-miR, und optional,
wobei das Effektormolekül ein Oligonukleotid ist, bevorzugt eine siRNA wie etwa eine BNA-basierte siRNA, oder ein Antisense-Oligonukleotid wie etwa ein BNA-basiertes Antisense-Oligonukleotid, zum Beispiel ein AON basierend auf 2'-O,4'-Aminoethylen-verbrückter Nukleinsäure, das in der Lage ist, bevorzugt wenn es innerhalb einer Säugetierzelle vorhanden ist, eine mRNA als Ziel hat, die an der Expression des Proteins ApoB beteiligt ist.

16. Pharmazeutische Kombination oder pharmazeutische Zusammensetzung nach Anspruch 15, wobei das Bindungsmolekül, das eine Bindungsstelle für ein Zelloberflächenmolekül umfasst, umfasst durch das dritte Konjugat, ein Ligand für ein Zelloberflächenmolekül oder ein Antikörper ist, der eine Bindungsstelle für ein Zelloberflächenmolekül oder mindestens eine Domäne oder ein Fragment davon umfasst, die die Bindungsstelle umfassen, und optional,
wobei das dritte Konjugat eines oder mehrere ist von: einem Antikörper-Toxin-Konjugat, einem Rezeptor-Ligand-Toxin-Konjugat, einem Antikörper-Wirkstoff-Konjugat, einem Rezeptor-Ligand-Wirkstoff-Konjugat, einem Antikörper-Nukleinsäure-Konjugat oder einem Rezeptor-Ligand-Nukleinsäure-Konjugat, und optional,
wobei das Bindungsmolekül, das eine Bindungsstelle für ein Zelloberflächenmolekül umfasst, umfasst durch das dritte Konjugat, das in der Lage ist, an ein Zelloberflächenmoleküle zu binden von: CD71, CA125, EpCAM(17-1A), CD52, CEA, CD44v6, FAP, EGF-IR, Integrin, Syndecan-1, vaskuläres Integrin alpha-V beta-3, HER2, EGFR, CD20, CD22, Folatrezeptor 1, CD146, CD56, CD19, CD138, CD27L-Rezeptor, PSMA, CanAg, Integrin-alphaV, CA6, CD33, Mesothelin, Cripto, CD3, CD30, CD239, CD70, CD123, CD352, DLL3, CD25, EphrinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, Asialoglykoproteinrezeptor (ASGPR), bevorzugt eines von: HER2, CD71, ASGPR und EGFR, mehr bevorzugt CD71, und optional
wobei das Bindungsmolekül, das eine Bindungsstelle für ein Zelloberflächenmolekül umfasst, umfasst durch das dritte Konjugat, eines ist oder umfasst von: einen Antikörper, bevorzugt einen monoklonalen Antikörper wie etwa einen humanen monoklonalen Antikörper, ein lgG, ein Molekül, das einen Single-Domain-Antikörper umfasst oder daraus besteht, mindestens eine VHH-Domäne, bevorzugt eine Kameliden-VH, eine variable schwere Kette eines neuen Antigenrezeptors (VNAR)-Domäne, ein Fab, ein scFv, ein Fv, ein dAb, ein F(ab)2 und ein Fcab-Fragment.

17. Pharmazeutische Kombination oder pharmazeutische Zusammensetzung nach Anspruch 15 oder 16 zur Verwendung als Arzneimittel und optional
zur Verwendung bei der Behandlung oder Prophylaxe einer Krankheit oder Gesundheitsproblems, an dem ein Expressionsprodukt eines oder mehrere der Gene beteiligt ist: apoB, TTR, PCSK9, ALAS1, AT3, GO, CC5, X-Gen von HBV, S-Gen von HBV, AAT und LDH, und optional zur Verwendung bei der Behandlung oder Prophylaxe einer Krankheit oder Gesundheitsproblems, an dem ein Expressionsprodukt des Gens apoB beteiligt ist, und optional
zur Verwendung bei der Behandlung oder Prophylaxe von einem Krebs, einer Infektionskrankheit, einer Virusinfektion, Hypercholesterinämie, primärer Hyperoxalurie, Hämophilie A, Hämophilie B, AAT-bedingter Lebererkrankung, akuter hepatischer Porphyrie, TTR-vermittelter Amyloidose, hereditärer TTR-Amyloidose (hATTR), komplementvermittelter Erkrankung, Hepatitis-B-Infektion oder einer Autoimmunerkrankung.

18. In-vitro- oder Ex-vivo-Verfahren zum Übertragen des zweiten Konjugats oder des dritten Konjugats nach einem der Ansprüche 14-15 von außerhalb einer Zelle in das Innere dieser Zelle, bevorzugt anschließendes Übertragen des Effektormoleküls, das vom zweiten Konjugat oder dem dritten Konjugat nach einem der Ansprüche 14-15 umfasst wird, in das Zytosol dieser Zelle, umfassend die Schritte von:
Bereitstellen einer Zelle, die ASGPR auf ihrer Oberfläche exprimiert, und, wenn das dritte Konjugat in die Zelle übertragen werden soll, die das Zelloberflächenmolekül exprimiert, für welches das dritte Konjugat ein Bindungsmolekül zum Binden an das Zelloberflächenmolekül umfasst, wobei die Zelle bevorzugt aus einer Leberzelle, einer viral infizierten Zelle und einer Tumorzelle ausgewählt ist;
Bereitstellen des zweiten Konjugats oder des dritten Konjugats nach einem der Ansprüche 14-15 zum Übertragen in die Zelle, die in Schritt a) bereitgestellt wurde;
Bereitstellen des Saponin-Konjugats nach einem der Ansprüche 1-14;
Inkontaktbringen der Zelle aus Schritt a) in vitro oder ex vivo mit dem zweiten Konjugat oder dem dritten Konjugat aus Schritt b) und dem Saponin-Konjugat aus Schritt c), wodurch die Übertragung des zweiten Konjugats oder des dritten Konjugats von außerhalb der Zelle in diese Zelle bewirkt wird, und bevorzugt anschließend die Übertragung des zweiten Konjugats oder des dritten Konjugats in das Zytosol dieser Zelle bewirkt wird, oder bevorzugt dadurch anschließend die Übertragung zumindest des Effektormoleküls, das durch das zweite Konjugat oder das dritte Konjugat umfasst ist, in das Zytosol dieser Zelle bewirkt wird.

## Revendications

1. Conjugué de saponine comprenant au moins une saponine liée de manière covalente à un ligand pour le récepteur de l'asialoglycoprotéine (ASGPR), où le ligand pour l'ASGPR comprend au moins une fraction N-acétyl-galactosamine (GalNAc), de préférence trois ou quatre fractions GalNAc, plus préférablement le ligand pour l'ASGPR comprend ou est constitué de (GalNAc)₃Tris, où l'au moins une saponine est choisie parmi des saponines triterpénoïdes monodesmosidiques et des saponines triterpénoïdes bidesmosidiques, où la saponine est un dérivé de saponine comprenant une chaîne saccharide liée à la structure centrale d'aglycone, qui est choisie parmi le groupe A :
GIcA-
GaI-(1→2)-[Xyl-(1→3)]-GIcA-,
Glc-(1→2)-[GIc-(1→4)]-GIcA-,
Glc-(1→2)-Ara-(1→3)-[Gal-(1→2)]-GIcA-,
Xyl-(1→2)-Ara-(1→3)-[Gal-(1→2)]-GlcA-,
Rha-(1→2)-Gal-(1→3)-[Glc-(1→2)]-GIcA-,
Ara-(1→4)-Rha-(1→2)-Glc-(1→2)-Rha-(1→2)-GlcA-,
Ara-(1→4)-Fuc-(1→2)-Glc-(1→2)-Rha-(1→2)-GlcA-,
Ara-(1→4)-Rha-(1→2)-Gal-(1→2)-Rha-(1→2)-GIcA-,
Ara-(1→4)-Fuc-(1→2)-Gal-(1→2)-Rha-(1→2)-GlcA-,
Ara-(1→4)-Rha-(1→2)-Glc-(1→2)-Fuc-(1→2)-GIcA-,
Ara-(1→4)-Fuc-(1→2)-Glc-(1→2)-Fuc-(1→2)-GlcA-,
Ara-(1→4)-Rha-(1→2)-Gal-(1→2)-Fuc-(1→2)-GlcA-,
Ara-(1→4)-Fuc-(1→2)-Gal-(1→2)-Fuc-(1→2)-GIcA-,
Xyl-(1→4)-Rha-(1→2)-Glc-(1→2)-Rha-(1→2)-GIcA-,
Xyl-(1→4)-Fuc-(1→2)-GIc-(1→2)-Rha-(1→2)-GIcA-,
Xyl-(1→4)-Rha-(1→2)-Gal-(1→2)-Rha-(1→2)-GIcA-,
Xyl-(1→4)-Fuc-(1→2)-Gal-(1→2)-Rha-(1→2)-GIcA-,
Xyl-(1→4)-Rha-(1→2)-Glc-(1→2)-Fuc-(1→2)-GIcA-,
Xyl-(1→4)-Fuc-(1→2)-GIc-(1→2)-Fuc-(1→2)-GIcA-,
Xyl-(1→4)-Rha-(1→2)-Gal-(1→2)-Fuc-(1→2)-GIcA-,
Xyl-(1→4)-Fuc-(1→2)-Gal-(1→2)-Fuc-(1→2)-GIcA-, et
des dérivés de ceux-ci,
où ladite chaîne saccharide comprend un groupe carboxyle qui a été dérivatisé et le groupe carboxyle qui est dérivatisé fait partie d'une fraction d'acide glucuronique.

2. Conjugué de saponine selon la revendication 1, où la saponine comprend une structure centrale d'aglycone choisie dans le groupe constitué de :
acide 2alpha-hydroxy oléanolique ;
acide 16alpha-hydroxy oléanolique ;
hédérénine (acide 23-hydroxy oléanolique) ;
acide 16alpha,23-dihydroxy oléanolique ;
gypsogénine ;
acide quillaïque ;
protoaescigénine-21(2-méthylbut-2-énoate)-22-acétate ;
23-oxo-barringtogénol C-21,22-bis(2-méthylbut-2-énoate) ;
23-oxo-barringtogénol C-21(2-méthylbut-2-énoate)-16,22-diacétate ;
digitogénine ;
3,16,28-trihydroxy oléanane-12-ène ;
acide gypsogénique,
et
des dérivés de ceux-ci,
de préférence, la saponine comprend une structure centrale d'aglycone choisie parmi l'acide quillaïque et la gypsogénine ou les dérivés de ceux-ci, plus préférablement, la structure centrale d'aglycone de la saponine est l'acide quillaïque ou un dérivé de celui-ci.

3. Conjugué de saponine selon la revendication 1 ou 2, où
• la saponine comprend une chaîne saccharide liée à la structure centrale d'aglycone, qui est choisie parmi le groupe B :
GIc-,
Gal-,
Rha-(1→2)-[Xyl-(1→4)]-Rha-,
Rha-(1→2)-[Ara-(1→3)-Xyl-(1→4)]-Rha-,
Ara-,
XyI-,
Xyl-(1→4)-Rha-(1→2)-[R1-(→4)]-Fuc-, où R1 est l'acide 4E-méthoxycinnamique,
Xyl-(1→4)-Rha-(1→2)-[R2-(→4)]-Fuc-, où R2 est l'acide 4Z-méthoxycinnamique,
Xyl-(1→4)-[Gal-(1→3)]-Rha-(1→2)-4-OAc-Fuc-,
Xyl-(1→4)-[Glc-(1→3)]-Rha-(1→2)-3,4-di-OAc-Fuc-,
Xyl-(1→4)-[Glc-(1→3)]-Rha-(1→2)-[R3-(→4)]-3-OAc-Fuc- où R3 est l'acide 4E-méthoxycinnamique,
Glc-(1→3)-Xyl-(1→4)-[Glc-(1→3)]-Rha-(1→2)-4-OAc-Fuc-,
Glc-(1→3)-Xyl-(1→4)-Rha-(1→2)-4-OAc-Fuc-,
(Ara- ou Xyl-) (1→3)-(Ara- ou Xyl-)(1→4)-(Rha- ou Fuc-)(1→2)-[4-OAc-(Rhaou Fuc-)(1→4)]-(Rha- ou Fuc-),
Xyl-(1→3)-Xyl-(1→4)-Rha-(1→2)-[Qui-(1→4)]-Fuc-,
Api-(1→3)-Xyl-(1→4)-[Glc-(1→3)]-Rha-(1→2)-Fuc-,
Xyl-(1→4)-[Gal-(1→3)]-Rha-(1→2)-Fuc-,
Xyl-(1→4)-[Glc-(1→3)]-Rha-(1→2)-Fuc-,
AraI/Xyl-(1→4)-Rha/Fuc-(1→4)-[Glc/Gal-(1→2)]-Fuc-,
Api-(1→3)-XyI-(1→4)-[GIc-(1→3)]-Rha-(1→2)-[R4-(→4)]-Fuc- où R4 est l'acide 5-O-[5-O-Ara/Api-3,5-dihydroxy-6-méthyl-octanoyl]-3,5-dihydroxy-6-méthyl-octanoïque),
Api-(1→3)-XyI-(1→4)-Rha-(1→2)-[R5-(→4)]-Fuc- où R5 est l'acide 5-O-[5-O-Ara/Api-3,5-dihydroxy-6-méthyl-octanoyl]-3,5-dihydroxy-6-méthyl-octanoïque),
Api-(1→3)-XyI-(1→4)-Rha-(1→2)-[Rha-(1→3)]-4-OAc-Fuc-,
Api-(1→3)-XyI-(1→4)- [GIc-(1→3)]-Rha-(1→2)-[Rha-(1→3)]-4-OAc-Fuc-,
6-OAc-GIc-(1→3)-XyI-(1→4)-Rha-(1→2)-[3-OAc-Rha-(1→3)]-Fuc-,
GIc-(1→3)-XyI-(1→4)-Rha-(1→2)-[3-OAc--Rha-(1→3)]-Fuc-,
XyI-(1→3)-XyI-(1→4)-Rha-(1→2)-[Qui-(1→4)]-Fuc-,
GIc-(1→3)-[XyI-(1→4)]-Rha-(1→2)-[Qui-(1→4)]-Fuc-,
GIc-(1→3)-XyI-(1→4)-Rha-(1→2)-[XyI-(1→3)-4-OAc-Qui-(1→4)]-Fuc-,
XyI-(1→3)-XyI-(1→4)-Rha-(1→2)-[3,4-di-OAc-Qui-(1→4)j-Fuc-,
GIc-(1→3)-[XyI-(1→4)]-Rha-(1→2)-Fuc-,
6-OAc-GIc-(1→3)-[XyI-(1→4)]-Rha-(1→2)-Fuc-,
GIc-(1→3)-[XyI-(1→3)-XyI-(1→4)]-Rha-(1→2)-Fuc-,
XyI-(1→3)-XyI-(1→4)-Rha-(1→2)-[XyI-(1→3)-4-OAc-Qui-(1→4)]-Fuc-,
Api/XyI-(1→3)-XyI-(1→4)-[GIc-(1→3)]-Rha-(1→2)-[Rha-(1→3)]-4OAc-Fuc-,
Api-(1→3)-XyI-(1→4)-[GIc-(1→3)]-Rha-(1→2)-[Rha-(1→3)-4OAc-Fuc-Api/XyI-(1→3)-XyI-(1→4)-[GIc-(1→3)-Rha-(1→2)-[R6-(→4)]-Fuc- où R6 est l'acide 5-O-[5-O-Rha-(1→2)-Ara/Api-3,5-dihydroxy-6-méthyl-octanoyl]-3,5-dihydroxy-6-méthyl-octanoïque),
Api/XyI-(1→3)-XyI-(1→4)-[GIc-(1→3)]-Rha-(1→2)-[R7-(→4)]-Fuc- où R7 est l'acide 5-O-[5-O-Ara/Api-3,5-dihydroxy-6-méthyl-octanoyl]-3,5-dihydroxy-6-méthyl-octanoïque),
Api/XyI-(1→3)-XyI-(1→4)-[GIc-(1→3)]-Rha-(1→2)-[R8-(→4)]-Fuc- où R8 est l'acide 5-O-[5-O-Aral/Api-3,5-dihydroxy-6-méthyl-octanoyl]-3,5-dihydroxy-6-méthyl-octanoïque),
Api-(1→3)-XyI-(1→4)-Rha-(1→2)-[R9-(→4)]-Fuc- où R9 est l'acide 5-O-[5-O-Aral/Api-3,5-dihydroxy-6-méthyl-octanoyl]-3,5-dihydroxy-6-méthyl-octanoïqu e),
XyI-(1→3)-XyI-(1→4)-Rha-(1→2)-[R10-(→4)]-Fuc- où R10 est l'acide 5-O-[5-O-Ara/Api-3,5-dihydroxy-6-méthyl-octanoyl]-3,5-dihydroxy-6-méthyl-octanoïque),
Api-(1→3)-XyI-(1→4)-Rha-(1→2)-[R11-(→3)]-Fuc- où R11 est l'acide 5-O-[5-O-Ara/Api-3,5-dihydroxy-6-méthyl-octanoyl]-3,5-dihydroxy-6-méthyl-octanoïque),
XyI-(1→3)-XyI-(1→4)-Rha-(1→2)-[R12-(→3)]-Fuc- où R12 est l'acide 5-O-[5-O-Ara/Api-3,5-dihydroxy-6-méthyl-octanoyl]-3,5-dihydroxy-6-méthyl-octanoïque)
GIc-(1→3)-[GIc-(1→6)]-GaI-, et
des dérivés de ceux-ci,
ou
• la saponine est un glycoside triterpénique bidesmosidique comprenant une première chaîne saccharide choisie dans le groupe A liée à la structure centrale d'aglycone et comprenant une deuxième chaîne saccharide choisie dans le groupe B liée à la structure centrale d'aglycone.

4. Conjugué de saponine selon l'une quelconque des revendications 1 à 3, où la saponine est choisie dans le groupe constitué de : la saponine d'écorce de *Quillaja*, NP-005236, NP-012672, NP-017777, NP-017778, NP-017774, NP-018110, NP-017772, NP-018109, NP-017888, NP-017889, NP-018108, SA1641, AE X55, NP-017674, NP-017810, AG1, NP-003881, NP-017676, NP-017677, NP-017706, NP-017705, NP-017773, NP-017775, SA1657, AG2, SO1861, GE1741, SO1542, SO1584, SO1658, SO1674, SO1832, SO1862, SO1904, QS-7, QS1861, QS-7api, QS1862, QS-17, QS-18, QS-21 A-apio, QS-21 A-xylo, QS-21 B-apio, QS-21 B-xylo, bêta-Aescine, Aescine la, saponine de graines de thé I, saponine de graines de thé J, Assamsaponine F, acide Primula 1 et des stéréoisomères de ceux-ci, des dérivés de ceux-ci et des combinaisons de ceux-ci, de préférence la saponine est choisie dans le groupe constitué de QS-21, un dérivé de QS-21, SO1861, un dérivé de SO1861, SA1641, un dérivé de SA1641, GE1741, un dérivé de GE1741 et des combinaisons de ceux-ci, plus préférablement la saponine est choisie dans le groupe constitué d'un dérivé de QS-21, d'un dérivé de SO1861 et des combinaisons de ceux-ci, le plus préférablement la saponine est un dérivé de SO1861.

5. Conjugué de saponine selon l'une quelconque des revendications 1 à 4, où la saponine est un dérivé de saponine où
i. le dérivé de saponine comprend une structure centrale d'aglycone comprenant un groupe aldéhyde qui a été dérivatisé ;
ii. le dérivé de saponine comprend une chaîne saccharide, de préférence une chaîne saccharide choisie dans le groupe B tel que défini dans la revendication 3, la chaîne saccharide comprenant un groupe acétoxy (Me(CO)O-) qui a été dérivatisé ; ou
iii. le dérivé de saponine comprend à la fois les dérivations i. et ii..

6. Conjugué de saponine selon l'une quelconque des revendications 1 à 5, où la saponine est l'une quelconque ou plusieurs parmi : SO1861, SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, saponine de Quillaja, Saponinum album, QS-18, Quil-A, Gyp1, gypsoside A, AG1, AG2, SO1542, SO1584, SO1658, SO1674, SO1832, SO1904, des stéréoisomères de ceux-ci, des dérivés de ceux-ci et des combinaisons de ceux-ci, de préférence la saponine est choisie dans le groupe constitué de QS-21, un dérivé de QS-21, SO1861, un dérivé de SO1861, SA1641, un dérivé de SA1641, GE1741, un dérivé de GE1741 et des combinaisons de ceux-ci, plus préférablement la saponine est choisie dans le groupe constitué d'un dérivé de QS-21, d'un dérivé de SO1861 et des combinaisons de ceux-ci, le plus préférablement la saponine est un dérivé de SO1861.

7. Conjugué de saponine selon l'une quelconque des revendications 1 à 6, où la saponine est un dérivé de saponine de la saponine d'acide quillaïque ou de la saponine de gypsogénine selon la revendication 2 et est représentée par la molécule 1 : 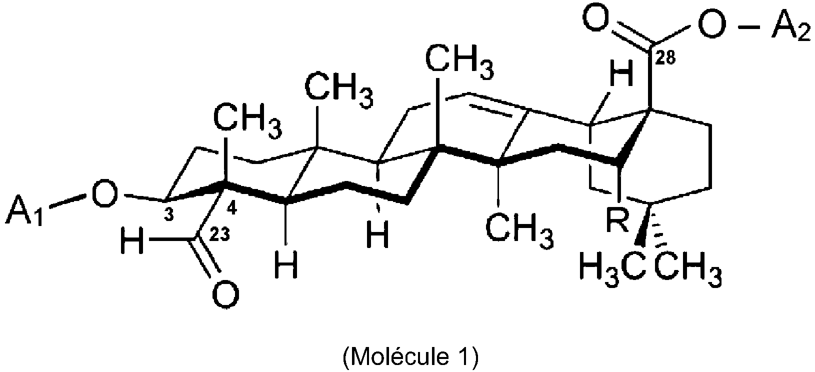 où
A₁ représente une chaîne saccharide choisie dans le groupe A tel que défini dans la revendication 1, et A₁ comprend ou est constitué d'une fraction d'acide glucuronique, et le groupe carboxyle de ladite fraction d'acide glucuronique de A₁ a été dérivatisé ;
A₂ représente de l'hydrogène, un monosaccharide ou un oligosaccharide linéaire ou ramifié, de préférence A₂ représente une chaîne saccharide choisie dans le groupe B tel que défini dans la revendication 3, plus préférablement A₂ représente une chaîne saccharide choisie dans le groupe B tel que défini dans la revendication 3 et A₂ comprend au moins un groupe acétoxy (Me(CO)O-), tel qu'un, deux, trois ou quatre groupes acétoxy,
où de préférence A₁ et A₂ sont tous deux une chaîne oligosaccharidique ;
et R est de l'hydrogène dans la gypsogénine ou de l'hydroxyle dans l'acide quillaïque ;
où, éventuellement, le dérivé de saponine correspond à la saponine représentée par la Molécule 1 où la dérivatisation suivante est présente :
- le groupe carboxyle d'une fraction d'acide glucuronique de A₁ a été dérivatisé par transformation en une liaison amide par réaction avec du 2-amino-2-méthyl-1,3-propanediol (AMPD) ou du N-(2-aminoéthyl)maléimide (AEM), fournissant ainsi une saponine-Glu-AMPD telle qu'un QS-21-Glu-AMPD ou un SO1861-Glu-AMPD ou une saponine-Glu-AEM telle qu'un QS-21-Glu-AEM ou un SO1861Glu-AEM,
où A₁ est de préférence Gal-(1→2)-[Xyl-(1→3)]-GIcA-.

8. Conjugué de saponine selon l'une quelconque des revendications 1 à 7, où l'au moins une saponine et le ligand pour l'ASGPR sont liés de manière covalente directement ou via au moins un lieur, et éventuellement
où la fraction GalNAc est liée à la saponine S, de préférence via un lieur de saponine L_{S}, comme montré dans la formule (ll)s : 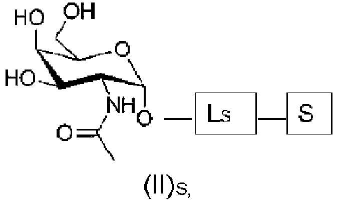 ou
où les fractions GalNAc sont chacune liées de manière covalente séparément via l'oxygène en position «1 » de la fraction GalNAc à une fraction de pontage centrale B, qui forme efficacement un pont entre les fractions GalNAc et la fraction saponine, de préférence via un lieur de fraction saponine L_{S}, comme montré dans la formule (lll)s : 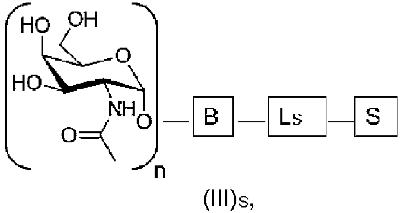
où n est un nombre entier supérieur ou égal à 2, de préférence n est 3, L_{S} est un lieur de fraction saponine et S est la fraction saponine, et éventuellement
où les fractions GalNAc sont liées à la fraction de pontage B via des lieurs GalNAc L_{GAL} comme montré dans la formule (IV)s : 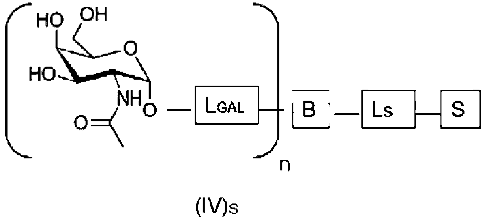
où n est un nombre entier supérieur ou égal à 2, de préférence n est 3, L_{S} est un lieur de fraction saponine et S est la fraction saponine.

9. Conjugué de saponine selon la revendication 8, où le lieur de fraction saponine L_{S} représente toute fraction chimique adapté pour lier de manière covalente une saponine à la GalNAc comme dans la formule (ll)s ou à la fraction de pontage B comme dans les formules (lll)s et (IV)s, et éventuellement
où le lieur de fraction saponine L_{S} est le résultat d'une réaction de couplage entre au moins un premier précurseur L_{S1} lié de manière covalente à la GalNAc ou à la fraction de pontage B et un deuxième précurseur L_{S2} lié de manière covalente à la fraction saponine, où L_{S1} est un précurseur du lieur de fraction saponine L_{S} qui est lié de manière covalente à la GalNAc ou à la fraction de pontage B et L_{S2} est un précurseur du lieur de fraction saponine L_{S} qui est lié de manière covalente à la fraction saponine, et éventuellement
où le lieur de fraction saponine L_{S} est le résultat d'une réaction de couplage entre au moins un premier précurseur L_{S1} lié de manière covalente à la GalNAc ou à la fraction de pontage B et un deuxième précurseur L_{S2} lié de manière covalente à la fraction saponine, où la réaction de couplage est choisie dans le groupe constitué d'une cycloaddition d'azide-alkyne, d'un couplage thiol-maléimide, d'une réaction de Staudinger, d'une ouverture de cycle nucléophile d'électrophiles hétérocycliques tendus, d'une réaction carbonyle de type non aldol et d'un ajout à une double liaison carbone-carbone, de préférence où la réaction de couplage est une cycloaddition d'azide-alkyne, un couplage thiol-maléimide, une réaction de Staudinger, une ouverture de cycle nucléophile d'électrophiles hétérocycliques tendus, plus préférablement où la réaction de couplage est une cycloaddition d'azide-alkyne, ou un couplage thiol-maléimide, et éventuellement,
où le groupe de liaison saponine L_{S} est le résultat d'une réaction de couplage entre un premier précurseur L_{S1} lié de manière covalente à la GalNAc ou à la fraction de pontage B, le premier précurseur L_{S1} comprenant un azide ;
et un deuxième précurseur L_{S2} lié de manière covalente à la fraction saponine, le deuxième précurseur L_{S2} comprenant un alcyne et comprenant de préférence une hydrazone résultant d'un couplage hydrazide/aldéhyde à un aldéhyde de la fraction saponine, et éventuellement
où la structure du précurseur L_{S1} est l'azide suivant de formule (XVII) : 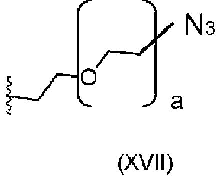
où a représente un nombre entier supérieur ou égal à 0, de préférence a représente un nombre entier choisi parmi 1, 2 et 3, plus préférablement a représente 2, ou
où la structure du précurseur L_{S1} comprend l'azide suivant de formule (XVIII) : 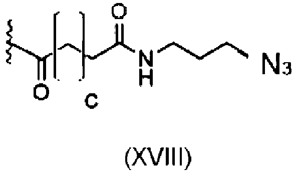
où c représente un nombre entier supérieur ou égal à 0, de préférence c représente un nombre entier compris entre 5 et 15, plus préférablement c représente 9, et éventuellement où la structure du précurseur L_{S2} comprend l'hydrazone suivante de formule (XIX) : 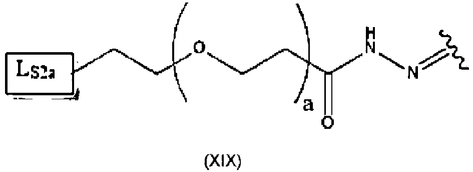
où a représente un nombre entier supérieur ou égal à 0, de préférence a représente un nombre entier compris entre 2 et 6, plus préférablement a représente 4, et où L_{S2a} représente une fraction contenant de l'alcyne.

10. Conjugué de saponine selon la revendication 9, où L_{S2a} comprend moins de 20 atomes de carbone, de préférence L_{S2a} représente une fraction selon la formule (XX) :

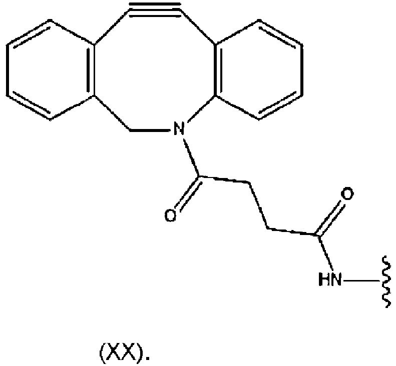

11. Conjugué de saponine selon l'une quelconque des revendications 8 à 10, où la fraction de pontage est un composé de formule (XV) : 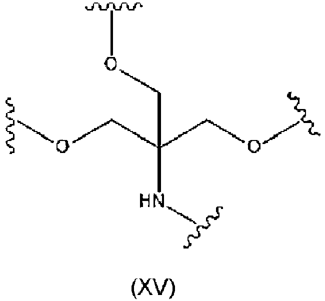
où les atomes d'oxygène du composé de formule (XV) sont liés à la GalNAc ou aux lieurs GalNAc L_{GAL}, et l'atome d'azote du composé de formule (XV) est lié au lieur de fraction saponine L_{S}, et éventuellement
où L_{GAL} représente toute fraction chimique adaptée pour lier de manière covalente la GalNAc à la fraction de pontage B, et éventuellement
où L_{GAL} comprend 2 à 25 atomes de carbone, de préférence 7 à 15 atomes de carbone, plus préférablement 11 atomes de carbone, et où L_{GAL} comprend au moins un, de préférence deux fractions amide, et éventuellement
où L_{GAL} est un composé selon la formule (XVI) :

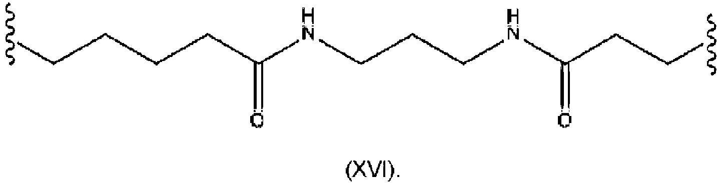

12. Conjugué de saponine selon l'une quelconque des revendications 8 à 11, où le ligand pour l'ASGPR est la (GalNAc)₃Tris représentée par la molécule I' : 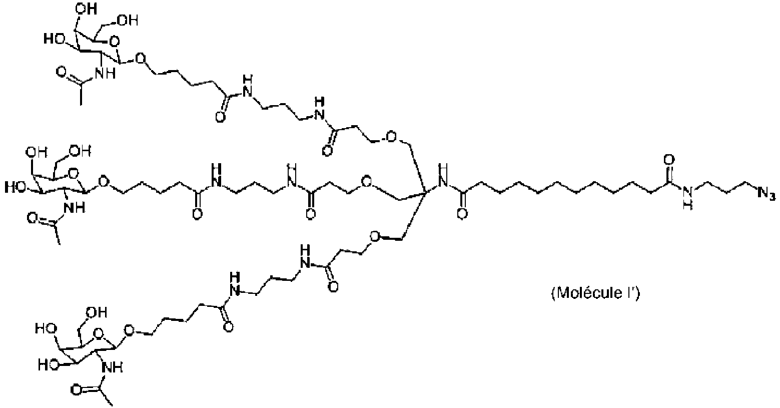 ou où le ligand pour l'ASGPR est la mono-GalNAc représenté par la molécule Il' :

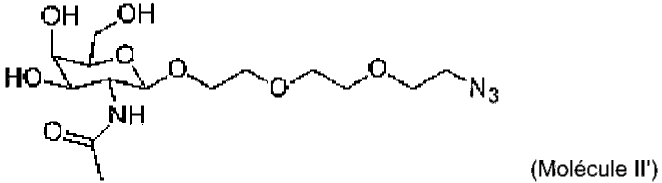

13. Conjugué de saponine selon l'une quelconque des revendications 1 à 12, où l'au moins une saponine est liée de manière covalente à la molécule I' ou à la molécule II' selon la revendication 12 via un lieur représenté par la molécule III' : 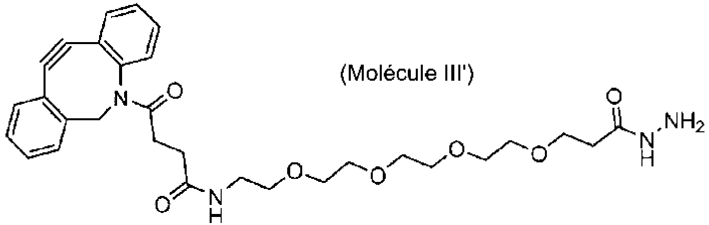 où la fraction hydrazide de la molécule III' a formé une liaison hydrazone covalente avec un groupe aldéhyde dans la saponine, et où le groupe dibenzocyclooctyne a formé une liaison covalente avec le groupe azide de la molécule I' ou de la molécule II'.

14. Conjugué de saponine selon l'une quelconque des revendications 1 à 13, où l'au moins une saponine est liée de manière covalente au ligand pour l'ASGPR via au moins un lieur clivable, et éventuellement
où le lieur clivable est soumis à un clivage dans des conditions acides, des conditions réductrices, des conditions enzymatiques et/ou des conditions induites par lumière, et de préférence le lieur clivable comprend une liaison clivable choisie parmi une liaison hydrazone et une liaison hydrazide soumise à un clivage dans des conditions acides, et/ou une liaison susceptible de protéolyse, par exemple protéolyse par cathepsine B, et/ou une liaison susceptible de clivage dans des conditions réductrices telles qu'une liaison disulfure, ou le lieur clivable est soumis à un clivage *in vivo* dans des conditions acides telles que celles présentes par exemple dans des endosomes et/ou des lysosomes de cellules mammifères, de préférence de cellules humaines, de préférence le lieur clivable est soumis à un clivage *in vivo* à un pH de 4,0 à 6,5, et plus préférablement à un pH ≤ 5,5.

15. Combinaison pharmaceutique comprenant :
- une première composition pharmaceutique comprenant le conjugué de saponine selon l'une quelconque des revendications 1 à 14 et comprenant éventuellement un excipient pharmaceutiquement acceptable et/ou un diluant pharmaceutiquement acceptable ; et
- une deuxième composition pharmaceutique comprenant un deuxième conjugué d'une molécule effectrice et d'un ligand pour l'ASGPR, où le ligand pour l'ASGPR comprend de préférence au moins une fraction GalNAc, de préférence trois ou quatre fractions GalNAc, plus préférablement, le ligand pour l'ASGPR comprend ou est constitué de (GalNAc)₃Tris, ou un troisième conjugué d'une molécule effectrice et d'une molécule de liaison comprenant un site de liaison pour une molécule de surface cellulaire, et comprenant éventuellement un excipient pharmaceutiquement acceptable et/ou un diluant pharmaceutiquement acceptable, ou
une composition pharmaceutique comprenant :
- le conjugué de saponine selon l'une quelconque des revendications 1 à 14 ;
- un deuxième conjugué d'une molécule effectrice et d'un ligand pour l'ASGPR, où le ligand pour l'ASGPR comprend de préférence au moins une fraction GalNAc, de préférence trois ou quatre fractions GalNAc, plus préférablement, le ligand pour l'ASGPR comprend ou est constitué de (GalNAc)₃Tris, ou un troisième conjugué d'une molécule effectrice et d'une molécule de liaison comprenant un site de liaison pour une molécule de surface cellulaire, et comprenant éventuellement un excipient pharmaceutiquement acceptable et/ou un diluant pharmaceutiquement acceptable, et éventuellement
où la molécule effectrice comprend ou est constitué d'au moins l'un parmi une petite molécule telle qu'une molécule de médicament, une toxine telle qu'une toxine protéique, un oligonucléotide tel qu'un BNA, un acide xénonucléique ou un ARNsi, une enzyme, un peptide, une protéine, ou toute combinaison de ceux-ci, de préférence, la molécule effectrice est une toxine, une enzyme ou un oligonucléotide, et éventuellement
où le ligand pour l'ASGPR comprend ou est (GalNAc)₃Tris, et/ou où le ligand pour l'ASGPR et la molécule effectrice sont conjugués via une liaison covalente, de préférence via au moins un lieur, et éventuellement
où la molécule effectrice est un oligonucléotide choisi parmi un ou plusieurs des éléments suivants : ARN interférent court (ARNsi), ARN court en épingle à cheveux (ARNsh), micro-ARN en forme d'anti-épingle à cheveux (ARNmi), ARN simple brin, ARN aptamère, ARN double brin (ARNds), anti-micro-ARN (anti-ARNmi, anti-miR), oligonucléotide antisens (ASO), ADN, ADN antisens, acide nucléique bloqué (LNA), acide nucléique ponté (BNA), acide nucléique ponté 2'-O,4'-aminoéthylène (BNA^{NC}), ARNsi à base de BNA et oligonucléotide antisens à base de BNA (BNA-AON), et éventuellement
où la molécule effectrice est un oligonucléotide choisi parmi un ou plusieurs des éléments suivants : anti-ARNmi, BNA-AON ou ARNsi, tel que ARNsi à base de BNA, choisi parmi ARNsi modifié chimiquement, ARNsi métaboliquement stable et chimiquement modifié, ARNsi métaboliquement stable et éventuellement
où la molécule effectrice est un oligonucléotide capable, par exemple lorsqu'il est présent à l'intérieur d'une cellule mammifère, d'effectuer le silençage de l'un quelconque des gènes suivants : apolipoprotéine B (apoB), HSP27, transthyrétine (TTR), proprotéine convertase subtilisine/kexine de type 9 (PCSK9), delta-aminolévulinate synthase 1 (ALAS 1), antithrombine 3 (AT3), glycolate oxydase (GO), composant du complément C5 (CC5), gène X du virus de l'hépatite B (VHB), gène S du VHB, alpha-1 antitrypsine (AAT) et la lactate déshydrogénase (LDH), et/ou est un oligonucléotide capable, par exemple lorsqu'il est présent à l'intérieur d'une cellule mammifère, de cibler un ARNmi aberrant, et éventuellement
où la molécule effectrice est un oligonucléotide, de préférence un ARNsi tel qu'un ARNsi à base de BNA, ou un oligonucléotide antisens tel qu'un oligonucléotide antisens à base de BNA, par exemple un AON à base d'acide nucléique ponté 2'-O,4'-aminoéthylène, capable, de préférence lorsqu'il est présent à l'intérieur d'une cellule mammifère, d'effectuer le silençage du gène de l'apolipoprotéine B (apoB), et éventuellement
où la molécule effectrice est un oligonucléotide capable, par exemple lorsqu'il est présent à l'intérieur d'une cellule mammifère, de cibler un ARNm impliqué dans l'expression de l'une quelconque des protéines suivantes : apoB, HSP27, TTR, PCSK9, ALAS1 , AT3, GO, CC5, produit d'expression du gène X du VHB, produit d'expression du gène S du VHB, AAT et LDH, ou est capable, par exemple lorsqu'il est présent à l'intérieur d'une cellule mammifère, d'antagoniser ou de restaurer une fonction de ARNmi telle que l'inhibition d'un ARNmi oncogène (onco-miR) ou la suppression de l'expression d'un onco-miR, et éventuellement, où la molécule effectrice est un oligonucléotide, de préférence un ARNsi tel qu'un ARNsi à base de BNA, ou un oligonucléotide antisens tel qu'un oligonucléotide antisens à base de BNA, par exemple un AON à base d'acide nucléique ponté 2'-O,4'-aminoéthylène, capable, de préférence lorsqu'il est présent à l'intérieur d'une cellule mammifère, de cibler un ARNm impliqué dans l'expression de la protéine apoB.

16. Combinaison pharmaceutique ou composition pharmaceutique selon la revendication 15, où la molécule de liaison comprenant un site de liaison pour une molécule de surface cellulaire, comprise par le troisième conjugué, est un ligand pour une molécule de surface cellulaire ou un anticorps comprenant un site de liaison pour une molécule de surface cellulaire ou au moins un domaine ou fraction de celui-ci comprenant le site de liaison, et où, éventuellement, le troisième conjugué est l'un quelconque ou plusieurs des éléments suivants : un conjugué anticorps-toxine, un conjugué récepteur-ligand-toxine, un conjugué anticorps-médicament, un conjugué récepteur-ligand-médicament, un conjugué anticorps-acide nucléique ou un conjugué récepteur-ligand-acide nucléique, et éventuellement
où la molécule de liaison comprenant un site de liaison pour une molécule de surface cellulaire, comprise par le troisième conjugué, est capable de se lier à l'une quelconque des molécules de surface cellulaire suivantes : CD71, CA125, EpCAM(17-1A), CD52, CEA, CD44v6, FAP, EGF-IR, intégrine, syndécane-1, intégrine vasculaire alpha-V bêta-3, HER2, EGFR, CD20, CD22, récepteur du folate 1, CD146, CD56, CD19, CD138, récepteur CD27L, PSMA, CanAg, intégrine alphaV, CA6, CD33, mésothéline, Cripto, CD3, CD3O, CD239, CD70, CD123, CD352, DLL3, CD25, ephrinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, récepteur de l'asialoglycoprotéine (ASGPR), de préférence l'une quelconque parmi : HER2, CD71, ASGPR et EGFR, plus préférablement CD71, et éventuellement
où la molécule de liaison comprenant un site de liaison pour une molécule de surface cellulaire, comprise par le troisième conjugué, est ou comprend l'un quelconque des éléments suivants : un anticorps, de préférence un anticorps monoclonal tel qu'un anticorps monoclonal humain, une IgG, une molécule comprenant ou constituée d'un anticorps à domaine unique, au moins un domaine V_{HH}, de préférence un V_{H} de camélidés, un domaine de nouveau récepteur d'antigène (V_{NAR}) variable à chaîne lourde, un Fab, un scFv, un Fv, un dAb, un F(ab)2 et une fraction Fcab.

17. Combinaison pharmaceutique ou composition pharmaceutique selon les revendications 15 ou 16, pour une utilisation comme médicament, et éventuellement
pour une utilisation dans le traitement ou la prophylaxie d'une maladie ou d'un problème de santé dans lequel est impliqué un produit d'expression d'un quelconque ou plusieurs des gènes suivants : apoB, TTR, PCSK9, ALAS1, A13, GO, CC5, gène X du VHB, gène S du VHB, AAT et LDH, et éventuellement pour une utilisation dans le traitement ou la prophylaxie d'une maladie ou d'un problème de santé dans lequel un produit d'expression est impliqué du gène apoB, et éventuellement pour une utilisation dans le traitement ou la prophylaxie d'un cancer, d'une maladie infectieuse, d'une infection virale, d'une hypercholestérolémie, d'une hyperoxalurie primaire, d'une hémophilie A, d'une hémophilie B, d'une maladie hépatique liée à l'AAT, d'une porphyrie hépatique aiguë, d'une amylose médiée par TTR, d'une amylose héréditaire TTR (hATTR), d'une maladie médiée par complément, d'une infection par hépatite B ou d'une maladie auto-immune.

18. Méthode *in vitro* ou *ex vivo* pour transférer le deuxième conjugué ou le troisième conjugué selon l'une quelconque des revendications 14 à 15 de l'extérieur d'une cellule à l'intérieur de ladite cellule, de préférence en transférant par la suite la molécule effectrice comprise par le deuxième conjugué ou le troisième conjugué selon l'une quelconque des revendications 14 à 15 dans le cytosol de ladite cellule, comprenant les étapes de :
fournir une cellule qui exprime l'ASGPR sur sa surface et, lorsque le troisième conjugué doit être transféré dans la cellule, qui exprime la molécule de surface cellulaire pour laquelle le troisième conjugué comprend une molécule de liaison pour se lier à ladite molécule de surface cellulaire, la cellule étant de préférence choisie parmi une cellule hépatique, une cellule infectée par un virus et une cellule tumorale ;
fournir le deuxième conjugué ou le troisième conjugué selon l'une quelconque des revendications 14 à 15 pour le transfert dans la cellule fournie à l'étape a) ;
fournir le conjugué de saponine selon l'une quelconque des revendications 1 à 14 ;
mettre en contact la cellule de l'étape a) *in vitro* ou *ex vivo* avec le deuxième conjugué ou le troisième conjugué de l'étape b) et le conjugué de saponine de l'étape c), effectuant ainsi le transfert du deuxième conjugué ou du troisième conjugué de l'extérieur de la cellule vers ladite cellule, et de préférence effectuant ainsi par la suite le transfert du deuxième conjugué ou du troisième conjugué dans le cytosol de ladite cellule, ou de préférence effectuant ainsi par la suite le transfert d'au moins la molécule effectrice comprise par le deuxième conjugué ou le troisième conjugué dans le cytosol de ladite cellule.
